# EUROPEAN PATENT SPECIFICATION

(11) **EP 3 122 738 B1**
(45) Date of publication and mention of the grant of the patent: **12.12.2018**
(21) Application number: 15712614.5
(22) Date of filing: 26.03.2015
(51) Int. Cl.: C07D 401/06, A01N 43/40, A01N 43/78, C07D 405/06, C07D 411/06, A61K 31/443, A61K 31/4436, A61K 31/4439, A61P 33/00, A01P 7/00

(54) **N-ACYLIMINO HETEROCYCLIC COMPOUNDS FOR CONTROLLING INVERTEBRATE PESTS**
N-ACYLIMINO HETEROCYCLISCHE VERBINDUNGEN ZUR KONTROLLE VON WIRBELLOSEN SCHÄDLINGEN
COMPOSÉS N-ACYLIMINO HÉTÉROCYCLIQUES POUR LUTTER CONTRE LES NUISIBLES INVERTÉBRÉS

(30) Priority: 27.03.2014 US 201461970949 P
(43) Date of publication of application: 01.02.2017
(73) Proprietor: BASF SE, 67056 Ludwigshafen am Rhein (DE)
(72) Inventor: GOCKEL, Birgit, 67063 Ludwigshafen (DE); MCLAUGHLIN, Martin, John, 67098 Bad Dürkheim (DE); KÖRBER, Karsten, 69214 Eppelheim (DE); VON DEYN, Wolfgang, 67435 Neustadt (DE); DIETZ, Jochen, 76227 Karlsruhe (DE); POHLMAN, Matthias, 67251 Freinsheim (DE); KOJIMA, Kenichi, Raleigh Wake, NC 27615 (US)
(74) Representative: BASF IP Association
(86) International application number: PCT/EP2015/056532
(87) International publication number: WO 2015/144813

(56) References cited:
- EP-A1- 2 633 756
- EP-A2- 2 634 174

## Description

The present invention relates to N-acylimino heterocyclic compounds, including their stereoisomers, tautomers and salts, and to compositions comprising such compounds. The invention also relates to the use of the N-acylimino heterocyclic compounds, their stereoisomers, their tautomers and their salts, for combating invertebrate pests. Furthermore the invention relates also to methods of combating invertebrate pests, which comprises applying such compounds.

### Background of Invention

Invertebrate pests, such as insects, acaridae and nematode pests destroy growing and harvested crops and attack wooden dwelling and commercial structures, causing large economic loss to the food supply and to property. While a large number of pesticidal agents are known, due to the ability of target pests to develop resistance to said agents, there is an ongoing need for new agents for combating animal pests. In particular, animal pests such as insects and acaridae are difficult to be effectively controlled.

EP 259738 discloses compounds of the formula A, which have insecticidal activity: 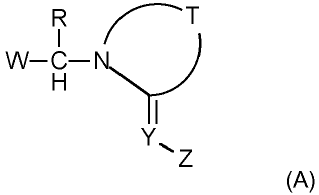 where W is a substituted pyridyl radical or a 5-or 6-membered heterocyclic radical, R is hydrogen or alkyl, T together with the atoms to which it is bound forms a 5- or 6-membered heterocyclic ring, Y is inter alia a nitrogen atom and Z is an electron withdrawing group selected from nitro and cyano.

Pesticidal compounds, which are similar to those of EP 259738, are known from EP 639569, where the moiety electron withdrawing moiety Z is an electron withdrawing group such as alkoxcarbonyl, arylcarbonyl, heterocyclic carbonyl, C₁-C₄-alkylsulfonyl, sulfamoyl or C₁-C₄-acyl.

US 2013/0150414 describe, inter alia, pesticidal compounds of the formula B 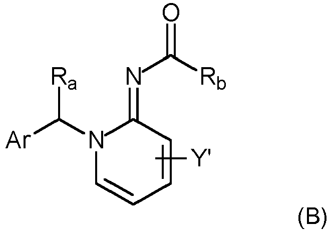 wherein Ar is an aryl or 5- or 6-membered heterocyclic group, Rₐ is hydrogen or alkyl, Y' is hyddrogen, halogen, a hydroxyl group, an alkyl group or an alkoxy group and R_{b} is an alkyl group substituted with halogen or an alkoxy group, optionally substituted with halogen.

Pesticidal compounds, which are similar to those of US 2013/0150414, are known from WO 2013/129688.
EP2633756 describes iminopyridine pesticides. EP2634174 describes nitrogen-containing heterocyclic pesticides having a 2-imino group.

The pesticidal activity of the compounds is not satisfactory. It is therefore an object of the present invention to provide compounds having a good pesticidal activity, especially against difficult to control insects and acarid pests.

### Summary of Invention

It has been found that these objects are solved by N-substituted acyl-imino compounds of the general formula (I) described below, by their stereoisomers, their tautomers and their salts. Therefore, the present invention relates to N-acylimino compound of formula (I): 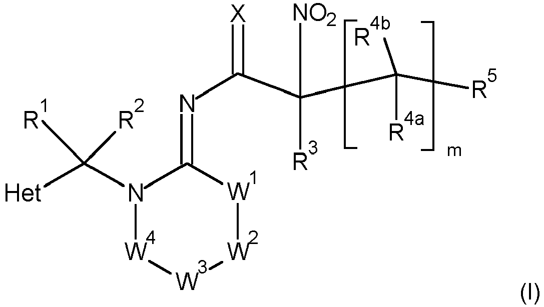 wherein
- m: is an integer selected from 0, 1, 2, 3, 4, 5 or 6, in particular 0 or 1;
- X: is O or S, in particular O;
- Het: is a 5- or 6- membered carbon-bound or nitrogen-bound heterocyclic or heteroaromatic ring, comprising 1, 2, 3, 4 or 5 carbon atoms and 1, 2 or 3 heteroatoms as ring members, which are independently selected from sulfur, oxygen or nitrogen, wherein the carbon, sulfur and nitrogen ring members can independently be partly or completely oxidized, and wherein each ring is optionally substituted by k identical or different substituents R⁶, wherein k is an integer selected from 0, 1, 2, 3 or 4;
- W¹-W²-W³-W⁴: represents a carbon chain group connected to N and C=N, and thus forming a saturated, unsaturated, or partially unsaturated 5 or 6 membered nitrogen containing heterocycle, wherein
W¹, W², W³ and W⁴ each individually represent CR^{v}R^{w}, wherein
each R^{w} independently from each other, is selected from the group consisting hydrogen, halogen, cyano, azido, nitro, SCN, SF₅, C₁-C₁₀-alkyl, C₃-C₈-cycloalkyl, C₂-C₁₀-alkenyl, C₂-C₁₀-alkynyl,
wherein each of the four last mentioned radicals may be unsubstituted, partly or completely halogenated and/or may optionally be substituted with 1, 2 or 3 identical or different radicals R⁷,
OR⁸, NR^{9a}R^{9b}, S(O)ₙR⁸, S(O)ₙNR^{9a}R^{9b}, C(=O)R^{7a}, C(=O)NR^{9a}R^{9b}, C(=O)OR⁸, C(=S)R^{7a}, C(=S)NR^{9a}R^{9b}, C(=S)OR⁸, C(=S)SR⁸, C(=NR¹⁷)R^{7a}, C(=NR¹⁷)NR^{9a}R^{9b} and Si(R¹¹)₂R¹²,
each R^{v} independently from each other, are selected from the group consisting of hydrogen, halogen, cyano, C₁-C₁₀-alkyl, C₃-C₈-cycloalkyl, C₂-C₁₀-alkenyl and C₂-C₁₀-alkynyl,
wherein each of the four last mentioned radicals may be unsubstituted, partly or completely halogenated or may optionally be further substituted with 1, 2 or 3 identical or different radicals R⁷; or
R^{v} and R^{w} present in one of the groups may together form =O, =CR¹³R¹⁴, =S, =NR¹⁷, =NOR¹⁶ or =NNR^{9a}R^{9b}, or
two R^{w} of adjacent carbon atoms, may form both together and together with the existing bond a double bond between the adjacent carbon atoms; and wherein one of W² or W³ may optionally represent a single or a double bond between the adjacent carbon atoms;
- R¹ and R²: are independently from each other selected from the group consisting of hydrogen, halogen, CN, SCN, nitro, C₁-C₆-alkyl, C₃-C₆-cycloalkyl,
wherein each of the two aforementioned radicals is unsubstituted, partly or completely halogenated or may carry 1, 2 or 3 identical or different radicals R⁷,
Si(R¹¹)₂R¹², OR⁸, OSO₂R⁸, S(O)ₙR⁸, S(O)ₙNR^{9a}R^{9b}, NR^{9a}R^{9b}, C(=O)NR^{9a}R^{9b}, C(=S)NR^{9a}R^{9b}, C(=O)OR⁸, C(=O)R^{7a}, C(=S)R^{7a}, phenyl, benzyl,
wherein the phenyl ring in the last two radicals is unsubstituted or optionally substituted with 1, 2, 3, 4 or 5 identical or different substituents R¹⁰,
a 3-, 4-, 5-, 6- or 7- membered saturated, partly saturated or unsaturated aromatic heterocyclic ring comprising 1, 2 or 3 identical or different heteroatoms as ring members, which are selected from oxygen, nitrogen and sulfur,
wherein the heterocyclic ring is optionally substituted with 1, 2, 3 or 4 identical or different substituents R¹⁰, and wherein the nitrogen and/or the sulfur atom(s) of the heterocyclic ring may optionally be oxidized,
or
R¹ and R² form, together with the carbon atom, which they attached to, a 3-, 4-, 5- or 6-membered saturated or partly unsaturated carbocyclic or heterocyclic ring,
wherein each of the carbon atoms of said cycle are unsubstituted or may carry 1 or 2 identical or different radicals R⁷,
or
R¹ and R² may together be =O, =CR¹³R¹⁴, =S, =NR¹⁷, =NOR¹⁶ or =NNR^{9a}R^{9b};
- R³: is selected from the group consisting of hydrogen, halogen, NO₂, C₁-C₆-alkyl, C₂-C₆-alkenyl, C₂-C₆-alkynyl, C₃-C₈-cycloalkyl and C₃-C₈-cycloalkenyl,
wherein each of the five last mentioned radicals is unsubstituted, partly or completely halogenated or carries 1 or 2 identical or different radicals R⁷, it being also possible for cycloalkyl and cycloalkenyl to carry 1, 2, 3, 4, 5 or 6 identical or different C₁-C₄-alkyl groups,
S(O)ₙNR^{9a}R^{9b}, NR^{9a}R^{9b}, C(=O)OR⁸, C(=O)NR^{9a}R^{9b}, C(=S)NR^{9a}R^{9b}, C(=O)R^{7a}, C(=S)R^{7a}, NR^{9a}-C(=O)R^{7a}, NR^{9a}-C(=S)R^{7a}, NR^{9a}-S(O)ₙR^{8a}, phenyl, benzyl,
wherein the phenyl ring in the last two mentioned radicals is unsubstituted or may be substituted with one or more, e.g. 1, 2, 3, 4 or 5 identical or different substituents R¹⁰,
and a 3-, 4-, 5-, 6- or 7- membered saturated, partly saturated or unsaturated aromatic heterocyclic ring comprising 1, 2 or 3 heteroatoms as ring members, which are identical or different and selected from oxygen, nitrogen and sulfur,
wherein the heterocyclic ring is optionally substituted with 1, 2, 3 or 4 identical or different substituents R¹⁰, and wherein the nitrogen and/or the sulfur atom(s) of the heterocyclic ring may optionally be oxidized;
- R^{4a} and R^{4b}: are selected each independently from one another and independently from the integer of m from the group consisting of hydrogen, halogen, CN, C₁-C₆-alkyl, C₁-C₆-alkoxy, di-(C₁-C₆-alkyl)amino, C₁-C₆-alkylthio, and C₃-C₈-cycloalkyl,
wherein each of the 5 last mentioned radicals is unsubstituted, partly or completely halogenated,
it being also possible for R^{4b} to be selected from
C₁-C₆-alkylsulfinyl, C₁-C₆-alkylsulfonyl,
wherein each of the two last mentioned radicals is unsubstituted, partly or completely halogenated,
C₁-C₆-alkyl or C₃-C₈-cycloalkyl,
wherein each of the two last mentioned radicals carries 1, 2 or 3 identical or different radicals R⁷,
SH, S(O)ₙNR^{9a}R^{9b}, NR^{9a}R^{9b}, C(=O)OR⁸, C(=O)NR^{9a}R^{9b}, C(=S)NR^{9a}R^{9b}, C(=O)R^{7a}, C(=S)R^{7a},
a moiety Q¹-phenyl,
wherein the phenyl ring is optionally substituted with 1, 2, 3, 4 or 5 identical or different substituents R¹⁰,
and a moiety Q¹-Het^{#},
wherein
Q¹ is a single bond, NR^{9a}, C₁-C₄-alkylene or NR^{9a}-C₁-C₄-alkylene,
wherein the heteroatom in the last moiety is bound to the carbon atom of CR^{4a}R^{4b};
or
R^{4a} and R^{4b} may form together with the carbon atom they are bound to, a 3-, 4-, 5-or 6- membered aliphatic ring,
wherein each of the carbon atoms of the ring may be unsubstituted, partly or completely halogenated, and/or may carry 1 or 2 identical or different radicals selected from C₁-C₄-alkyl, C₁-C₄-haloalkyl, C₁-C₄-alkoxy and C₁-C₄-haloalkoxy;
or
R^{4a} and R^{4b} may together form =O, =CR¹³R¹⁴, =S, =NR¹⁷, =NOR¹⁶ or =NNR^{9a}R^{9b};
- R⁵: is selected from the group consisting of hydrogen, halogen, CN, C₁-C₆-alkyl, C₁-C₆-alkoxy, C₁-C₆-alkylthio, C₁-C₆-alkylsulfinyl, C₁-C₆-alkylsulfonyl, C₂-C₆-alkenyl, C₂-C₆-alkenylthio, C₂-C₆-alkenylsulfinyl, C₂-C₆-alkenylsulfonyl, C₂-C₆-alkynyl, C₂-C₆-alkynylthio, C₂-C₆-alkynylsulfinyl, C₂-C₆-alkynylsulfonyl, C₃-C₈-cycloalkyl, C₃-C₈-cycloalkoxy, C₃-C₈-cycloalkylthio, C₃-C₈-cycloalkylsulfinyl and C₃-C₈-cycloalkylsulfonyl,
wherein each of the 18 last mentioned radicals is unsubstituted, partly or completely halogenated and/or carries 1, 2 or 3 identical or different radicals R⁷, it being also possible for cycloalkyl radicals to carry 1, 2, 3, 4, 5 or 6 identical or different C₁-C₄-alkyl groups,
S(O)ₙNR^{9a}R^{9b}, O-NR^{9a}R^{9b}, NR^{9a}R^{9b}, NR^{9a}NR^{9a}R^{9b}, C(=O)OR⁸, C(=O)NR^{9a}R^{9b}, C(=S)NR^{9a}R^{9b}, C(=O)R^{7a}, C(=S)R^{7a}, NR^{9a}-C(=O)R^{7a}, NR^{9a}-C(=S)R^{7a}, NR^{9a}C(=O)NR^{9a}R^{9b}, NR^{9a}C(=S)NR^{9a}R^{9b}, NR^{9a}-S(O)ₙR^{8a}, a moiety Q-phenyl,
wherein the phenyl ring is optionally substituted with 1, 2, 3, 4 or 5 identical or different substituents R¹⁰,
a moiety Y-R^{5a},
and a moiety Q-Het^{#},
wherein
Y is O, S, S(=O) or S(=O)₂;
R^{5a} is selected from the group consisting of C(=O)NR^{9a}R^{9b}, C(=S)NR^{9a}R^{9b}, C(=O)R⁷, C(=S)R⁷;
phenyl or CH₂-phenyl,
optionally substituted with one or more, e.g. 1, 2, 3, 4 or 5 identical or different substituents R¹⁰;
a 3-, 4-, 5-, 6- or 7- membered saturated, partly saturated or unsaturated aromatic heterocyclic ring comprising 1, 2 or 3 heteroatoms selected from oxygen, nitrogen and /or sulfur, optionally substituted with 1, 2, 3 or 4 identical or different substituents R^{y},
wherein the nitrogen and/or the sulfur atom(s) of the heterocyclic ring may optionally be oxidized, and
R^{y} is selected from the group consisting of
hydrogen, halogen, cyano, nitro, C₁-C₁₀-alkyl, C₂-C₁₀-alkenyl, wherein the two last mentioned radicals may optionally be substituted with one or more identical or different radical R¹⁸,
phenyl,
optionally substituted with halogen, cyano, nitro, C₁-C₆-alkyl, C₁-C₆-haloalkyl, C₁-C₆-alkoxy or C₁-C₆-haloalkoxy, C₁-C₄ alkyl and C₁-C₄ alkoxy, wherein both of the last two mentioned aliphatic radicals may be unsubstituted, partly or completely halogenated,
OR¹⁹, -S(O)ₙR¹⁹, S(O)ₙNR^{20a}R^{20b}, NR^{20a}R^{20b}, C(=O)R¹⁸, C(=O)OR¹⁹, -C(=NR^{20a})R¹⁸, C(=O)NR^{20a}R^{20b}, C(=S)NR^{20a}R^{20b},
or
two R^{y} present together on one atom of a partly saturated heterocyclic may be =O, =CR^{21a}R^{21b}; =NR^{20a}, =NOR¹⁹ or =NNR^{20a}; or
two R^{y} on adjacent carbon atoms may be a bridge selected from CH₂CH₂CH₂CH₂, CH=CH-CH=CH, N=CH-CH=CH, CH=N-CH=CH, N=CH-N=CH, CH₂CH₂CH₂, CH=CHCH₂, CH₂CH₂NR^{17a}, CH₂CH=N, CH=CH-NR^{17a}, and form together with the carbon atoms to which the two R^{y} are bonded a 5-membered or 6-membered fused partly saturated or unsaturated, aromatic carbocyclic or heterocyclic ring, wherein the ring may optionally be substituted with one or two substituents selected from =O, OH, CH₃, OCH₃, halogen, cyano, C₁-C₄-alkoxy, C₁-C₄-haloalkoxy, C₁-C₄-alkyl and C₁-C₄-haloalkyl;
Q irrespectively of its occurrence, is a single bond, CH₂, S, O, S(=O), S(=O)₂, NR^{9a}, -O-S(=O)₂-, -NR^{9a}-S(=O)₂-, -O-C(=O)-, -NR^{9a}-C(=O)-, -O-C₁-C₄-alkylene, -S-C₁-C₄-alkylene, NR^{9a}-C₁-C₄-alkylene, -O-C(=O)-C₁-C₄-alkylene or -NR^{9a}-C(=O)-C₁-C₄-alkylene,
wherein the heteroatom in the last 7 moieties is bound to the carbon atom of C(NO₂)R³ or CR^{4a}R^{4b}, respectively;
or, if m is 1,
- R³ and R^{4a}: may also form both together and together with the existing bond a double bond between the adjacent carbon atoms;
or, if m is 1,
one of R^{4a} or R^{4b} together with R⁵ may also form with the carbon atom they are bound to, a 3-, 4-, 5- or 6- membered saturated or partially unsaturated carbocycle or heterocycle,
wherein each of the carbon atoms of the carbocycle or heterocycle may be unsubstituted or may carry 1 or 2 identical or different radicals R^{7b}, and wherein the heterocylce has 1 or 2 non-adjacent identical or different heteroatoms or heteroatom moieties as ring members, which are selected from O, S, N, and N-R^{17c};
or, if m = 0,
R³ and R⁵ together may also form with the carbon atom they are bound to, a 3-, 4-, 5- or 6-membered saturated or partially unsaturated carbocycle or heterocycle,
wherein each of the carbon atoms of the carbocycle or heterocycle may be unsubstituted or may carry 1 or 2 radicals R^{7b}, and where the heterocylce has 1 or 2 non-adjacent identical or different heteroatoms or heteroatom moieties as ring members, which are selected from O, S, N and N-R^{17c}, or
R³ and R⁵ may together form =O or =S;
where, independently of their occurrence,
- n: is 0, 1 or 2;
- R⁶: is selected from the group consisting of
halogen, cyano, azido, nitro, SCN, SF₅,
C₁-C₁₀-alkyl, C₃-C₈-cycloalkyl, C₂-C₁₀-alkenyl, C₂-C₁₀-alkynyl,
wherein each of the four last mentioned radicals may be partially or completely halogenated and/or may optionally be substituted independently from one another with 1, 2 or 3 identical or different radicals R⁷,
OR⁸, NR^{17a}R^{17b}, S(O)ₙR⁸, S(O)ₙNR^{17a}R^{17b}, C(=O)R^{7a}, C(=O)NR^{17a}R^{17b}, C(=O)OR⁸, C(=S)R^{7a}, C(=S)NR^{17a}R^{17b}, C(=S)OR⁸, C(=S)SR⁸, C(=NR¹⁷)R^{7a}, C(=NR¹⁷)NR^{17a}R^{17b}, Si(R¹¹)₂R¹²,
phenyl, optionally substituted with 1, 2, 3, 4 or 5 identical or different substituents R¹⁰,
a 3-, 4-, 5-, 6- or 7- membered saturated, partly saturated or unsaturated aromatic heterocyclic ring comprising 1, 2 or 3 heteroatoms as ring members, which are identical or different and selected from oxygen, nitrogen and sulfur,
wherein the heterocyclic ring is optionally substituted with 1, 2, 3 or 4 identical or different substituents R¹⁰, and wherein the nitrogen and/or the sulfur atom(s) of the heterocyclic ring may optionally be oxidized,
or two of R⁶ present on one ring carbon may together form =O, =CR¹³R¹⁴, =S, =NR¹⁷, =NOR¹⁶, =NNR^{9a}R^{9b};
or two R⁶ together form a linear C₂-C₇ alkylene chain, thus forming, together with the ring atom(s) to which they are bound, a 3-, 4-, 5-, 6-, 7- or 8-membered ring,
wherein 1 or 2 CH₂ moieties of the alkylene chain may be replaced by 1 or 2 heteroatom moieties selected from O, S and NR^{17c} and/or
1 or 2 of the CH₂ groups of the alkylene chain may be replaced by a group C=O, C=S and/or C=NR¹⁷; and
wherein the alkylene chain is unsubstituted or may be substituted with 1, 2, 3, 4, 5 or 6 identical or different radicals selected from the group consisting of
halogen, C₁-C₆-haloalkyl, C₁-C₆-alkoxy, C₁-C₆-haloalkoxy, C₁-C₆-alkylthio, C₁-C₆-haloalkylthio, C₃-C₈-cycloalkyl, C₃-C₈-halocycloalkyl, C₂-C₆-alkenyl, C₂-C₆-haloalkenyl, C₂-C₆-alkynyl, C₂-C₆-haloalkynyl,
phenyl which may be substituted with 1, 2, 3, 4 or 5 identical or different radicals R¹⁰, and
a 3-, 4-, 5-, 6- or 7-membered saturated, partially unsaturated or aromatic heterocyclic ring containing 1, 2 or 3 heteroatoms or heteroatom groups selected from N, O, S, NO, SO and SO₂, as ring members, wherein the heterocyclic ring may be substituted with 1, 2, 3, 4 or 5 identical or different radicals R¹⁰;
- R⁷: independently of its occurrence, is selected from the group consisting of cyano, azido, nitro, -OH, -SH, -SCN, SF₅, C₁-C₆-alkyl, C₁-C₆-haloalkyl, C₃-C₈-cycloalkyl, C₃-C₈-halocycloalkyl, C₂-C₆-alkenyl, C₂-C₆-haloalkenyl, C₂-C₆-alkynyl, C₂-C₆-haloalkynyl, C₁-C₆-alkoxy, C₁-C₆-haloalkoxy, C₁-C₆-alkylthio, C₁-C₆-alkylsulfinyl, C₁-C₆-alkylsulfonyl, C₁-C₆-haloalkylthio, Si(R¹¹)₂R¹², OR⁸, OSO₂R⁸, S(O)ₙR⁸, S(O)ₙNR^{17a}R^{17b}, NR^{17a}R^{17b}, C(=O)NR^{17a}R^{17b}, C(=S)NR^{17a}R^{17b}, C(=O)OR⁸, C(=O)R¹⁵, C(=S)R¹⁵, C(=NR¹⁷)R¹⁵,
phenyl, phenyl-C₁-C₄-alkyl, phenoxy,
wherein the phenyl ring in the last three groups is unsubstituted or may be substituted with 1, 2, 3, 4 or 5 identical or different radicals selected from halogen, C₁-C₆-alkyl, cyano, azido, nitro, C₁-C₁₀-alkyl, C₁-C₆-haloalkyl, C₁-C₆-alkoxy, C₁-C₆ haloalkoxy, (C₁-C₆-alkoxy)carbonyl, (C₁-C₆-alkyl)amino and di-(C₁-C₆-alkyl)amino, and
a 3-, 4-, 5-, 6- or 7- membered saturated, partly saturated or unsaturated aromatic heterocyclic ring comprising 1, 2 or 3 heteroatoms as ring members, which are identical or different and selected from oxygen, nitrogen and sulfur,
wherein the heterocyclic ring is unsubstituted or may be substituted with 1, 2, 3 or 4 identical or different radicals selected from halogen, C₁-C₆-alkyl, cyano, azido, nitro, C₁-C₁₀-alkyl, C₁-C₆-haloalkyl, C₁-C₆-alkoxy, C₁-C₆ haloalkoxy, (C₁-C₆-alkoxy)carbonyl, (C₁-C₆-alkyl)amino and di-(C₁-C₆-alkyl)amino, and
wherein the nitrogen and/or the sulfur atom(s) of the heterocyclic ring may optionally be oxidized,
or
two R⁷ present on one carbon atom may together form =O, =CR¹³R¹⁴, =S, =NR¹⁷, =NOR¹⁶, =NNR^{9a}R^{9b},
or
two R⁷ may form a 3-, 4-, 5-, 6-, 7- or 8-membered saturated or partly unsaturated carbocyclic or heterocyclic ring together with the carbon atoms to which the two R⁷ are bonded, wherein the heterocyclic ring comprises 1, 2 or 3 heteroatoms as ring members, which are identical or different and selected from oxygen, nitrogen and sulfur,
wherein the heterocyclic ring is unsubstituted or may be substituted with 1, 2, 3 or 4 identical or different radicals selected from halogen, C₁-C₆-alkyl, cyano, azido, nitro, C₁-C₁₀-alkyl, C₁-C₆-haloalkyl, C₁-C₆-alkoxy, C₁-C₆ haloalkoxy, (C₁-C₆-alkoxy)carbonyl, (C₁-C₆-alkyl)amino and di-(C₁-C₆-alkyl)amino;
- R^{7a}: independently of its occurrence, is selected from the group consisting of hydrogen, C₁-C₆-alkyl, C₁-C₆-haloalkyl, C₁-C₆-alkoxy, C₁-C₆-haloalkoxy, C₁-C₆-alkylthio, C₁-C₆-alkylsulfinyl, C₁-C₆-alkylsulfonyl, C₁-C₆-haloalkylthio, C₃-C₈-cycloalkyl, C₃-C₈-halocycloalkyl, C₃-C₆-cycloalkyl- C₁-C₄-alkyl, C₂-C₆-alkenyl, C₂-C₆-haloalkenyl, C₂-C₆-alkynyl, C₂-C₆-haloalkynyl,
phenyl, optionally substituted with 1, 2, 3, 4 or 5 identical or different substituents R¹⁰,
benzyl, wherein the phenyl moiety of benzyl is unsubstituted or substituted by 1, 2 or 3 identical or different radicals selected from the group consisting of halogen,CN, C₁-C₄-alkyl, C₁-C₄-haloalkyl, C₁-C₄-alkoxy and C₁-C₄-haloalkoxy, and
a 3-, 4-, 5-, 6- or 7- membered saturated, partly saturated or unsaturated aromatic heterocyclic ring comprising 1, 2 or 3 heteroatoms as ring members, which are identical or different and selected from oxygen, nitrogen and sulfur,
wherein the heterocyclic ring is optionally substituted with 1, 2, 3 or 4 identical or different substituents R¹⁰, and
wherein the nitrogen and/or the sulfur atom(s) of the heterocyclic ring may optionally be oxidized;
- R^{7b}: is selected from the group consisting of halogen, cyano, C₁-C₁₀-alkyl, C₃-C₈-cycloalkyl, C₂-C₁₀-alkenyl, C₂-C₁₀-alkynyl,
wherein each of the four last mentioned radicals is unsubstituted, partly or completely halogenated, and/or may be substituted with 1, 2 or 3 identical or different radicals R⁷,
OR⁸, NR^{17a}R^{17b}, S(O)ₙR⁸, S(O)ₙNR^{17a}R^{17b}, C(=O)R^{7a}, C(=O)NR^{17a}R^{17b}, C(=O)OR⁸, C(=S)R^{7a}, C(=S)NR^{17a}R^{17b}, C(=S)OR⁸, C(=NR¹⁷)R^{7a}, C(=NR¹⁷)NR^{17a}R^{17b}, Si(R¹¹)₂R¹²; phenyl, optionally substituted with 1, 2, 3, 4 or 5 identical or different substituents R¹⁰,
a 3-, 4-, 5-, 6- or 7- membered saturated, partly saturated or unsaturated aromatic heterocyclic ring comprising 1, 2 or 3 heteroatoms as ring members, which are identical or different and selected from oxygen, nitrogen and sulfur,
wherein the heterocyclic ring is optionally substituted with 1, 2, 3 or 4 identical or different substituents R¹⁰, and wherein the nitrogen and/or the sulfur atom(s) of the heterocyclic ring may optionally be oxidized,
or
two of R^{7b} present on one ring carbon may together form =O, =S or =CR¹³R¹⁴,
or
two R^{7b} together form a linear C₂-C₇ alkylene chain, thus forming, together with the ring atom(s) to which they are bound, a 3-, 4-, 5-, 6-, 7- or 8-membered ring,
wherein 1 or 2 CH₂ moieties of the alkylene chain may be replaced by 1 or 2 heteroatom moieties selected from O, S and NR^{17c} and/or
1 or 2 of the CH₂ groups of the alkylene chain may be replaced by a group C=O, C=S and/or C=NR¹⁷; and
wherein the alkylene chain is unsubstituted or may be substituted with 1, 2, 3, 4, 5 or 6 identical or different radicals selected from the group consisting of
halogen, C₁-C₄-alkyl, C₁-C₆-haloalkyl, C₁-C₆-alkoxy, C₁-C₆-haloalkoxy, C₁-C₆-alkylthio, C₁-C₆-haloalkylthio, C₃-C₈-cycloalkyl, C₃-C₈-halocycloalkyl, C₂-C₆-alkenyl, C₂-C₆-haloalkenyl, C₂-C₆-alkynyl, C₂-C₆-haloalkynyl, phenyl which may be substituted with 1, 2, 3, 4 or 5 identical or different radicals R¹⁰, and
a 3-, 4-, 5-, 6- or 7-membered saturated, partially unsaturated or aromatic heterocyclic ring containing 1, 2 or 3 heteroatoms or heteroatom groups selected from N, O, S, NO, SO and SO₂, as ring members, wherein the heterocyclic ring may be substituted with 1, 2, 3, 4 or 5 identical or different radicals R¹⁰;
- R⁸: independently of its occurrence, is selected from the group consisting of hydrogen, C₁-C₆-alkyl, C₁-C₆-haloalkyl, C₃-C₈-cycloalkyl, C₃-C₈-cycloalkyl-C₁-C₄-alkyl, C₃-C₈-halocycloalkyl, C₂-C₆-alkenyl, C₂-C₆-haloalkenyl, C₂-C₆-alkynyl, C₂-C₆ haloalkynyl, C(=O)R¹⁵, C(=O)NR^{17a}R^{17b}, C(=S)NR^{17a}R^{17b}, C(=O)OR¹⁶,
phenyl, phenyl-C₁-C-₄-alkyl, wherein the phenyl ring in the last two mentioned radicals is unsubstituted or substituted with 1, 2, 3, 4 or 5 identical or different substituents R¹⁰, and
a 3-, 4-, 5-, 6- or 7- membered saturated, partly saturated or unsaturated aromatic heterocyclic ring comprising 1, 2 or 3 heteroatoms as ring members, which are identical or different and selected from oxygen, nitrogen and sulfur,
wherein the heterocyclic ring is optionally substituted with 1, 2, 3 or 4 identical or different substituents R¹⁰, and wherein the nitrogen and/or the sulfur atom(s) of the heterocyclic ring may optionally be oxidized,
- R^{8a}: independently of its occurrence, is selected from the group consisting of
C₁-C₆-alkyl, C₃-C₈-cycloalkyl, C₃-C₈-cycloalkyl-C₁-C₄-alkyl, C₂-C₆-alkenyl, C₂-C₆-alkynyl,
wherein each of the five last mentioned radicals is unsubstituted, partly or completely halogenated,
phenyl, benzyl,
wherein the phenyl ring in the last two mentioned radicals is unsubstituted or may be substituted with 1, 2, 3, 4 or 5 identical or different substituents R¹⁰,
and a 3-, 4-, 5-, 6- or 7- membered saturated, partly saturated or unsaturated aromatic heterocyclic ring comprising 1, 2 or 3 heteroatoms as ring members, which are identical or different and selected from oxygen, nitrogen and sulfur,
wherein the heterocyclic ring is optionally substituted with 1, 2, 3 or 4 identical or different substituents R¹⁰, and wherein the nitrogen and/or the sulfur atom(s) of the heterocyclic ring may optionally be oxidized,
- R⁹: independently of its occurrence, is selected from the group consisting of hydrogen, C₁-C₆-alkyl, C₁-C₆-haloalkyl, C₁-C₆-alkoxy, C₁-C₆-haloalkoxy, C₃-C₈-cycloalkyl, C₃-C₈-cycloalkyl-C₁-C₄-alkyl, C₃-C₈-halocycloalkyl, C₂-C₆-alkenyl, C₂-C₆-haloalkenyl, C₂-C₆-alkynyl, C₂-C₆ haloalkynyl,
phenyl, optionally substituted with 1, 2, 3, 4 or 5 identical or different substituents R¹⁰;
a 3-, 4-, 5-, 6- or 7- membered saturated, partly saturated or unsaturated aromatic C-bound heterocyclic ring comprising 1, 2 or 3 heteroatoms as ring members, which are identical or different and selected from oxygen, nitrogen and sulfur,
wherein the heterocyclic ring is optionally substituted with 1, 2, 3 or 4 identical or different substituents R¹⁰, and
wherein the nitrogen and/or the sulfur atom(s) of the heterocyclic ring may optionally be oxidized,
- R^{9a} and R^{9b}: are each independently from one another selected from the group consisting of
hydrogen, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₃-C₈-cycloalkyl, C₃-C₈-halocycloalkyl, C₃-C₈-cycloalkyl-C₁-C₄-alkyl, C₂-C₆-alkenyl, C₂-C₆-haloalkenyl, C₂-C₆-alkynyl, C₂-C₆ haloalkynyl,
S(O)ₙR¹⁶, -S(O)ₙNR^{17a}R^{17b}, C(=O)R¹⁵, C(=O)OR¹⁶, C(=O)NR^{17a}R^{17b}, C(=S)R¹⁵, C(=S)SR¹⁶, C(=S)NR^{17a}R^{17b}, C(=NR¹⁷)R¹⁵; phenyl, benzyl, 1-phenethyl or 2-phenethyl,
wherein the phenyl ring in the last four mentioned radicals is unsubstituted or may be substituted with 1, 2, 3, 4 or 5 identical or different substituents R¹⁰; and
a 3-, 4-, 5-, 6- or 7- membered saturated, partly saturated or unsaturated aromatic C-bound heterocyclic ring comprising 1, 2 or 3 heteroatoms as ring members, which are identical or different and selected from oxygen, nitrogen and sulfur,
wherein the heterocyclic ring is optionally substituted with 1, 2, 3 or 4 identical or different substituents R¹⁰, and
wherein the nitrogen and/or the sulfur atom(s) of the heterocyclic ring may optionally be oxidized,
or,
R^{9a} and R^{9b} are together a C₂-C₇ alkylene chain and form a 3-, 4-, 5-, 6-, 7- or 8-membered saturated, partly saturated or unsaturated aromatic ring together with the nitrogen atom they are bonded to,
wherein the alkylene chain may contain one or two heteroatoms, which are, independently of each other, selected from oxygen, sulfur or nitrogen,
where the alkylene chain may optionally be substituted with 1, 2, 3 or 4 radicals selected from
halogen, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₃-C₈-cycloalkyl, C₃-C₈-halocycloalkyl, C₂-C₆-alkenyl, C₂-C₆-haloalkenyl, C₂-C₆-alkynyl, C₂-C₆ haloalkynyl,
phenyl, optionally substituted with 1, 2, 3, 4 or 5 identical or different substituents R¹⁰, and
a 3-, 4-, 5-, 6- or 7- membered saturated, partly saturated or unsaturated aromatic C-bound heterocyclic ring comprising 1, 2 or 3 heteroatoms as ring members, which are identical or different and selected from oxygen, nitrogen and sulfur,
wherein the heterocyclic ring is optionally substituted with 1, 2, 3 or 4 identical or different substituents R¹⁰, and
wherein the nitrogen and/or the sulfur atom(s) of the heterocyclic ring may optionally be oxidized,
or
- R^{9a} and R^{9b}: together may form =CR¹³R¹⁴, =NR¹⁷, =NOR¹⁶, =NNR^{17a}R^{17b} moiety;
- R¹⁰: independently of its occurrence, is selected from the group consisting of halogen, cyano, azido, nitro, SCN, SF₅, C₁-C₁₀-alkyl, C₁-C₄-haloalkyl, C₃-C₈-cycloalkyl, C₂-C₁₀-alkenyl, C₂-C₁₀-alkynyl,
wherein each of the four last mentioned radicals may optionally be substituted with 1, 2, 3, 4 or 5 identical or different radicals R⁷,
Si(R¹¹)₂R¹², OR¹⁶, OS(O)ₙR¹⁶, -S(O)ₙR¹⁶, S(O)ₙNR^{17a}R^{17b}, NR^{17a}R^{17b}, C(=O)R¹⁵, C(=S)R¹⁵, C(=O)OR¹⁶, -C(=NR¹⁷)R¹⁵, C(=O)NR^{17a}R^{17b}, C(=S)NR^{17a}R^{17b}, phenyl,
optionally substituted with 1, 2, 3, 4 or 5 identical or different radicals selected from OH, halogen, cyano, nitro, C₁-C₆-alkyl, C₁-C₆-haloalkyl, C₁-C₆-alkoxy and C₁-C₆-haloalkoxy, and
a 3-, 4-, 5-, 6- or 7- membered saturated, partly saturated or unsaturated aromatic heterocyclic ring comprising 1, 2 or 3 heteroatoms as ring members, which are identical or different and selected from oxygen, nitrogen and sulfur,
wherein the heterocyclic ring is unsubstituted or may be substituted with 1, 2, 3, 4 or 5 identical or different substituents selected from halogen, cyano, NO₂, C₁-C₆-alkyl, C₁-C₆-haloalkyl, C₁-C₆-alkoxy and C₁-C₆-haloalkoxy, and
wherein the nitrogen and/or the sulfur atom(s) of the heterocyclic ring may optionally be oxidized;
or
two R¹⁰ present together on one carbon ring atom of a saturated or partly unsaturated heterocyclic radical may form =O, =CR¹³R¹⁴, =S, =NR¹⁷, =NOR¹⁶, =NNR^{17a}R^{17b};
or,
two R¹⁰ on adjacent carbon ring atoms may together be a bivalent radical selected from CH₂CH₂CH₂CH₂, CH=CH-CH=CH, N=CH-CH=CH, CH=N-CH=CH, N=CH-N=CH, OCH₂CH₂CH₂, OCH=CHCH₂, CH₂OCH₂CH₂, OCH₂CH₂O, OCH₂OCH₂, CH₂CH₂CH₂, CH=CHCH₂, CH₂CH₂O, CH=CHO, CH₂OCH₂, CH₂C(=O)O, C(=O)OCH₂, O(CH₂)O, SCH₂CH₂CH₂, SCH=CHCH₂, CH₂SCH₂CH₂, SCH₂CH₂S, SCH₂SCH₂, CH₂CH₂S, CH=CHS, CH₂SCH₂, CH₂C(=S)S, C(=S)SCH₂, S(CH₂)S, CH₂CH₂NR¹⁷, CH₂CH=N, CH=CH-NR¹⁷, OCH=N, SCH=N and form together with said adjacent carbon ring atoms a 5-membered or 6-membered partly saturated or unsaturated, aromatic carbocyclic or heterocyclic ring, wherein the ring may optionally be substituted with one or two substituents independently selected from =O, OH, CH₃, OCH₃, halogen, cyano, halomethyl and halomethoxy;
- R¹¹ and R¹²: independently of their occurrence, are selected from the group consisting of C₁-C₆-alkyl, C₁-C₆-haloalkyl, C₁-C₆-alkoxy, C₁-C₆-alkoxy-C₁-C₄-alkyl, C₂-C₆-alkenyl, C₂-C₆-haloalkenyl, C₂-C₆-alkynyl, C₂-C₆-haloalkynyl, C₃-C₈-cycloalkyl, C₃-C₈-halocycloalkyl, C₃-C₈-CyCloalkyl-C₁-C₄-alkyl, C₃-C₈-halocycloalkyl-C₁-C₄-alkyl, C₁-C₆-haloalkoxy-C₁-C₄-alkyl,
phenyl and benzyl,
wherein the phenyl ring in last two radicals is unsubstituted or substituted with 1, 2, 3, 4 or 5 identical or different radicals selected from halogen, OH, cyano, NO₂, C₁-C₆-alkyl, C₁-C₆-haloalkyl, C₁-C₆-alkoxy and C₁-C₆-haloalkoxy;
- R¹³ and R¹⁴: independently of their occurrence, are selected from the group consisting of hydrogen, halogen, CN, C₁-C₆-alkyl, C₃-C₆-cycloalkyl, C₁-C₄-alkoxy-C₁-C₄-alkyl, phenyl and benzyl;
- R¹⁵: independently of its occurrence, is selected from the group consisting of hydrogen, C₁-C₆-alkyl, C₂-C₆-alkenyl, C₂-C₆-alkynyl, C₃-C₈-cycloalkyl,
wherein the four last mentioned aliphatic and cycloaliphatic radicals may be unsubstituted, partially or completely halogenated and/or oxygenated and/or may carry 1 or 2 identical or different radicals selected from C₁-C₄ alkoxy;
C₃-C₆-cycloalkyl-C₁-C₄-alkyl, phenyl, benzyl and pyridyl, wherein the last three radicals may be unsubstituted, partially or completely halogenated and/or may carry 1, 2 or 3 substituents selected from C₁-C₆-alkyl, C₁-C₆-haloalkyl, C₁-C₆-alkoxy, C₁-C₆ haloalkoxy, (C₁-C₆-alkoxy)carbonyl, (C₁-C₆-alkyl)amino and di-(C₁-C₆-alkyl)amino;
- R¹⁶: independently of its occurrence, is selected from the group consisting of hydrogen, C₁-C₆-alkyl, C₂-C₆-alkenyl, C₂-C₆-alkynyl, C₃-C₈-cycloalkyl, C₃-C₈-cycloalkyl-C₁-C₄-alkyl, wherein the five last mentioned aliphatic and cycloaliphatic radicals may be unsubstituted, partially or completely halogenated and/or oxygenated and/or may carry 1 or 2 radicals selected from C₁-C₄ alkoxy,
phenyl, benzyl and pyridyl,
wherein the last three radicals may be unsubstituted, partially or completely halogenated and/or may carry 1, 2 or 3 identical or different substituents selected from C₁-C₆-alkyl, C₁-C₆-haloalkyl, C₁-C₆-alkoxy, C₁-C₆ haloalkoxy, (C₁-C₆-alkoxy)carbonyl, (C₁-C₆-alkyl)amino and di-(C₁-C₆-alkyl)amino;
- R¹⁷: independently of its occurrence, is selected from the group consisting of hydrogen, C₁-C₆-alkoxy, C₁-C₆-haloalkoxy, C₁-C₆-alkylthio, trimethylsilyl, triethylsilyl, *tert*butyldimethylsilyl, C₁-C₆-alkyl, C₂-C₆-alkenyl, C₂-C₆-alkynyl, C₃-C₈-cycloalkyl,
wherein the four last mentioned radicals may be unsubstituted, partially or completely halogenated and/or oxygenated and/or may carry 1 or 2 radicals selected from C₁-C₄-alkoxy,
C₃-C₆-cycloalkyl-C₁-C₄-alkyl, phenyl, benzyl, pyridyl, phenoxy, wherein the four last mentioned radicals may be unsubstituted, partially or completely halogenated and/or may carry 1, 2 or 3 identical or different
substituents selected from C₁-C₆-alkyl, C₁-C₆-haloalkyl, C₁-C₆-alkoxy, C₁-C₆ haloalkoxy and (C₁-C₆-alkoxy)carbonyl,
- R^{17a} and R^{17b}: are each independently from one another selected from the group consisting of hydrogen, C₁-C₆-alkoxy, C₁-C₆-haloalkoxy, C₁-C₆-alkylthio, C₁-C₆-alkylsulfinyl, C₁-C₆-alkylsulfonyl, C₁-C₆-haloalkylthio, trimethylsilyl, triethylsilyl, *tert*butyldimethylsilyl, C₁-C₆-alkyl, C₂-C₆-alkenyl, C₂-C₆-alkynyl, C₃-C₈-cycloalkyl,
wherein the four last mentioned radicals may be unsubstituted, partially or completely halogenated and/or oxygenated and/or may carry 1 or 2 identical or different radicals selected from C₁-C₄-alkoxy,
phenyl, benzyl, pyridyl, phenoxy,
wherein the four last mentioned radicals may be unsubstituted, partially or completely halogenated and/or may carry 1, 2 or 3 identical or different substituents selected from C₁-C₆-alkyl, C₁-C₆-haloalkyl, C₁-C₆-alkoxy, C₁-C₆ haloalkoxy and (C₁-C₆-alkoxy)carbonyl,
or,
R^{17a} and R^{17b} may together be a C₂-C₆ alkylene chain forming a 3- to 7-membered saturated, partly saturated or unsaturated ring together with the nitrogen atom R^{17a} and R^{17b} are bonded to,
wherein the alkylene chain may contain 1 or 2 heteroatoms selected, independently of each other, from oxygen, sulfur and nitrogen, and may optionally be substituted with halogen, C₁-C₄-haloalkyl, C₁-C₄-alkoxy or C₁-C₄-haloalkoxy, and
wherein the nitrogen and/or the sulfur atom(s) of the heterocyclic ring may optionally be oxidized;
or
R^{17a} and R^{17b} together may form a =CR¹³R¹⁴, =NR¹⁷ or =NOR¹⁶ moiety;
- R^{17c}: independently of its occurrence, is selected from the group consisting of hydrogen, CN, C₁-C₆-alkyl, C₂-C₆-alkenyl, C₂-C₆-alkynyl, C₃-C₈-cycloalkyl, C₃-C₈-cycloalkyl-C₁-C₄-alkyl,
wherein the five last mentioned radicals may be unsubstituted, partially or completely halogenated and/or oxygenated and/or may carry 1 or 2 radicals independently selected from C₁-C₄ alkoxy,
phenyl, benzyl and pyridyl, wherein the last three radicals may be unsubstituted, partially or completely halogenated and/or may carry 1, 2 or 3 identical or different substituents selected from C₁-C₆-alkyl, C₁-C₆-haloalkyl, C₁-C₆-alkoxy, C₁-C₆ haloalkoxy, (C₁-C₆-alkoxy)carbonyl, (C₁-C₆-alkyl)amino and di-(C₁-C₆-alkyl)amino;
- R¹⁸: is each independently from one another selected from the group consisting of hydrogen, halogen, cyano, nitro, OH, SH, SCN, SF₅, C₁-C₆-alkoxy, C₁-C₆-haloalkoxy, C₁-C₆-alkylthio, C₁-C₆-alkylsulfinyl, C₁-C₆-alkylsulfonyl, C₁-C₆-haloalkylthio, trimethylsilyl, triethylsilyl, *tert*butyldimethylsilyl, C₁-C₆-alkyl, C₂-C₆-alkenyl, C₂-C₆-alkynyl, C₃-C₈-cycloalkyl,
wherein the four last mentioned aliphatic and cyclo-aliphatic radicals may be unsubstituted, partially or completely halogenated and/or oxgenated and/or may carry 1 or 2 radicals selected from C₁-C₄ alkoxy;
phenyl, benzyl, pyridyl, phenoxy,
wherein the last four radicals may be unsubstituted, partially or completely halogenated and/or to carry 1, 2 or 3 substituents selected from C₁-C₆-alkyl, C₁-C₆-haloalkyl, C₁-C₆-alkoxy, C₁-C₆ haloalkoxy, (C₁-C₆-alkoxy)carbonyl, (C₁-C₆-alkyl)amino or di-(C₁-C₆-alkyl)amino,
or
two R¹⁸ present on the same carbon atom may together be =O, =CH(C₁-C₄), =C(C₁-C₄-alkyl)C₁-C₄-alkyl, =N(C₁-C₆-alkyl) or =NO(C₁-C₆-alkyl);
- R¹⁹: is each independently from one another selected from the group consisting of hydrogen, cyano, C₁-C₆-alkoxy, C₁-C₆-haloalkoxy, C₁-C₆-alkylthio, C₁-C₆-alkylsulfinyl, C₁-C₆-alkylsulfonyl, C₁-C₆-haloalkylthio, trimethylsilyl, triethylsilyl, *tert*butyldimethylsilyl, C₁-C₆-alkyl, C₂-C₆-alkenyl, C₂-C₆-alkynyl, C₃-C₈-cycloalkyl,
wherein the four last mentioned radicals may be unsubstituted, partially or completely halogenated and/or oxygenated and/or may carry 1 or 2 radicals selected from C₁-C₄ alkoxy,
phenyl, benzyl, pyridyl, phenoxy,
wherein the last four radicals may be unsubstituted, partially or completely halogenated and/or carry 1, 2 or 3 substituents selected from C₁-C₆-alkyl, C₁-C₆-haloalkyl, C₁-C₆-alkoxy, C₁-C₆ haloalkoxy or (C₁-C₆-alkoxy)carbonyl;
- R^{20a}, R^{20b}: are each independently from one another selected from the group consisting of hydrogen, cyano, C₁-C₆-alkoxy, C₁-C₆-haloalkoxy, C₁-C₆-alkylthio, C₁-C₆-alkylsulfinyl, C₁-C₆-alkylsulfonyl, C₁-C₆-haloalkylthio, trimethylsilyl, triethylsilyl, *tert*butyldimethylsilyl, C₁-C₆-alkyl, C₂-C₆-alkenyl, C₂-C₆-alkynyl, C₃-C₈-cycloalkyl,
wherein the four last mentioned aliphatic and cyclo-aliphatic radicals may be unsubstituted, partially or completely halogenated and/or oxygenated and/or may carry 1 or 2 identical or different radicals selected from C₁-C₄-alkoxy,
phenyl, benzyl, pyridyl, phenoxy,
wherein the four last mentioned radicals may be unsubstituted, partially or completely halogenated and/or carry 1, 2 or 3 identical or different substituents selected from C₁-C₆-alkyl, C₁-C₆-haloalkyl, C₁-C₆-alkoxy, C₁-C₆ haloalkoxy and (C₁-C₆-alkoxy)carbonyl,
or,
R^{20a} and R^{20b} may together be a C₂-C₆ alkylene chain forming a 3- to 7-membered saturated, partly saturated or unsaturated ring together with the nitrogen atom R^{20a} and R^{20b} are bonded to, wherein the alkylene chain may contain 1 or 2 heteroatoms selected from oxygen, sulfur and nitrogen, and may optionally be substituted with halogen, C₁-C₄-haloalkyl, C₁-C₄-alkoxy or C₁-C₄-haloalkoxy, and wherein the nitrogen and/or the sulfur atom(s) of the heterocyclic ring may optionally be oxidized;
- R^{21a}, R^{21b}: are each independently from one another selected from the group consisting of hydrogen, C₁-C₄ alkyl, C₁-C₆ cycloalkyl, C₁-C₄ alkoxyalkyl, phenyl and benzyl;
- Het^{#}: represents a 3-, 4-, 5-, 6- or 7- membered saturated, partly saturated or unsaturated aromatic heterocyclic ring comprising 1, 2 or 3 heteroatoms as ring members, which are selected from oxygen, nitrogen and/or sulfur,
wherein the heterocyclic ring is optionally substituted with 1, 2, 3, 4 or 5identical or different substituents R¹⁰, and wherein the nitrogen and/or the sulfur atom(s) of the heterocyclic ring may optionally be oxidized,
the stereoisomers, the tautomers and the salts thereof.

Moreover, the present invention relates to and includes the following embodiments:
- agricultural and veterinary compositions comprising an amount of at least one compound of the formula (I) or a stereoisomer, tautomer or salt thereof;
- the use of the compounds of formula (I), the stereoisomers, the tautomers or the salts thereof for combating invertebrate pests, excluding the use on the human or animal body by therapy;
- the use of the compounds of formula (I), the stereoisomers, the tautomers or the salts thereof for protecting growing plants from attack or infestation by invertebrate pests;
- the use of the compounds of formula (I), the stereoisomers, the tautomers or the salts, thereof for protecting plant proparagation material, especially seeds, from soil insects;
- the use of the compounds of formula (I), the stereoisomers, the tautomers or the salts thereof for protecting the seedlings roots and shoots of plants from soil and foliar insects;
- a non-therapeutic method for combating or controlling invertebrate pests, which method comprises contacting said pest or its food supply, habitat or breeding grounds with a pesticidally effective amount of at least one compound of the formula (I) or a stereoisomer, a tautomer or salt thereof;
- a method for protecting growing plants from attack or infestation by invertebrate pests, which method comprises contacting a plant, or soil or water in which the plant is growing, with a pesticidally effective amount of at least one compound of the formula (I) or a stereoisomer, a tautomer or salt thereof, in particular a method protecting crop plants from attack or infestation by animal pests, which comprises contacting the crop plants with a pesticidally effective amount of at least one compound of the formula (I) or stereoisomer, a tautomer or salt thereof;
- a method for the protection of plant propagation, especially seeds, from soil insects and of the seedlings' roots and shoots from soil and foliar insects comprising contacting the seeds before sowing and/or after pregermination with at least one compound of the formula (I) or stereoisomer, a tautomer or salt thereof;
- seeds comprising a compound of the formula (I) or an enantiomer, diastereomer or salt thereof;
- compounds of formula (I), the stereoisomers, the tautomers or the salts, in particular the veterinary acceptable salts, thereof for use in combating parasites in and on animals, in particular for the use in the treatment of animals infested or infected by parasites, for the prevention of animals of getting infected or infested by parasites or for the protection of animals against infestation or infection by parasites.

The present invention also relates to plant propagation materials, in particular as mentioned above to seeds, containing at least one compound of formula (I), a stereoisomer, a tautomer and/or an agriculturally acceptable salt thereof.

### Detailed Description of Invention

The present invention relates to every possible stereoisomer of the compounds of formula (I), i.e. to single enantiomers, diastereomers and E/Z-isomers as well as to mixtures thereof and also to the salts thereof. The present invention relates to each isomer alone, or mixtures or combinations of the isomers in any proportion to each other. Depending on the substitution pattern, the compounds of the formula (I) may have one or more centers of chirality, in which case they are present as mixtures of enantiomers or diastereomers. One center of chirality is the carbon ring atom carrying radical R¹. Another centre of chirality may be the carbon atom which carries R³, in particular if R³ is different from NO₂ and the group [CR^{4a}R^{4b}]ₘR⁵. The invention provides both the pure enantiomers or diastereomers and their mixtures and the use according to the invention of the pure enantiomers or diastereomers of the compound I or its mixtures. Suitable compounds of the formula (I) also include all possible geometrical stereoisomers (cis/trans isomers) and mixtures thereof.

The present invention also relates to potential tautomers of the compounds of formula (I) and also to the salts of such tautomers. The present invention relates to the tautomer as such as well as to mixtures or combinations of the tautomers in any proportion to each other. The term "tautomers" encompasses isomers, which are derived from the compounds of formula (I) by the shift of an H-atom involving at least one H-atom located at a nitrogen, oxygen or sulphur atom. Examples of tautomeric forms are keto-enol forms, imine-enamine forms, urea-isourea forms, thiourea-isothiourea forms, (thio)amide-(thio)imidate forms etc.

The compounds of the present invention, i.e. the compounds of formula (I), their stereoisomers, their tautomers as well as their salts, in particular their agriculturally acceptable salts and their veterinarily acceptable salts, may be amorphous or may exist in one ore more different crystalline states (polymorphs) or modifications which may have a different macroscopic properties such as stability or show different biological properties such as activities. The present invention includes both amorphous and crystalline compounds of the formula (I), mixtures of different crystalline states or modifications of the respective stereoisomers or tautomers, as well as amorphous or crystalline salts thereof.

Salts of the compounds of the formula (I) are preferably agriculturally salts as well as veterinarily acceptable salts. They can be formed in a customary method, e.g. by reacting the compound with an acid of the anion in question if the compound of formula (I) has a basic functionality or by reacting an acidic compound of formula (I) with a suitable base.

Suitable agriculturally or veterinary useful salts are especially the salts of those cations or anions, in particular the acid addition salts of those acids, whose cations and anions, respectively, do not have any adverse effect on the action of the compounds according to the present invention. Suitable cations are in particular the ions of the alkali metals, preferably lithium, sodium and potassium, of the alkaline earth metals, preferably calcium, magnesium and barium, and of the transition metals, preferably manganese, copper, zinc and iron, and also ammonium (NH₄⁺) and substituted ammonium in which one to four of the hydrogen atoms are replaced by C₁-C₄-alkyl, C₁-C₄-hydroxyalkyl, C₁-C₄-alkoxy, C₁-C₄-alkoxy-C₁-C₄-alkyl, hydroxy-C₁-C₄-alkoxy-C₁-C₄-alkyl, phenyl or benzyl. Examples of substituted ammonium ions comprise methylammonium, isopropylammonium, dimethylammonium, diisopropylammonium, trimethylammonium, tetramethylammonium, tetraethylammonium, tetrabutylammonium, 2-hydroxyethylammonium, 2-(2-hydroxyethoxy)ethyl-ammonium, bis(2-hydroxyethyl)ammonium, benzyltrimethylammonium and benzyltriethylammonium, furthermore phosphonium ions, sulfonium ions, preferably tri(C₁-C₄-alkyl)sulfonium, and sulfoxonium ions, preferably tri(C₁-C₄-alkyl)sulfoxonium.

Anions of useful acid addition salts are primarily chloride, bromide, fluoride, hydrogen sulfate, sulfate, dihydrogen phosphate, hydrogen phosphate, phosphate, nitrate, hydrogen carbonate, carbonate, hexafluorosilicate, hexafluorophosphate, benzoate, and the anions of C₁-C₄-alkanoic acids, preferably formate, acetate, propionate and butyrate. They can be formed by reacting the compounds of the formulae I with an acid of the corresponding anion, preferably of hydrochloric acid, hydrobromic acid, sulfuric acid, phosphoric acid or nitric acid.

The organic moieties mentioned in the above definitions of the variables are - like the term halogen - collective terms for individual listings of the individual group members. The prefix Cₙ-Cₘ indicates in each case the possible number of carbon atoms in the group.

"Halogen" will be taken to mean fluoro, chloro, bromo and iodo.
The term "partially or completely halogenated" will be taken to mean that 1 or more, e.g. 1, 2, 3, 4 or 5 or all of the hydrogen atoms of a given radical have been replaced by a halogen atom, in particular by fluorine or chlorine. For example, partially or completely halogenated alkyl is also termed haloalkyl, partially or completely halogenated cycloalkyl is also termed halocycloalkyl, partially or completely halogenated alkylenyl is also termed haloalkenyl, partially or completely halogenated alkylynyl is also termed haloalkynyl, partially or completely halogenated alkoxy is also termed haloalkoxy, partially or completely halogenated alkylthio is also termed haloalkthio, partially or completely halogenated alkylsulfinyl is also termed haloalkylsulfinyl, partially or completely halogenated alkylsulfonyl is also termed haloalsulfonyl, partially or completely halogenated cycloalkylalkyl is also termed halocycloalkylalkyl.

The term "Cₙ-Cₘ-alkyl" as used herein, and also in Cₙ-Cₘ-alkylamino, di-Cₙ-Cₘ-alkylamino, Cₙ-Cₘ-alkylaminocarbonyl, di-(Cₙ-Cₘ-alkylamino)carbonyl, Cₙ-Cₘ-alkylthio, Cₙ-Cₘ-alkylsulfinyl and Cₙ-Cₘ-alkylsulfonyl, refers to a branched or unbranched saturated hydrocarbon group having n to m, e.g. 1 to 10 carbon atoms, preferably 1 to 6 carbon atoms, for example methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl, 1-ethylpropyl, hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, heptyl, octyl, 2-ethylhexyl, nonyl and decyl and their isomers. C₁-C₄-alkyl means for example methyl, ethyl, propyl, 1-methylethyl, butyl, 1-methylpropyl, 2-methylpropyl or 1,1-dimethylethyl.

The term "Cₙ-Cₘ-haloalkyl" as used herein, and also in Cₙ-Cₘ-haloalkylthio (= Cₙ-Cₘ-haloalkylsulfenyl), Cₙ-Cₘ-haloalkylsulfinyl and Cₙ-Cₘ-haloalkylsulfonyl, refers to a straight-chain or branched alkyl group having n to m carbon atoms, e.g. 1 to 10 in particular 1 to 6 carbon atoms (as mentioned above), where some or all of the hydrogen atoms in these groups may be replaced by halogen atoms as mentioned above, for example C₁-C₄-haloalkyl, such as chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl, pentafluoroethyl and the like. The term C₁-C₁₀-haloalkyl in particular comprises C₁-C₂-fluoroalkyl, which is synonym with methyl or ethyl, wherein 1, 2, 3, 4 or 5 hydrogen atoms are substituted by fluorine atoms, such as fluoromethyl, difluoromethyl, trifluoromethyl, 1-fluoroethyl, 2-fluoroethyl, 2,2-difluoroethyl, 2,2,2-trifluoroethyl and pentafluoromethyl. "Halomethyl" is methyl in which 1, 2 or 3 of the hydrogen atoms are replaced by halogen atoms. Examples are bromomethyl, chloromethyl, fluoromethyl, dichloromethyl, trichloromethyl, difluoromethyl, trifluoromethyl, chlorofluoromethyl, dichlorofluoromethyl, chlorodifluoromethyl and the like. Similarly, "Cₙ-Cₘ-alkoxy", "Cₙ-Cₘ-alkylthio", or "Cₙ-Cₘ-alkylsulfenyl", respectively, "Cₙ-Cₘ-alkylsulfinyl" or "Cₙ-Cₘ-alkylsulfonyl" refer to straight-chain or branched alkyl groups having n to m carbon atoms, e.g. 1 to 10, in particular 1 to 6 or 1 to 4 carbon atoms (as mentioned above) bonded through O, S, S(=O) or S(=O)₂ linkages, respectively, at any bond in the alkyl group. Accordingly, the terms "Cₙ-Cₘ-haloalkoxy", "Cₙ-Cₘ-haloalkylthio" or "Cₙ-Cₘ-alkylsulfenyl", respectively, "Cₙ-Cₘ-haloalkylsulfinyl" or "Cₙ-Cₘ-haloalkylsulfonyl", refer to straight-chain or branched alkyl groups having n to m carbon atoms, e.g. 1 to 10, in particular 1 to 6 or 1 to 4 carbon atoms (as mentioned above) bonded through O, S, S(=O) or S(=O)₂ linkages, respectively, at any bond in the haloalkyl group, where some or all of the hydrogen atoms in these groups may be replaced by halogen atoms as mentioned above.

The term "C₁-Cₘ-alkoxy" is a C₁-Cₘ-alkyl group, as defined above, attached via an oxygen atom. C₁-C₂-Alkoxy is methoxy or ethoxy. C₁-C₄-Alkoxy is, for example, methoxy, ethoxy, n-propoxy, 1-methylethoxy (isopropoxy), butoxy, 1-methylpropoxy (sec-butoxy), 2-methylpropoxy (isobutoxy) or 1,1-dimethylethoxy (tert-butoxy). C₁-C₆-Alkoxy includes the meanings given for C₁-C₄-alkoxy and also includes, for example, pentoxy, 1-methylbutoxy, 2-methylbutoxy, 3-methylbutoxy, 1,1-dimethylpropoxy, 1,2-dimethylpropoxy, 2,2-dimethylpropoxy, 1-ethylpropoxy, hexoxy, 1-methylpentoxy, 2-methylpentoxy, 3-methylpentoxy, 4-methylpentoxy, 1,1-dimethylbutoxy, 1,2-dimethylbutoxy, 1,3-dimethylbutoxy, 2,2-dimethylbutoxy, 2,3-dimethylbutoxy, 3,3-dimethylbutoxy, 1-ethylbutoxy, 2-ethylbutoxy, 1,1,2-trimethylpropoxy, 1,2,2-trimethylpropoxy, 1-ethyl-1-methylpropoxy or 1-ethyl-2-methylpropoxy. C₁-C₈-Alkoxy includes the meanings given for C₁-C₆-alkoxy and also includes, for example, heptyloxy, octyloxy, 2-ethylhexyloxy and positional isomers thereof. C₁-C₁₀-Alkoxy includes the meanings given for C₁-C₈-alkoxy and also includes, for example, nonyloxy, decyloxy and positional isomers thereof.

The term "C₁-Cₘ-alkylthio" is a C₁-Cₘ-alkyl group, as defined above, attached via a sulphur atom. C₁-C₂-Alkylthio is methylthio or ethylthio. C₁-C₄-Alkylthio is, for example, methylthio, ethylthio, n-propylthio, 1-methylethylthio (isopropylthio), butylthio, 1-methylpropylthio (sec-butylthio), 2-methylpropylthio (isobutylthio) or 1,1-dimethylethylthio (tert-butylthio). C₁-C₆-Alkylthio includes the meanings given for C₁-C₄-alkylthio and also includes, for example, pentylthio, 1-methylbutylthio, 2-methylbutylthio, 3-methylbutylthio, 1,1-dimethylpropylthio, 1,2-dimethylpropylthio, 2,2-dimethylpropylthio, 1-ethylpropylthio, hexylthio, 1-methylpentylthio, 2-methylpentylthio, 3-methylpentylthio, 4-methylpentylthio, 1,1-dimethylbutylthio, 1,2-dimethylbutylthio, 1,3-dimethylbutylthio, 2,2-dimethylbutylthio, 2,3-dimethylbutylthio, 3,3-dimethylbutylthio, 1-ethylbutylthio, 2-ethylbutylthio, 1,1,2-trimethylpropylthio, 1,2,2-trimethylpropylthio, 1-ethyl-1-methylpropylthio or 1-ethyl-2-methylpropylthio. C₁-C₈-Alkylthio includes the meanings given for C₁-C₆-alkylthio and also includes, for example, heptylthio, octylthio, 2-ethylhexylthio and positional isomers thereof. C₁-C₁₀-Alkylthio includes the meanings given for C₁-C₈-alkylthio and also includes, for example, nonylthio, decylthio and positional isomers thereof.

The term "C₁-Cₘ-alkylsulfinyl" is a C₁-Cₘ-alkyl group, as defined above, attached via a S(=O) group. The term "C₁-Cₘ-alkylsulfonyl" is a C₁-Cₘ-alkyl group, as defined above, attached via a S(=O)2 group.

The term "C₁-Cₘ-haloalkyloxy" is a C₁-Cₘ-haloalkyl group, as defined above, attached via an oxygen atom. Examples include C₁-C₂-haloalkoxy, such as chloromethoxy, bromomethoxy, dichloromethoxy, trichloromethoxy, fluoromethoxy, difluoromethoxy, trifluoromethoxy, chlorofluoromethoxy, dichlorofluoromethoxy, chlorodifluoromethoxy, 1-chloroethoxy, 1-bromoethoxy, 1-fluoroethoxy, 2-fluoroethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloroethoxy and pentafluoroethoxy.

The term "C₁-Cₘ-haloalkylthio" is a C₁-Cₘ-haloalkyl group, as defined above, attached via a sulfur atom. Examples include C₁-C₂-haloalkylthio, such as chloromethylthio, bromomethylthio, dichloromethylthio, trichloromethylthio, fluoromethylthio, difluoromethylthio, trifluoromethylthio, chlorofluoromethylthio, dichlorofluoromethylthio, chlorodifluoromethylthio, 1-chloroethylthio, 1-bromoethylthio, 1-fluoroethylthio, 2-fluoroethylthio, 2,2-difluoroethylthio, 2,2,2-trifluoroethylthio, 2-chloro-2-fluoroethylthio, 2-chloro-2,2-difluoroethylthio, 2,2-dichloro-2-fluoroethylthio, 2,2,2-trichloroethylthio and pentafluoroethylthio and the like.

Similarly the terms C₁-C₂-fluoroalkoxy and C₁-C₂-fluoroalkylthio refer to C₁-C₂-fluoroalkyl which is bound to the remainder of the molecule via an oxygen atom or a sulfur atom, respectively.

The term "C₁-Cₘ-haloalkylsulfinyl" is a C₁-Cₘ-haloalkyl group, as defined above, attached via a S(=O) group. The term "C₁-Cₘ-haloalkylsulfonyl" is a C₁-Cₘ-haloalkyl group, as defined above, attached via a S(=O)₂ group.

The term "C₂-Cₘ-alkenyl" as used herein denotes a linear or branched ethylenically unsaturated hydrocarbon group having 2 to m, e.g. 2 to 10 or 2 to 6 carbon atoms and a C=C-double bond in any position, such as ethenyl, 1-propenyl, 2-propenyl, 1-methyl-ethenyl, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl and 1-ethyl-2-methyl-2-propenyl.

The term "C₂-Cₘ-haloalkenyl" as used herein, which is also expressed as "C₂-Cₘ-alkenyl which is partially or completely halogenated", refers to C₂-Cₘ-alkenyl, where some or all of the hydrogen atoms in these groups are replaced by halogen atoms as mentioned above, in particular fluorine, chlorine and bromine, for example 1-fluoroethenyl, 2-fluoroethenyl, 2,2-difluoroethenyl, 1,2,2-trifluoroethenyl, 1-fluoro-2-propenyl, 2-fluoro-2-propenyl, 3-fluoro-2-propenyl, 1-fluoro-1-propenyl, 1,2-difluoro-1-propenyl, 3,3-difluoropropen-2-yl, 1-chloroethenyl, 2-chloroethenyl, 2,2,-dichloroethenyl, 1-chloro-2-propenyl, and the like.

The term "C₂-Cₘ-alkynyl" as used herein refers to a linear or branched unsaturated hydrocarbon group having 2 to m, e.g. 2 to 10 or 2 to 6 carbon atoms and containing at least one triple bond, such as ethynyl, propynyl, 1-butynyl, 2-butynyl, and the like.

The term "C₂-Cₘ-haloalkynyl" as used herein, which is also expressed as "C₂-Cₘ-alkynyl which is partially or completely halogenated", refers to C₂-Cₘ-alkynyl, where some or all of the hydrogen atoms in these groups are replaced by halogen atoms as mentioned above, in particular fluorine, chlorine and bromine. Examples of C₂-Cₘ-haloalkynyl include 1-fluoro-2-propenyl, 2-fluoro-2-propenyl, 3-fluoro-2-propenyl, 1-fluoro-2-propynyl and 1,1-difluoro-2-propenyl, and the like.

The term "C₃-Cₘ-cycloalkyl" as used herein refers to a monocyclic and polycyclic 3- to m-membered saturated cycloaliphatic radical, e.g. cyclopropyl, cyclobutyl, cyclopentyl, cyclohexyl, cycloheptyl, cyclooctyl, cyclodecyl, bicyclo[2.2.1]heptyl, bicyclo[3.1.1]heptyl, bicyclo[2.2.2]octyl and bicyclo[3.2.1]octyl. Preferably, the term cycloalkyl denotes a monocyclic saturated hydrocarbon radical.

The term "C₃-Cₘ-cycloalkenyl" as used herein refers to a monocyclic and polycyclic 3- to m-membered unsaturated cycloaliphatic radical, e.g. cyclopropenyl, cyclobutenyl, cyclopentenyl, cyclohexenyl, cycloheptenyl, cyclooctenyl, cyclodecenyl, bicyclo[2.2.1]heptenyl, bicyclo[3.1.1]heptenyl, bicyclo[2.2.2]octenyl and bicyclo[3.2.1 ]octenyl. Preferably, the term cycloalkenyl denotes a monocyclic unsaturated hydrocarbon radical.

The term "C₃-Cₘ-halocycloalkyl" as used herein, which is also expressed as "cycloalkyl which is partially or completely halogenated", refers C₃-Cₘ-cycloalkyl as mentioned above, in which some or all of the hydrogen atoms are replaced by halogen atoms as mentioned above, in particular fluorine, chlorine and bromine. Examples of C₃-Cₘ-halocycloalkyl include 1-fluorocycloprpyl, 2-fluorocyclopropyl, 2,2-difluorocyclopropyl, 1-chlorocyclcopropyl, 2-chlorocyclopropyl, 2,2-dichlorocyclopropyl, 2,3-difluorocyclopropyl, 1-fluorocycobutyl etc.

The term "C₃-Cₘ-cycloalkyl-C₁-C₄-alkyl" refers to a C₃-Cₘ-cycloalkyl group as defined above, which is bound to the remainder of the molecule via a C₁-C₄-alkyl group, as defined above. Examples for C₃-Cₘ-cycloalkyl-C₁-C₄-alkyl are cyclopropylmethyl, cyclopropylethyl, cyclopropylpropyl, cyclobutylmethyl, cyclobutylethyl, cyclobutylpropyl, cyclopentylmethyl, cyclopentylethyl, cyclopentylpropyl, cyclohexylmethyl, cyclohexylethyl and cyclohexylpropyl.

The term "C₃-Cₘ-halocycloalkyl-C₁-C₄-alkyl" refers to a C₃-Cₘ-halocycloalkyl group as defined above which is bound to the remainder of the molecule via a C₁-C₄-alkyl group, as defined above.

The term "C₁-C₄-alkoxy-C₁-C₄-alkyl" as used herein refers to alkyl having 1 to 4 carbon atoms, e.g. like specific examples mentioned above, wherein one hydrogen atom of the alkyl radical is replaced by an C₁-C₄-alkoxy group. Examples are methoxymethyl, ethoxymethyl, propoxymethyl, isopropoxymethyl, n-butoxymethyl, sec-butoxymethyl, isobutoxymethyl, tert-butoxymethyl, 1-methoxyethyl, 1-ethoxyethyl, 1-propoxyethyl, 1-isopropoxyethyl, 1-n-butoxyethyl, 1-sec-butoxyethyl, 1-isobutoxyethyl, 1-tert-butoxyethyl, 2-methoxyethyl, 2-ethoxyethyl, 2-propoxyethyl, 2-isopropoxyethyl, 2-n-butoxyethyl, 2-sec-butoxyethyl, 2-isobutoxyethyl, 2-tert-butoxyethyl, 1-methoxypropyl, 1-ethoxypropyl, 1-propoxypropyl, 1-isopropoxypropyl, 1-n-butoxypropyl, 1-sec-butoxypropyl, 1-isobutoxypropyl, 1-tert-butoxypropyl, 2-methoxypropyl, 2-ethoxypropyl, 2-propoxypropyl, 2-isopropoxypropyl, 2-n-butoxypropyl, 2-sec-butoxypropyl, 2-isobutoxypropyl, 2-tert-butoxypropyl, 3-methoxypropyl, 3-ethoxypropyl, 3-propoxypropyl, 3-isopropoxypropyl, 3-n-butoxypropyl, 3-sec-butoxypropyl, 3-isobutoxypropyl, 3-tert-butoxypropyl and the like.

The term C₁-C₄-haloalkoxy-C₁-C₄-alkyl is a straight-chain or branched alkyl group having from 1 to 4 carbon atoms, wherein one of the hydrogen atoms is replaced by a C₁-C₄-alkoxy group and wherein at least one, e.g. 1, 2, 3, 4 or all of the remaining hydrogen atoms, either in the alkoxy moiety or in the alkyl moiety or in both, are replaced by halogen atoms. Examples are difluoromethoxymethyl (CHF₂OCH₂), trifluoromethoxymethyl, 1-difluoromethoxyethyl, 1-trifluoromethoxyethyl, 2-difluoromethoxyethyl, 2-trifluoromethoxyethyl, difluoro-methoxy-methyl (CH₃OCF₂), 1,1-difluoro-2-methoxyethyl, 2,2-difluoro-2-methoxyethyl and the like.

The term "C₁-Cₘ-alkoxycarbonyl" is a C₁-Cₘ-alkoxy group, as defined above, attached via an carbonyl group atom. C₁-C₂-Alkoxycarbonyl is methoxycarbonyl or ethoxycarbonyl. C₁-C₄-Alkoxy is, for example, methoxycarbonyl, ethoxycarbonyl, n-propoxycarbonyl, 1-methylethoxycarbonyl, butoxycarbonyl, 1-methylpropoxycarbonyl, 2-methylpropoxycarbonyl or 1,1-dimethylethoxycarbonyl. C₁-C₆-Alkoxycarbonyl includes the meanings given for C₁-C₄-alkoxycarbonyl and also includes, for example, pentoxycarbonyl, 1-methylbutoxycarbonyl, 2-methylbutoxycarbonyl, 3-methylbutoxycarbonyl, 1,1-dimethylpropoxycarbonyl, 1,2-dimethylpropoxycarbonyl, 2,2-dimethylpropoxycarbonyl, 1-ethylpropoxycarbonyl, hexoxycarbonyl, 1-methylpentoxycarbonyl, 2-methylpentoxycarbonyl, 3-methylpentoxycarbonyl, 4-methylpentoxycarbonyl, 1,1-dimethylbutoxycarbonyl, 1,2-dimethylbutoxycarbonyl, 1,3-dimethylbutoxycarbonyl, 2,2-dimethylbutoxycarbonyl, 2,3-dimethylbutoxycarbonyl, 3,3-dimethylbutoxycarbonyl, 1-ethylbutoxy, 2-ethylbutoxycarbonyl, 1,1,2-trimethylpropoxycarbonyl, 1,2,2-trimethylpropoxycarbonyl, 1-ethyl-1-methylpropoxycarbonyl or 1-ethyl-2-methylpropoxycarbonyl.

The term "aryl" as used herein refers to an aromatic hydrocarbon radical such as naphthyl or in particular phenyl.

The term "3- to 6-membered carbocyclic ring" as used herein refers to cyclopropane, cyclobutane, cyclopentane and cyclohexane rings. The term "3- to 7-membered carbocyclic ring" as used herein refers to cyclopropane, cyclobutane, cyclopentane, cyclohexane and cycloheptane rings.

The term "3-, 4-, 5-, 6- or 7-membered saturated, partially unsaturated or aromatic heterocyclic ring containing 1, 2 or 3 heteroatoms" or "containing heteroatom groups", wherein those heteroatom(s) (group(s)) are selected from N, O, S, NO, SO and SO₂ and are ring members, as used herein refers to monocyclic radicals, the monocyclic radicals being saturated, partially unsaturated or aromatic. The heterocyclic radical may be attached to the remainder of the molecule via a carbon ring member or via a nitrogen ring member.

Examples of 3-, 4-, 5-, 6- or 7-membered saturated heterocyclic rings include: oxiranyl, aziridinyl, azetidinyl, 2 tetrahydrofuranyl, 3-tetrahydrofuranyl, 2 tetrahydrothienyl, 3 tetrahydrothienyl, 2-pyrrolidinyl, 3-pyrrolidinyl, 3 pyrazolidinyl, 4 pyrazolidinyl, 5-pyrazolidinyl, 2 imidazolidinyl, 4 imidazolidinyl, 2-oxazolidinyl, 4-oxazolidinyl, 5 oxazolidinyl, 3-isoxazolidinyl, 4 isoxazolidinyl, 5 isoxazolidinyl, 2 thiazolidinyl, 4-thiazolidinyl, 5-thiazolidinyl, 3 isothiazolidinyl, 4-isothiazolidinyl, 5 isothiazolidinyl, 1,2,4-oxadiazolidin-3-yl, 1,2,4 oxadiazolidin 5 yl, 1,2,4-thiadiazolidin-3-yl, 1,2,4 thiadiazolidin-5-yl, 1,2,4 triazolidin-3-yl, 1,3,4-oxadiazolidin-2-yl, 1,3,4 thiadiazolidin-2-yl, 1,3,4 triazolidin-2-yl, 2-tetrahydropyranyl, 4 tetrahydropyranyl, 1,3-dioxan-5-yl, 1,4-dioxan-2-yl, 2-piperidinyl, 3-piperidinyl, 4-piperidinyl, 3-hexahydropyridazinyl, 4 hexahydropyridazinyl, 2-hexahydropyrimidinyl, 4-hexahydropyrimidinyl, 5 hexahydropyrimidinyl, 2-piperazinyl, 1,3,5-hexahydrotriazin-2-yl and 1,2,4 hexahydrotriazin-3-yl, 2-morpholinyl, 3-morpholinyl, 2-thiomorpholinyl, 3-thiomorpholinyl, 1-oxothiomorpholin-2-yl, 1-oxothiomorpholin-3-yl, 1,1-dioxothiomorpholin-2-yl, 1,1-dioxothiomorpholin-3-yl, hexahydroazepin-1-, -2-, -3- or -4-yl, hexahydrooxepinyl, hexahydro-1,3-diazepinyl, hexahydro-1,4-diazepinyl, hexahydro-1,3-oxazepinyl, hexahydro-1,4-oxazepinyl, hexahydro-1,3-dioxepinyl, hexahydro-1,4-dioxepinyl and the like. Examples of 3-, 4-, 5-, 6- or 7-membered partially unsaturated heterocyclic rings include: 2,3-dihydrofur-2-yl, 2,3-dihydrofur-3-yl, 2,4-dihydrofur-2-yl, 2,4-dihydrofur-3-yl, 2,3-dihydrothien-2-yl, 2,3 dihydrothien-3-yl, 2,4 dihydrothien-2-yl, 2,4-dihydrothien-3-yl, 2-pyrrolin-2-yl, 2-pyrrolin-3-yl, 3 pyrrolin-2-yl, 3-pyrrolin-3-yl, 2-isoxazolin-3-yl, 3-isoxazolin-3-yl, 4 isoxazolin 3 yl, 2-isoxazolin-4-yl, 3-isoxazolin-4-yl, 4-isoxazolin-4-yl, 2 isoxazolin-5-yl, 3-isoxazolin-5-yl, 4-isoxazolin-5-yl, 2-isothiazolin-3-yl, 3 isothiazolin-3-yl, 4-isothiazolin-3-yl, 2-isothiazolin-4-yl, 3-isothiazolin-4-yl, 4 isothiazolin-4-yl, 2-isothiazolin-5-yl, 3-isothiazolin-5-yl, 4-isothiazolin-5-yl, 2,3 dihydropyrazol-1-yl, 2,3-dihydropyrazol-2-yl, 2,3-dihydropyrazol-3-yl, 2,3 dihydropyrazol-4-yl, 2,3-dihydropyrazol-5-yl, 3,4-dihydropyrazol-1-yl, 3,4 dihydropyrazol-3-yl, 3,4-dihydropyrazol-4-yl, 3,4-dihydropyrazol-5-yl, 4,5 dihydropyrazol-1-yl, 4,5-dihydropyrazol-3-yl, 4,5-dihydropyrazol-4-yl, 4,5 dihydropyrazol-5-yl, 2,3-dihydrooxazol-2-yl, 2,3-dihydrooxazol-3-yl, 2,3 dihydrooxazol-4-yl, 2,3-dihydrooxazol-5-yl, 3,4-dihydrooxazol-2-yl, 3,4 dihydrooxazol-3-yl, 3,4-dihydrooxazol-4-yl, 3,4-dihydrooxazol-5-yl, 3,4 dihydrooxazol-2-yl, 3,4-dihydrooxazol-3-yl, 3,4-dihydrooxazol-4-yl, 2-, 3-, 4-, 5- or 6-di- or tetrahydropyridinyl, 3-di- or tetrahydropyridazinyl, 4 di- or tetrahydropyridazinyl, 2-di- or tetrahydropyrimidinyl, 4-di- or tetrahydropyrimidinyl, 5 di- or tetrahydropyrimidinyl, di- or tetrahydropyrazinyl, 1,3,5-di- or tetrahydrotriazin-2-yl, 1,2,4-di- or tetrahydrotriazin-3-yl, 2,3,4,5-tetrahydro[1H]azepin-1-, -2-, -3-, -4-, -5-, -6- or -7-yl, 3,4,5,6-tetrahydro[2H]azepin-2-, -3-, -4-, -5-, -6- or -7-yl, 2,3,4,7 tetrahydro[1H]azepin-1-, -2-, -3-, -4-, -5-, -6- or -7-yl, 2,3,6,7 tetrahydro[1H]azepin-1-, -2-, -3-, -4-, -5-, -6- or -7-yl, tetrahydrooxepinyl, such as 2,3,4,5-tetrahydro[1H]oxepin-2-, -3-, -4-, -5-, -6- or -7-yl, 2,3,4,7 tetrahydro[1H]oxepin-2-, -3-, -4-, -5-, -6- or -7-yl, 2,3,6,7 tetrahydro[1H]oxepin-2-, -3-, -4-, -5-, -6- or -7-yl, tetrahydro-1,3-diazepinyl, tetrahydro-1,4-diazepinyl, tetrahydro-1,3-oxazepinyl, tetrahydro-1,4-oxazepinyl, tetrahydro-1,3-dioxepinyl and tetrahydro-1,4-dioxepinyl.

Examples of 5- or 6-membered aromatic heterocyclic rings, also termed heteroaromatic rings or hetaryl, include: 2-furyl, 3-furyl, 2-thienyl, 3-thienyl, 2-pyrrolyl, 3-pyrrolyl, 3-pyrazolyl, 4-pyrazo¬lyl, 5-pyrazolyl, 2-oxazolyl, 4-oxazolyl, 5-oxazolyl, 2-thiazolyl, 4 thiazolyl, 5-thiazo¬lyl, 2-imidazolyl, 4-imidazolyl, 1,3,4-triazol-2-yl, 2-pyridinyl, 3-pyridinyl, 4-pyridinyl, 3-pyridazinyl, 4-pyridazinyl, 2-pyrimidinyl, 4-pyrimidinyl, 5-pyrimidinyl and 2-pyrazinyl.

A "C₂-Cₘ-alkylene" is divalent branched or preferably non-branched or linear saturated aliphatic chain having 2 to m, e.g. 2 to 7 carbon atoms, for example CH₂CH₂, -CH(CH₃)-, CH₂CH₂CH₂, CH(CH₃)CH₂, CH₂CH(CH₃), CH₂CH₂CH₂CH₂, CH₂CH₂CH₂CH₂CH₂, CH₂CH₂CH₂CH₂CH₂CH₂, and CH₂CH₂CH₂CH₂CH₂CH₂CH₂.

Embodiments of the present invention as well preferred compounds of the present invention are outlined in the following paragraphs. The remarks made below concerning preferred embodiments of the variables of the compounds of formula (I), especially with respect to their substituents X, W¹, W², W³, W⁴, Het, R¹, R², R³, R^{4a}, R^{4b} and R⁵ and their variables k and m are valid both on their own and, in particular, in every possible combination with each other.

When # appears in a formula showing a preferred substructure of a compound of the present invention, it denotes the attachment bond in the remainder molecule.

According to a special embodiment of the invention, preference is given to those N-acylimino compounds of the formula (I), wherein the variables, either independently of one another or in combination with one another have the following meanings:
- m: is an integer selected from 0, 1, 2, 3, 4, 5 or 6, in particular 0 or 1;
- X: is O or S, in particular O;
- Het: is a 5- or 6- membered carbon-bound or nitrogen-bound heterocyclic or heteroaromatic ring, comprising 1, 2, 3, 4 or 5 carbon atoms and 1, 2 or 3 heteroatoms as ring members, which are independently selected from sulfur, oxygen or nitrogen, wherein the carbon, sulfur and nitrogen ring members can independently be partly or completely oxidized, and wherein each ring is optionally substituted by k identical or different substituents R⁶, wherein k is an integer selected from 0, 1, 2, 3 or 4;
- W¹-W²-W³-W⁴: represents a carbon chain group connected to N and C=N, and thus forming a saturated, unsaturated, or partially unsaturated 5- or 6-membered nitrogen containing heterocycle, wherein W¹, W², W³ and W⁴ each individually represent CR^{v}R^{w}, wherein each R^{w} independently from each other, is selected from the group consisting
hydrogen, halogen, cyano, azido, nitro, SCN, SF₅, C₁-C₁₀-alkyl, C₃-C₈-cycloalkyl, C₂-C₁₀-alkenyl, C₂-C₁₀-alkynyl,
wherein each of the four last mentioned radicals may be unsubstituted, partly or completely halogenated and/or may optionally be substituted with 1, 2 or 3 identical or different radicals R⁷,
OR⁸, NR^{9a}R^{9b}, S(O)ₙR⁸, S(O)ₙNR^{9a}R^{9b}, C(=O)R^{7a}, C(=O)NR^{9a}R^{9b}, C(=O)OR⁸, C(=S)R^{7a}, C(=S)NR^{9a}R^{9b}, C(=S)OR⁸, C(=S)SR⁸, C(=NR¹⁷)R^{7a}, C(=NR¹⁷)NR^{9a}R^{9b} and Si(R¹¹)₂R¹²,
each R^{v} independently from each other, are selected from the group consisting of hydrogen, halogen, cyano, C₁-C₁₀-alkyl, C₃-C₈-cycloalkyl, C₂-C₁₀-alkenyl and C₂-C₁₀-alkynyl,
wherein each of the four last mentioned radicals may be unsubstituted, partly or completely halogenated or may optionally be further substituted with 1, 2 or 3 identical or different radicals R⁷; or
R^{v} and R^{w} present in one of the groups may together form =O, =CR¹³R¹⁴, =S, =NR¹⁷, =NOR¹⁶ or =NNR^{9a}R^{9b}, or
two R^{w} of adjacent carbon atoms, may form both together and together with the existing bond a double bond between the adjacent carbon atoms;
and wherein one of W² or W³ may optionally represent a single or a double bond between the adjacent carbon atoms;
- R¹ and R²: are independently from each other selected from the group consisting of hydrogen, halogen, CN, SCN, nitro, C₁-C₆-alkyl, C₃-C₆-cycloalkyl, wherein each of the two aforementioned radicals are unsubstituted, partly or completely halogenated or may carry 1, 2 or 3 identical or different radicals R⁷,
Si(R¹¹)₂R¹², OR⁸, OSO₂R⁸, S(O)ₙR⁸, S(O)ₙNR^{9a}R^{9b}, NR^{9a}R^{9b}, C(=O)NR^{9a}R^{9b}, C(=S)NR^{9a}R^{9b}, C(=O)OR⁸, C(=O)R^{7a}, C(=S)R^{7a},
phenyl, benzyl,
wherein the phenyl ring in the last two radicals is unsubstituted or optionally substituted with 1, 2, 3, 4 or 5 identical or different substituents R¹⁰,
a 3-, 4-, 5-, 6- or 7- membered saturated, partly saturated or unsaturated aromatic heterocyclic ring comprising 1, 2 or 3 identical or different heteroatoms as ring members, which are selected from oxygen, nitrogen and sulfur,
wherein the heterocyclic ring is optionally substituted with 1, 2, 3 or 4 identical or different substituents R¹⁰, and wherein the nitrogen and/or the sulfur atom(s) of the heterocyclic ring may optionally be oxidized,
or
- R¹ and R²: form, together with the carbon atom, which they attached to, a 3-, 4-, 5- or 6-membered saturated or partly unsaturated carbocyclic or heterocyclic ring, wherein each of the carbon atoms of said cycle are unsubstituted or may carry 1 or 2 identical or different radicals R⁷,
or
- R¹ and R²: may together be =O, =CR¹³R¹⁴, =S, =NR¹⁷, =NOR¹⁶ or =NNR^{9a}R^{9b};
- R³: is selected from the group consisting of hydrogen, halogen, NO₂, C₁-C₆-alkyl, C₂-C₆-alkenyl, C₂-C₆-alkynyl, C₃-C₈-cycloalkyl and C₃-C₈-cycloalkenyl,
wherein each of the five last mentioned radicals is unsubstituted, partly or completely halogenated or carries 1 or 2 identical or different radicals R⁷, it being also possible for cycloalkyl and cycloalkenyl to carry 1, 2, 3, 4, 5 or 6 identical or different C₁-C₄-alkyl groups,
S(O)ₙNR^{9a}R^{9b}, NR^{9a}R^{9b}, C(=O)OR⁸, C(=O)NR^{9a}R^{9b}, C(=S)NR^{9a}R^{9b}, C(=O)R^{7a}, C(=S)R^{7a}, NR^{9a}-C(=O)R^{7a}, NR^{9a}-C(=S)R^{7a}, NR^{9a}-S(O)ₙR^{8a},
phenyl, benzyl,
wherein the phenyl ring in the last two mentioned radicals is unsubstituted or may be substituted with one or more, e.g. 1, 2, 3, 4 or 5 identical or different substituents R¹⁰,
and a 3-, 4-, 5-, 6- or 7- membered saturated, partly saturated or unsaturated aromatic heterocyclic ring comprising 1, 2 or 3 heteroatoms as ring members, which are identical or different and selected from oxygen, nitrogen and sulfur,
wherein the heterocyclic ring is optionally substituted with 1, 2, 3 or 4 identical or different substituents R¹⁰, and wherein the nitrogen and/or the sulfur atom(s) of the heterocyclic ring may optionally be oxidized;
- R^{4a} and R^{4b}: are selected each independently from one another and independently from the integer of m from the group consisting of hydrogen, halogen, CN, C₁-C₆-alkyl, C₁-C₆-alkoxy, di-(C₁-C₆-alkyl)amino, C₁-C₆-alkylthio, and C₁-C₈-cycloalkyl,
wherein each of the 4 last mentioned radicals is unsubstituted, partly or completely halogenated,
it being also possible for R^{4b} to be selected from
C₁-C₆-alkylsulfinyl, C₁-C₆-alkylsulfonyl,
wherein each of the two last mentioned radicals is unsubstituted, partly or completely halogenated,
C₁-C₆-alkyl or C₃-C₈-cycloalkyl,
wherein each of the two last mentioned radicals carries 1, 2 or 3 identical or different radicals R⁷,
SH, S(O)ₙNR^{9a}R^{9b}, NR^{9a}R^{9b}, C(=O)OR⁸, C(=O)NR^{9a}R^{9b}, C(=S)NR^{9a}R^{9b}, C(=O)R^{7a}, C(=S)R^{7a},
a moiety Q¹-phenyl, wherein the phenyl ring is optionally substituted with 1, 2, 3, 4 or 5 identical or different substituents R¹⁰,
and a moiety Q¹-Het^{#}, wherein Q¹ is a single bond, NR^{9a}, C₁-C₄-alkylene or NR^{9a}-C₁-C₄-alkylene, wherein the heteroatom in the last moiety is bound to the carbon atom of CR⁴R⁵;
or
- R^{4a} and R^{4b}: may form together with the carbon atom they are bound to, a 3-, 4-, 5- or 6-membered aliphatic ring,
wherein each of the carbon atoms of the ring may be unsubstituted, partly or completely halogenated, and/or may carry 1 or 2 identical or different radicals selected from C₁-C₄-alkyl, C₁-C₄-haloalkyl, C₁-C₄-alkoxy and C₁-C₄-haloalkoxy;
or
- R^{4a} and R^{4b}: may together form =O, =CR¹³R¹⁴, =S, =NR¹⁷, =NOR¹⁶ or =NNR^{9a}R^{9b};
- R⁵: is selected from the group consisting of hydrogen, halogen, CN, C₁-C₆-alkyl, C₁-C₆-alkoxy, C₁-C₆-alkylthio, C₁-C₆-alkylsulfinyl, C₁-C₆-alkylsulfonyl, C₂-C₆-alkenyl, C₂-C₆-alkenylthio, C₂-C₆-alkenylsulfinyl, C₂-C₆-alkenylsulfonyl, C₂-C₆-alkynyl, C₂-C₆-alkynylthio, C₂-C₆-alkynylsulfinyl, C₂-C₆-alkynylsulfonyl, C₃-C₈-cycloalkyl, C₃-C₈-cycloalkoxy, C₃-C₈-cycloalkylthio, C₃-C₈-cycloalkylsulfinyl and C₃-C₈-cycloalkylsulfonyl,
wherein each of the 18 last mentioned radicals is unsubstituted, partly or completely halogenated an/or carries 1, 2 or 3 identical or different radicals R⁷, it being also possible for cycloalkyl radicals to carry 1, 2, 3, 4, 5 or 6 identical or different C₁-C₄-alkyl groups,
S(O)ₙNR^{9a}R^{9b}, O-NR^{9a}R^{9b}, NR^{9a}R^{9b}, NR^{9a}NR^{9a}R^{9b}, C(=O)OR⁸, C(=O)NR^{9a}R^{9b}, C(=S)NR^{9a}R^{9b}, C(=O)R^{7a}, C(=S)R^{7a}, NR^{9a}-C(=O)R^{7a}, NR^{9a}-C(=S)R^{7a}, NR^{9a}C(=O)NR^{9a}R^{9b}, NR^{9a}C(=S)NR^{9a}R^{9b}, NR^{9a}-S(O)ₙR^{8a},
a moiety Q-phenyl, wherein the phenyl ring is optionally substituted with 1, 2, 3, 4 or 5 identical or different substituents R¹⁰,
a moiety Y-R^{5a},
and a moiety Q-Het^{#},
wherein
Y is O, S, S(=O) or S(=O)₂;
R^{5a} is selected from the group consisting of C(=O)NR^{9a}R^{9b}, C(=S)NR^{9a}R^{9b}, C(=O)R⁷, C(=S)R⁷;
phenyl or CH₂-phenyl, optionally substituted with one or more, e.g. 1, 2, 3, 4 or 5 identical or different substituents R¹⁰;
a 3-, 4-, 5-, 6- or 7- membered saturated, partly saturated or unsaturated aromatic heterocyclic ring comprising 1, 2 or 3 heteroatoms selected from oxygen, nitrogen and /or sulfur, optionally substituted with 1, 2, 3 or 4 identical or different substituents R^{y},
wherein the nitrogen and/or the sulfur atom(s) of the heterocyclic ring may optionally be oxidized, and
R^{y} is selected from the group conisisting of
hydrogen, halogen, cyano, nitro, C₁-C₁₀-alkyl, C₂-C₁₀-alkenyl,
wherein the two last mentioned radicals may optionally be substituted with one or more identical or different radical R¹⁸,
phenyl,
optionally substituted with halogen, cyano, nitro, C₁-C₆-alkyl, C₁-C₆-haloalkyl, C₁-C₆-alkoxy or C₁-C₆-haloalkoxy, C₁-C₄ alkyl and C₁-C₄ alkoxy, wherein both of the last two mentioned aliphatic radicals may be unsubstituted, partly or completely halogenated, OR¹⁹, -S(O)ₙR¹⁹, S(O)ₙNR^{20a}R^{20b}, NR^{20a}R^{20b}, C(=O)R¹⁸, C(=O)OR¹⁹, -C(=NR^{20a})R¹⁸, C(=O)NR^{20a}R^{20b}, C(=S)NR^{20a}R^{20b},
or
two R^{y} present together on one atom of a partly saturated heterocyclic may be =O, =CR^{21a}R^{21b}, =NR^{20a}, =NOR¹⁹ or =NNR^{20a};
or
two R^{y} on adjacent carbon atoms may be a bridge selected from CH₂CH₂CH₂CH₂, CH=CH-CH=CH, N=CH-CH=CH, CH=N-CH=CH, N=CH-N=CH, CH₂CH₂CH₂, CH=CHCH₂, CH₂CH₂NR^{17a}, CH₂CH=N, CH=CH-NR^{17a}, and form together with the carbon atoms to which the two R^{y} are bonded to a 5-membered or 6-membered fused partly saturated or unsaturated, aromatic carbocyclic or heteocyclic ring, wherein the ring may optionally be substituted with one or two substituents selected from =O, OH, CH₃, OCH₃, halogen, cyano, C₁-C₄-alkoxy, C₁-C₄-haloalkoxy, C₁-C₄-alkyl and C₁-C₄-haloalkyl;
- Q: irrespectively of its occurrence, is a single bond, CH₂, S, O, S(=O), S(=O)₂, NR^{9a}, -O-S(=O)₂-, -NR^{9a}-S(=O)₂-, -O-C(=O)-, -NR^{9a}-C(=O)-, -O-C₁-C₄-alkylene, -S-C₁-C₄-alkylene, NR^{9a}-C₁-C₄-alkylene, -O-C(=O)-C₁-C₄-alkylene or -NR^{9a}-C(=O)-C₁-C₄-alkylene, wherein the heteroatom in the last 6 moieties is bound to the carbon atom of C(CN)R³ or CR^{4a}R^{4b}, respectively;
or, if m is 1,
R³ and R^{4a} may also form both together and together with the existing bond a double bond between the adjacent carbon atoms; or, if m is 1,
one of R^{4a} or R^{4b} together with R⁵ may also form with the carbon atom they are bound to, a 3-, 4-, 5- or 6-membered saturated partially unsaturated heterocycle,
wherein each of the carbon atoms of the heterocycle may be unsubstituted or may carry 1 or 2 identical or different radicals R^{7b}, and wherein the heterocylce has 1 or 2 non-adjacent identical or different heteroatoms or heteroatom moieties as ring members, which are selected from S, N, NH and N-R^{17c};

or, if m = 0,
R³ and R⁵ together may also form with the carbon atom they are bound to, a 3-, 4-, 5- or 6-membered saturated partially unsaturated carbocycle or heterocycle,
wherein each of the carbon atoms of the carbocycle or heterocycle may be unsubstituted or may carry 1 or 2 radicals R^{7b}, and where the heterocylce has 1 or 2 non-adjacent identical or different heteroatoms or heteroatom moieties as ring members, which are selected from O, S, N and N-R^{17c},
or
- R³ and R⁵: may together form =O or =S;
where, independently of their occurrence,
n is 0, 1 or 2;
R⁶ is selected from the group consisting of
halogen, cyano, azido, nitro, SCN, SF₅,
C₁-C₁₀-alkyl, C₃-C₈-cycloalkyl, C₂-C₁₀-alkenyl, C₂-C₁₀-alkynyl, wherein each of of the four last mentioned radicals may optionally be substituted independently from one another with 1, 2 or 3 identical or different radicals R⁷,
OR⁸, NR^{17a}R^{17b}, S(O)ₙR⁸, S(O)ₙNR^{17a}R^{17b}, C(=O)R^{7a}, C(=O)NR^{17a}R^{17b}, C(=O)OR⁸, C(=S)R^{7a}, C(=S)NR^{17a}R^{17b}, C(=S)OR⁸, C(=S)SR⁸, C(=NR¹⁷)R^{7a}, C(=NR¹⁷)NR^{17a}R^{17b}, Si(R¹¹)₂R¹²,
phenyl, optionally substituted with 1, 2, 3, 4 or 5 identical or different substituents R¹⁰,
a 3-, 4-, 5-, 6- or 7- membered saturated, partly saturated or unsaturated aromatic heterocyclic ring comprising 1, 2 or 3 heteroatoms as ring members, which are identical or different and selected from oxygen, nitrogen and sulfur,
wherein the heterocyclic ring is optionally substituted with 1, 2, 3 or 4 identical or different substituents R¹⁰, and wherein the nitrogen and/or the sulfur atom(s) of the heterocyclic ring may optionally be oxidized,
or two of R⁶ present on one ring carbon may together form =O, =CR¹³R¹⁴, =S, =NR¹⁷, =NOR¹⁶, =NNR^{9a}R^{9b};
or two R⁶ together form a linear C₂-C₇ alkylene chain, thus forming, together with the ring atom(s) to which they are bound, a 3-, 4-, 5-, 6-, 7- or 8-membered ring,
wherein 1 or 2 CH₂ moieties of the alkylene chain may be replaced by 1 or 2 heteroatom moieties selected from O, S and NR^{17c} and/or
1 or 2 of the CH₂ groups of the alkylene chain may be replaced by a group C=O, C=S and/or C=NR¹⁷; and
wherein the alkylene chain is unsubstituted or may be substituted with 1, 2, 3, 4, 5 or 6 identical or different radicals selected from the group consisting of halogen, C₁-C₆-haloalkyl, C₁-C₆-alkoxy, C₁-C₆-haloalkoxy, C₁-C₆-alkylthio, C₁-C₆-haloalkylthio, C₃-C₈-cycloalkyl, C₃-C₈-halocycloalkyl, C₂-C₆-alkenyl, C₂-C₆-haloalkenyl, C₂-C₆-alkynyl, C₂-C₆-haloalkynyl,
phenyl which may be substituted with 1, 2, 3, 4 or 5 identical or different radicals R¹⁰, and
a 3-, 4-, 5-, 6- or 7-membered saturated, partially unsaturated or aromatic heterocyclic ring containing 1, 2 or 3 heteroatoms or heteroatom groups selected from N, O, S, NO, SO and SO₂, as ring members, wherein the heterocyclic ring may be substituted with 1, 2, 3, 4 or 5 identical or different radicals R¹⁰;
- R⁷: independently of its occurrence, is selected from the group consisting of cyano, azido, nitro, -OH, -SH, -SCN, SF₅, C₁-C₆-alkyl, C₁-C₆-haloalkyl, C₃-C₈-cycloalkyl, C₃-C₈-halocycloalkyl, C₂-C₆-alkenyl, C₂-C₆-haloalkenyl, C₂-C₆-alkynyl, C₂-C₆-haloalkynyl, C₁-C₆-alkoxy, C₁-C₆-haloalkoxy, C₁-C₆-alkylthio, C₁-C₆-alkylsulfinyl, C₁-C₆-alkylsulfonyl, C₁-C₆-haloalkylthio, Si(R¹¹)₂R¹², OR⁸, OSO₂R⁸, S(O)ₙR⁸, S(O)ₙNR^{17a}R^{17b}, NR^{17a}R^{17b}, C=O)NR^{17a}R^{17b}, C(=S)NR^{17a}R^{17b}, C(=O)OR⁸, C(=O)R¹⁵, C(=S)R¹⁵, C(=NR¹⁷)R¹⁵,
phenyl, phenyl-C₁-C₄-alkyl, phenoxy,
wherein the phenyl ring in the last three groups is unsubstituted or may be substituted with 1, 2, 3, 4 or 5 identical or different radicals selected from halogen, C₁-C₆-alkyl, cyano, azido, nitro, C₁-C₁₀-alkyl, C₁-C₆-haloalkyl, C₁-C₆-alkoxy, C₁-C₆ haloalkoxy, (C₁-C₆-alkoxy)carbonyl, (C₁-C₆-alkyl)amino and di-(C₁-C₆-alkyl)amino, and
a 3-, 4-, 5-, 6- or 7- membered saturated, partly saturated or unsaturated aromatic heterocyclic ring comprising 1, 2 or 3 heteroatoms as ring members, which are identical or different and selected from oxygen, nitrogen and sulfur,
wherein the heterocyclic ring is unsubstituted or may be substituted with 1, 2, 3 or 4 identical or different radicals selected from halogen, C₁-C₆-alkyl, cyano, azido, nitro, C₁-C₁₀-alkyl, C₁-C₆-haloalkyl, C₁-C₆-alkoxy, C₁-C₆ haloalkoxy, (C₁-C₆-alkoxy)carbonyl, (C₁-C₆-alkyl)amino and di-(C₁-C₆-alkyl)amino, and wherein the nitrogen and/or the sulfur atom(s) of the heterocyclic ring may optionally be oxidized,
or
two R⁷ present on one carbon atom may together form =O, =CR¹³R¹⁴, =S, =NR¹⁷, =NOR¹⁶, =NNR^{9a}R^{9b},
or
two R⁷ may form a 3-, 4-, 5-, 6-, 7- or 8-membered saturated or partly unsaturated carbocyclic or heterocyclic ring together with the carbon atoms to which the two R⁷ are bonded, wherein the heterocyclic ring comprises 1, 2 or 3 heteroatoms as ring members, which are identical or different and selected from oxygen, nitrogen and sulfur,
wherein the heterocyclic ring is unsubstituted or may be substituted with 1, 2, 3 or 4 identical or different radicals selected from halogen, C₁-C₆-alkyl, cyano, azido, nitro, C₁-C₁₀-alkyl, C₁-C₆-haloalkyl, C₁-C₆-alkoxy, C₁-C₆ haloalkoxy, (C₁-C₆-alkoxy)carbonyl, (C₁-C₆-alkyl)amino and di-(C₁-C₆-alkyl)amino;
- R^{7a}: independently of its occurrence, is selected from the group consisting of hydrogen, C₁-C₆-alkyl, C₁-C₆-haloalkyl, C₁-C₆-alkoxy, C₁-C₆-haloalkoxy, C₁-C₆-alkylthio, C₁-C₆-alkylsulfinyl, C₁-C₆-alkylsulfonyl, C₁-C₆-haloalkylthio, C₃-C₈-cycloalkyl, C₃-C₈-halocycloalkyl, C₂-C₆-alkenyl, C₂-C₆-haloalkenyl, C₂-C₆-alkynyl, C₂-C₆-haloalkynyl,
phenyl, optionally substituted with 1, 2, 3, 4 or 5 identical or different substituents R¹⁰, and
a 3-, 4-, 5-, 6- or 7- membered saturated, partly saturated or unsaturated aromatic heterocyclic ring comprising 1, 2 or 3 heteroatoms as ring members, which are identical or different and selected from oxygen, nitrogen and sulfur,
wherein the heterocyclic ring is optionally substituted with 1, 2, 3 or 4 identical or different substituents R¹⁰, and
wherein the nitrogen and/or the sulfur atom(s) of the heterocyclic ring may optionally be oxidized;
- R^{7b}: is selected from the group consisting of halogen, cyano, C₁-C₁₀-alkyl, C₃-C₈-cycloalkyl, C₂-C₁₀-alkenyl, C₂-C₁₀-alkynyl,
wherein each of the four last mentioned radicals is unsubstituted, partly or completely halogenated, and/or may be substituted with 1, 2 or 3 identical or different radicals R⁷,
OR⁸, NR^{17a}R^{17b}, S(O)ₙR⁸, S(O)ₙNR^{17a}R^{17b}, C(=O)R^{7a}, C(=O)NR^{17a}R^{17b}, C(=O)OR⁸, C(=S)R^{7a}, C(=S)NR^{17a}R^{17b}, C(=S)OR⁸, C(=NR¹⁷)R^{7a}, C(=NR¹⁷)NR^{17a}R^{17b}, Si(R¹¹)₂R¹²; phenyl, optionally substituted with 1, 2, 3, 4 or 5 identical or different substituents R¹⁰,
a 3-, 4-, 5-, 6- or 7- membered saturated, partly saturated or unsaturated aromatic heterocyclic ring comprising 1, 2 or 3 heteroatoms as ring members, which are identical or different and selected from oxygen, nitrogen and sulfur,
wherein the heterocyclic ring is optionally substituted with 1, 2, 3 or 4 identical or different substituents R¹⁰, and wherein the nitrogen and/or the sulfur atom(s) of the heterocyclic ring may optionally be oxidized,
or
two of R^{7b} present on one ring carbon may together form =O, =S or =CR¹³R¹⁴,
or
two R^{7b} together form a linear C₂-C₇ alkylene chain, thus forming, together with the ring atom(s) to which they are bound, a 3-, 4-, 5-, 6-, 7- or 8-membered ring,
wherein 1 or 2 CH₂ moieties of the alkylene chain may be replaced by 1 or 2 heteroatom moieties selected from O, S and NR^{17c} and/or
1 or 2 of the CH₂ groups of the alkylene chain may be replaced by a group C=O, C=S and/or C=NR¹⁷; and
wherein the alkylene chain is unsubstituted or may be substituted with 1, 2, 3, 4, 5 or 6 identical or different radicals selected from the group consisting of halogen, C₁-C₆-haloalkyl, C₁-C₆-alkoxy, C₁-C₆-haloalkoxy, C₁-C₆-alkylthio, C₁-C₆-haloalkylthio, C₃-C₈-cycloalkyl, C₃-C₈-halocycloalkyl, C₂-C₆-alkenyl, C₂-C₆-haloalkenyl, C₂-C₆-alkynyl, C₂-C₆-haloalkynyl, phenyl which may be substituted with 1, 2, 3, 4 or 5 identical or different radicals R¹⁰, and
a 3-, 4-, 5-, 6- or 7-membered saturated, partially unsaturated or aromatic heterocyclic ring containing 1, 2 or 3 heteroatoms or heteroatom groups selected from N, O, S, NO, SO and SO₂, as ring members, wherein the heterocyclic ring may be substituted with 1, 2, 3, 4 or 5 identical or different radicals R¹⁰;
- R⁸: independently of its occurrence, is selected from the group consisting of hydrogen, C₁-C₆-alkyl, C₁-C₆-haloalkyl, C₃-C₈-cycloalkyl, C₃-C₈-cycloalkyl-C₁-C₄-alkyl, C₃-C₈-halocycloalkyl, C₂-C₆-alkenyl, C₂-C₆-haloalkenyl, C₂-C₆-alkynyl, C₂-C₆ haloalkynyl, C(=O)R¹⁵, C(=O)NR^{17a}R^{17b}, C(=S)NR^{17a}R^{17b}, C(=O)OR¹⁶,
phenyl, phenyl-C₁-C-₄-alkyl, wherein the phenyl ring in the last two mentioned radicals is unsubstituted or substituted with 1, 2, 3, 4 or 5 identical or different substituents R¹⁰, and
a 3-, 4-, 5-, 6- or 7- membered saturated, partly saturated or unsaturated aromatic heterocyclic ring comprising 1, 2 or 3 heteroatoms as ring members, which are identical or different and selected from oxygen, nitrogen and sulfur,
wherein the heterocyclic ring is optionally substituted with 1, 2, 3 or 4 identical or different substituents R¹⁰, and
wherein the nitrogen and/or the sulfur atom(s) of the heterocyclic ring may optionally be oxidized,
- R^{8a}: independently of its occurrence, is selected from the group consisting of C₁-C₆-alkyl, C₃-C₈-cycloalkyl, C₃-C₈-cycloalkyl-C₁-C₄-alkyl, C₂-C₆-alkenyl, C₂-C₆-alkynyl,
wherein each of the five last mentioned radicals is unsubstituted, partly or completely halogenated,
phenyl, benzyl,
wherein the phenyl ring in the last two mentioned radicals is unsubstituted or may be substituted with 1, 2, 3, 4 or 5 identical or different substituents R¹⁰,
and a 3-, 4-, 5-, 6- or 7- membered saturated, partly saturated or unsaturated aromatic heterocyclic ring comprising 1, 2 or 3 heteroatoms as ring members, which are identical or different and selected from oxygen, nitrogen and sulfur,
wherein the heterocyclic ring is optionally substituted with 1, 2, 3 or 4 identical or different substituents R¹⁰, and wherein the nitrogen and/or the sulfur atom(s) of the heterocyclic ring may optionally be oxidized,
- R⁹: independently of its occurrence, is selected from the group consisting of hydrogen, C₁-C₆-alkyl, C₁-C₆-haloalkyl, C₁-C₆-alkoxy, C₁-C₆-haloalkoxy, C₃-C₈-cycloalkyl, C₃-C₈-cycloalkyl-C₁-C₄-alkyl, C₃-C₈-halocycloalkyl, C₂-C₆-alkenyl, C₂-C₆-haloalkenyl, C₂-C₆-alkynyl, C₂-C₆ haloalkynyl,
phenyl, optionally substituted with 1, 2, 3, 4 or 5 identical or different substituents R¹⁰;
a 3-, 4-, 5-, 6- or 7- membered saturated, partly saturated or unsaturated aromatic C-bound heterocyclic ring comprising 1, 2 or 3 heteroatoms as ring members, which are identical or different and selected from oxygen, nitrogen and sulfur,
wherein the heterocyclic ring is optionally substituted with 1, 2, 3 or 4 identical or different substituents R¹⁰, and
wherein the nitrogen and/or the sulfur atom(s) of the heterocyclic ring may optionally be oxidized,
- R^{9a} and R^{9b}: are each independently from one another selected from the group consisting of
hydrogen, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₃-C₈-cycloalkyl, C₃-C₈-halocycloalkyl, C₃-C₈-cycloalkyl-C₁-C₄-alkyl, C₂-C₆-alkenyl, C₂-C₆-haloalkenyl, C₂-C₆-alkynyl, C₂-C₆ haloalkynyl,
S(O)ₙR¹⁶, -S(O)ₙNR^{17a}R^{17b}, C(=O)R¹⁵, C(=O)OR¹⁶, C(=O)NR^{17a}R^{17b}, C(=S)R¹⁵ C(=S)SR¹⁶, C(=S)NR^{17a}R^{17b}, C(=NR¹⁷)R¹⁵;
phenyl, benzyl, 1-phenethyl or 2-phenethyl,
wherein the phenyl ring in the last four mentioned radicals is unsubstituted or may be substituted with 1, 2, 3, 4 or 5 identical or different substituents R¹⁰; and a 3-, 4-, 5-, 6- or 7- membered saturated, partly saturated or unsaturated aromatic C-bound heterocyclic ring comprising 1, 2 or 3 heteroatoms as ring members, which are identical or different and selected from oxygen, nitrogen and sulfur,
wherein the heterocyclic ring is optionally substituted with 1, 2, 3 or 4 identical or different substituents R¹⁰, and
wherein the nitrogen and/or the sulfur atom(s) of the heterocyclic ring may optionally be oxidized,
or,
R^{9a} and R^{9b} are together a C₂-C₇ alkylene chain and form a 3-, 4-, 5-, 6-, 7- or 8-membered saturated, partly saturated or unsaturated aromatic ring together with the nitrogen atom they are bonded to,
wherein the alkylene chain may contain one or two heteroatoms, which are, independently of each other, selected from oxygen, sulfur or nitrogen, where the alkylene chain may optionally be substituted with 1, 2, 3 or 4 radicals selected from
halogen, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₃-C₈-cycloalkyl, C₃-C₈-halocycloalkyl, C₂-C₆-alkenyl, C₂-C₆-haloalkenyl, C₂-C₆-alkynyl, C₂-C₆ haloalkynyl, phenyl, optionally substituted with 1, 2, 3, 4 or 5 identical or different substituents R¹⁰, and
a 3-, 4-, 5-, 6- or 7- membered saturated, partly saturated or unsaturated aromatic C-bound heterocyclic ring comprising 1, 2 or 3 heteroatoms as ring members, which are identical or different and selected from oxygen, nitrogen and sulfur,
wherein the heterocyclic ring is optionally substituted with 1, 2, 3 or 4 identical or different substituents R¹⁰, and
wherein the nitrogen and/or the sulfur atom(s) of the heterocyclic ring may optionally be oxidized,
or
- R^{9a} and R^{9b}: together may form =CR¹³R¹⁴, =NR¹⁷, =NOR¹⁶, =NNR^{17a}R^{17b} moiety;
- R¹⁰: independently of its occurrence, is selected from the group consisting of halogen, cyano, azido, nitro, SCN, SF₅, C₁-C₁₀-alkyl, C₃-C₈-cycloalkyl, C₂-C₁₀-alkenyl, C₂-C₁₀-alkynyl,
wherein each of the four last mentioned radicals may optionally be substituted with 1, 2, 3, 4 or 5 identical or different radicals R⁷,
Si(R¹¹)₂R¹², OR¹⁶, OS(O)ₙR¹⁶, -S(O)ₙR¹⁶, S(O)ₙNR^{17a}R^{17b}, NR^{17a}R^{17b}, C(=O)R¹⁵, C(=S)R¹⁵, C(=O)OR¹⁶, -(=NR¹⁷)R¹⁵, C(=O)NR^{17a}R^{17b}, C(=S)NR^{17a}R^{17b},
phenyl,
optionally substituted with 1, 2, 3, 4 or 5 identical or different radicals selected from OH, halogen, cyano, nitro, C₁-C₆-alkyl, C₁-C₆-haloalkyl, C₁-C₆-alkoxy and C₁-C₆-haloalkoxy, and
a 3-, 4-, 5-, 6- or 7- membered saturated, partly saturated or unsaturated aromatic heterocyclic ring comprising 1, 2 or 3 heteroatoms as ring members, which are identical or different and selected from oxygen, nitrogen and sulfur,
wherein the heterocyclic ring is unsubstituted or may be substituted with 1, 2, 3, 4 or 5 identical or different substituents selected from halogen, cyano, NO₂, C₁-C₆-alkyl, C₁-C₆-haloalkyl, C₁-C₆-alkoxy and C₁-C₆-haloalkoxy, and
wherein the nitrogen and/or the sulfur atom(s) of the heterocyclic ring may optionally be oxidized;
or
two R¹⁰ present together on one carbon ring atom of a saturated or partly unsaturated heterocyclic radical may form =O, =CR¹³R¹⁴, =S, =NR¹⁷, =NOR¹⁶, =NNR^{17a}R^{17b};
or,
two R¹⁰ on adjacent carbon ring atoms may together be a bivalent radical selected from CH₂CH₂CH₂CH₂, CH=CH-CH=CH, N=CH-CH=CH, CH=N-CH=CH, N=CH-N=CH, OCH₂CH₂CH₂, OCH=CHCH₂, CH₂OCH₂CH₂, OCH₂CH₂O, OCH₂OCH₂, CH₂CH₂CH₂, CH=CHCH₂, CH₂CH₂O, CH=CHO, CH₂OCH₂, CH₂C(=O)O, C(=O)OCH₂, O(CH₂)O, SCH₂CH₂CH₂, SCH=CHCH₂, CH₂SCH₂CH₂, SCH₂CH₂S,
SCH₂SCH₂, CH₂CH₂S, CH=CHS, CH₂SCH₂, CH₂C(=S)S, C(=S)SCH₂, S(CH₂)S, CH₂CH₂NR¹⁷, CH₂CH=N, CH=CH-NR¹⁷, OCH=N, SCH=N and form together with said adjacent carbon ring atoms a 5-membered or 6-membered partly saturated or unsaturated, aromatic carbocyclic or heterocyclic ring, wherein the ring may optionally be substituted with one or two substituents independently selected from =O, OH, CH₃, OCH₃, halogen, cyano, halomethyl and halomethoxy;
- R¹¹ and R¹²: independently of their occurrence, are selected from the group consisting of C₁-C₆-alkyl, C₁-C₆-haloalkyl, C₁-C₆-alkoxy, C₁-C₆-alkoxy-C₁-C₄-alkyl, C₂-C₆-alkenyl, C₂-C₆-haloalkenyl, C₂-C₆-alkynyl, C₂-C₆-haloalkynyl, C₃-C₈-cycloalkyl, C₃-C₈-halocycloalkyl, C₃-C₈-cycloalkyl-C₁-C₄-alkyl, C₃-C₈-halocycloalkyl-C₁-C₄-alkyl, C₁-C₆-haloalkoxy-C₁-C₄-alkyl,
phenyl and benzyl,
wherein the phenyl ring in last two radicals is unsubstituted or substituted with 1, 2, 3, 4 or 5 identical or different radicals selected from halogen, OH, cyano, NO₂, C₁-C₆-alkyl, C₁-C₆-haloalkyl, C₁-C₆-alkoxy and C₁-C₆-haloalkoxy;
- R¹³ and R¹⁴: independently of their occurrence, are selected from the group consisting of hydrogen, halogen, CN, C₁-C₆-alkyl, C₃-C₆-cycloalkyl, C₁-C₄-alkoxy-C₁-C₄-alkyl, phenyl and benzyl;
- R¹⁵: independently of its occurrence, is selected from the group consisting of hydrogen, C₁-C₆-alkyl, C₂-C₆-alkenyl, C₂-C₆-alkynyl, C₃-C₈-cycloalkyl,
wherein the four last mentioned aliphatic and cycloaliphatic radicals may be unsubstituted, partially or completely halogenated and/or oxygenated and/or may carry 1 or 2 identical or different radicals selected from C₁-C₄ alkoxy;
phenyl, benzyl and pyridyl, wherein the last three radicals may be unsubstituted, partially or completely halogenated and/or to carry 1, 2 or 3 substituents selected from C₁-C₆-alkyl, C₁-C₆-haloalkyl, C₁-C₆-alkoxy, C₁-C₆ haloalkoxy, (C₁-C₆-alkoxy)carbonyl, (C₁-C₆-alkyl)amino or di-(C₁-C₆-alkyl)amino;
- R¹⁶: independently of its occurrence, is selected from the group consisting of hydrogen, C₁-C₆-alkyl, C₂-C₆-alkenyl, C₂-C₆-alkynyl, C₃-C₈-cycloalkyl, C₃-C₈-cycloalkyl-C₁-C₄-alkyl, wherein the five last mentioned aliphatic and cycloaliphatic radicals may be unsubstituted, partially or completely halogenated and/or oxygenated and/or may carry 1 or 2 radicals selected from C₁-C₄ alkoxy,
phenyl, benzyl and pyridyl,
wherein the last three radicals may be unsubstituted, partially or completely halogenated and/or may carry 1, 2 or 3 identical or different substituents
selected from C₁-C₆-alkyl, C₁-C₆-haloalkyl, C₁-C₆-alkoxy, C₁-C₆ haloalkoxy, (C₁-C₆-alkoxy)carbonyl, (C₁-C₆-alkyl)amino and di-(C₁-C₆-alkyl)amino;
- R¹⁷: independently of its occurrence, is selected from the group consisting of hydrogen, C₁-C₆-alkoxy, C₁-C₆-haloalkoxy, C₁-C₆-alkylthio, trimethylsilyl, triethylsilyl, *tert*butyldimethylsilyl, C₁-C₆-alkyl, C₂-C₆-alkenyl, C₂-C₆-alkynyl, C₃-C₈-cycloalkyl,
wherein the four last mentioned radicals may be unsubstituted, partially or completely halogenated and/or oxygenated and/or may carry 1 or 2 radicals selected from C₁-C₄-alkoxy,
phenyl, benzyl, pyridyl, phenoxy,
wherein the four last mentioned radicals may be unsubstituted, partially or completely halogenated and/or may carry 1, 2 or 3 identical or different substituents selected from C₁-C₆-alkyl, C₁-C₆-haloalkyl, C₁-C₆-alkoxy, C₁-C₆ haloalkoxy and (C₁-C₆-alkoxy)carbonyl,
- R^{17a} and R^{17b}: are each independently from one another selected from the group consisting of hydrogen, C₁-C₆-alkoxy, C₁-C₆-haloalkoxy, C₁-C₆-alkylthio, C₁-C₆-alkylsulfinyl, C₁-C₆-alkylsulfonyl, C₁-C₆-haloalkylthio, trimethylsilyl, triethylsilyl, *tert*butyldimethylsilyl, C₁-C₆-alkyl, C₂-C₆-alkenyl, C₂-C₆-alkynyl, C₃-C₈-cycloalkyl,
wherein the four last mentioned radicals may be unsubstituted, partially or completely halogenated and/or oxygenated and/or may carry 1 or 2 identical or different radicals selected from C₁-C₄-alkoxy,
phenyl, benzyl, pyridyl, phenoxy,
wherein the four last mentioned radicals may be unsubstituted, partially or completely halogenated and/or may carry 1, 2 or 3 identical or different substituents selected from C₁-C₆-alkyl, C₁-C₆-haloalkyl, C₁-C₆-alkoxy, C₁-C₆ haloalkoxy and (C₁-C₆-alkoxy)carbonyl,
or,
R^{17a} and R^{17b} may together be a C₂-C₆ alkylene chain forming a 3- to 7-membered saturated, partly saturated or unsaturated ring together with the nitrogen atom R^{17a} and R^{17b} are bonded to,
wherein the alkylene chain may contain 1 or 2 heteroatoms selected, independently of each other, from oxygen, sulfur or nitrogen, and may optionally be substituted with halogen, C₁-C₄-haloalkyl, C₁-C₄-alkoxy or C₁-C₄-haloalkoxy, and
wherein the nitrogen and/or the sulfur atom(s) of the heterocyclic ring may optionally be oxidized;
or
R^{17a} and R^{17b} together may form a =CR¹³R¹⁴, =NR¹⁷ or =NOR¹⁶ moiety;
- R^{17c}: independently of its occurrence, is selected from the group consisting of hydrogen, CN, C₁-C₆-alkyl, C₂-C₆-alkenyl, C₂-C₆-alkynyl, C₃-C₈-cycloalkyl, C₃-C₈-cycloalkyl-C₁-C₄-alkyl,
wherein the five last mentioned radicals may be unsubstituted, partially or completely halogenated and/or oxygenated and/or may carry 1 or 2 radicals independently selected from C₁-C₄ alkoxy,
phenyl, benzyl and pyridyl, wherein the last three radicals may be unsubstituted, partially or completely halogenated and/or may carry 1, 2 or 3 identical or different substituents substituents selected from C₁-C₆-alkyl, C₁-C₆-haloalkyl, C₁-C₆-alkoxy, C₁-C₆ haloalkoxy, (C₁-C₆-alkoxy)carbonyl, (C₁-C₆-alkyl)amino and di-(C₁-C₆-alkyl)amino;
- R¹⁸: is each independently from one another selected from the group consisting of hydrogen, halogen, cyano, nitro, OH, SH, SCN, SF₅, C₁-C₆-alkoxy, C₁-C₆-haloalkoxy, C₁-C₆-alkylthio, C₁-C₆-alkylsulfinyl, C₁-C₆-alkylsulfonyl, C₁-C₆-haloalkylthio, trimethylsilyl, triethylsilyl, *tert*butyldimethylsilyl, C₁-C₆-alkyl, C₂-C₆-alkenyl, C₂-C₆-alkynyl, C₃-C₈-cycloalkyl,
wherein the four last mentioned aliphatic and cyclo-aliphatic radicals may be unsubstituted, partially or completely halogenated and/or oxgenated and/or may carry 1 or 2 radicals selected from C₁-C₄ alkoxy;
phenyl, benzyl, pyridyl, phenoxy,
wherein the last four radicals may be unsubstituted, partially or completely halogenated and/or to carry 1, 2 or 3 substituents selected from C₁-C₆-alkyl, C₁-C₆-haloalkyl, C₁-C₆-alkoxy, C₁-C₆ haloalkoxy, (C₁-C₆-alkoxy)carbonyl, (C₁-C₆-alkyl)amino or di-(C₁-C₆-alkyl)amino,
or
two R¹⁸ present on the same carbon atom may together be =O, =CH(C₁-C₄), =C(C₁-C₄-alkyl)C₁-C₄-alkyl, =N(C₁-C₆-alkyl) or =NO(C₁-C₆-alkyl);
- R¹⁹: is each independently from one another selected from the group consisting of hydrogen, cyano, C₁-C₆-alkoxy, C₁-C₆-haloalkoxy, C₁-C₆-alkylthio, C₁-C₆-alkylsulfinyl, C₁-C₆-alkylsulfonyl, C₁-C₆-haloalkylthio, trimethylsilyl, triethylsilyl, *tert*butyldimethylsilyl, C₁-C₆-alkyl, C₂-C₆-alkenyl, C₂-C₆-alkinyl, C₃-C₈-cycloalkyl,
wherein the four last mentioned radicals may be unsubstituted, partially or completely halogenated and/or oxygenated and/or may carry 1 or 2 radicals selected from C₁-C₄ alkoxy,
phenyl, benzyl, pyridyl, phenoxy,
wherein the last four radicals may be unsubstituted, partially or completely halogenated and/or carry 1, 2 or 3 substituents selected from C₁-C₆-alkyl, C₁-C₆-haloalkyl, C₁-C₆-alkoxy, C₁-C₆ haloalkoxy or (C₁-C₆-alkoxy)carbonyl;
- R^{20a}, R^{20b}: are each independently from one another selected from the group consisting of hydrogen, cyano, C₁-C₆-alkoxy, C₁-C₆-haloalkoxy, C₁-C₆-alkylthio, C₁-C₆-alkylsulfinyl, C₁-C₆-alkylsulfonyl, C₁-C₆-haloalkylthio, trimethylsilyl, triethylsilyl, *tert*butyldimethylsilyl, C₁-C₆-alkyl, C₂-C₆-alkenyl, C₂-C₆-alkinyl, C₃-C₈-cycloalkyl,
wherein the four last mentioned aliphatic and cyclo-aliphatic radicals may be unsubstituted, partially or completely halogenated and/or oxygenated and/or may carry 1 or 2 identical or different radicals selected from C₁-C₄-alkoxy,
phenyl, benzyl, pyridyl, phenoxy,
wherein the four last mentioned radicals may be unsubstituted, partially or completely halogenated and/or carry 1, 2 or 3 identical or different substituents selected from C₁-C₆-alkyl, C₁-C₆-haloalkyl, C₁-C₆-alkoxy, C₁-C₆ haloalkoxy and (C₁-C₆-alkoxy)carbonyl,
or,
R^{20a} and R^{20b} may together be a C₂-C₆ alkylene chain forming a 3- to 7-membered saturated, partly saturated or unsaturated ring together with the nitrogen atom R^{20a} and R^{20b} are bonded to, wherein the alkylene chain may contain 1 or 2 heteroatoms selected from oxygen, sulfur or nitrogen, and may optionally be substituted with halogen, C₁-C₄-haloalkyl, C₁-C₄-alkoxy or C₁-C₄-haloalkoxy, and wherein the nitrogen and/or the sulfur atom(s) of the heterocyclic ring may optionally be oxidized;
- R^{21a}, R^{21b}: are each independently from one another selected from the group consisting of hydrogen, C₁-C₄ alkyl, C₁-C₆ cycloalkyl, C₁-C₄ alkoxyalkyl, phenyl and benzyl;
- Het^{#}: represents a 3-, 4-, 5-, 6- or 7- membered saturated, partly saturated or unsaturated aromatic heterocyclic ring comprising 1, 2 or 3 heteroatoms as ring members, which are selected from oxygen, nitrogen and/or sulfur,
wherein the heterocyclic ring is optionally substituted with 1, 2, 3 or 4 identical or different substituents R¹⁰, and wherein the nitrogen and/or the sulfur atom(s) of the heterocyclic ring may optionally be oxidized,
the stereoisomers, the tautomers and the salts thereof.

Preferred are compounds of formula (I), wherein Het is selected from the group consisting of radicals of formulae Het-1 to Het-24, with preference given to compounds of the formula (I), their stereoisomers, there tautomers and their salts, where Het is selected from the radicals of the formulae Het-1, Het-11 and Het-24: 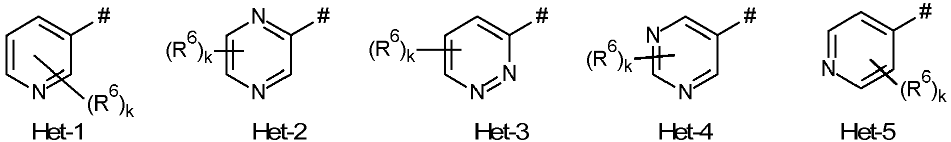 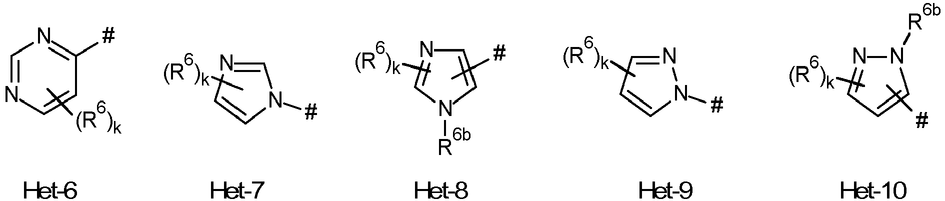 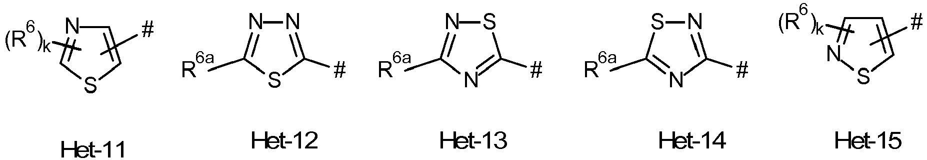 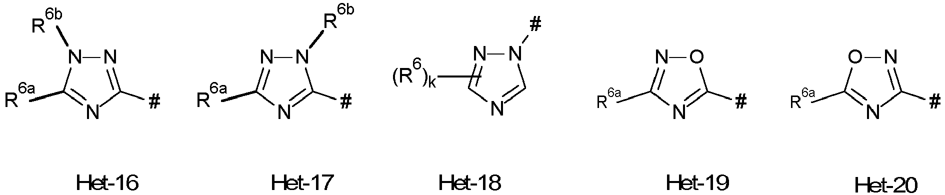 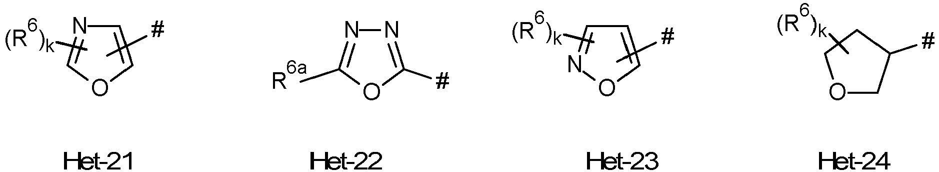 wherein # denotes the bond in formula (I), and wherein R⁶ and k are as defined above and where R^{6a} is hydrogen or has one of the meanings given for R⁶ and where R^{6b} is hydrogen or a C-bound radical mentioned as R⁶ and where R^{6b} is in particular hydrogen, C₁-C₄-alkyl or C₁-C₄-haloalkyl. In particular k is 0, 1 or 2, especially 0 or 1. In formulae Het-1, Het-2, Het-3, Het-4, Het-7, Het-8, Het-9, Het-10, Het-11, Het-18 and Het-21, k is especially 1. In particular R^{6a} in formulae Het-12, Het-13, Het-14, Het-16, Het-17, Het-19, Het 20 and Het-22 is different from hydrogen.

Irrespectively of its occurrence, R⁶ is preferably selected from the group consisting of halogen, cyano, C₁-C₆-alkyl, C₃-C₈-cycloalkyl, C₂-C₆-alkenyl, C₂-C₆-alkynyl, wherein each of the four last mentioned radicals may optionally be partly or completely halogenated, in particular by fluorine or chlorine, or may be (further) substituted independently from one another with one or more (e.g. 1, 2 or 3) identical or different R⁷, or R⁶ may also be a radical selected from the group consisting of OR⁸, NR^{17a}R^{17b}, S(O)ₙR⁸, S(O)ₙNR^{17a}R^{17b}, C(=O)R^{7a}, C(=O)NR^{17a}R^{17b}, C(=O)OR⁸, C(=S)R^{7a}, C(=S)NR^{17a}R^{17b}, C(=NR¹⁷)R^{7a}, C(=NR¹⁷)NR^{17a}R^{17b}. Irrespectively of its occurrence, R⁶ is in particular selected from the group consisting of halogen, such as chlorine or fluorine, C₁-C₄-alkyl, such as methyl or ethyl, C₁-C₄-alkoxy, such as methoxy or ethoxy, C₁-C₄-haloalkoxy, such as difluoromethoxy or trifluoromethoxy, and C₁-C₄-haloalkyl, such as difluoromethyl, trifluoromethyl, 2,2-difluoroethyl, 2,2,2-trifluoroethyl or pentafluoroethyl, more preferably from halogen, C₁-C₄-alkyl and C₁-C₄-haloalkyl, even more preferably from fluorine, chlorine, C₁-C₂-alkyl, such as methyl or ethyl and C₁-C₂-haloalkyl such as difluoromethyl, trifluoromethyl, 2,2-difluoroethyl, 2,2,2-trifluoroethyl or pentafluoroethyl.

Irrespectively of its occurrence, R^{6a} is preferably selected from the group consisting of hydrogen, halogen, cyano, C₁-C₆-alkyl, C₃-C₈-cycloalkyl, C₂-C₆-alkenyl and C₂-C₆-alkynyl, wherein the carbon atoms of the aforementioned aliphatic and cycloaliphatic radicals may optionally be partly or completely halogenated, in particular by fluorine or chlorine, or may further substituted independently from one another with one or more identical or different R⁷, or R^{6a} may also be a radical selected from the group consisting of OR⁸, NR^{17a}R^{17b}, S(O)ₙR⁸, S(O)ₙNR^{17a}R^{17b}, C(=O)R^{7a}, C(=O)NR^{17a}R^{17b}, C(=O)OR⁸, C(=S)R^{7a}, C(=S)NR^{17a}R^{17b}, C(=NR¹⁷)R^{7a}, C(=NR¹⁷)NR^{17a}R^{17b}. Irrespectively of its occurrence, R^{6a} is in particular selected from the group consisting of hydrogen, halogen, such as chlorine or fluorine, C₁-C₄-alkyl, such as methyl or ethyl, C₁-C₄-alkoxy, such as methoxy or ethoxy, C₁-C₄-haloalkoxy, such as difluoromethoxy or trifluoromethoxy, and C₁-C₄-haloalkyl, such as difluoromethyl, trifluoromethyl, 2,2-difluoroethyl, 2,2,2-trifluoroethyl or pentafluoroethyl, more preferably from halogen, C₁-C₄-alkyl and C₁-C₄-haloalkyl, even more preferably from fluorine, chlorine, C₁-C₂-alkyl, such as methyl or ethyl and C₁-C₂-haloalkyl such as difluoromethyl, trifluoromethyl, 2,2-difluoroethyl, 2,2,2-trifluoroethyl or pentafluoroethyl.

Irrespectively of its occurrence, R^{6b} is in particular selected from the group consisting of hydrogen, C₁-C₄-alkyl, such as methyl or ethyl, and C₁-C₄-haloalkyl, such as difluoromethyl, trifluoromethyl, 2,2-difluoroethyl, 2,2,2-trifluoroethyl or pentafluoroethyl, more preferably C₁-C₂-alkyl, such as methyl or ethyl and C₁-C₂-haloalkyl such as difluoromethyl, trifluoromethyl, 2,2-difluoroethyl, 2,2,2-trifluoroethyl or pentafluoroethyl.

Particularly preferred are compounds of formula (I), wherein Het is selected from the group consisting of radicals of formulae Het-1, Het-11a and Het-24, 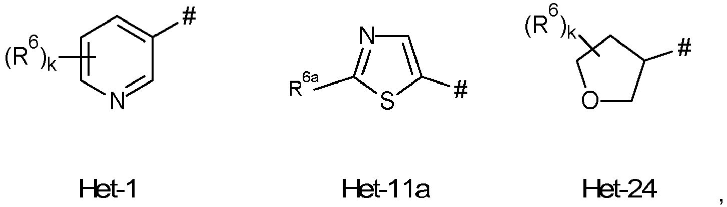 where
- R⁶: is selected from the group consisting of halogen, such as chlorine or fluorine, C₁-C₄-alkyl, such as methyl or ethyl, C₁-C₄-alkoxy, such as methoxy or ethoxy, C₁-C₄-haloalkoxy, such as difluoromethoxy or trifluoromethoxy, and C₁-C₄-haloalkyl, such as difluoromethyl, trifluoromethyl, 2,2-difluoroethyl, 2,2,2-trifluoroethyl or pentafluoroethyl, more preferably from halogen, C₁-C₄-alkyl and C₁-C₄-haloalkyl, even more preferably from fluorine, chlorine, C₁-C₂-alkyl, such as methyl or ethyl and C₁-C₂-haloalkyl such as difluoromethyl, trifluoromethyl, 2,2-difluoroethyl, 2,2,2-trifluoroethyl or pentafluoroethyl; and where
- R^{6a}: is selected from the group consisting of hydrogen, halogen, such as chlorine or fluorine, C₁-C₄-alkyl, such as methyl or ethyl, C₁-C₄-alkoxy, such as methoxy or ethoxy, C₁-C₄-haloalkoxy, such as difluoromethoxy or trifluoromethoxy, and C₁-C₄-haloalkyl, such as difluoromethyl, trifluoromethyl, 2,2-difluoroethyl, 2,2,2-trifluoroethyl or pentafluoroethyl, more preferably from halogen, C₁-C₄-alkyl and C₁-C₄-haloalkyl, even more preferably from fluorine, chlorine, C₁-C₂-alkyl, such as methyl or ethyl and C₁-C₂-haloalkyl such as difluoromethyl, trifluoromethyl, 2,2-difluoroethyl, 2,2,2-trifluoroethyl or pentafluoroethyl and
- k: is 0, 1 or 2.

A particularly preferred group of embodiments relates to compounds of formula (I) to the stereoisomers, the tautomers and to the salts thereof, wherein Het is a radical of formula Het-1, where k is 0, 1 or 2, in particular 1 or 2 and especially 1 and where R⁶ is as defined above and in particular selected from the group consisting of halogen, such as chlorine or fluorine, C₁-C₄-alkyl, such as methyl or ethyl, C₁-C₄-alkoxy, such as methoxy or ethoxy, C₁-C₄-haloalkoxy, such as difluoromethoxy or trifluoromethoxy, and C₁-C₄-haloalkyl, such as difluoromethyl, trifluoromethyl, 2,2-difluoroethyl, 2,2,2-trifluoroethyl or pentafluoroethyl, more preferably from halogen, C₁-C₄-alkyl and C₁-C₄-haloalkyl, even more preferably from fluorine, chlorine, C₁-C₂-alkyl, such as methyl or ethyl and C₁-C₂-haloalkyl such as difluoromethyl, trifluoromethyl, 2,2-difluoroethyl, 2,2,2-trifluoroethyl or pentafluoroethyl. Amongst the compounds of this particular group of embodiments, a particular sub-group of embodiments relates to compounds of the formula (I), to the stereoisomers, the tautomers and to the salts thereof, wherein Het is a radical of formula Het-1a 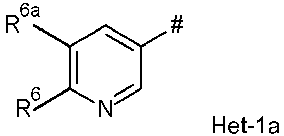 where
- R⁶: is as defined above and in particular selected from the group consisting of halogen, such as chlorine or fluorine, C₁-C₄-alkyl, such as methyl or ethyl, and C₁-C₄-haloalkyl, such as difluoromethyl, trifluoromethyl, 2,2-difluoroethyl, 2,2,2-trifluoroethyl or pentafluoroethyl, and even more preferably from fluorine, chlorine, C₁-C₂-alkyl, such as methyl or ethyl and C₁-C₂-haloalkyl such as difluoromethyl, trifluoromethyl, 2,2-difluoroethyl, 2,2,2-trifluoroethyl or pentafluoroethyl;
- R^{6a}: is as defined above and in particular selected from the group consisting of hydrogen, halogen, such as chlorine or fluorine and C₁-C₄-alkyl, such as methyl or ethyl, more preferably is hydrogen. A special embodiment of the radical Het-1a is 6-chloropyridin-3-yl, i.e. R^{6a} is hydrogen and
- R⁶: is chlorine. A further special embodiment of the radical Het-1a is 6-(trifluoromethyl)pyridin-3-yl, i.e. R^{6a} is hydrogen and R⁶ is trifluoromethyl.

Another particularly preferred group of embodiments relates to compounds of formula (I) to the stereoisomers, the tautomers and to the salts thereof, wherein Het is a radical of formula Het-11, where k is 0, 1 or 2, in particular 0 or 1, and where Het is in particular a radical of formula Het-11a, 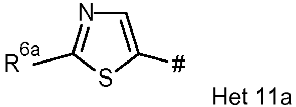 where R^{6a} is as defined above and wherein R^{6a} is in particular selected from the group consisting of hydrogen, halogen, such as chlorine or fluorine, C₁-C₄-alkyl, such as methyl or ethyl, C₁-C₄-alkoxy, such as methoxy or ethoxy, C₁-C₄-haloalkoxy, such as difluoromethoxy or trifluoromethoxy, and C₁-C₄-haloalkyl, such as difluoromethyl, trifluoromethyl, 2,2-difluoroethyl, 2,2,2-trifluoroethyl or pentafluoroethyl, more preferably from halogen, C₁-C₄-alkyl and C₁-C₄-haloalkyl, even more preferably from fluorine, chlorine, C₁-C₂-alkyl, such as methyl or ethyl and C₁-C₂-haloalkyl such as difluoromethyl, trifluoromethyl, 2,2-difluoroethyl, 2,2,2-trifluoroethyl or pentafluoroethyl. A special embodiment of the radical Het-11a is 2-chlorothiazol-5-yl, i.e. R^{6a} is chlorine.

Another particularly preferred group of embodiments relates to compounds of formula (I) to the stereoisomers, the tautomers and to the salts thereof, wherein Het is a radical of formula Het-24, where k is 0, 1 or 2, in particular 0 or 1, and where R⁶, if present, is as defined above and in particular selected from the group consisting of halogen, such as chlorine or fluorine, C₁-C₄-alkyl, such as methyl or ethyl, and C₁-C₄-haloalkyl, such as difluoromethyl, trifluoromethyl, 2,2-difluoroethyl, 2,2,2-trifluoroethyl or pentafluoroethyl, and even more preferably from fluorine, chlorine, C₁-C₂-alkyl, such as methyl or ethyl and C₁-C₂-haloalkyl such as difluoromethyl, trifluoromethyl, 2,2-difluoroethyl, 2,2,2-trifluoroethyl or pentafluoroethyl.

Preferred are compounds of formula (I), wherein R¹ and R² are independently from each other selected from the group consisting of hydrogen, halogen, such as fluorine or chlorine, CN, C₁-C₆-alkyl, in particular C₁-C₄-alkyl, such as methyl, ethyl, n-propyl or isopropyl, C₃-C₆-cycloalkyl, such as cyclopropyl or cyclobutyl, C₁-C₆-haloalkyl, in particular C₁-C₂-haloalkyl, such as fluoromethyl, difluoromethyl, trifluoromethyl, 1,1-difluoroethyl, 2-fluoroethyl, 2,2-difluoroethyl or 2,2,2-trifluoroethyl, or C₃-C₆-halocycloalkyl such as 1-fluorocyclopropyl or 2,2-difluorocyclopropyl.

Preferred are also compounds of formula (I), wherein R¹ and R² form, together with the carbon atom, which they attached to, a 3- to 5 membered saturated carbocyclic ring such as cyclopropyl, cyclobutyl or cyclopentyl.

Even more preferred are compounds of formula (I), wherein R¹ and R² are independently from each other selected from the group consisting of hydrogen, halogen, cyano, C₁-C₃-alkyl, such as methyl ethyl or isopropyl, or C₁-C₃-haloalkyl such as fluoromethyl, difluoromethyl, trifluoromethyl, 1,1-difluoroethyl, 2-fluoroethyl, 2,2-difluoroethyl or 2,2,2-trifluoroethyl.

Preferably at least one of the radicals R¹ and R² is hydrogen.

Even more preferred are compounds of formula (I), wherein R¹ and R² are both hydrogen or R¹ is methyl and R² is hydrogen.

In a particular group of embodiments, R¹ and R² are both hydrogen.

According to a first group of embodiments A, compounds of formula (I) are preferred, wherein R³ is selected from the group consisting of hydrogen, halogen, NO₂, C₁-C₆-alkyl, in particular C₁-C₄-alkyl, C₁-C₆-haloalkyl, in particular C₁-C₄-haloalkyl, C₂-C₆-alkenyl, in particular C₂-C₄-alkenyl, C₂-C₆-haloalkenyl, in particular C₂-C₄-haloalkenyl, C₃-C₆-cycloalkyl, such as cyclopropyl or cyclobutyl, C₃-C₆-halocycloalkyl, such as 1-fluorocyclopropyl, 2-fluorocyclopropyl, 2,2-difluorocyclopropyl, 2-chlorocyclopropyl, 2,2-dichlorocyclopropyl, 1-fluorocyclobutyl, 2-fluorocyclobutyl, 3-fluorocyclobutyl, 2,2-difluorocyclobutyl or 3,3-difluorocyclobutyl, C₃-C₆-cycloalkenyl such as cyclobutenyl, NR^{9a}R^{9b} and NR^{9a}-C(=O)R^{7a}.

In the context of R³, the radicals R^{7a} is preferably selected from the group consisting of hydrogen, C₁-C₄-alkyl, such as methyl, ethyl, n-propyl, isopropyl, n-butyl, 2-butyl or isobutyl, and C₁-C₄-haloalkyl, such as fluoromethyl, difluoromethyl, trifluoromethyl, 1,1-difluoroethyl, 2-fluoroethyl, 2,2-difluoroethyl or 2,2,2-trifluoroethyl, C₂-C₄-alkenyl, such as ethenyl, 2-propenyl, isopropenyl, n-butenyl, 2-butenyl or isobutenyl, and C₂-C₄-haloalkenyl, such as 1,1-difluoroethenyl, 2-fluoroethenyl, 2,2-difluoroethenyl or 2,2,2-trifluoroethenyl. In the context of R³, the radicals R^{9a} and R^{9b} are preferably selected from the group consisting of hydrogen, C₁-C₄-alkyl, such as methyl, ethyl, n-propyl, isopropyl, n-butyl, 2-butyl or isobutyl, and C₁-C₄-haloalkyl, such as fluoromethyl, difluoromethyl, trifluoromethyl, 1,1-difluoroethyl, 2-fluoroethyl, 2,2-difluoroethyl or 2,2,2-trifluoroethyl, or NR^{9a}R^{9b} may also be a saturated N-bound 3-, 4-, 5- or 6-membered heterocycle, which in addition to the nitrogen atom may have 1 further heteroatom as ring members, which is selected from O and N and where the N-bound 3-, 4-, 5- or 6-membered heterocycle may be unsubstituted or carry 1, 2, 3 or 4 radicals selected from C₁-C₄-alkyl and C₁-C₄-haloalkyl. Examples of radicals NR^{9a}R^{9b} include, but are not limited to methylamino, ethylamino, n-propylamino, isopropylamino, n-butylamino, 2-butylamino, isobutylamino, dimethylamino, diethylamino, di-n-propylamino, di-n-butylamino, N-methyl-N-ethylamino, N-methyl-N-propylamino, N-methyl-N-n-propylamino, N-methyl-N-isopropylamino, N-methyl-N-n-butylamino, N-methyl-N-2-butylamino, N-methyl-N-isobutylamino, 1-pyrrolidinyl, 1-piperidinyl, 1-piperazinyl, 4-methyl-1-piperazinyl and 4-morpholinyl. Examples of radicals NR^{9a}C(=O)R^{7a} include, but are not limited NH-C(=O)H, NH-C(=O)CH₃, NH-C(=O)CH₂CH₃ and NH-C(=O)CH(CH₃)₂.

In this group of embodiments A, the radical R³ is in particular selected from the group consisting of hydrogen, fluorine, chlorine, C₁-C₄-alkyl, such as methyl, ethyl, n-propyl, isopropyl or n-butyl, and C₂-C₄-alkenyl, such as ethenyl, 2-propenyl, isopropenyl, n-butenyl, 2-butenyl or isobutenyl.

In this group of embodiments A, the radical R³ is especially selected from the group consisting of hydrogen and C₁-C₄-alkyl, such as methyl, ethyl, n-propyl, isopropyl or n-butyl.

In this group of embodiments A, preference is given to compounds of formula (I), wherein R⁵ is selected from the group consisting of hydrogen, halogen, CN, NR^{9a}R^{9b}, C₁-C₆-alkyl, in particular C₁-C₄-alkyl, C₁-C₆-haloalkyl, in particular C₁-C₄-haloalkyl, C₁-C₆-alkoxy, in particular C₁-C₄-alkoxy, C₁-C₆-haloalkoxy, in particular C₁-C₄-haloalkoxy, C₂-C₆-alkenyl, in particular C₂-C₄-alkenyl, C₂-C₆-haloalkenyl, in particular C₂-C₄-haloalkenyl, C₃-C₆-cycloalkyl, C₃-C₆-halocycloalkyl, C₃-C₆-cycloalkoxy, C₃-C₆-halocycloalkoxy, C(=O)OR⁸, C(=O)NR^{9a}R^{9b}, C(=S)NR^{9a}R^{9b}, C(=O)R^{7a}, C(=S)R^{7a} and Q-phenyl, where phenyl is unsubstituted or substituted with 1, 2, 3, 4 or 5 identical or different substituents R¹⁰.

In context of R⁵, Het^{#} is preferably 5- or 6-membered hetaryl such as pyridyl, thienyl, furyl, pyrrolyl, thiazolyl, isothiazolyl, pyrazolyl, imidazolyl, oxazolyl or isoxazolyl, which is unsubstituted or substituted by 1, 2 or 3 radicals R¹⁰. In this context, R¹⁰ is preferably selected from the group consisting of halogen, such as chlorine or fluorine, CN, C₁-C₄-alkyl, such as methyl, ethyl, n-propyl and isopropyl, C₁-C₄-haloalkyl, in particular C₁-C₂-haloalkyl, such as fluoromethyl, difluoromethyl, trifluoromethyl, 1,1-difluoroethyl, 2-fluoroethyl, 2,2-difluoroethyl or 2,2,2-trifluoroethyl, C₁-C₄-alkoxy, such as methoxy, ethoxy, n-propoxy and isopropoxy, and C₁-C₄-haloalkoxy, in particular C₁-C₂-haloalkoxy, such as fluoromethoxy, difluoromethoxy, trifluoromethoxy, 1,1-difluoroethoxy, 2-fluoroethoxy, 2,2-difluoroethoxy or 2,2,2-trifluoroethoxy.

In context of R⁵, the radical R^{7a} is preferably selected from the group consisting of hydrogen, C₁-C₄-alkyl, C₁-C₄-haloalkyl, C₃-C₆-cycloalkyl, C₃-C₆-cycloalkyl-C₁-C₄-alkyl, phenyl and benzyl, where the phenyl ring in the last two radicals is unsubstituted or substituted by 1, 2 or 3 identical or different radicals selected from the group consisting of halogen, such as chlorine or fluorine, CN, C₁-C₄-alkyl, such as methyl, ethyl, n-propyl and isopropyl, C₁-C₄-haloalkyl, in particular C₁-C₂-haloalkyl, such as fluoromethyl, difluoromethyl, trifluoromethyl, 1,1-difluoroethyl, 2-fluoroethyl, 2,2-difluoroethyl or 2,2,2-trifluoroethyl, C₁-C₄-alkoxy, such as methoxy, ethoxy, n-propoxy and isopropoxy, and C₁-C₄-haloalkoxy, in particular C₁-C₂-haloalkoxy, such as fluoromethoxy, difluoromethoxy, trifluoromethoxy, 1,1-difluoroethoxy, 2-fluoroethoxy, 2,2-difluoroethoxy or 2,2,2-trifluoroethoxy. R^{7a} is in particular C₁-C₄-alkyl.

In context of R⁵, the radical R⁸ is preferably selected from the group consisting of C₁-C₄-alkyl, C₁-C₄-haloalkyl, C₃-C₆-cycloalkyl, C₃-C₆-cycloalkyl-C₁-C₄-alkyl, phenyl and benzyl, where the phenyl ring in the last two radicals is unsubstituted or substituted by 1, 2 or 3 identical or different radicals selected from the group consisting of halogen, such as chlorine or fluorine, CN, C₁-C₄-alkyl, such as methyl, ethyl, n-propyl and isopropyl, C₁-C₄-haloalkyl, in particular C₁-C₂-haloalkyl, such as fluoromethyl, difluoromethyl, trifluoromethyl, 1,1-difluoroethyl, 2-fluoroethyl, 2,2-difluoroethyl or 2,2,2-trifluoroethyl, C₁-C₄-alkoxy, such as methoxy, ethoxy, n-propoxy and isopropoxy, and C₁-C₄-haloalkoxy, in particular C₁-C₂-haloalkoxy, such as fluoromethoxy, difluoromethoxy, trifluoromethoxy, 1,1-difluoroethoxy, 2-fluoroethoxy, 2,2-difluoroethoxy or 2,2,2-trifluoroethoxy. R⁸ is in particular C₁-C₄-alkyl.

In context of R⁵, the radicals R^{9a} and R^{9b} are preferably selected from the group consisting of hydrogen, C₁-C₄-alkyl, such as methyl, ethyl, n-propyl, isopropyl, n-butyl, 2-butyl or isobutyl, and C₁-C₄-haloalkyl, such as fluoromethyl, difluoromethyl, trifluoromethyl, 1,1-difluoroethyl, 2-fluoroethyl, 2,2-difluoroethyl or 2,2,2-trifluoroethyl, or NR^{9a}R^{9b} may also be a saturated N-bound 3-, 4-, 5- or 6-membered heterocycle, which in addition to the nitrogen atom may have 1 further heteroatom as ring members, which is selected from O and N and where the N-bound 3-, 4-, 5- or 6-membered heterocycle may be unsubstituted or carry 1, 2, 3 or 4 radicals selected from C₁-C₄-alkyl and C₁-C₄-haloalkyl. Examples of such radicals NR^{9a}R^{9b} include, but are not limited to methylamino, ethylamino, n-propylamino, isopropylamino, n-butylamino, 2-butylamino, isobutylamino, dimethylamino, diethylamino, di-n-propylamino, di-n-butylamino, N-methyl-N-ethylamino, N-methyl-N-propylamino, N-methyl-N-n-propylamino, N-methyl-N-isopropylamino, N-methyl-N-n-butylamino, N-methyl-N-2-butylamino, N-methyl-N-isobutylamino, 1-pyrrolidinyl, 1-piperidinyl, 1-piperazinyl, 4-methyl-1-piperazinyl and 4-morpholinyl.

In context of R⁵, the radical R¹⁰ is preferably selected from the group consisting of halogen, such as chlorine or fluorine, CN, C₁-C₄-alkyl, such as methyl, ethyl, n-propyl and isopropyl, C₁-C₄-haloalkyl, in particular C₁-C₂-haloalkyl, such as fluoromethyl, difluoromethyl, trifluoromethyl, 1,1-difluoroethyl, 2-fluoroethyl, 2,2-difluoroethyl or 2,2,2-trifluoroethyl, C₁-C₄-alkoxy, such as methoxy, ethoxy, n-propoxy and isopropoxy, and C₁-C₄-haloalkoxy, in particular C₁-C₂-haloalkoxy, such as fluoromethoxy, difluoromethoxy, trifluoromethoxy, 1,1-difluoroethoxy, 2-fluoroethoxy, 2,2-difluoroethoxy or 2,2,2-trifluoroethoxy.

In this group of embodiments A, the radical R⁵ is in particular selected from the group consisting of hydrogen, halogen, such as fluorine or chlorine, CN, C₁-C₆-alkyl, in particular C₁-C₄-alkyl, such as methyl, ethyl, n-propyl, isopropyl, n-butyl, 2-butyl or isobutyl, C₁-C₆-haloalkyl, in particular C₁-C₄-haloalkyl, such as fluoromethyl, difluoromethyl, trifluoromethyl, 1,1-difluoroethyl, 2-fluoroethyl, 2,2-difluoroethyl or 2,2,2-trifluoroethyl, C₁-C₆-alkoxy, in particular C₁-C₄-alkoxy, such as methoxy, ethoxy, n-propoxy, isopropoxy, n-butoxy, 2-butoxy or isobutoxy, C₁-C₆-haloalkoxy, in particular C₁-C₄-haloalkoxy, such as fluoromethoxy, difluoromethoxy, trifluoromethoxy, 1,1-difluoroethoxy, 2-fluoroethoxy, 2,2-difluoroethoxy or 2,2,2-trifluoroethoxy, C₂-C₆-alkenyl, in particular C₂-C₄-alkenyl, such as ethenyl, 2-propenyl, isopropenyl, n-butenyl, 2-butenyl or isobutenyl, and C₂-C₆-haloalkenyl, in particular C₂-C₄-haloalkenyl, such as 1,1-difluoroethenyl, 2-fluoroethenyl, 2,2-difluoroethenyl or 2,2,2-trifluoroethenyl, C₃-C₆-cycloalkyl, such as cyclopropyl, cyclobutyl, cyclopentyl or cyclohexyl, C₃-C₆-halocycloalkyl, such as 1-fluorocyclopropyl, 2-fluorocyclopropyl, 2,2-difluorocyclopropyl, 2-chlorocyclopropyl, 2,2-dichlorocyclopropyl, 1-fluorocyclobutyl, 2-fluorocyclobutyl, 3-fluorocyclobutyl, 2,2-difluorocyclobutyl or 3,3-difluorocyclobutyl, C₃-C₆-cycloalkoxy, such as cyclopropoxy, cyclobutoxy, cyclopentoxy or cyclohexoxy, and C₃-C₆-halocycloalkoxy, such as 1-fluorocyclopropoxy, 2-fluorocyclopropoxy, 2,2-difluorocyclopropoxy, 2-chlorocyclopropoxy, 2,2-dichlorocyclopropoxy, 1-fluorocyclobutoxy, 2-fluorocyclobutoxy, 3-fluorocyclobutoxy, 2,2-difluorocyclobutoxy or 3,3-difluorocyclobutoxy.

The variable m is preferably 0, 1 or 2, and especially 0 or 1, in particular with regard to the group of embodiments A.

According to particular embodiments A, wherein m=0,
R³ and R⁵ are independently selected from hydrogen, halogen, such as fluorine or chlorine, and wherein in particular each of R³ and R⁵ is fluorine.

If R^{4a} and R^{4b} are present, i.e. if m is different from 0 and in particular 1 or 2, and especially 1, the variables R^{4a} and R^{4b} are in particular selected, independently from one another, from the group consisting of hydrogen, C₁-C₄-alkyl, in particular methyl, and halogen, in particular fluorine or chlorine, or R^{4a} and R^{4b} together are =O.

According to a second group of embodiments B, compounds of formula (I) are preferred, wherein m is 0, and
- R³ and R⁵: together with the carbon atom, to which they are bound, form a 3-, 4-, 5- or 6-membered saturated carbocyle, a 4-, 5- or 6-membered unsaturated carbocyle, an epoxide ring or an oxetane ring,
wherein each of the carbon atoms of the saturated or unsaturated carbocycle may be unsubstituted or may carry 1 or 2 identical or different radicals R^{7b}, wherein R^{7b} is as defined above and wherein
- R^{7b}: is in particular selected from the group consisting of halogen, C₁-C₆-alkyl, C₁-C₆-alkoxy, and C₂-C₆-alkynyl,
wherein the carbon atoms of the aforementioned aliphatic radicals may optionally be partly or completely halogenated, and
one or two radicals R^{7b} may also be C₃-C₆-cycloalkyl, which is unsubstituted or carries 1, 2, 3, 4 or 5 radicals selected from
fluorine, chlorine and methyl,
phenyl, optionally substituted with 1, 2, 3, 4 or 5 identical or different substituents R^{10a}, or a 3-, 4-, 5-, 6- or 7- membered saturated, partly saturated or unsaturated aromatic heterocyclic ring comprising 1, 2 or 3 heteroatoms as ring members, which are identical or different and selected from oxygen, nitrogen and sulfur,
wherein the heterocyclic ring is optionally substituted with 1, 2, 3 or 4 identical or different substituents R^{10a}, and
wherein the nitrogen and/or the sulfur atom(s) of the heterocyclic ring may optionally be oxidized,
in particular a 5- or 6-membered hetaryl having 1 or 2 heteroatoms selected from O, S and N,
wherein the 5- or 6-membered hetaryl is unsubstituted or carries 1, 2 or 3 identical or different substituents R^{10a},
or two of R^{7b} present on one ring carbon together form =O or =S,
or two R^{7b} together form a linear C₂-C₇ alkylene chain, thus forming, together with the carbon atom to which they are bound, a 3-, 4-, 5- or 6-membered spiro ring,
wherein 1 or 2 CH₂ moieties of the alkylene chain may be replaced by 1 or 2 heteroatom moieties independently selected from O and S, and wherein the alkylene chain is unsubstituted or may be substituted with 1, 2, 3, 4, 5 or 6 identical or different radicals selected from the group consisting of halogen, C₁-C₄-alkyl, C₁-C₄-haloalkyl, C₁-C₄-alkoxy and C₁-C₄-haloalkoxy, wherein
^{R10a} has one of the meanings given for R¹⁰ and is in particular selected from the group consisting of halogen, C₁-C₄-alkyl, C₁-C₄-haloalkyl, C₁-C₄-alkoxy and C₁-C₄-haloalkoxy.

According to the second group of embodiments B, particular preference is given to compounds of formula (I), wherein
- R³ and R⁵: together with the carbon atom, to which they are bound, form a 3- or 4-membered saturated carbocyle, i.e. a 1,1-cyclopropylidene or 1,1-cyclobutylidene radical, or a 5-membered monounsaturated carbocycle, such as an 1,1-cyclopent-3-enylidene radical, wherein each of the carbon atoms of the saturated or unsaturated carbocycle may be unsubstituted or may carry 1 or 2 identical or different radicals R^{7b}, wherein
- R^{7b}: is as defined above and in particular is selected from the group consisting of halogen, C₁-C₄-alkyl, and wherein one radical R^{7b} may also be
phenyl, optionally substituted with 1, 2, 3, 4 or 5, in particular 1, 2 or 3, identical or different substituents R^{10a}, wherein
- R^{10a}: is as defined above, and in particular is selected from the group consisting of halogen, such as fluorine or chlorine, C₁-C₄-alkyl, CN, C₁-C₄-haloalkyl, C₁-C₄-alkoxy and C₁-C₄-haloalkoxy.

According to the second group of embodiments B, more particular preference is given to compounds of formula (I), wherein R³ and R⁵ together form the following moieties: CH₂-CH₂, CHF-CH₂, CF₂-CH₂, CHF-CHF, CF₂-CHF, CF₂-CF₂, CHCl-CH₂, CCl₂-CH₂, CHCI-CHCI, CCl₂-CHCl, CCl₂-CCl₂, CH(CH₃)-CH₂, C(CH₃)₂-CH₂, CH(CH₃)-CH(CH₃), C(CH₃)₂-CH(CH₃), C(CH₃)₂-C(CH₃)₂, CH(OCH₃)-CH₂, CH(CH₂CH₃)-CH₂, CH(CH₂CH₂CH₃)-CH₂, CH(CH₂CH₂CH₂CH₃)-CH₂, CH(CH₃)-CH₂-CH₂, CH₂-CH(CH₃)-CH₂, C(CH₃)₂-CH₂-CH₂, CH₂-C(CH₃)₂-CH₂, CH(CH₃)-CH(CH₃)-CH₂, CH(CH₃)-CH₂-CH(CH₃), CH(CH₃)-CH(CH₃)-CH(CH₃), C(CH₃)₂-CH(CH₃)-CH₂, CH(CH₃)-C(CH₃)₂-CH₂, C(CH₃)₂-CH₂-C(CH₃)₂, C(CH₃)₂-C(CH₃)₂-CH₂, C(CH₃)₂-C(CH₃)₂-C(CH₃)₂, CHF-CH₂-CH₂, CH₂-CHF-CH₂, CF₂-CH₂-CH₂, CH₂-CF₂-CH₂, CHF-CHF-CH₂, CHF-CH₂-CHF, CHF-CHF-CHF, CF₂-CHF-CH₂, CHF-CF₂-CH₂, CF₂-CH₂-CF₂, CF₂-CF₂-CH₂, CF₂-CF₂-CF₂, CHCl-CH₂-CH₂, CH₂-CHCl-CH₂, CCl₂-CH₂-CH₂, CH₂-CCl₂-CH₂, CHCl-CHCl-CH₂, CHCl-CH₂-CHCl, CHCl-CHCl-CHCl, CCl₂-CHCl-CH₂, CHCl-CCl₂-CH₂, CCl₂-CH₂-CCl₂, CCl₂-CCl₂-CH₂, CCl₂-CCl₂-CCl₂, CH₂O, CH(CH₃)-O, (CH₃)₂-O, CH₂-O-CH₂, CH₂-CH₂-O, or a radical CH(Ar)-CH₂, where Ar is selected from phenyl, 2-chlorophenyl, 2-fluorophenyl, 2-methylphenyl, 2-methoxyphenyl, 2-cyanophenyl, 3-chlorophenyl, 3-fluorophenyl, 3-methylphenyl, 3-methoxyphenyl, 3-cyanophenyl, 4-chlorophenyl, 4-fluorophenyl, 4-methylphenyl, 4-methoxyphenyl and 4-cyanophenyl,
thereby forming, together with the carbon atom they are bound to, an unsubstituted or substituted 1,1-cyclopropylidene or 1,1-cyclobutylidene radical.

According to a third group of embodiments C, compounds of formula (I) are preferred, wherein R³ and R⁵ together form =S or =O, in particular =O.

According to a fourth group of embodiments D, compounds of formula (I) are preferred, wherein X, Het, W¹-W²-W³-W⁴; R¹, R², R^{4b} and R⁵ are as defined herein, and specifically, individually or in combination, have the meanings given as preferred meanings,
m is 1, and
R³ and R^{4a} form both together and together with the existing bond a double bond between the adjacent carbon atoms. In this embodiment, D the radical R^{4b} is as defined above and in particular selected from the group consisting of hydrogen, C₁-C₄-alkyl, in particular methyl, and halogen, in particular fluorine or chlorine.

In this group of embodiments D, preference is given to compounds of formula (I), wherein R⁵ is selected from the group consisting of hydrogen, halogen, CN, NR^{9a}R^{9b}, C₁-C₆-alkyl, in particular C₁-C₄-alkyl, C₁-C₆-haloalkyl, in particular C₁-C₄-haloalkyl, C₁-C₆-alkoxy, in particular C₁-C₄-alkoxy, C₁-C₆-haloalkoxy, in particular C₁-C₄-haloalkoxy, C₂-C₆-alkenyl, in particular C₂-C₄-alkenyl, C₂-C₆-haloalkenyl, in particular C₂-C₄-haloalkenyl, C₃-C₆-cycloalkyl, C₃-C₆-halocycloalkyl, C₃-C₆-cycloalkoxy, C₃-C₆-halocycloalkoxy, C(=O)OR⁸, C(=O)NR^{9a}R^{9b}, C(=S)NR^{9a}R^{9b}, C(=O)R^{7a}, C(=S)R^{7a} and Q-phenyl, where phenyl is unsubstituted or substituted with 1, 2, 3, 4 or 5 identical or different substituents R¹⁰.

In this group of embodiments D, the radical R⁵ is in particular selected from the group consisting of hydrogen, halogen, such as fluorine or chlorine, CN, C₁-C₆-alkyl, in particular C₁-C₄-alkyl, such as methyl, ethyl, n-propyl, isopropyl, n-butyl, 2-butyl or isobutyl, C₁-C₆-haloalkyl, in particular C₁-C₄-haloalkyl, such as fluoromethyl, difluoromethyl, trifluoromethyl, 1,1-difluoroethyl, 2-fluoroethyl, 2,2-difluoroethyl or 2,2,2-trifluoroethyl, C₁-C₆-alkoxy, in particular C₁-C₄-alkoxy, such as methoxy, ethoxy, n-propoxy, isopropoxy, n-butoxy, 2-butoxy or isobutoxy, C₁-C₆-haloalkoxy, in particular C₁-C₄-haloalkoxy, such as fluoromethoxy, difluoromethoxy, trifluoromethoxy, 1,1-difluoroethoxy, 2-fluoroethoxy, 2,2-difluoroethoxy or 2,2,2-trifluoroethoxy, C₂-C₆-alkenyl, in particular C₂-C₄-alkenyl, such as ethenyl, 2-propenyl, isopropenyl, n-butenyl, 2-butenyl or isobutenyl, and C₂-C₆-haloalkenyl, in particular C₂-C₄-haloalkenyl, such as 1,1-difluoroethenyl, 2-fluoroethenyl, 2,2-difluoroethenyl or 2,2,2-trifluoroethenyl, C₃-C₆-cycloalkyl, such as cyclopropyl, cyclobutyl, cyclopentyl or cyclohexyl, C₃-C₆-halocycloalkyl, such as 1-fluorocyclopropyl, 2-fluorocyclopropyl, 2,2-difluorocyclopropyl, 2-chlorocyclopropyl, 2,2-dichlorocyclopropyl, 1-fluorocyclobutyl, 2-fluorocyclobutyl, 3-fluorocyclobutyl, 2,2-difluorocyclobutyl or 3,3-difluorocyclobutyl, C₃-C₆-cycloalkoxy, such as cyclopropoxy, cyclobutoxy, cyclopentoxy or cyclohexoxy, and C₃-C₆-halocycloalkoxy, such as 1-fluorocyclopropoxy, 2-fluorocyclopropoxy, 2,2-difluorocyclopropoxy, 2-chlorocyclopropoxy, 2,2-dichlorocyclopropoxy, 1-fluorocyclobutoxy, 2-fluorocyclobutoxy, 3-fluorocyclobutoxy, 2,2-difluorocyclobutoxy or 3,3-difluorocyclobutoxy.

According to a fifth group of embodiments E, compounds of formula (I) are preferred, wherein X, Het, W¹-W²-W³-W⁴; R¹, R², R³ and R^{4b} are as defined herein, and specifically, individually or in combination, have the meanings given as preferred meanings,
m is 1, and
R^{4b} and R⁵ form together and with the carbon atom they are bound to a 3-, 4-, 5- or 6-membered saturated or partially unsaturated carbocycle or heterocycle,
wherein each of the carbon atoms of the carbocycle or heterocycle may be unsubstituted or may carry 1 or 2 identical or different radicals R^{7b}, and wherein the heterocylce has 1 or 2 non-adjacent identical or different heteroatoms or heteroatom moieties as ring members, which are selected from O, S, N, NH and N-R^{17c}.

Preferred are compounds of formula (I), wherein W¹-W²-W³-W⁴ represents a carbon chain group connected to N and C=N, which is selected from the group consisting of CR^{w6}=CR^{w5}-CR^{w4}=CR^{w3}, CR^{w6}=CR^{w5}-CHR^{w4}-CHR^{w3}, CHR^{w6}-CHR^{w5}-CHR^{w4}-CHR^{w3}, CHR^{w6}-CHR^{w5}-CR^{w4}=CR^{w3}, and CHR^{w6}-CHR^{w5}-CHR^{w4}-CHR^{w3}, where in the five aforementioned radicals the carbon atom which carries R^{w6} is bound to the nitrogen atom and where R^{w3}, R^{w4}, R^{w5} and R^{w6}, independently of each other, have one of the meanings given for R^{w}. In this context, R^{w} is preferably selected from the group consisting of hydrogen, halogen, such as fluorine or chlorine, CN, C₁-C₄-alkyl, such as methyl, ethyl, n-propyl and isopropyl, C₁-C₄-haloalkyl, in particular C₁-C₂-haloalkyl, such as fluoromethyl, difluoromethyl, trifluoromethyl, 1,1-difluoroethyl, 2-fluoroethyl, 2,2-difluoroethyl or 2,2,2-trifluoroethyl, C₁-C₄-alkoxy, such as methoxy, ethoxy, n-propoxy and isopropoxy, and C₁-C₄-haloalkoxy, in particular C₁-C₂-haloalkoxy, such as fluoromethoxy, difluoromethoxy, trifluoromethoxy, 1,1-difluoroethoxy, 2-fluoroethoxy, 2,2-difluoroethoxy or 2,2,2-trifluoroethoxy. Preferably, at most one of R^{w3}, R^{w4}, R^{w5} and R^{w6} is different from hydrogen.

In a particularly preferred group of embodiments R^{w3}, R^{w4} and R^{w6} are hydrogen while R^{w5} has one of the meanings given for R^{w}, and where R^{w5} is in particular selected from the group consisting of hydrogen, halogen, such as fluorine or chlorine, CN, C₁-C₄-alkyl, such as methyl, ethyl, n-propyl and isopropyl, C₁-C₄-haloalkyl, in particular C₁-C₂-haloalkyl, such as fluoromethyl, difluoromethyl, trifluoromethyl, 1,1-difluoroethyl, 2-fluoroethyl, 2,2-difluoroethyl or 2,2,2-trifluoroethyl, C₁-C₄-alkoxy, such as methoxy, ethoxy, n-propoxy and isopropoxy, and C₁-C₄-haloalkoxy, in particular C₁-C₂-haloalkoxy, such as fluoromethoxy, difluoromethoxy, trifluoromethoxy, 1,1-difluoroethoxy, 2-fluoroethoxy, 2,2-difluoroethoxy or 2,2,2-trifluoroethoxy.

In another particularly preferred group of embodiments R^{w3}, R^{w4} and R^{w5} are hydrogen while R^{w6} has one of the meanings given for R^{w}, and where R^{w6} is in particular selected from the group consisting of hydrogen, halogen, such as fluorine or chlorine, CN, C₁-C₄-alkyl, such as methyl, ethyl, n-propyl and isopropyl, C₁-C₄-haloalkyl, in particular C₁-C₂-haloalkyl, such as fluoromethyl, difluoromethyl, trifluoromethyl, 1,1-difluoroethyl, 2-fluoroethyl, 2,2-difluoroethyl or 2,2,2-trifluoroethyl, C₁-C₄-alkoxy, such as methoxy, ethoxy, n-propoxy and isopropoxy, and C₁-C₄-haloalkoxy, in particular C₁-C₂-haloalkoxy, such as fluoromethoxy, difluoromethoxy, trifluoromethoxy, 1,1-difluoroethoxy, 2-fluoroethoxy, 2,2-difluoroethoxy or 2,2,2-trifluoroethoxy.

Especially, all of R^{w3}, R^{w4}, R^{w5} and R^{w6} are hydrogen.

Preferred are compounds of formula (I), wherein the moiety of the formula (A) 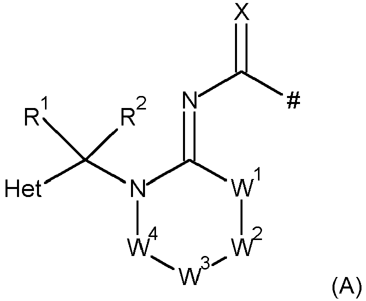 represents a radical selected from the group consisting of W.Het-1, W.Het-2, W.Het-3, W.Het-4, W.Het-5, W.Het-6, W.Het-7, W.Het-8, W.Het-9, W.Het-10, W.Het-11 and W.Het-12, wherein the radical of formula (A) is in particular selected rom the radicals W.Het-1, W.Het-2, W.Het-5, W.Het-6, W.Het-9 and W.Het-10. 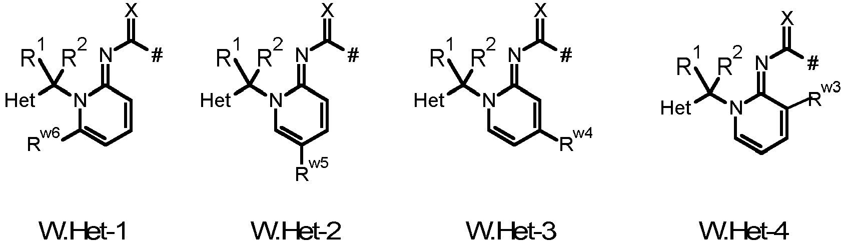 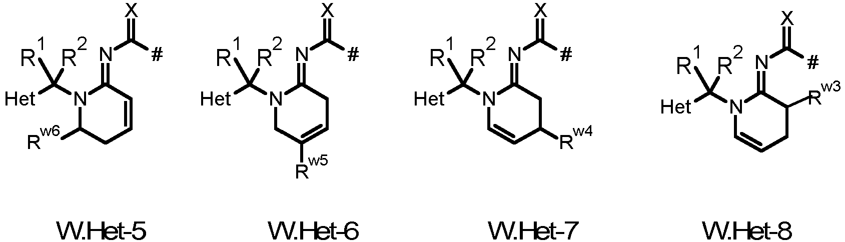 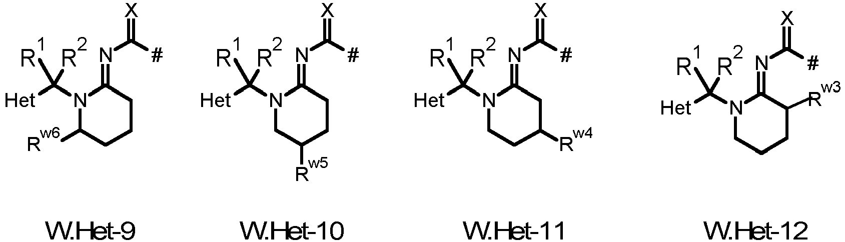 wherein # denotes the bond to the remainder of the molecule and where R¹, R² and Het are as defined herein and where R¹, R² and Het, individually or in combination have the meanings given as preferred meanings, and wherein R^{w3}, R^{w4},^{,} R^{w5} and R^{w6} are as defined above and in particular selected from the group consisting of hydrogen, halogen, such as fluorine or chlorine, CN, C₁-C₄-alkyl, such as methyl, ethyl, n-propyl and isopropyl, C₁-C₄-haloalkyl, in particular C₁-C₂-haloalkyl, such as fluoromethyl, difluoromethyl, trifluoromethyl, 1,1-difluoroethyl, 2-fluoroethyl, 2,2-difluoroethyl or 2,2,2-trifluoroethyl, C₁-C₄-alkoxy, such as methoxy, ethoxy, n-propoxy and isopropoxy, and C₁-C₄-haloalkoxy, in particular C₁-C₂-haloalkoxy, such as fluoromethoxy, difluoromethoxy, trifluoromethoxy, 1,1-difluoroethoxy, 2-fluoroethoxy, 2,2-difluoroethoxy or 2,2,2-trifluoroethoxy.

Particularly preferred are compounds of formula (I), wherein the moiety of the formula A is selected from the group consisting of W.Het-1, W.Het-2, W.Het-3 and W.Het-4, wherein R^{w6}, R^{w5}, R^{w4}, and R^{w3} are as defined above and in particular selected from the group consisting of hydrogen, halogen, such as fluorine or chlorine, CN, C₁-C₄-alkyl, such as methyl, ethyl, n-propyl and isopropyl, C₁-C₄-haloalkyl, in particular C₁-C₂-haloalkyl, such as fluoromethyl, difluoromethyl, trifluoromethyl, 1,1-difluoroethyl, 2-fluoroethyl, 2,2-difluoroethyl or 2,2,2-trifluoroethyl, C₁-C₄-alkoxy, such as methoxy, ethoxy, n-propoxy and isopropoxy, and C₁-C₄-haloalkoxy, in particular C₁-C₂-haloalkoxy, such as fluoromethoxy, difluoromethoxy, trifluoromethoxy, 1,1-difluoroethoxy, 2-fluoroethoxy, 2,2-difluoroethoxy or 2,2,2-trifluoroethoxy and where R^{w6}, R^{w5}, R^{w4}, and R^{w3} are especially hydrogen.

Likewise, particularly preferred are compounds of formula (I), wherein the moiety of the formula A is selected from the group consisting of W.Het-1, W.Het-5 and W.Het-9, wherein R^{w6} is as defined above and in particular selected from the group consisting of hydrogen, halogen, such as fluorine or chlorine, CN, C₁-C₄-alkyl, such as methyl, ethyl, n-propyl and isopropyl, C₁-C₄-haloalkyl, in particular C₁-C₂-haloalkyl, such as fluoromethyl, difluoromethyl, trifluoromethyl, 1,1-difluoroethyl, 2-fluoroethyl, 2,2-difluoroethyl or 2,2,2-trifluoroethyl, C₁-C₄-alkoxy, such as methoxy, ethoxy, n-propoxy and isopropoxy, and C₁-C₄-haloalkoxy, in particular C₁-C₂-haloalkoxy, such as fluoromethoxy, difluoromethoxy, trifluoromethoxy, 1,1-difluoroethoxy, 2-fluoroethoxy, 2,2-difluoroethoxy or 2,2,2-trifluoroethoxy and where R^{w6} is especially hydrogen.

Likewise, particularly preferred are compounds of formula (I), wherein the moiety of the formula A is selected from the group consisting of W.Het-2, W.Het-6 and W.Het-10, wherein R^{w5} is as defined above and in particular selected from the group consisting of hydrogen, halogen, such as fluorine or chlorine, CN, C₁-C₄-alkyl, such as methyl, ethyl, n-propyl and isopropyl, C₁-C₄-haloalkyl, in particular C₁-C₂-haloalkyl, such as fluoromethyl, difluoromethyl, trifluoromethyl, 1,1-difluoroethyl, 2-fluoroethyl, 2,2-difluoroethyl or 2,2,2-trifluoroethyl, C₁-C₄-alkoxy, such as methoxy, ethoxy, n-propoxy and isopropoxy, and C₁-C₄-haloalkoxy, in particular C₁-C₂-haloalkoxy, such as fluoromethoxy, difluoromethoxy, trifluoromethoxy, 1,1-difluoroethoxy, 2-fluoroethoxy, 2,2-difluoroethoxy or 2,2,2-trifluoroethoxy and where R^{w5} is especially hydrogen.

Likewise, particularly preferred are compounds of formula (I), wherein the moiety of the formula A is selected from the group consisting of W.Het-1, W.Het-2, W.Het-3 and W.Het-4, especially from the group consisting of W.Het-1 and W.Het-2, wherein R^{w3}, R^{w4}, R^{w5} and R^{w6}, independently of each other, are as defined above and in particular selected from the group consisting of hydrogen, halogen, such as fluorine or chlorine, CN, C₁-C₄-alkyl, such as methyl, ethyl, n-propyl and isopropyl, C₁-C₄-haloalkyl, in particular C₁-C₂-haloalkyl, such as fluoromethyl, difluoromethyl, trifluoromethyl, 1,1-difluoroethyl, 2-fluoroethyl, 2,2-difluoroethyl or 2,2,2-trifluoroethyl, C₁-C₄-alkoxy, such as methoxy, ethoxy, n-propoxy and isopropoxy, and C₁-C₄-haloalkoxy, in particular C₁-C₂-haloalkoxy, such as fluoromethoxy, difluoromethoxy, trifluoromethoxy, 1,1-difluoroethoxy, 2-fluoroethoxy, 2,2-difluoroethoxy or 2,2,2-trifluoroethoxy and where at most one of R^{w3}, R^{w4}, R^{w5} and R^{w6} are especially hydrogen.

In the moieties W.Het-1, W.Het-2, W.Het-3, W.Het-4, W.Het-5, W.Het-6, W.Het-7, W.Het-8, W.Het-9, W.Het-10, W.Het-11 and W.Het-12, the heterocycle Het is in particular selected from the group consisting of the radicals of formulae Het-1 to Het-24, as defined above, and in particular selected from the group consisting of the radicals of the formulae Het-1 or Het-1a, Het-11a and Het-24.

In the moieties W.Het-1, W.Het-2, W.Het-3, W.Het-4, W.Het-5, W.Het-6, W.Het-7, W.Het-8, W.Het-9, W.Het-10, W.Het-11 and W.Het-12, the radicals R¹ and R² are, independently from each other, in particular selected from the group consisting of hydrogen, halogen, such as fluorine or chlorine, CN, C₁-C₆-alkyl, in particular C₁-C₄-alkyl, such as methyl, ethyl, n-propyl or isopropyl, C₃-C₆-cycloalkyl, such as cyclopropyl or cyclobutyl, C₁-C₆-haloalkyl, in particular C₁-C₂-haloalkyl, such as fluoromethyl, difluoromethyl, trifluoromethyl, 1,1-difluoroethyl, 2-fluoroethyl, 2,2-difluoroethyl or 2,2,2-trifluoroethyl, or C₃-C₆-halocycloalkyl such as 1-fluorocyclopropyl or 2,2-difluorocyclopropyl, or R¹ and R² may together be =CR¹³R¹⁴ or R¹ and R² form, together with the carbon atom, which they attached to, a 3- to 5 membered saturated carbocyclic ring such as cyclopropyl, cyclobutyl or cyclopentyl.

In particular embodiments of moieties W.Het-1, W.Het-2, W.Het-3, W.Het-4, W.Het-5, W.Het-6, W.Het-7, W.Het-8, W.Het-9, W.Het-10, W.Het-11 and W.Het-12, the radicals R¹ and R² are, independently from each other, more particularly selected from the group consisting of hydrogen, halogen, cyano, C₁-C₃-alkyl, such as methyl ethyl or isopropyl, or C₁-C₃-haloalkyl such as fluoromethyl, difluoromethyl, trifluoromethyl, 1,1-difluoroethyl, 2-fluoroethyl, 2,2-difluoroethyl or 2,2,2-trifluoroethyl, where in particular at least one of the radicals R¹ and R² is hydrogen.

Especially, R¹ and R² in the moieties W.Het-1, W.Het-2, W.Het-3, W.Het-4, W.Het-5, W.Het-6, W.Het-7, W.Het-8, W.Het-9, W.Het-10, W.Het-11 and W.Het-12 are both hydrogen.

A particular group 1 of embodiments relates to compounds of the formula (I), to their stereoisomers, their tautomers and their salts, wherein the moiety of formula (A) represents a radical selected from the group consisting of W.Het-1, wherein Het is selected from the group consisting of radicals of formulae Het-1, Het-11a and Het-24.

A further particular group 2 of embodiments relates to compounds of the formula (I), to their stereoisomers, their tautomers and their salts, wherein the moiety of formula (A) represents a radical selected from the group consisting of W.Het-2, wherein Het is selected from the group consisting of radicals of formulae Het-1, Het-11a and Het-24.

A further particular group 3 of embodiments relates to compounds of the formula (I), to their stereoisomers, their tautomers and their salts, wherein the moiety of formula (A) represents a radical selected from the group consisting of W.Het-5, wherein Het is selected from the group consisting of radicals of formulae Het-1, Het-11a and Het-24.

A further particular group 4 of embodiments relates to compounds of the formula (I), to their stereoisomers, their tautomers and their salts, wherein the moiety of formula (A) represents a radical selected from the group consisting of W.Het-6, wherein Het is selected from the group consisting of radicals of formulae Het-1, Het-11a and Het-24.

A further particular group 5 of embodiments relates to compounds of the formula (I), to their stereoisomers, their tautomers and their salts, wherein the moiety of formula (A) represents a radical selected from the group consisting of W.Het-9, wherein Het is selected from the group consisting of radicals of formulae Het-1, Het-11a and Het-24.

A further particular group 6 of embodiments relates to compounds of the formula (I), to their stereoisomers, their tautomers and their salts, wherein the moiety of formula (A) represents a radical selected from the group consisting of W.Het-10, wherein Het is selected from the group consisting of radicals of formulae Het-1, Het-11a and Het-24.

A special group 1a of embodiments relates to compounds of the formula (I), to their stereoisomers, their tautomers and their salts, wherein the moiety of formula (A) represents a radical selected from the group consisting of W.Het-1, wherein Het is a radicals of formulae Het-1a.

A further special group 2a of embodiments relates to compounds of the formula (I), to their stereoisomers, their tautomers and their salts, wherein the moiety of formula (A) represents a radical selected from the group consisting of W.Het-2, wherein Het is a radicals of formulae Het-1a.

A further special group 3a of embodiments relates to compounds of the formula (I), to their stereoisomers, their tautomers and their salts, wherein the moiety of formula (A) represents a radical selected from the group consisting of W.Het-5, wherein Het is a radicals of formulae Het-1a.

A further special group 4a of embodiments relates to compounds of the formula (I), to their stereoisomers, their tautomers and their salts, wherein the moiety of formula (A) represents a radical selected from the group consisting of W.Het-6, wherein Het is a radicals of formulae Het-1a.

A further special group 5a of embodiments relates to compounds of the formula (I), to their stereoisomers, their tautomers and their salts, wherein the moiety of formula (A) represents a radical selected from the group consisting of W.Het-9, wherein Het is a radicals of formulae Het-1a.

A further special group 6a of embodiments relates to compounds of the formula (I), to their stereoisomers, their tautomers and their salts, wherein the moiety of formula (A) represents a radical selected from the group consisting of W.Het-10, wherein Het is a radicals of formulae Het-1a.

In embodiments 1, 3, 5, 1a, 3a and 5a the radical R^{w6} is as defined above and in particular selected from the group consisting of hydrogen, halogen, such as fluorine or chlorine, CN, C₁-C₄-alkyl, such as methyl, ethyl, n-propyl and isopropyl, C₁-C₄-haloalkyl, in particular C₁-C₂-haloalkyl, such as fluoromethyl, difluoromethyl, trifluoromethyl, 1,1-difluoroethyl, 2-fluoroethyl, 2,2-difluoroethyl or 2,2,2-trifluoroethyl, C₁-C₄-alkoxy, such as methoxy, ethoxy, n-propoxy and isopropoxy, and C₁-C₄-haloalkoxy, in particular C₁-C₂-haloalkoxy, such as fluoromethoxy, difluoromethoxy, trifluoromethoxy, 1,1-difluoroethoxy, 2-fluoroethoxy, 2,2-difluoroethoxy or 2,2,2-trifluoroethoxy. In embodiments 1, 3, 5, 1a, 3a and 5a the radical R^{w6} is especially hydrogen.

In embodiments 2, 4, 6, 2a, 4a and 6a the radical R^{w5} is as defined above and in particular selected from the group consisting of hydrogen, halogen, such as fluorine or chlorine, CN, C₁-C₄-alkyl, such as methyl, ethyl, n-propyl and isopropyl, C₁-C₄-haloalkyl, in particular C₁-C₂-haloalkyl, such as fluoromethyl, difluoromethyl, trifluoromethyl, 1,1-difluoroethyl, 2-fluoroethyl, 2,2-difluoroethyl or 2,2,2-trifluoroethyl, C₁-C₄-alkoxy, such as methoxy, ethoxy, n-propoxy and isopropoxy, and C₁-C₄-haloalkoxy, in particular C₁-C₂-haloalkoxy, such as fluoromethoxy, difluoromethoxy, trifluoromethoxy, 1,1-difluoroethoxy, 2-fluoroethoxy, 2,2-difluoroethoxy or 2,2,2-trifluoroethoxy. In embodiments 2, 4, 6, 2a, 4a and 6a the radical R^{w5} is especially hydrogen.

In embodiments 1, 2, 3, 4, 5, 6, 1a, 2a, 3a, 4a, 5a and 6a the radicals R¹ and R² are, independently from each other, in particular selected from the group consisting of hydrogen, halogen, such as fluorine or chlorine, CN, C₁-C₆-alkyl, in particular C₁-C₄-alkyl, such as methyl, ethyl, n-propyl or isopropyl, C₃-C₆-cycloalkyl, such as cyclopropyl or cyclobutyl, C₁-C₆-haloalkyl, in particular C₁-C₂-haloalkyl, such as fluoromethyl, difluoromethyl, trifluoromethyl, 1,1-difluoroethyl, 2-fluoroethyl, 2,2-difluoroethyl or 2,2,2-trifluoroethyl, or C₃-C₆-halocycloalkyl such as 1-fluorocyclopropyl or 2,2-difluorocyclopropyl, or R¹ and R² may together be =CR¹³R¹⁴ or R¹ and R² form, together with the carbon atom which they attached to, a 3- to 5 membered saturated carbocyclic ring such as cyclopropyl, cyclobutyl or cyclopentyl.

In embodiments 1, 2, 3, 4, 5, 6, 1a, 2a, 3a, 4a, 5a and 6a the radicals R¹ and R² are, independently from each other, more particularly selected from the group consisting of hydrogen, halogen, cyano, C₁-C₃-alkyl, such as methyl ethyl or isopropyl, or C₁-C₃-haloalkyl such as fluoromethyl, difluoromethyl, trifluoromethyl, 1,1-difluoroethyl, 2-fluoroethyl, 2,2-difluoroethyl or 2,2,2-trifluoroethyl, where in particular at least one of the radicals R¹ and R² is hydrogen and where especially both R¹ and R² are hydrogen or R¹ is methyl and R² is hydrogen.

In the compounds of formula (I), where the moiety of formula (A) is selected from the moieties of formulae W.Het-1, W.Het-2, W.Het-3, W.Het-4, W.Het-5, W.Het-6, W.Het-7, W.Het-8, W.Het-9, W.Het-10, W.Het-11 and W.Het-12 and likewise in the embodiments 1, 2, 3, 4, 5, 6, 1a, 2a, 3a, 4a, 5a and 6a, the variables m, R¹, R², R³, R^{4a}, R^{4b} and R⁵ are as defined above and in particular have the preferred meanings.

In the compounds of formula (I), where the moiety of formula (A) is selected from the moieties of formulae W.Het-1, W.Het-2, W.Het-3, W.Het-4, W.Het-5, W.Het-6, W.Het-7, W.Het-8, W.Het-9, W.Het-10, W.Het-11 and W.Het-12 and likewise in the embodiments 1, 2, 3, 4, 5, 6, 1a, 2a, 3a, 4a, 5a and 6a, the variables m, R¹, R², R³, R^{4a}, R^{4b} and R⁵, independently of each other or in particular in combination, in particular have the following meanings:
- m: is 0 or 1;
- R¹ and R²: are, independently from each other, selected from the group consisting of hydrogen, halogen, such as fluorine or chlorine, CN, C₁-C₆-alkyl, in particular C₁-C₄-alkyl, such as methyl, ethyl, n-propyl or isopropyl, C₃-C₆-cycloalkyl, such as cyclopropyl or cyclobutyl, C₁-C₆-haloalkyl, in particular C₁-C₂-haloalkyl, such as fluoromethyl, difluoromethyl, trifluoromethyl, 1,1-difluoroethyl, 2-fluoroethyl, 2,2-difluoroethyl or 2,2,2-trifluoroethyl, or C₃-C₆-halocycloalkyl such as 1-fluorocyclopropyl or 2,2-difluorocyclopropyl, or R¹ and R² form, together with the carbon atom which they attached to, a 3- to 5 membered saturated carbocyclic ring such as cyclopropyl, cyclobutyl or cyclopentyl;
- R³: is selected from the group consisting of hydrogen, halogen, NO₂, C₁-C₆-alkyl, in particular C₁-C₄-alkyl, C₁-C₆-haloalkyl, in particular C₁-C₄-haloalkyl, C₂-C₆-alkenyl, in particular C₂-C₄-alkenyl, such as ethenyl, 2-propenyl, isopropenyl, n-butenyl, 2-butenyl or isobutenyl, C₂-C₆-haloalkenyl, in particular C₂-C₄-haloalkenyl, such as 1,1-difluoroethenyl, 2-fluoroethenyl, 2,2-difluoroethenyl or 2,2,2-trifluoroethenyl, C₃-C₆-cycloalkyl, such as cyclopropyl or cyclobutyl, C₃-C₆-cycloalkenyl, such as cyclobutenyl, C₃-C₆-halocycloalkyl, such as 1-fluorocyclopropyl, 2-fluorocyclopropyl, 2,2-difluorocyclopropyl, 2-chlorocyclopropyl, 2,2-dichlorocyclopropyl, 1-fluorocyclobutyl, 2-fluorocyclobutyl, 3-fluorocyclobutyl, 2,2-difluorocyclobutyl or 3,3-difluorocyclobutyl, NR^{9a}R^{9b} and NR^{9a}-C(=O)R^{7a}, where R^{7a}, R^{9a} and R^{9b} are as defined above and in particular have the preferred meanings;
- R⁵: is selected from the group consisting of hydrogen, halogen, CN, NR^{9a}R^{9b}, C₁-C₆-alkyl, in particular C₁-C₄-alkyl, C₁-C₆-haloalkyl, in particular C₁-C₄-haloalkyl, C₂-C₆-alkenyl, in particular C₂-C₄-alkenyl, such as ethenyl, 2-propenyl, isopropenyl, n-butenyl, 2-butenyl or isobutenyl, C₂-C₆-haloalkenyl, in particular C₂-C₄-haloalkenyl, such as 1,1-difluoroethenyl, 2-fluoroethenyl, 2,2-difluoroethenyl or 2,2,2-trifluoroethenyl, C₁-C₆-alkoxy, in particular C₁-C₄-alkoxy, C₁-C₆-haloalkoxy, in particular C₁-C₄-haloalkoxy, C₃-C₆-cycloalkyl, C₃-C₆-halocycloalkyl, C₃-C₆-halocycloalkyl, C₃-C₆-cycloalkoxy or C₃-C₆-halocycloalkoxy, C(=O)OR⁸, C(=O)NR^{9a}R^{9b}, C(=S)NR^{9a}R^{9b}, C(=O)R^{7a}, C(=S)R^{7a}, Q-phenyl, where phenyl is unsubstituted or substituted with 1, 2, 3, 4 or 5 identical or different substituents R¹⁰, or Q-Het^{#}, where Het^{#} is unsubstituted or substituted with 1, 2, 3, 4 or 5 identical or different substituents R¹⁰,
and where Q, irrespectively of its occurrence, is a bond, O, OCH₂, NR^{9a}, such as NH or N(CH₃),
NR^{9a}-C(=O), such as NH-C(=O), N(CH₃)-C(=O), N(CH₂CH₃)-C(=O) or N(CH(CH₃)₂)-C(=O), OC(=O), NR^{9a}CH₂, such as NHCH₂ or N(CH₃)CH₂, OC(=O)CH₂, or NR^{9a}C(=O)CH₂ such as NHC(=O)CH₂, N(H)-C(=O)CH₃, N(H)-C(=O)CH₂CH₃ or N(H)-C(=O)CH(CH₃)₂,
where R^{7a}, R^{9a}, R^{9b} and R¹⁰ are as defined above and in particular have the preferred meanings;
and if m =1
- R^{4a} and R^{4b}: are selected, independently from one another, from the group consisting of hydrogen, C₁-C₄-alkyl, in particular methyl, and halogen, in particular fluorine or chlorine, or R^{4a} and R^{4b} together are =O, or R^{4a} together with R³ may also form both together and together with the existing bond a double bond between the adjacent carbon atoms.

In the compounds of formula (I), where the moiety of formula (A) is selected from the moieties of formulae W.Het-1, W.Het-2, W.Het-3, W.Het-4, W.Het-5, W.Het-6, W.Het-7, W.Het-8, W.Het-9, W.Het-10, W.Het-11 and W.Het-12 and likewise in the embodiments 1, 2, 3, 4, 5, 6, 1a, 2a, 3a, 4a, 5a and 6a, the variables m, R¹, R², R³, R^{4a}, R^{4b} and R⁵, independently of each other or in particular in combination, more particularly have the following meanings:
- R¹ and R²: are, independently from each other, selected from the group consisting of hydrogen, halogen, cyano, C₁-C₃-alkyl, such as methyl, ethyl n-propyl, isopropyl or n-butyl, or C₁-C₃-haloalkyl such as fluoromethyl, difluoromethyl, trifluoromethyl, 1,1-difluoroethyl, 2-fluoroethyl, 2,2-difluoroethyl or 2,2,2-trifluoroethyl, where in particular at least one of the radicals R¹ and R² is hydrogen, and wherein especially both R¹ and R² are hydrogen or R¹ is methyl and R² is hydrogen;
- R³: is selected from the group consisting of hydrogen, fluorine, chlorine, C₁-C₄-alkyl, such as methyl, ethyl, n-propyl, isopropyl or n-butyl, and C₂-C₆-alkenyl, in particular C₂-C₄-alkenyl, such as ethenyl, 2-propenyl, isopropenyl, n-butenyl, 2-butenyl or isobutenyl;
- R⁵: is selected from the group consisting of hydrogen, halogen, such as fluorine or chlorine, CN, C₁-C₆-alkyl, in particular C₁-C₄-alkyl, such as methyl, ethyl, n-propyl, isopropyl, n-butyl, 2-butyl or isobutyl, C₁-C₆-haloalkyl, in particular C₁-C₄-haloalkyl, such as fluoromethyl, difluoromethyl, trifluoromethyl, 1,1-difluoroethyl, 2-fluoroethyl, 2,2-difluoroethyl or 2,2,2-trifluoroethyl, C₂-C₆-alkenyl, in particular C₂-C₄-alkenyl, such as ethenyl, 2-propenyl, isopropenyl, n-butenyl, 2-butenyl or isobutenyl, C₂-C₆-haloalkenyl, in particular C₂-C₄-haloalkenyl, such as 1,1-difluoroethenyl, 2-fluoroethenyl, 2,2-difluoroethenyl or 2,2,2-trifluoroethenyl, C₁-C₆-alkoxy, in particular C₁-C₄-alkoxy, such as methoxy, ethoxy, n-propoxy, isopropoxy, n-butoxy, 2-butoxy or isobutoxy, C₁-C₆-haloalkoxy, in particular C₁-C₄-haloalkoxy, such as fluoromethoxy, difluoromethoxy, trifluoromethoxy, 1,1-difluoroethoxy, 2-fluoroethoxy, 2,2-difluoroethoxy or 2,2,2-trifluoroethoxy, C₃-C₆-cycloalkyl, such as cyclopropyl, cyclobutyl, cyclopentyl or cyclohexyl, C₃-C₆-halocycloalkyl, such as 1-fluorocyclopropyl, 2-fluorocyclopropyl, 2,2-difluorocyclopropyl, 2-chlorocyclopropyl, 2,2-dichlorocyclopropyl, 1-fluorocyclobutyl, 2-fluorocyclobutyl, 3-fluorocyclobutyl, 2,2-difluorocyclobutyl or 3,3-difluorocyclobutyl, C₃-C₆-halocycloalkyl such as cyclopropoxy, cyclobutoxy, cyclopentoxy or cyclohexoxy, and C₃-C₆-halocycloalkoxy, such as 1-fluorocyclopropoxy, 2-fluorocyclopropoxy, 2,2-difluorocyclopropoxy, 2-chlorocyclopropoxy, 2,2-dichlorocyclopropoxy, 1-fluorocyclobutoxy, 2-fluorocyclobutoxy, 3-fluorocyclobutoxy, 2,2-difluorocyclobutoxy or 3,3-difluorocyclobutoxy;
and if m =1
- R^{4a} and R^{4b}: are selected, independently from one another, from the group consisting of hydrogen and methyl or R^{4a} and R^{4b} together are =O, or R^{4a} together with R³ may also form both together and together with the existing bond a double bond between the adjacent carbon atoms.

In the compounds of formula (I), where the moiety of formula (A) is selected from the moieties of formulae W.Het-1, W.Het-2, W.Het-3, W.Het-4, W.Het-5, W.Het-6, W.Het-7, W.Het-8, W.Het-9, W.Het-10, W.Het-11 and W.Het-12 and likewise in the embodiments 1, 2, 3, 4, 5, 6, 1a, 2a, 3a, 4a, 5a and 6a, where m = 0, the variables R¹, R², R³, and R⁵, independently of each other or in particular in combination, in particular may also have the following meanings:
R¹ and R² are, independently from each other, selected from the group consisting of hydrogen, halogen, cyano, C₁-C₃-alkyl, such as methyl ethyl or isopropyl, or C₁-C₃-haloalkyl such as fluoromethyl, difluoromethyl, trifluoromethyl, 1,1-difluoroethyl, 2-fluoroethyl, 2,2-difluoroethyl or 2,2,2-trifluoroethyl, where in particular at least one of the radicals R¹ and R² is hydrogen, and wherein especially both R¹ and R² are hydrogen or R¹ is methyl and R² is hydrogen; and
R³ and R⁵ are, independently from each other, selected from hydrogen, halogen, such as fluorine or chlorine, and wherein in particular each of R³ and R⁵ is fluorine.

In the compounds of formula (I), where the moiety of formula (A) is selected from the moieties of formulae W.Het-1, W.Het-2, W.Het-3, W.Het-4, W.Het-5, W.Het-6, W.Het-7, W.Het-8, W.Het-9, W.Het-10, W.Het-11 and W.Het-12 and likewise in the embodiments 1, 2, 3, 4, 5, 6, 1a, 2a, 3a, 4a, 5a and 6a, where m = 0, the variables R¹, R², R³, and R⁵, independently of each other or in particular in combination, in particular may also have the following meanings:
- R¹ and R²: are, independently from each other, selected from the group consisting of hydrogen, halogen, such as fluorine or chlorine, CN, C₁-C₆-alkyl, in particular C₁-C₄-alkyl, such as methyl, ethyl, n-propyl or isopropyl, C₃-C₆-cycloalkyl, such as cyclopropyl or cyclobutyl, C₁-C₆-haloalkyl, in particular C₁-C₂-haloalkyl, such as fluoromethyl, difluoromethyl, trifluoromethyl, 1,1-difluoroethyl, 2-fluoroethyl, 2,2-difluoroethyl or 2,2,2-trifluoroethyl, or C₃-C₆-halocycloalkyl such as 1-fluorocyclopropyl or 2,2-difluorocyclopropyl, or R¹ and R² form, together with the carbon atom, which they attached to, a 3- to 5 membered saturated carbocyclic ring such as cyclopropyl, cyclobutyl or cyclopentyl;
- R³ and R⁵: together with the carbon atom, to which they are bound, form a 3-, 4-, 5- or 6-membered saturated carbocyle, a 4-, 5- or 6-membered unsaturated carbocyle, an epoxide ring or an oxetane ring, wherein each of the carbon atoms of the saturated or unsaturated carbocycle may be unsubstituted or may carry 1 or 2 identical or different radicals R^{7b}, wherein R^{7b} is as defined above and wherein
- R^{7b}: is in particular selected from the group consisting of halogen, C₁-C₆-alkyl, C₁-C₆-alkoxy, and C₂-C₆-alkynyl, and wherein the carbon atoms of the aforementioned aliphatic radicals may optionally be partly or completely halogenated, and wherein one or two identical or different radicals R^{7b} may also be
C₃-C₆-cycloalkyl, which is unsubstituted or carries 1, 2, 3, 4 or 5 identical or different radicals selected from fluorine, chlorine or methyl,
phenyl, optionally substituted with 1, 2, 3, 4 or 5 identical or different substituents R^{10a}, or a
a 3-, 4-, 5-, 6- or 7- membered saturated, partly saturated or unsaturated aromatic heterocyclic ring comprising 1, 2 or 3 heteroatoms as ring members, which are identical or different and selected from oxygen, nitrogen and sulfur, where the heterocyclic ring is optionally substituted with 1, 2, 3 or 4 identical or different substituents R^{10a}, and wherein the nitrogen and/or the sulfur atom(s) of the heterocyclic ring may optionally be oxidized, in particular a 5- or 6-membered hetaryl having 1 or 2 heteroatoms selected from O, S and N, where the 5- or 6-membered hetaryl is unsubstituted or carries 1, 2 or 3 identical or different substituents R^{10a}, or two of R^{7b} present on one ring carbon may together form =O or =S, or two R^{7b} together form a linear C₂-C₇ alkylene chain, thus forming, together with the carbon atom to which they are bound, a 3-, 4-, 5- or 6-membered spiro ring, wherein 1 or 2 CH₂ moieties of the alkylene chain may be replaced by 1 or 2 heteroatom moieties independently selected from O and S, and wherein the alkylene chain is unsubstituted or may be substituted with 1, 2, 3, 4, 5 or 6 radicals selected from the group consisting of halogen, C₁-C₄-alkyl, C₁-C₄-haloalkyl, C₁-C₄-alkoxy and C₁-C₄-haloalkoxy, where
- R^{10a}: has one of the meanings give for R¹⁰ and where R^{10a} is in particular selected from the group consisting of halogen, C₁-C₄-alkyl, C₁-C₄-haloalkyl, C₁-C₄-alkoxy and C₁-C₄-haloalkoxy.

In the compounds of formula (I), where the moiety of formula (A) is selected from the moieties of formulae W.Het-1, W.Het-2, W.Het-3, W.Het-4, W.Het-5, W.Het-6, W.Het-7, W.Het-8, W.Het-9, W.Het-10, W.Het-11 and W.Het-12 and likewise in the embodiments 1, 2, 3, 4, 5, 6, 1a, 2a, 3a, 4a, 5a and 6a, where m = 0, the variables R¹, R², R³, and R⁵, independently of each other or in particular in combination, more particular may also have the following meanings:
- R¹ and R²: are, independently from each other, selected from the group consisting of hydrogen, halogen, cyano, C₁-C₃-alkyl, such as methyl ethyl or isopropyl, or C₁-C₃-haloalkyl such as fluoromethyl, difluoromethyl, trifluoromethyl, 1,1-difluoroethyl, 2-fluoroethyl, 2,2-difluoroethyl or 2,2,2-trifluoroethyl, where in particular at least one of the radicals R¹ and R² is hydrogen and where especially both R¹ and R² are hydrogen or R¹ is methyl and R² is hydrogen;
- R³ and R⁵: together with the carbon atom, to which they are bound, form a 3- or 4-membered saturated carbocyle, i.e. a 1,1-cyclopropylidene or 1,1-cyclobutylidene radical, or a 5-membered monounsaturated carbocycle, such as such as an 1,1-cyclopent-3-enylidene radical, wherein each of the carbon atoms of the saturated or unsaturated carbocycle may be unsubstituted or may carry 1, 2, 3, 4, 5 or 6 identical or different radicals R^{7b}, wherein
- R^{7b}: is as defined above and in particular selected from the group consisting of halogen, C₁-C₄-alkyl, and wherein one radical R^{7b} may also be
phenyl, optionally substituted with 1, 2, 3, 4 or 5, in particular 1, 2 or 3, identical or different substituents R^{10a}, wherein
- R^{10a} is: as defined above, and in particular selected from the group consisting of halogen, such as fluorine or chlorine, C₁-C₄-alkyl, CN, C₁-C₄-haloalkyl, C₁-C₄-alkoxy and C₁-C₄-haloalkoxy.

In the compounds of formula (I), where the moiety of formula (A) is selected from the moieties of formulae W.Het-1, W.Het-2, W.Het-3, W.Het-4, W.Het-5, W.Het-6, W.Het-7, W.Het-8, W.Het-9, W.Het-10, W.Het-11 and W.Het-12 and likewise in the embodiments 1, 2, 3, 4, 5, 6, 1a, 2a, 3a, 4a, 5a and 6a, where m = 0, the variables R¹, R², R³, and R⁵, independently of each other or in particular in combination, especially may also have the following meanings:
- R¹ and R²: are hydrogen;
- R³ and R⁵: together form a moiety selected from the group consisting of:
CH₂-CH₂, CHF-CH₂, CF₂-CH₂, CHF-CHF, CF₂-CHF, CF₂-CF₂, CHCl-CH₂, CCl₂-CH₂, CHCl-CHCl, CCl₂-CHCl, CCl₂-CCl₂, CH(CH₃)-CH₂, C(CH₃)₂-CH₂, CH(CH₃)-CH(CH₃), C(CH₃)₂-CH(CH₃), C(CH₃)₂-C(CH₃)₂, CH(OCH₃)-CH₂, CH(CH₂CH₃)-CH₂, CH(CH₂CH₂CH₃)-CH₂, CH(CH₂CH₂CH₂CH₃)-CH₂, CH(CH₃)-CH₂-CH₂, CH₂-CH(CH₃)-CH₂, C(CH₃)₂-CH₂-CH₂, CH₂-C(CH₃)₂-CH₂, CH(CH₃)-CH(CH₃)-CH₂, CH(CH₃)-CH₂-CH(CH₃), CH(CH₃)-CH(CH₃)-CH(CH₃), C(CH₃)₂-CH(CH₃)-CH₂, CH(CH₃)-C(CH₃)₂-CH₂, C(CH₃)₂-CH₂-C(CH₃)₂, C(CH₃)₂-C(CH₃)₂-CH₂, C(CH₃)₂-C(CH₃)₂-C(CH₃)₂, CH₂-CH₂-CH₂, CHF-CH₂-CH₂, CH₂-CHF-CH₂, CF₂-CH₂-CH₂, CH₂-CF₂-CH₂, CHF-CHF-CH₂, CHF-CH₂-CHF, CHF-CHF-CHF, CF₂-CHF-CH₂, CHF-CF₂-CH₂, CF₂-CH₂-CF₂, CF₂-CF₂-CH₂, CF₂-CF₂-CF₂, CHCl-CH₂-CH₂, CH₂-CHCl-CH₂, CCl₂-CH₂-CH₂, CH₂-CCl₂-CH₂, CHCl-CHCl-CH₂, CHCl-CH₂-CHCl, CHCl-CHCl-CHCl, CCl₂-CHCl-CH₂, CHCl-CCl₂-CH₂, CCl₂-CH₂-CCl₂, CCl₂-CCl₂-CH₂, CCl₂-CCl₂-CCl₂, CH₂O, CH(CH₃)-O, (CH₃)₂-O, CH₂-O-CH₂, CH₂-CH₂-O, or a radical CH(Ar)-CH₂, where Ar is selected from phenyl, 2-chlorophenyl, 2-fluorophenyl, 2-methylphenyl, 2-methoxyphenyl, 2-cyanophenyl, 3-chlorophenyl, 3-fluorophenyl, 3-methylphenyl, 3-methoxyphenyl, 3-cyanophenyl, 4-chlorophenyl, 4-fluorophenyl, 4-methylphenyl, 4-methoxyphenyl and 4-cyanophenyl,
thereby forming, together with the carbon atom they are bound to, an unsubstituted or substituted 1,1-cyclopropylidene or 1,1-cyclobutylidene radical.

In the compounds of formula (I), in particular in those compounds of formula (I) where the moiety of formula (A) is selected from the moieties of formulae W.Het-1, W.Het-2, W.Het-3, W.Het-4, W.Het-5, W.Het-6, W.Het-7, W.Het-8, W.Het-9, W.Het-10, W.Het-11 and W.Het-12 and likewise in the embodiments 1, 2, 3, 4, 5, 6, 1a, 2a, 3a, 4a, 5a and 6a, the variable X is in particular O.

In the compounds of formula (I), where the moiety of formula (A) is selected from the moieties of formulae W.Het-1, W.Het-2, W.Het-3, W.Het-4, W.Het-5, W.Het-6, W.Het-7, W.Het-8, W.Het-9, W.Het-10, W.Het-11 and W.Het-12 and likewise in the embodiments 1, 2, 3, 4, 5, 6, 1a, 2a, 3a, 4a, 5a and 6a, wherein R¹, R², R^{4b} and R⁵ are as defined herein, and specifically, individually or in combination, have the meanings given as preferred meanings,
m = 1, and
R³ and R^{4a} form both together and together with the existing bond a double bond between the adjacent carbon atoms.

In the compounds of formula (I), where the moiety of formula (A) is selected from the moieties of formulae W.Het-1, W.Het-2, W.Het-3, W.Het-4, W.Het-5, W.Het-6, W.Het-7, W.Het-8, W.Het-9, W.Het-10, W.Het-11 and W.Het-12 and likewise in the embodiments 1, 2, 3, 4, 5, 6, 1a, 2a, 3a, 4a, 5a and 6a, wherein R¹, R², R^{4b} and R⁵ are as defined herein, and specifically, individually or in combination, have the meanings given as preferred meanings,
m is 1, and
R^{4a} and R⁵ form together and with the carbon atom they are bound to a 3-, 4-, 5- or 6-membered saturated or partially unsaturated carbocycle or heterocycle,
wherein each of the carbon atoms of the carbocycle or heterocycle may be unsubstituted or may carry 1 or 2 identical or different radicals R^{7b}, and wherein the heterocylce has 1 or 2 non-adjacent identical or different heteroatoms or heteroatom moieties as ring members, which are selected from O, S, N, NH and N-R^{17c}.

Apart from that, the variables Het^{#}, Q, Q¹, R^{v}, R^{w}, R^{y}, R⁶, R⁷, R^{7a}, R⁸, R^{8a}, R⁹, R^{9a}, R^{9b}, R^{9c}, R^{9d}, R¹⁰, R¹¹, R¹², R¹³, R¹⁴, R¹⁵, R¹⁶, R¹⁷, R^{17a}, R^{17b} and R^{17c}, irrespectively of their occurrence, in particular have the following meanings, if not stated otherwise:
Het^{#} irrespectively of its occurrence, is 5- or 6-membered hetaryl such as pyridyl, thienyl, furyl, pyrrolyl, thiazolyl, isothiazolyl, pyrazolyl, imidazolyl, oxazolyl or isoxazolyl, which is unsubstituted or substituted by 1, 2 or 3 radicals R¹⁰.
Q is, irrespectively of its occurrence, selected from a single bond, NR^{9a}, CH₂, and NR^{9a}CH₂, and it is in particular a single bond, NH, N(C₁-C₄-alkyl), CH₂, NHCH₂ or N(C₁-C₄-alkyl)CH₂.
Q¹ is, irrespectively of its occurrence, a single bond, NR^{9a}, CH₂ or NR^{9a}CH₂, wherein the heteroatom in the last moiety is bound to the carbon atom of CR^{4a}R^{4b}, and it is in particular a single bond, NH, CH₂, N(C₁-C₄-alkyl), NHCH₂ or N(C₁-C₄-alkyl)CH₂, wherein the heteroatom in the last moiety is bound to the carbon atom of CR^{4a}R^{4b}.
R^{v} is hydrogen.
R^{w} irrespectively of its occurrence, is selected from the group consisting of hydrogen, halogen, such as fluorine or chlorine, CN, C₁-C₄-alkyl, such as methyl, ethyl, n-propyl and isopropyl, C₁-C₄-haloalkyl, in particular C₁-C₂-haloalkyl, such as fluoromethyl, difluoromethyl, trifluoromethyl, 1,1-difluoroethyl, 2-fluoroethyl, 2,2-difluoroethyl or 2,2,2-trifluoroethyl, C₁-C₄-alkoxy, such as methoxy, ethoxy, n-propoxy and isopropoxy, and C₁-C₄-haloalkoxy, in particular C₁-C₂-haloalkoxy, such as fluoromethoxy, difluoromethoxy, trifluoromethoxy, 1,1-difluoroethoxy, 2-fluoroethoxy, 2,2-difluoroethoxy or 2,2,2-trifluoroethoxy or two R^{w}, which are bound to an adjacent carbon atom, form together and together with the existing bound a C=C-double bond. R^{w} is more particularly hydrogen, chlorine, fluorine or methyl and especially hydrogen.
R^{y} is independently selected from the group consisting of
hydrogen, halogen, such as fluorine or chlorine, cyano, -S(O)ₙR¹⁹, S(O)ₙNR^{20a}R^{20b}, NR^{20a}R^{20b}, C(=O)R¹⁸, C(=O)OR¹⁹, -C(=NR^{20a})R¹⁸, C(=O)NR^{20a}R^{20b}, C(=S)NR^{20a}R^{20b},
phenyl, optionally substituted with halogen, cyano, nitro, C₁-C₆-alkyl, C₁-C₆-haloalkyl, C₁-C₆-alkoxy or C₁-C₆-haloalkoxy, C₁-C₄ alkyl and C₁-C₄ alkoxy, wherein both of the last two mentioned aliphatic radicals may be unsubstituted, partly or completely halogenated,
   or
two R^{y} on adjacent carbon atoms may be a bridge selected from CH=CH-CH=CH and CH=CHCH₂, and form together with the carbon atoms to which the two R^{y} are bonded to a fused 5-membered or 6-membered aromatic carbocyclic ring, wherein the ring may optionally be substituted with one or two substituents selected from halogen, cyano, halomethyl and halomethoxy.
R⁶ irrespectively of its occurrence, is selected from the group consisting of halogen, such as chlorine or fluorine, C₁-C₄-alkyl, such as methyl or ethyl, C₁-C₄-alkoxy, such as methoxy or ethoxy, C₁-C₄-haloalkoxy, such as difluoromethoxy or trifluormethoxy, and C₁-C₄-haloalkyl, such as difluoromethyl, trifluoromethyl, 2,2-difluoroethyl, 2,2,2-trifluoroethyl or pentafluoroethyl, more preferably from halogen, C₁-C₄-alkyl and C₁-C₄-haloalkyl, even more preferably from fluorine, chlorine, C₁-C₂-alkyl, such as methyl or ethyl and C₁-C₂-haloalkyl such as difluoromethyl, trifluoromethyl, 2,2-difluoroethyl, 2,2,2-trifluoroethyl or pentafluoroethyl.
R⁷ irrespectively of its occurrence, is selected from the group consisting of CN, C₁-C₄-alkoxy, such as methoxy or ethoxy, C₁-C₄-haloalkoxy, such as difluoromethoxy or trifluormethoxy, such as difluoromethyl, trifluoromethyl, 2,2-difluoroethyl, 2,2,2-trifluoroethyl or pentafluoroethyl, more preferably from halogen, C₁-C₄-alkyl and C₁-C₄-haloalkyl, even more preferably from fluorine, chlorine, C₁-C₂-alkyl, such as methyl or ethyl and C₁-C₂-haloalkyl such as difluoromethyl, trifluoromethyl, 2,2-difluoroethyl, 2,2,2-trifluoroethyl or pentafluoroethyl, C₃-C₆-cycloalkyl such as cyclopropyl, cyclobutyl or cyclopropyl, and C₃-C₆-halocycloalkyl.
R^{7a} irrespectively of its occurrence, is selected from the group consisting of hydrogen, C₁-C₄-alkyl, C₁-C₄-haloalkyl, C₃-C₆-cycloalkyl, C₃-C₆-cycloalkyl- C₁-C₄-alkyl, phenyl and benzyl, where the phenyl ring in the last two radicals is unsubstitued or substituted by 1, 2 or 3 identical or different radicals selected from the group consisting of halogen, such as chlorine or fluorine, CN, C₁-C₄-alkyl, such as methyl, ethyl, n-propyl and isopropyl, C₁-C₄-haloalkyl, in particular C₁-C₂-haloalkyl, such as fluoromethyl, difluoromethyl, trifluoromethyl, 1,1-difluoroethyl, 2-fluoroethyl, 2,2-difluoroethyl or 2,2,2-trifluoroethyl, C₁-C₄-alkoxy, such as methoxy, ethoxy, n-propoxy and isopropoxy, and C₁-C₄-haloalkoxy, in particular C₁-C₂-haloalkoxy, such as fluoromethoxy, difluoromethoxy, trifluoromethoxy, 1,1-difluoroethoxy, 2-fluoroethoxy, 2,2-difluoroethoxy or 2,2,2-trifluoroethoxy.
R⁸ irrespectively of its occurrence, is selected from the group consisting of C₁-C₄-alkyl, C₁-C₄-haloalkyl, C₃-C₆-cycloalkyl, C₃-C₆-cycloalkyl-C₁-C₄-alkyl, phenyl and benzyl, where the phenyl ring in the last two radicals is unsubstitued or substituted by 1, 2 or 3 identical or different radicals selected from the group consisting of halogen, such as chlorine or fluorine, CN, C₁-C₄-alkyl, such as methyl, ethyl, n-propyl and isopropyl, C₁-C₄-haloalkyl, in particular C₁-C₂-haloalkyl, such as fluoromethyl, difluoromethyl, trifluoromethyl, 1,1-difluoroethyl, 2-fluoroethyl, 2,2-difluoroethyl or 2,2,2-trifluoroethyl, C₁-C₄-alkoxy, such as methoxy, ethoxy, n-propoxy and isopropoxy, and C₁-C₄-haloalkoxy, in particular C₁-C₂-haloalkoxy, such as fluoromethoxy, difluoromethoxy, trifluoromethoxy, 1,1-difluoroethoxy, 2-fluoroethoxy, 2,2-difluoroethoxy or 2,2,2-trifluoroethoxy.
R^{8a} irrespectively of its occurrence, is selected from the group consisting of C₁-C₄-alkyl, C₁-C₄-haloalkyl, C₃-C₆-cycloalkyl, C₃-C₆-cycloalkyl-C₁-C₄-alkyl, phenyl and benzyl, where the phenyl ring in the last two radicals is unsubstitued or substituted by 1, 2 or 3 identical or different radicals selected from the group consisting of halogen, such as chlorine or fluorine, CN, C₁-C₄-alkyl, such as methyl, ethyl, n-propyl and isopropyl, C₁-C₄-haloalkyl, in particular C₁-C₂-haloalkyl, such as fluoromethyl, difluoromethyl, trifluoromethyl, 1,1-difluoroethyl, 2-fluoroethyl, 2,2-difluoroethyl or 2,2,2-trifluoroethyl, C₁-C₄-alkoxy, such as methoxy, ethoxy, n-propoxy and isopropoxy, and C₁-C₄-haloalkoxy, in particular C₁-C₂-haloalkoxy, such as fluoromethoxy, difluoromethoxy, trifluoromethoxy, 1,1-difluoroethoxy, 2-fluoroethoxy, 2,2-difluoroethoxy or 2,2,2-trifluoroethoxy.
R⁹ irrespectively of its occurrence, is selected from the group consisting of C₁-C₄-alkyl, C₁-C₄-haloalkyl, C₃-C₆-cycloalkyl, C₃-C₆-cycloalkyl-C₁-C₄-alkyl, phenyl and benzyl, where the phenyl ring in the last two radicals is unsubstitued or substituted by 1, 2 or 3 identical or different radicals selected from the group consisting of halogen, such as chlorine or fluorine, CN, C₁-C₄-alkyl, such as methyl, ethyl, n-propyl and isopropyl, C₁-C₄-haloalkyl, in particular C₁-C₂-haloalkyl, such as fluoromethyl, difluoromethyl, trifluoromethyl, 1,1-difluoroethyl, 2-fluoroethyl, 2,2-difluoroethyl or 2,2,2-trifluoroethyl, C₁-C₄-alkoxy, such as methoxy, ethoxy, n-propoxy and isopropoxy, and C₁-C₄-haloalkoxy, in particular C₁-C₂-haloalkoxy, such as fluoromethoxy, difluoromethoxy, trifluoromethoxy, 1,1-difluoroethoxy, 2-fluoroethoxy, 2,2-difluoroethoxy or 2,2,2-trifluoroethoxy.
R^{9a} and R^{9b} irrespectively of their occurrence, are preferably selected from the group consisting of hydrogen, C₁-C₄-alkyl, such as methyl, ethyl, n-propyl, isopropyl, n-butyl, 2-butyl or isobutyl, and C₁-C₄-haloalkyl, such as fluoromethyl, difluoromethyl, trifluoromethyl, 1,1-difluoroethyl, 2-fluoroethyl, 2,2-difluoroethyl or 2,2,2-trifluoroethyl, or NR^{9a}R^{9b} may also be a saturated N-bound 3-, 4-, 5- or 6-membered heterocycle, which in addition to the nitrogen atom may have 1 further heteroatom as ring members, which is selected from O and N and where the N-bound 3-, 4-, 5- or 6-membered heterocycle may be unsubstituted or carry 1, 2, 3 or 4 radicals selected from C₁-C₄-alkyl and C₁-C₄-haloalkyl. Examples of such radicals NR^{9a}R^{9b} include, but are not limited to methylamino, ethylamino, n-propylamino, isopropylamino, n-butylamino, 2-butylamino, isobutylamino, dimethylamino, diethylamino, di-n-propylamino, di-n-butylamino, N-methyl-N-ethylamino, N-methyl-N-propylamino, N-methyl-N-n-propylamino, N-methyl-N-isopropylamino, N-methyl-N-n-butylamino, N-methyl-N-2-butylamino, N-methyl-N-isobutylamino, 1-pyrrolidinyl, 1-piperidinyl, 1-piperazinyl, 4-methyl-1-piperazinyl and 4-morpholinyl.
R^{9c} and R^{9d} irrespectively of their occurrence, are preferably selected from the group consisting of hydrogen, C₁-C₄-alkyl, such as methyl, ethyl, n-propyl, isopropyl, n-butyl, 2-butyl or isobutyl, and C₁-C₄-haloalkyl, such as fluoromethyl, difluoromethyl, trifluoromethyl, 1,1-difluoroethyl, 2-fluoroethyl, 2,2-difluoroethyl or 2,2,2-trifluoroethyl. Examples of such radicals NR^{9c}R^{9d} include, but are not limited to methylamino, ethylamino, n-propylamino, isopropylamino, n-butylamino, 2-butylamino, isobutylamino, dimethylamino, diethylamino, di-n-propylamino, di-n-butylamino, N-methyl-N-ethylamino, N-methyl-N-propylamino, N-methyl-N-n-propylamino, N-methyl-N-isopropylamino, N-methyl-N-n-butylamino, N-methyl-N-2-butylamino and N-methyl-N-isobutylamino.
R¹⁰ irrespectively of its occurrence, is selected from the group consisting of halogen, such as chlorine or fluorine, CN, C₁-C₄-alkyl, such as methyl, ethyl, n-propyl and isopropyl, C₁-C₄-haloalkyl, in particular C₁-C₂-haloalkyl, such as fluoromethyl, difluoromethyl, trifluoromethyl, 1,1-difluoroethyl, 2-fluoroethyl, 2,2-difluoroethyl or 2,2,2-trifluoroethyl, C₁-C₄-alkoxy, such as methoxy, ethoxy, n-propoxy and isopropoxy, and C₁-C₄-haloalkoxy, in particular C₁-C₂-haloalkoxy, such as fluoromethoxy, difluoromethoxy, trifluoromethoxy, 1,1-difluoroethoxy, 2-fluoroethoxy, 2,2-difluoroethoxy or 2,2,2-trifluoroethoxy.
R¹¹, R¹² independently of their occurrence, are selected from the group consisting of C₁-C₆-alkyl, C₁-C₆-alkoxy, C₃-C₈-cycloalkyl, C₃-C₈-cycloalkyl-C₁-C₄-alkyl, phenyl and benzyl, where the phenyl ring in last two radicals are unsubstituted or substituted with 1, 2, or 3 identical or different radicals selected from fluorine, chlorine, C₁-C₃-alkyl, C₁-C₂-haloalkyl, C₁-C₂-alkoxy and C₁-C₂-haloalkoxy.
R¹³, R¹⁴ independently of their occurrence, are selected from the group consisting of hydrogen, fluorine, chlorine, CN, C₁-C₄-alkyl, such as methyl, ethyl, n-propyl or n-butyl, C₃-C₆-cycloalkyl, such as cyclopropyl, cyclobutyl or cyclopentyl, and phenyl.
R¹⁵ irrespectively of its occurrence, is selected from the group consisting of hydrogen, C₁-C₄-alkyl, C₁-C₄-haloalkyl, C₃-C₆-cycloalkyl, C₃-C₆-cycloalkyl- C₁-C₄-alkyl, phenyl and benzyl, where the phenyl ring in the last two radicals is unsubstitued or substituted by 1, 2 or 3 identical or different radicals selected from the group consisting of halogen, such as chlorine or fluorine, CN, C₁-C₄-alkyl, such as methyl, ethyl, n-propyl and isopropyl, C₁-C₄-haloalkyl, in particular C₁-C₂-haloalkyl, such as fluoromethyl, difluoromethyl, trifluoromethyl, 1,1-difluoroethyl, 2-fluoroethyl, 2,2-difluoroethyl or 2,2,2-trifluoroethyl, C₁-C₄-alkoxy, such as methoxy, ethoxy, n-propoxy and isopropoxy, and C₁-C₄-haloalkoxy, in particular C₁-C₂-haloalkoxy, such as fluoromethoxy, difluoromethoxy, trifluoromethoxy, 1,1-difluoroethoxy, 2-fluoroethoxy, 2,2-difluoroethoxy or 2,2,2-trifluoroethoxy.
R¹⁶ irrespectively of its occurrence, is selected from the group consisting of C₁-C₄-alkyl, C₁-C₄-haloalkyl, C₃-C₆-cycloalkyl, C₃-C₆-cycloalkyl-C₁-C₄-alkyl, phenyl and benzyl, where the phenyl ring in the last two radicals is unsubstitued or substituted by 1, 2 or 3 identical or different radicals selected from the group consisting of halogen, such as chlorine or fluorine, CN, C₁-C₄-alkyl, such as methyl, ethyl, n-propyl and isopropyl, C₁-C₄-haloalkyl, in particular C₁-C₂-haloalkyl, such as fluoromethyl, difluoromethyl, trifluoromethyl, 1,1-difluoroethyl, 2-fluoroethyl, 2,2-difluoroethyl or 2,2,2-trifluoroethyl, C₁-C₄-alkoxy, such as methoxy, ethoxy, n-propoxy and isopropoxy, and C₁-C₄-haloalkoxy, in particular C₁-C₂-haloalkoxy, such as fluoromethoxy, difluoromethoxy, trifluoromethoxy, 1,1-difluoroethoxy, 2-fluoroethoxy, 2,2-difluoroethoxy or 2,2,2-trifluoroethoxy.
R¹⁷ irrespectively of its occurrence, is selected from the group consisting of hydrogen, C₁-C₆-alkyl, C₁-C₄-haloalkyl, C₁-C₆-alkoxy, C₁-C₄-haloalkoxy, C₃-C₆-alkenyl, C₃-C₆-cycloalkyl-C₁-C₄-alkyl, phenyl and benzyl, where the phenyl ring in the last two radicals is unsubstitued or substituted by 1, 2 or 3 identical or different radicals selected from the group consisting of halogen, such as chlorine or fluorine, CN, C₁-C₄-alkyl, such as methyl, ethyl, n-propyl and isopropyl, C₁-C₄-haloalkyl, in particular C₁-C₂-haloalkyl, such as fluoromethyl, difluoromethyl, trifluoromethyl, 1,1-difluoroethyl, 2-fluoroethyl, 2,2-difluoroethyl or 2,2,2-trifluoroethyl, C₁-C₄-alkoxy, such as methoxy, ethoxy, n-propoxy and isopropoxy, and C₁-C₄-haloalkoxy, in particular C₁-C₂-haloalkoxy, such as fluoromethoxy, difluoromethoxy, trifluoromethoxy, 1,1-difluoroethoxy, 2-fluoroethoxy, 2,2-difluoroethoxy or 2,2,2-trifluoroethoxy.
R^{17a} and R^{17b} irrespectively of their occurrence, are preferably selected from the group consisting of hydrogen, C₁-C₄-alkyl, such as methyl, ethyl, n-propyl, isopropyl, n-butyl, 2-butyl or isobutyl, and C₁-C₄-haloalkyl, such as fluoromethyl, difluoromethyl, trifluoromethyl, 1,1-difluoroethyl, 2-fluoroethyl, 2,2-difluoroethyl or 2,2,2-trifluoroethyl, or NR^{9a}R^{9b} may also be a saturated N-bound 3-, 4-, 5- or 6-membered heterocycle, which in addition to the nitrogen atom may have 1 further heteroatom as ring members, which is selected from O and N and where the N-bound 3-, 4-, 5- or 6-membered heterocycle may be unsubstituted or carry 1, 2, 3 or 4 radicals selected from C₁-C₄-alkyl and C₁-C₄-haloalkyl. Examples of such radicals NR^{9a}R^{9b} include, but are not limited to methylamino, ethylamino, n-propylamino, isopropylamino, n-butylamino, 2-butylamino, isobutylamino, dimethylamino, diethylamino, di-n-propylamino, di-n-butylamino, N-methyl-N-ethylamino, N-methyl-N-propylamino, N-methyl-N-n-propylamino, N-methyl-N-isopropylamino, N-methyl-N-n-butylamino, N-methyl-N-2-butylamino, N-methyl-N-isobutylamino, 1-pyrrolidinyl, 1-piperidinyl, 1-piperazinyl, 4-methyl-1-piperazinyl and 4-morpholinyl.
R^{17c} irrespectively of its occurrence, is selected from the group consisting of hydrogen, C₁-C₄-alkyl, C₁-C₄-haloalkyl, C₃-C₆-cycloalkyl, C₃-C₆-cycloalkyl- C₁-C₄-alkyl, phenyl and benzyl, where the phenyl ring in the last two radicals is unsubstitued or substituted by 1, 2 or 3 identical or different radicals selected from the group consisting of halogen, such as chlorine or fluorine, CN, C₁-C₄-alkyl, such as methyl, ethyl, n-propyl and isopropyl, C₁-C₄-haloalkyl, in particular C₁-C₂-haloalkyl, such as fluoromethyl, difluoromethyl, trifluoromethyl, 1,1-difluoroethyl, 2-fluoroethyl, 2,2-difluoroethyl or 2,2,2-trifluoroethyl, C₁-C₄-alkoxy, such as methoxy, ethoxy, n-propoxy and isopropoxy, and C₁-C₄-haloalkoxy, in particular C₁-C₂-haloalkoxy, such as fluoromethoxy, difluoromethoxy, trifluoromethoxy, 1,1-difluoroethoxy, 2-fluoroethoxy, 2,2-difluoroethoxy or 2,2,2-trifluoroethoxy.
R¹⁸ is each independently from one another selected from the group consisting of hydrogen, halogen, cyano, nitro, OH, SH, SCN, SF₅, C₁-C₆-alkoxy, C₁-C₆-haloalkoxy, C₁-C₆-alkylthio, C₁-C₆-alkylsulfinyl, C₁-C₆-alkylsulfonyl, C₁-C₆-haloalkylthio, trimethylsilyl, triethylsilyl, *tert*butyldimethylsilyl, C₁-C₆-alkyl, C₂-C₆-alkenyl, C₂-C₆-alkynyl, C₃-C₈-cycloalkyl, wherein the four last mentioned aliphatic and cyclo-aliphatic radicals may be unsubstituted, partially or completely halogenated and/or oxgenated and/or may carry 1 or 2 radicals selected from C₁-C₄ alkoxy,
phenyl, benzyl, pyridyl, phenoxy, wherein the last four radicals may be unsubstituted, partially or completely halogenated and/or to carry 1, 2 or 3 substituents selected from C₁-C₆-alkyl, C₁-C₆-haloalkyl, C₁-C₆-alkoxy, C₁-C₆ haloalkoxy, (C₁-C₆-alkoxy)carbonyl, (C₁-C₆-alkyl)amino or di-(C₁-C₆-alkyl)amino,
or two R¹⁸ present on the same carbon atom may together be =O, =CH(C₁-C₄), =C(C₁-C₄-alkyl)C₁-C₄-alkyl, =N(C₁-C₆-alkyl) or =NO(C₁-C₆-alkyl).
R¹⁹ is each independently from one another selected from the group consisting of hydrogen, cyano, C₁-C₆-alkoxy, C₁-C₆-haloalkoxy, C₁-C₆-alkylthio, C₁-C₆-alkylsulfinyl, C₁-C₆-alkylsulfonyl, C₁-C₆-haloalkylthio, trimethylsilyl, triethylsilyl, *tert*butyldimethylsilyl, C₁-C₆-alkyl, C₂-C₆-alkenyl, C₂-C₆-alkynyl, C₃-C₈-cycloalkyl, wherein the four last mentioned radicals may be unsubstituted, partially or completely halogenated and/or oxygenated and/or may carry 1 or 2 radicals selected from C₁-C₄ alkoxy,
phenyl, benzyl, pyridyl, phenoxy, wherein the last four radicals may be unsubstituted, partially or completely halogenated and/or carry 1, 2 or 3 substituents selected from C₁-C₆-alkyl, C₁-C₆-haloalkyl, C₁-C₆-alkoxy, C₁-C₆ haloalkoxy or (C₁-C₆-alkoxy)carbonyl.
R^{20a}, R^{20b} are each independently from one another selected from the group consisting of hydrogen, cyano, C₁-C₆-alkoxy, C₁-C₆-haloalkoxy, C₁-C₆-alkylthio, C₁-C₆-alkylsulfinyl, C₁-C₆-alkylsulfonyl, C₁-C₆-haloalkylthio, trimethylsilyl, triethylsilyl, *tert*butyldimethylsilyl, C₁-C₆-alkyl, C₂-C₆-alkenyl, C₂-C₆-alkynyl, C₃-C₈-cycloalkyl, wherein the four last mentioned aliphatic and cyclo-aliphatic radicals may be unsubstituted, partially or completely halogenated and/or oxygenated and/or may carry 1 or 2 identical or different radicals selected from C₁-C₄-alkoxy,
phenyl, benzyl, pyridyl, phenoxy, wherein the four last mentioned radicals may be unsubstituted, partially or completely halogenated and/or carry 1, 2 or 3 identical or different substituents selected from C₁-C₆-alkyl, C₁-C₆-haloalkyl, C₁-C₆-alkoxy, C₁-C₆ haloalkoxy and (C₁-C₆-alkoxy)carbonyl,
or, R^{20a} and R^{20b} may together be a C₂-C₆ alkylene chain forming a 3- to 7-membered saturated, partly saturated or unsaturated ring together with the nitrogen atom R^{20a} and R^{20b} are bonded to, wherein the alkylene chain may contain 1 or 2 heteroatoms selected from oxygen, sulfur or nitrogen, and may optionally be substituted with halogen, C₁-C₄-haloalkyl, C₁-C₄-alkoxy or C₁-C₄-haloalkoxy, and wherein the nitrogen and/or the sulfur atom(s) of the heterocyclic ring may optionally be oxidized.
R^{21a}, R^{21b} are each independently from one another selected from the group consisting of hydrogen, C₁-C₄ alkyl, C₁-C₆ cycloalkyl, C₁-C₄ alkoxy-C₁-C₄-alkyl, phenyl and benzyl.

A special group of embodiments relates to the compounds of formula (I)-A.1a, to their tautomers, to their stereoisomers and to their salts, where m, R¹, R², R³, R^{4a}, R^{4b} and R⁵ are as defined above and where R¹, R², R³, [CR^{4a}R^{4b}]ₘ and R⁵ have in particular one of the meanings given in any of lines 1 to 996 of the following table A.

A further special group of embodiments relates to the compounds of formula (I)-A.1a, to their tautomers, to their stereoisomers and to their salts, where m=1, R³ and R^{4a} form both together and together with the existing bond a double bond between the adjacent carbon atoms, and R¹, R², R^{4b} and R⁵ have one of the meanings given in any of lines 1 to 452 of the following table B.

A further special group of embodiments relates to the compounds of formula (I)-A.2a, to their tautomers, to their stereoisomers and to their salts, where m, R¹, R², R³, R^{4a}, R^{4b} and R⁵ are as defined above and where R¹, R², R³, [CR^{4a}R^{4b}]ₘ and R⁵ have in particular one of the meanings given in any of lines 1 to 996 of the following table A.

A further special group of embodiments relates to the compounds of formula (I)-A.2a, to their tautomers, to their stereoisomers and to their salts, where m=1, R³ and R^{4a} form both together and together with the existing bond a double bond between the adjacent carbon atoms, and R¹, R², R^{4b} and R⁵ have one of the meanings given in any of lines 1 to 452 of the following table B.

A further special group of embodiments relates to the compounds of formula (I)-A.1b, to their tautomers, to their stereoisomers and to their salts, where m, R¹, R², R³, R^{4a}, R^{4b} and R⁵ are as defined above and where R¹, R², R³, [CR^{4a}R^{4b}]ₘ and R⁵ have in particular one of the meanings given in any of lines 1 to 996 of the following table A.

A further special group of embodiments relates to the compounds of formula (I)-A.1b, to their tautomers, to their stereoisomers and to their salts, where m=1, R³ and R^{4a} form both together and together with the existing bond a double bond between the adjacent carbon atoms, and R¹, R², R^{4b} and R⁵ have one of the meanings given in any of lines 1 to 452 of the following table B.

A further special group of embodiments relates to the compounds of formula (I)-A.2b, to their tautomers, to their stereoisomers and to their salts, where m, R¹, R², R³, R^{4a}, R^{4b} and R⁵ are as defined above and where R¹, R², R³, [CR^{4a}R^{4b}]ₘ and R⁵ have in particular one of the meanings given in any of lines 1 to 996 of the following table A.

A further special group of embodiments relates to the compounds of formula (I)-A.2b, to their tautomers, to their stereoisomers and to their salts, where m=1, R³ and R^{4a} form both together and together with the existing bond a double bond between the adjacent carbon atoms, and R¹, R², R^{4b} and R⁵ have one of the meanings given in any of lines 1 to 452 of the following table B.

A further special group of embodiments relates to the compounds of formula (I)-A.1c, to their tautomers, to their stereoisomers and to their salts, where m, R¹, R², R³, R^{4a}, R^{4b} and R⁵ are as defined above and where R¹, R², R³, [CR^{4a}R^{4b}]ₘ and R⁵ have in particular one of the meanings given in any of lines 1 to 996 of the following table A.

A further special group of embodiments relates to the compounds of formula (I)-A.1c, to their tautomers, to their stereoisomers and to their salts, where m=1, R³ and R^{4a} form both together and together with the existing bond a double bond between the adjacent carbon atoms, and R¹, R², R^{4b} and R⁵ have one of the meanings given in any of lines 1 to 452 of the following table B.

A further special group of embodiments relates to the compounds of formula (I)-A.2c, to their tautomers, to their stereoisomers and to their salts, where m, R¹, R², R³, R^{4a}, R^{4b} and R⁵ are as defined above and where R¹, R², R³, [CR^{4a}R^{4b}]ₘ and R⁵ have in particular one of the meanings given in any of lines 1 to 996 of the following table A.

A further special group of embodiments relates to the compounds of formula (I)-A.2c, to their tautomers, to their stereoisomers and to their salts, where m=1, R³ and R^{4a} form both together and together with the existing bond a double bond between the adjacent carbon atoms, and R¹, R², R^{4b} and R⁵ have one of the meanings given in any of lines 1 to 452 of the following table B.

A further special group of embodiments relates to the compounds of formula (I)-A.1d, to their tautomers, to their stereoisomers and to their salts, where m, R¹, R², R³, R^{4a}, R^{4b} and R⁵ are as defined above and where R¹, R², R³, [CR^{4a}R^{4b}]ₘ and R⁵ have in particular one of the meanings given in any of lines 1 to 996 of the following table A.

A further special group of embodiments relates to the compounds of formula (I)-A.1d, to their tautomers, to their stereoisomers and to their salts, where m=1, R³ and R^{4a} form both together and together with the existing bond a double bond between the adjacent carbon atoms, and R¹, R², R^{4b} and R⁵ have one of the meanings given in any of lines 1 to 452 of the following table B.

A further special group of embodiments relates to the compounds of formula (I)-A.2d, to their tautomers, to their stereoisomers and to their salts, where m, R¹, R², R³, R^{4a}, R^{4b} and R⁵ are as defined above and where R¹, R², R³, [CR^{4a}R^{4b}]ₘ and R⁵ have in particular one of the meanings given in any of lines 1 to 996 of the following table A.

A further special group of embodiments relates to the compounds of formula (I)-A.2d, to their tautomers, to their stereoisomers and to their salts, where m=1, R³ and R^{4a} form both together and together with the existing bond a double bond between the adjacent carbon atoms, and R¹, R², R^{4b} and R⁵ have one of the meanings given in any of lines 1 to 452 of the following table B.

A further special group of embodiments relates to the compounds of formula (I)-A.3a, to their tautomers, to their stereoisomers and to their salts, where m, R¹, R², R³, R^{4a}, R^{4b} and R⁵ are as defined above and where R¹, R², R³, [CR^{4a}R^{4b}]ₘ and R⁵ have in particular one of the meanings given in any of lines 1 to 996 of the following table A.

A further special group of embodiments relates to the compounds of formula (I)-A.3a, to their tautomers, to their stereoisomers and to their salts, where m=1, R³ and R^{4a} form both together and together with the existing bond a double bond between the adjacent carbon atoms, and R¹, R², R^{4b} and R⁵ have one of the meanings given in any of lines 1 to 452 of the following table B.

A further special group of embodiments relates to the compounds of formula (I)-A.4a, to their tautomers, to their stereoisomers and to their salts, where m, R¹, R², R³, R^{4a}, R^{4b} and R⁵ are as defined above and where R¹, R², R³, [CR^{4a}R^{4b}]ₘ and R⁵ have in particular one of the meanings given in any of lines 1 to 996 of the following table A.

A further special group of embodiments relates to the compounds of formula (I)-A.4a, to their tautomers, to their stereoisomers and to their salts, where m=1, R³ and R^{4a} form both together and together with the existing bond a double bond between the adjacent carbon atoms, and R¹, R², R^{4b} and R⁵ have one of the meanings given in any of lines 1 to 452 of the following table B.

A further special group of embodiments relates to the compounds of formula (I)-A.3b, to their tautomers, to their stereoisomers and to their salts, where m, R¹, R², R³, R^{4a}, R^{4b} and R⁵ are as defined above and where R¹, R², R³, [CR^{4a}R^{4b}]ₘ and R⁵ have in particular one of the meanings given in any of lines 1 to 996 of the following table A.

A further special group of embodiments relates to the compounds of formula (I)-A.3b, to their tautomers, to their stereoisomers and to their salts, where m=1, R³ and R^{4a} form both together and together with the existing bond a double bond between the adjacent carbon atoms, and R¹, R², R^{4b} and R⁵ have one of the meanings given in any of lines 1 to 452 of the following table B.

A further special group of embodiments relates to the compounds of formula (I)-A.4b, to their tautomers, to their stereoisomers and to their salts, where m, R¹, R², R³, R^{4a}, R^{4b} and R⁵ are as defined above and where R¹, R², R³, [CR^{4a}R^{4b}]ₘ and R⁵ have in particular one of the meanings given in any of lines 1 to 996 of the following table A.

A further special group of embodiments relates to the compounds of formula (I)-A.4b, to their tautomers, to their stereoisomers and to their salts, where m=1, R³ and R^{4a} form both together and together with the existing bond a double bond between the adjacent carbon atoms, and R¹, R², R^{4b} and R⁵ have one of the meanings given in any of lines 1 to 452 of the following table B.

A further special group of embodiments relates to the compounds of formula (I)-A.3c, to their tautomers, to their stereoisomers and to their salts, where m, R¹, R², R³, R^{4a}, R^{4b} and R⁵ are as defined above and where R¹, R², R³, [CR^{4a}R^{4b}]ₘ and R⁵ have in particular one of the meanings given in any of lines 1 to 996 of the following table A.

A further special group of embodiments relates to the compounds of formula (I)-A.3c, to their tautomers, to their stereoisomers and to their salts, where m=1, R³ and R^{4a} form both together and together with the existing bond a double bond between the adjacent carbon atoms, and R¹, R², R^{4b} and R⁵ have one of the meanings given in any of lines 1 to 452 of the following table B.

A further special group of embodiments relates to the compounds of formula (I)-A.4c, to their tautomers, to their stereoisomers and to their salts, where m, R¹, R², R³, R^{4a}, R^{4b} and R⁵ are as defined above and where R¹, R², R³, [CR^{4a}R^{4b}]ₘ and R⁵ have in particular one of the meanings given in any of lines 1 to 996 of the following table A.

A further special group of embodiments relates to the compounds of formula (I)-A.4c, to their tautomers, to their stereoisomers and to their salts, where m=1, R³ and R^{4a} form both together and together with the existing bond a double bond between the adjacent carbon atoms, and R¹, R², R^{4b} and R⁵ have one of the meanings given in any of lines 1 to 452 of the following table B.

A further special group of embodiments relates to the compounds of formula (I)-A.3d, to their tautomers, to their stereoisomers and to their salts, where m, R¹, R², R³, R^{4a}, R^{4b} and R⁵ are as defined above and where R¹, R², R³, [CR^{4a}R^{4b}]ₘ and R⁵ have in particular one of the meanings given in any of lines 1 to 996 of the following table A.

A further special group of embodiments relates to the compounds of formula (I)-A.3d, to their tautomers, to their stereoisomers and to their salts, where m=1, R³ and R^{4a} form both together and together with the existing bond a double bond between the adjacent carbon atoms, and R¹, R², R^{4b} and R⁵ have one of the meanings given in any of lines 1 to 452 of the following table B.

A further special group of embodiments relates to the compounds of formula (I)-A.4d, to their tautomers, to their stereoisomers and to their salts, where m, R¹, R², R³, R^{4a}, R^{4b} and R⁵ are as defined above and where R¹, R², R³, [CR^{4a}R^{4b}]ₘ and R⁵ have in particular one of the meanings given in any of lines 1 to 996 of the following table A.

A further special group of embodiments relates to the compounds of formula (I)-A.4d, to their tautomers, to their stereoisomers and to their salts, where m=1, R³ and R^{4a} form both together and together with the existing bond a double bond between the adjacent carbon atoms, and R¹, R², R^{4b} and R⁵ have one of the meanings given in any of lines 1 to 452 of the following table B.

A skilled person will readily appreciate that the compounds of formulae (I)-A.1a, (I)-A.2a, (I)-A.3a, (I)-A.4a, (I)-A.1b, (I)-A.2b, (I)-A.3b, (I)-A.4b, (I)-A.1c, (I)-A.2c, (I)-A.3c, (I)-A.4c, (I)-A.1d, (I)-A.2d, (I)-A.3d and (I)-A.4d, where R¹, R², R³, [CR^{4a}R^{4b}]ₘ and R⁵ have the meanings given in one of lines 2 to 17, 19 to 33, 35 to 48, 50 to 62, 64 to 75, 77 to 87, 89 to 420, 422 to 437, 439 to 453, 455 to 468, 470 to 482, 484 to 495, 497 to 507, 509 to 840 of the following table A may have S- or R-configuration with regard to the carbon atom carrying the radical R³. The aforementioned embodiments include both the S-enantiomer and the R-enantiomer as well as mixtures of these enantiomers, in particular racemic mixtures.

A skilled person will also readily appreciate that the compounds of formulae (I)-A.1a, (I)-A.2a, (I)-A.3a, (I)-A.4a, (I)-A.1b, (I)-A.2b, (I)-A.3b, (I)-A.4b, (I)-A.1c, (I)-A.2c, (I)-A.3c, (I)-A.4c, (I)-A.1d, (I)-A.2d, (I)-A.3d and (I)-A.4d, where R¹, R², R³, [CR^{4a}R^{4b}]ₘ and R⁵ have the meanings given in one of lines 421 to 840 and 919 to 996 of the following table A or given in one of lines 229 to 366 and 372 to 376 of the following table B may have S- or R-configuration with regard to the carbon atom carrying radicals R¹ and R². The aforementioned embodiments include both the S-enantiomer and the R-enantiomer as well as mixtures of these enantiomers, in particular racemic mixtures.

A skilled person will also readily appreciate that the compounds of formulae (I)-A.1a, (I)-A.2a, (I)-A.3a, (I)-A.4a, (I)-A.1b, (I)-A.2b, (I)-A.3b, (I)-A.4b, (I)-A.1c, (I)-A.2c, (I)-A.3c, (I)-A.4c, (I)-A.1d, (I)-A.2d, (I)-A.3d and (I)-A.4d, where R¹, R², R³, [CR^{4a}R^{4b}]ₘ and R⁵ have the meanings given in one of lines 422 to 437, 439 to 453, 455 to 468, 470 to 482, 484 to 495, 497 to 507, 509 to 840 and 919 to 996 of the following table A may have S- or R-configuration with regard to (i) the carbon atom carrying the radical R³ and (ii) the carbon atom carrying radicals R¹ and R². The aforementioned embodiments include (i),(ii)-diasteromers selected from the S,S-diastereomer, the S,R diastereomer, the R,S-diastereomer and the R,R-diastereomer as well as mixtures of these enantiomers, in particular racemic mixtures with regard to the configuration at either (i) or (ii).

A skilled person will also readily appreciate that the compounds of formulae (I)-A.1a, (I)-A.2a, (I)-A.3a, (I)-A.4a, (I)-A.1b, (I)-A.2b, (I)-A.3b, (I)-A.4b, (I)-A.1c, (I)-A.2c, (I)-A.3c, (I)-A.4c, (I)-A.1d, (I)-A.2d, (I)-A.3d and (I)-A.4d, where R¹, R² and R⁵ have the meanings given in one of lines 2 to 114, 116 to 228, 230 to 342 and 344 to 366 of the following table B may have E- or Z-configuration with regard to the NO₂ group and the radical R⁵. The aforementioned embodiments include the E- and Z-stereoisomers as well as mixtures of these stereoisomers.

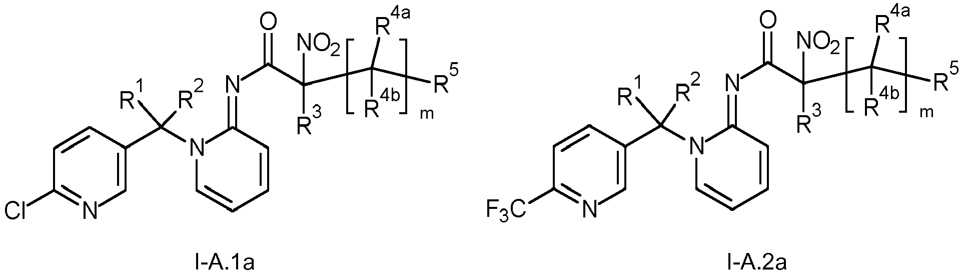

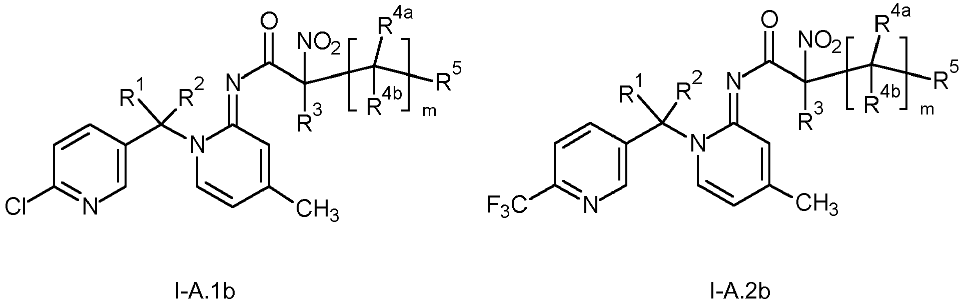

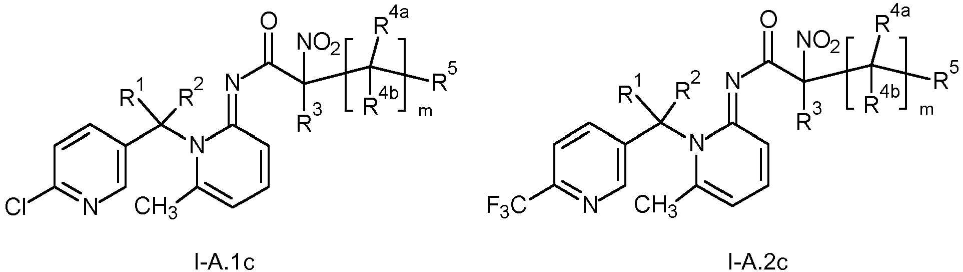

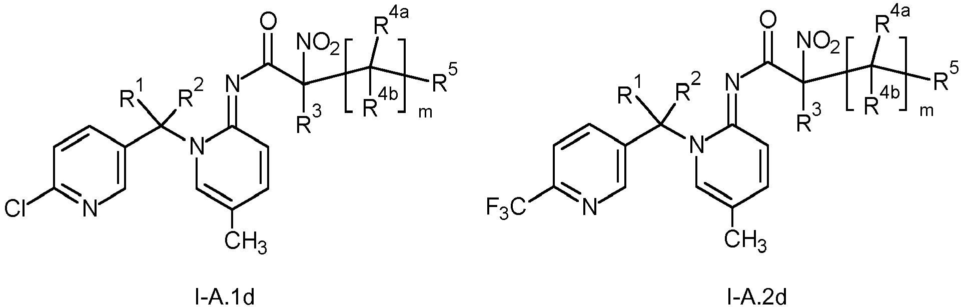

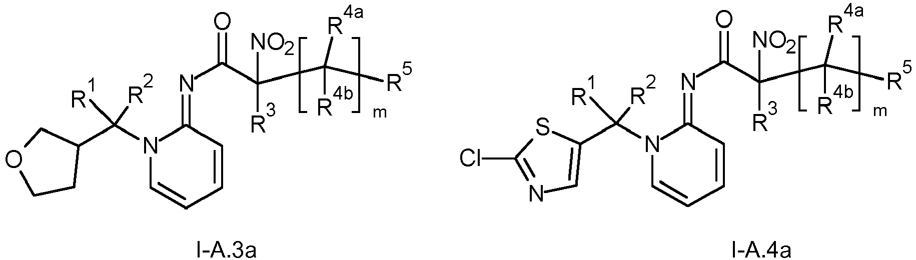

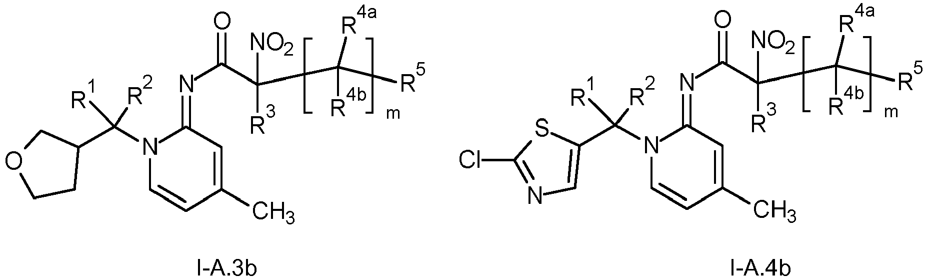

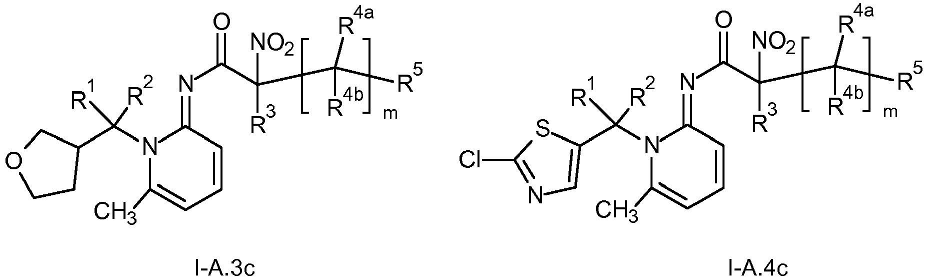

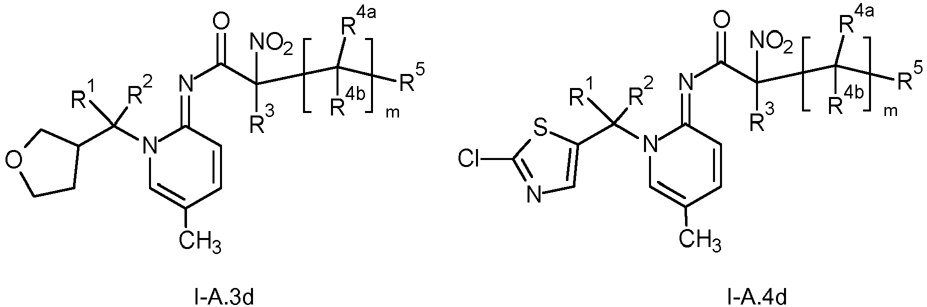

**Table A:**

| # | R¹ | R² | R³ | R⁵ | [CR^{4a}R^{4b}]ₘ |
|---|---|---|---|---|---|
| 1 | H | H | H | H | - |
| 2 | H | H | H | F | - |
| 3 | H | H | H | Cl | - |
| 4 | H | H | H | Br | - |
| 5 | H | H | H | CH₃ | - |
| 6 | H | H | H | CH₂CH₃ | - |
| 7 | H | H | H | CH₂CH=CH₂ | - |
| 8 | H | H | H | CH₂CH₂CH₃ | - |
| 9 | H | H | H | CH(CH₃)₂ | - |
| 10 | H | H | H | CH₂CH₂CH₂CH₃ | - |
| 11 | H | H | H | CH(CH₃)CH₂CH₃ | - |
| 12 | H | H | H | CH₂CH(CH₃)₂ | - |
| 13 | H | H | H | CN | - |
| 14 | H | H | H | c-C₃H₅ | - |
| 15 | H | H | H | c-C₄H₇ | - |
| 16 | H | H | H | c-C₅H₉ | - |
| 17 | H | H | H | c-C₆H₁₁ | - |
| 18 | H | H | F | F | - |
| 19 | H | H | F | Cl | - |
| 20 | H | H | F | Br | - |
| 21 | H | H | F | CH₃ | - |
| 22 | H | H | F | CH₂CH₃ | - |
| 23 | H | H | F | CH₂CH=CH₂ | - |
| 24 | H | H | F | CH₂CH₂CH₃ | - |
| 25 | H | H | F | CH(CH₃)₂ | - |
| 26 | H | H | F | CH₂CH₂CH₂CH₃ | - |
| 27 | H | H | F | CH(CH₃)CH₂CH₃ | - |
| 28 | H | H | F | CH₂CH(CH₃)₂ | - |
| 29 | H | H | F | CN | - |
| 30 | H | H | F | c-C₃H₅ | - |
| 31 | H | H | F | c-C₄H₇ | - |
| 32 | H | H | F | c-C₅H₉ | - |
| 33 | H | H | F | c-C₆H₁₁ | - |
| 34 | H | H | Cl | Cl | - |
| 35 | H | H | Cl | Br | - |
| 36 | H | H | Cl | CH₃ | - |
| 37 | H | H | Cl | CH₂CH₃ | - |
| 38 | H | H | Cl | CH₂CH=CH₂ | - |
| 39 | H | H | Cl | CH₂CH₂CH₃ | - |
| 40 | H | H | Cl | CH(CH₃)₂ | - |
| 41 | H | H | Cl | CH₂CH₂CH₂CH₃ | - |
| 42 | H | H | Cl | CH(CH₃)CH₂CH₃ | - |
| 43 | H | H | Cl | CH₂CH(CH₃)₂ | - |
| 44 | H | H | Cl | CN | - |
| 45 | H | H | Cl | c-C₃H₅ | - |
| 46 | H | H | Cl | c-C₄H₇ | - |
| 47 | H | H | Cl | c-C₅H₉ | - |
| 48 | H | H | Cl | c-C₆H₁₁ | - |
| 49 | H | H | Br | Br | - |
| 50 | H | H | Br | CH₃ | - |
| 51 | H | H | Br | CH₂CH₃ | - |
| 52 | H | H | Br | CH₂CH=CH₂ | - |
| 53 | H | H | Br | CH₂CH₂CH₃ | - |
| 54 | H | H | Br | CH(CH₃)₂ | - |
| 55 | H | H | Br | CH₂CH₂CH₂CH₃ | - |
| 56 | H | H | Br | CH(CH₃)CH₂CH₃ | - |
| 57 | H | H | Br | CH₂CH(CH₃)₂ | - |
| 58 | H | H | Br | CN | - |
| 59 | H | H | Br | c-C₃H₅ | - |
| 60 | H | H | Br | c-C₄H₇ | - |
| 61 | H | H | Br | c-C₅H₉ | - |
| 62 | H | H | Br | c-C₆H₁₁ | - |
| 63 | H | H | CH₃ | CH₃ | - |
| 64 | H | H | CH₃ | CH₂CH₃ | - |
| 65 | H | H | CH₃ | CH₂CH=CH₂ | - |
| 66 | H | H | CH₃ | CH₂CH₂CH₃ | - |
| 67 | H | H | CH₃ | CH(CH₃)₂ | - |
| 68 | H | H | CH₃ | CH₂CH₂CH₂CH₃ | - |
| 69 | H | H | CH₃ | CH(CH₃)CH₂CH₃ | - |
| 70 | H | H | CH₃ | CH₂CH(CH₃)₂ | - |
| 71 | H | H | CH₃ | CN | - |
| 72 | H | H | CH₃ | c-C₃H₅ | - |
| 73 | H | H | CH₃ | c-C₄H₇ | - |
| 74 | H | H | CH₃ | c-C₅H₉ | - |
| 75 | H | H | CH₃ | c-C₆H₁₁ | - |
| 76 | H | H | CH₂CH₃ | CH₂CH₃ | - |
| 77 | H | H | CH₂CH₃ | CH₂CH=CH₂ | - |
| 78 | H | H | CH₂CH₃ | CH₂CH₂CH₃ | - |
| 79 | H | H | CH₂CH₃ | CH(CH₃)₂ | - |
| 80 | H | H | CH₂CH₃ | CH₂CH₂CH₂CH₃ | - |
| 81 | H | H | CH₂CH₃ | CH(CH₃)CH₂CH₃ | - |
| 82 | H | H | CH₂CH₃ | CH₂CH(CH₃)₂ | - |
| 83 | H | H | CH₂CH₃ | CN | - |
| 84 | H | H | CH₂CH₃ | c-C₃H₅ | - |
| 85 | H | H | CH₂CH₃ | c-C₄H₇ | - |
| 86 | H | H | CH₂CH₃ | c-C₅H₉ | - |
| 87 | H | H | CH₂CH₃ | c-C₆H₁₁ | - |
| 88 | H | H | CH₂CH=CH₂ | CH₂CH=CH₂ | - |
| 89 | H | H | CH₂CH=CH₂ | CH₂CH₂CH₃ | - |
| 90 | H | H | CH₂CH=CH₂ | CH(CH₃)₂ | - |
| 91 | H | H | CH₂CH=CH₂ | CH₂CH₂CH₂CH₃ | - |
| 92 | H | H | CH₂CH=CH₂ | CH(CH₃)CH₂CH₃ | - |
| 93 | H | H | CH₂CH=CH₂ | CH₂CH(CH₃)₂ | - |
| 94 | H | H | CH₂CH=CH₂ | CN | - |
| 95 | H | H | CH₂CH=CH₂ | c-C₃H₅ | - |
| 96 | H | H | CH₂CH=CH₂ | c-C₄H₇ | - |
| 97 | H | H | CH₂CH=CH₂ | c-C₅H₉ | - |
| 98 | H | H | CH₂CH=CH₂ | c-C₆H₁₁ | - |
| 99 | H | H | H | F | CH₂ |
| 100 | H | H | H | Cl | CH₂ |
| 101 | H | H | H | Br | CH₂ |
| 102 | H | H | H | CH₂CH=CH₂ | CH₂ |
| 103 | H | H | H | CH₂CH₂CH₂CH₃ | CH₂ |
| 104 | H | H | H | CH(CH₃)CH₂CH₃ | CH₂ |
| 105 | H | H | H | CH₂CH(CH₃)₂ | CH₂ |
| 106 | H | H | H | CN | CH₂ |
| 107 | H | H | H | c-C₃H₅ | CH₂ |
| 108 | H | H | H | c-C₄H₇ | CH₂ |
| 109 | H | H | H | c-C₅H₉ | CH₂ |
| 110 | H | H | H | c-C₆H₁₁ | CH₂ |
| 111 | H | H | F | F | CH₂ |
| 112 | H | H | F | Cl | CH₂ |
| 113 | H | H | F | Br | CH₂ |
| 114 | H | H | F | CH₂CH=CH₂ | CH₂ |
| 115 | H | H | F | CH₂CH₂CH₂CH₃ | CH₂ |
| 116 | H | H | F | CH(CH₃)CH₂CH₃ | CH₂ |
| 117 | H | H | F | CH₂CH(CH₃)₂ | CH₂ |
| 118 | H | H | F | CN | CH₂ |
| 119 | H | H | F | c-C₃H₅ | CH₂ |
| 120 | H | H | F | c-C₄H₇ | CH₂ |
| 121 | H | H | F | c-C₅H₉ | CH₂ |
| 122 | H | H | F | c-C₆H₁₁ | CH₂ |
| 123 | H | H | Cl | F | CH₂ |
| 124 | H | H | Cl | Cl | CH₂ |
| 125 | H | H | Cl | Br | CH₂ |
| 126 | H | H | Cl | CH₂CH=CH₂ | CH₂ |
| 127 | H | H | Cl | CH₂CH₂CH₂CH₃ | CH₂ |
| 128 | H | H | Cl | CH(CH₃)CH₂CH₃ | CH₂ |
| 129 | H | H | Cl | CH₂CH(CH₃)₂ | CH₂ |
| 130 | H | H | Cl | CN | CH₂ |
| 131 | H | H | Cl | c-C₃H₅ | CH₂ |
| 132 | H | H | Cl | c-C₄H₇ | CH₂ |
| 133 | H | H | Cl | c-C₅H₉ | CH₂ |
| 134 | H | H | Cl | c-C₆H₁₁ | CH₂ |
| 135 | H | H | Br | F | CH₂ |
| 136 | H | H | Br | Cl | CH₂ |
| 137 | H | H | Br | Br | CH₂ |
| 138 | H | H | Br | CH₂CH=CH₂ | CH₂ |
| 139 | H | H | Br | CH₂CH₂CH₂CH₃ | CH₂ |
| 140 | H | H | Br | CH(CH₃)CH₂CH₃ | CH₂ |
| 141 | H | H | Br | CH₂CH(CH₃)₂ | CH₂ |
| 142 | H | H | Br | CN | CH₂ |
| 143 | H | H | Br | c-C₃H₅ | CH₂ |
| 144 | H | H | Br | c-C₄H₇ | CH₂ |
| 145 | H | H | Br | c-C₅H₉ | CH₂ |
| 146 | H | H | Br | c-C₆H₁₁ | CH₂ |
| 147 | H | H | CH₃ | F | CH₂ |
| 148 | H | H | CH₃ | Cl | CH₂ |
| 149 | H | H | CH₃ | Br | CH₂ |
| 150 | H | H | CH₃ | CH₂CH=CH₂ | CH₂ |
| 151 | H | H | CH₃ | CH₂CH₂CH₂CH₃ | CH₂ |
| 152 | H | H | CH₃ | CH(CH₃)CH₂CH₃ | CH₂ |
| 153 | H | H | CH₃ | CH₂CH(CH₃)₂ | CH₂ |
| 154 | H | H | CH₃ | CN | CH₂ |
| 155 | H | H | CH₃ | c-C₃H₅ | CH₂ |
| 156 | H | H | CH₃ | c-C₄H₇ | CH₂ |
| 157 | H | H | CH₃ | c-C₅H₉ | CH₂ |
| 158 | H | H | CH₃ | c-C₆H₁₁ | CH₂ |
| 159 | H | H | CH₂CH₃ | F | CH₂ |
| 160 | H | H | CH₂CH₃ | Cl | CH₂ |
| 161 | H | H | CH₂CH₃ | Br | CH₂ |
| 162 | H | H | CH₂CH₃ | CH₂CH=CH₂ | CH₂ |
| 163 | H | H | CH₂CH₃ | CH₂CH₂CH₂CH₃ | CH₂ |
| 164 | H | H | CH₂CH₃ | CH(CH₃)CH₂CH₃ | CH₂ |
| 165 | H | H | CH₂CH₃ | CH₂CH(CH₃)₂ | CH₂ |
| 166 | H | H | CH₂CH₃ | CN | CH₂ |
| 167 | H | H | CH₂CH₃ | c-C₃H₅ | CH₂ |
| 168 | H | H | CH₂CH₃ | c-C₄H₇ | CH₂ |
| 169 | H | H | CH₂CH₃ | c-C₅H₉ | CH₂ |
| 170 | H | H | CH₂CH₃ | c-C₆H₁₁ | CH₂ |
| 171 | H | H | CH₂CH=CH₂ | F | CH₂ |
| 172 | H | H | CH₂CH=CH₂ | Cl | CH₂ |
| 173 | H | H | CH₂CH=CH₂ | Br | CH₂ |
| 174 | H | H | CH₂CH=CH₂ | CH₂CH=CH₂ | CH₂ |
| 175 | H | H | CH₂CH=CH₂ | CH₂CH₂CH₂CH₃ | CH₂ |
| 176 | H | H | CH₂CH=CH₂ | CH(CH₃)CH₂CH₃ | CH₂ |
| 177 | H | H | CH₂CH=CH₂ | CH₂CH(CH₃)₂ | CH₂ |
| 178 | H | H | CH₂CH=CH₂ | CN | CH₂ |
| 179 | H | H | CH₂CH=CH₂ | c-C₃H₅ | CH₂ |
| 180 | H | H | CH₂CH=CH₂ | c-C₄H₇ | CH₂ |
| 181 | H | H | CH₂CH=CH₂ | c-C₅H₉ | CH₂ |
| 182 | H | H | CH₂CH=CH₂ | c-C₆H₁₁ | CH₂ |
| 183 | H | H | H | H | C=O |
| 184 | H | H | H | F | C=O |
| 185 | H | H | H | Cl | C=O |
| 186 | H | H | H | Br | C=O |
| 187 | H | H | H | CH₃ | C=O |
| 188 | H | H | H | CH₂CH₃ | C=O |
| 189 | H | H | H | CH₂CH=CH₂ | C=O |
| 190 | H | H | H | CH₂CH₂CH₃ | C=O |
| 191 | H | H | H | CH(CH₃)₂ | C=O |
| 192 | H | H | H | CH₂CH₂CH₂CH₃ | C=O |
| 193 | H | H | H | CH(CH₃)CH₂CH₃ | C=O |
| 194 | H | H | H | CH₂CH(CH₃)₂ | C=O |
| 195 | H | H | H | CN | C=O |
| 196 | H | H | H | c-C₃H₅ | C=O |
| 197 | H | H | H | c-C₄H₇ | C=O |
| 198 | H | H | H | c-C₅H₉ | C=O |
| 199 | H | H | H | c-C₆H₁₁ | C=O |
| 200 | H | H | F | H | C=O |
| 201 | H | H | F | F | C=O |
| 202 | H | H | F | Cl | C=O |
| 203 | H | H | F | Br | C=O |
| 204 | H | H | F | CH₃ | C=O |
| 205 | H | H | F | CH₂CH₃ | C=O |
| 206 | H | H | F | CH₂CH=CH₂ | C=O |
| 207 | H | H | F | CH₂CH₂CH₃ | C=O |
| 208 | H | H | F | CH(CH₃)₂ | C=O |
| 209 | H | H | F | CH₂CH₂CH₂CH₃ | C=O |
| 210 | H | H | F | CH(CH₃)CH₂CH₃ | C=O |
| 211 | H | H | F | CH₂CH(CH₃)₂ | C=O |
| 212 | H | H | F | CN | C=O |
| 213 | H | H | F | c-C₃H₅ | C=O |
| 214 | H | H | F | c-C₄H₇ | C=O |
| 215 | H | H | F | c-C₅H₉ | C=O |
| 216 | H | H | F | c-C₆H₁₁ | C=O |
| 217 | H | H | Cl | H | C=O |
| 218 | H | H | Cl | F | C=O |
| 219 | H | H | Cl | Cl | C=O |
| 220 | H | H | Cl | Br | C=O |
| 221 | H | H | Cl | CH₃ | C=O |
| 222 | H | H | Cl | CH₂CH₃ | C=O |
| 223 | H | H | Cl | CH₂CH=CH₂ | C=O |
| 224 | H | H | Cl | CH₂CH₂CH₃ | C=O |
| 225 | H | H | Cl | CH(CH₃)₂ | C=O |
| 226 | H | H | Cl | CH₂CH₂CH₂CH₃ | C=O |
| 227 | H | H | Cl | CH(CH₃)CH₂CH₃ | C=O |
| 228 | H | H | Cl | CH₂CH(CH₃)₂ | C=O |
| 229 | H | H | Cl | CN | C=O |
| 230 | H | H | Cl | c-C₃H₅ | C=O |
| 231 | H | H | Cl | c-C₄H₇ | C=O |
| 232 | H | H | Cl | c-C₅H₉ | C=O |
| 233 | H | H | Cl | c-C₆H₁₁ | C=O |
| 234 | H | H | Br | H | C=O |
| 235 | H | H | Br | F | C=O |
| 236 | H | H | Br | Cl | C=O |
| 237 | H | H | Br | Br | C=O |
| 238 | H | H | Br | CH₃ | C=O |
| 239 | H | H | Br | CH₂CH₃ | C=O |
| 240 | H | H | Br | CH₂CH=CH₂ | C=O |
| 241 | H | H | Br | CH₂CH₂CH₃ | C=O |
| 242 | H | H | Br | CH(CH₃)₂ | C=O |
| 243 | H | H | Br | CH₂CH₂CH₂CH₃ | C=O |
| 244 | H | H | Br | CH(CH₃)CH₂CH₃ | C=O |
| 245 | H | H | Br | CH₂CH(CH₃)₂ | C=O |
| 246 | H | H | Br | CN | C=O |
| 247 | H | H | Br | c-C₃H₅ | C=O |
| 248 | H | H | Br | c-C₄H₇ | C=O |
| 249 | H | H | Br | c-C₅H₉ | C=O |
| 250 | H | H | Br | c-C₆H₁₁ | C=O |
| 251 | H | H | CH₃ | H | C=O |
| 252 | H | H | CH₃ | F | C=O |
| 253 | H | H | CH₃ | Cl | C=O |
| 254 | H | H | CH₃ | Br | C=O |
| 255 | H | H | CH₃ | CH₃ | C=O |
| 256 | H | H | CH₃ | CH₂CH₃ | C=O |
| 257 | H | H | CH₃ | CH₂CH=CH₂ | C=O |
| 258 | H | H | CH₃ | CH₂CH₂CH₃ | C=O |
| 259 | H | H | CH₃ | CH(CH₃)₂ | C=O |
| 260 | H | H | CH₃ | CH₂CH₂CH₂CH₃ | C=O |
| 261 | H | H | CH₃ | CH(CH₃)CH₂CH₃ | C=O |
| 262 | H | H | CH₃ | CH₂CH(CH₃)₂ | C=O |
| 263 | H | H | CH₃ | CN | C=O |
| 264 | H | H | CH₃ | c-C₃H₅ | C=O |
| 265 | H | H | CH₃ | c-C₄H₇ | C=O |
| 266 | H | H | CH₃ | c-C₅H₉ | C=O |
| 267 | H | H | CH₃ | c-C₆H₁₁ | C=O |
| 268 | H | H | CH₂CH₃ | H | C=O |
| 269 | H | H | CH₂CH₃ | F | C=O |
| 270 | H | H | CH₂CH₃ | Cl | C=O |
| 271 | H | H | CH₂CH₃ | Br | C=O |
| 272 | H | H | CH₂CH₃ | CH₃ | C=O |
| 273 | H | H | CH₂CH₃ | CH₂CH₃ | C=O |
| 274 | H | H | CH₂CH₃ | CH₂CH=CH₂ | C=O |
| 275 | H | H | CH₂CH₃ | CH₂CH₂CH₃ | C=O |
| 276 | H | H | CH₂CH₃ | CH(CH₃)₂ | C=O |
| 277 | H | H | CH₂CH₃ | CH₂CH₂CH₂CH₃ | C=O |
| 278 | H | H | CH₂CH₃ | CH(CH₃)CH₂CH₃ | C=O |
| 279 | H | H | CH₂CH₃ | CH₂CH(CH₃)₂ | C=O |
| 280 | H | H | CH₂CH₃ | CN | C=O |
| 281 | H | H | CH₂CH₃ | c-C₃H₅ | C=O |
| 282 | H | H | CH₂CH₃ | c-C₄H₇ | C=O |
| 283 | H | H | CH₂CH₃ | c-C₅H₉ | C=O |
| 284 | H | H | CH₂CH₃ | c-C₆H₁₁ | C=O |
| 285 | H | H | CH₂CH=CH₂ | H | C=O |
| 286 | H | H | CH₂CH=CH₂ | F | C=O |
| 287 | H | H | CH₂CH=CH₂ | Cl | C=O |
| 288 | H | H | CH₂CH=CH₂ | Br | C=O |
| 289 | H | H | CH₂CH=CH₂ | CH₃ | C=O |
| 290 | H | H | CH₂CH=CH₂ | CH₂CH₃ | C=O |
| 291 | H | H | CH₂CH=CH₂ | CH₂CH=CH₂ | C=O |
| 292 | H | H | CH₂CH=CH₂ | CH₂CH₂CH₃ | C=O |
| 293 | H | H | CH₂CH=CH₂ | CH(CH₃)₂ | C=O |
| 294 | H | H | CH₂CH=CH₂ | CH₂CH₂CH₂CH₃ | C=O |
| 295 | H | H | CH₂CH=CH₂ | CH(CH₃)CH₂CH₃ | C=O |
| 296 | H | H | CH₂CH=CH₂ | CH₂CH(CH₃)₂ | C=O |
| 297 | H | H | CH₂CH=CH₂ | CN | C=O |
| 298 | H | H | CH₂CH=CH₂ | c-C₃H₅ | C=O |
| 299 | H | H | CH₂CH=CH₂ | c-C₄H₇ | C=O |
| 300 | H | H | CH₂CH=CH₂ | c-C₅H₉ | C=O |
| 301 | H | H | CH₂CH=CH₂ | c-C₆H₁₁ | C=O |
| 302 | H | H | H | H | CH(CH₃) |
| 303 | H | H | H | F | CH(CH₃) |
| 304 | H | H | H | Cl | CH(CH₃) |
| 305 | H | H | H | Br | CH(CH₃) |
| 306 | H | H | H | CH₃ | CH(CH₃) |
| 307 | H | H | H | CH₂CH₃ | CH(CH₃) |
| 308 | H | H | H | CH₂CH=CH₂ | CH(CH₃) |
| 309 | H | H | H | CH₂CH₂CH₃ | CH(CH₃) |
| 310 | H | H | H | CH(CH₃)₂ | CH(CH₃) |
| 311 | H | H | H | CH₂CH₂CH₂CH₃ | CH(CH₃) |
| 312 | H | H | H | CH(CH₃)CH₂CH₃ | CH(CH₃) |
| 313 | H | H | H | CH₂CH(CH₃)₂ | CH(CH₃) |
| 314 | H | H | H | CN | CH(CH₃) |
| 315 | H | H | H | c-C₃H₅ | CH(CH₃) |
| 316 | H | H | H | c-C₄H₇ | CH(CH₃) |
| 317 | H | H | H | c-C₅H₉ | CH(CH₃) |
| 318 | H | H | H | c-C₆H₁₁ | CH(CH₃) |
| 319 | H | H | F | H | CH(CH₃) |
| 320 | H | H | F | F | CH(CH₃) |
| 321 | H | H | F | Cl | CH(CH₃) |
| 322 | H | H | F | Br | CH(CH₃) |
| 323 | H | H | F | CH₃ | CH(CH₃) |
| 324 | H | H | F | CH₂CH₃ | CH(CH₃) |
| 325 | H | H | F | CH₂CH=CH₂ | CH(CH₃) |
| 326 | H | H | F | CH₂CH₂CH₃ | CH(CH₃) |
| 327 | H | H | F | CH(CH₃)₂ | CH(CH₃) |
| 328 | H | H | F | CH₂CH₂CH₂CH₃ | CH(CH₃) |
| 329 | H | H | F | CH(CH₃)CH₂CH₃ | CH(CH₃) |
| 330 | H | H | F | CH₂CH(CH₃)₂ | CH(CH₃) |
| 331 | H | H | F | CN | CH(CH₃) |
| 332 | H | H | F | c-C₃H₅ | CH(CH₃) |
| 333 | H | H | F | c-C₄H₇ | CH(CH₃) |
| 334 | H | H | F | c-C₅H₉ | CH(CH₃) |
| 335 | H | H | F | c-C₆H₁₁ | CH(CH₃) |
| 336 | H | H | Cl | H | CH(CH₃) |
| 337 | H | H | Cl | F | CH(CH₃) |
| 338 | H | H | Cl | Cl | CH(CH₃) |
| 339 | H | H | Cl | Br | CH(CH₃) |
| 340 | H | H | Cl | CH₃ | CH(CH₃) |
| 341 | H | H | Cl | CH₂CH₃ | CH(CH₃) |
| 342 | H | H | Cl | CH₂CH=CH₂ | CH(CH₃) |
| 343 | H | H | Cl | CH₂CH₂CH₃ | CH(CH₃) |
| 344 | H | H | Cl | CH(CH₃)₂ | CH(CH₃) |
| 345 | H | H | Cl | CH₂CH₂CH₂CH₃ | CH(CH₃) |
| 346 | H | H | Cl | CH(CH₃)CH₂CH₃ | CH(CH₃) |
| 347 | H | H | Cl | CH₂CH(CH₃)₂ | CH(CH₃) |
| 348 | H | H | Cl | CN | CH(CH₃) |
| 349 | H | H | Cl | c-C₃H₅ | CH(CH₃) |
| 350 | H | H | Cl | c-C₄H₇ | CH(CH₃) |
| 351 | H | H | Cl | c-C₅H₉ | CH(CH₃) |
| 352 | H | H | Cl | c-C₆H₁₁ | CH(CH₃) |
| 353 | H | H | Br | H | CH(CH₃) |
| 354 | H | H | Br | F | CH(CH₃) |
| 355 | H | H | Br | Cl | CH(CH₃) |
| 356 | H | H | Br | Br | CH(CH₃) |
| 357 | H | H | Br | CH₃ | CH(CH₃) |
| 358 | H | H | Br | CH₂CH₃ | CH(CH₃) |
| 359 | H | H | Br | CH₂CH=CH₂ | CH(CH₃) |
| 360 | H | H | Br | CH₂CH₂CH₃ | CH(CH₃) |
| 361 | H | H | Br | CH(CH₃)₂ | CH(CH₃) |
| 362 | H | H | Br | CH₂CH₂CH₂CH₃ | CH(CH₃) |
| 363 | H | H | Br | CH(CH₃)CH₂CH₃ | CH(CH₃) |
| 364 | H | H | Br | CH₂CH(CH₃)₂ | CH(CH₃) |
| 365 | H | H | Br | CN | CH(CH₃) |
| 366 | H | H | Br | c-C₃H₅ | CH(CH₃) |
| 367 | H | H | Br | c-C₄H₇ | CH(CH₃) |
| 368 | H | H | Br | c-C₅H₉ | CH(CH₃) |
| 369 | H | H | Br | c-C₆H₁₁ | CH(CH₃) |
| 370 | H | H | CH₃ | H | CH(CH₃) |
| 371 | H | H | CH₃ | F | CH(CH₃) |
| 372 | H | H | CH₃ | Cl | CH(CH₃) |
| 373 | H | H | CH₃ | Br | CH(CH₃) |
| 374 | H | H | CH₃ | CH₃ | CH(CH₃) |
| 375 | H | H | CH₃ | CH₂CH₃ | CH(CH₃) |
| 376 | H | H | CH₃ | CH₂CH=CH₂ | CH(CH₃) |
| 377 | H | H | CH₃ | CH₂CH₂CH₃ | CH(CH₃) |
| 378 | H | H | CH₃ | CH(CH₃)₂ | CH(CH₃) |
| 379 | H | H | CH₃ | CH₂CH₂CH₂CH₃ | CH(CH₃) |
| 380 | H | H | CH₃ | CH(CH₃)CH₂CH₃ | CH(CH₃) |
| 381 | H | H | CH₃ | CH₂CH(CH₃)₂ | CH(CH₃) |
| 382 | H | H | CH₃ | CN | CH(CH₃) |
| 383 | H | H | CH₃ | c-C₃H₅ | CH(CH₃) |
| 384 | H | H | CH₃ | c-C₄H₇ | CH(CH₃) |
| 385 | H | H | CH₃ | c-C₅H₉ | CH(CH₃) |
| 386 | H | H | CH₃ | c-C₆H₁₁ | CH(CH₃) |
| 387 | H | H | CH₂CH₃ | H | CH(CH₃) |
| 388 | H | H | CH₂CH₃ | F | CH(CH₃) |
| 389 | H | H | CH₂CH₃ | Cl | CH(CH₃) |
| 390 | H | H | CH₂CH₃ | Br | CH(CH₃) |
| 391 | H | H | CH₂CH₃ | CH₃ | CH(CH₃) |
| 392 | H | H | CH₂CH₃ | CH₂CH₃ | CH(CH₃) |
| 393 | H | H | CH₂CH₃ | CH₂CH=CH₂ | CH(CH₃) |
| 394 | H | H | CH₂CH₃ | CH₂CH₂CH₃ | CH(CH₃) |
| 395 | H | H | CH₂CH₃ | CH(CH₃)₂ | CH(CH₃) |
| 396 | H | H | CH₂CH₃ | CH₂CH₂CH₂CH₃ | CH(CH₃) |
| 397 | H | H | CH₂CH₃ | CH(CH₃)CH₂CH₃ | CH(CH₃) |
| 398 | H | H | CH₂CH₃ | CH₂CH(CH₃)₂ | CH(CH₃) |
| 399 | H | H | CH₂CH₃ | CN | CH(CH₃) |
| 400 | H | H | CH₂CH₃ | c-C₃H₅ | CH(CH₃) |
| 401 | H | H | CH₂CH₃ | c-C₄H₇ | CH(CH₃) |
| 402 | H | H | CH₂CH₃ | c-C₅H₉ | CH(CH₃) |
| 403 | H | H | CH₂CH₃ | c-C₆H₁₁ | CH(CH₃) |
| 404 | H | H | CH₂CH=CH₂ | H | CH(CH₃) |
| 405 | H | H | CH₂CH=CH₂ | F | CH(CH₃) |
| 406 | H | H | CH₂CH=CH₂ | Cl | CH(CH₃) |
| 407 | H | H | CH₂CH=CH₂ | Br | CH(CH₃) |
| 408 | H | H | CH₂CH=CH₂ | CH₃ | CH(CH₃) |
| 409 | H | H | CH₂CH=CH₂ | CH₂CH₃ | CH(CH₃) |
| 410 | H | H | CH₂CH=CH₂ | CH₂CH=CH₂ | CH(CH₃) |
| 411 | H | H | CH₂CH=CH₂ | CH₂CH₂CH₃ | CH(CH₃) |
| 412 | H | H | CH₂CH=CH₂ | CH(CH₃)₂ | CH(CH₃) |
| 413 | H | H | CH₂CH=CH₂ | CH₂CH₂CH₂CH₃ | CH(CH₃) |
| 414 | H | H | CH₂CH=CH₂ | CH(CH₃)CH₂CH₃ | CH(CH₃) |
| 415 | H | H | CH₂CH=CH₂ | CH₂CH(CH₃)₂ | CH(CH₃) |
| 416 | H | H | CH₂CH=CH₂ | CN | CH(CH₃) |
| 417 | H | H | CH₂CH=CH₂ | c-C₃H₅ | CH(CH₃) |
| 418 | H | H | CH₂CH=CH₂ | c-C₄H₇ | CH(CH₃) |
| 419 | H | H | CH₂CH=CH₂ | c-C₅H₉ | CH(CH₃) |
| 420 | H | H | CH₂CH=CH₂ | c-C₆H₁₁ | CH(CH₃) |
| 421 | CH₃ | H | H | H | - |
| 422 | CH₃ | H | H | F | - |
| 423 | CH₃ | H | H | Cl | - |
| 424 | CH₃ | H | H | Br | - |
| 425 | CH₃ | H | H | CH₃ | - |
| 426 | CH₃ | H | H | CH₂CH₃ | - |
| 427 | CH₃ | H | H | CH₂CH=CH₂ | - |
| 428 | CH₃ | H | H | CH₂CH₂CH₃ | - |
| 429 | CH₃ | H | H | CH(CH₃)₂ | - |
| 430 | CH₃ | H | H | CH₂CH₂CH₂CH₃ | - |
| 431 | CH₃ | H | H | CH(CH₃)CH₂CH₃ | - |
| 432 | CH₃ | H | H | CH₂CH(CH₃)₂ | - |
| 433 | CH₃ | H | H | CN | - |
| 434 | CH₃ | H | H | c-C₃H₅ | - |
| 435 | CH₃ | H | H | c-C₄H₇ | - |
| 436 | CH₃ | H | H | c-C₅H₉ | - |
| 437 | CH₃ | H | H | c-C₆H₁₁ | - |
| 438 | CH₃ | H | F | F | - |
| 439 | CH₃ | H | F | Cl | - |
| 440 | CH₃ | H | F | Br | - |
| 441 | CH₃ | H | F | CH₃ | - |
| 442 | CH₃ | H | F | CH₂CH₃ | - |
| 443 | CH₃ | H | F | CH₂CH=CH₂ | - |
| 444 | CH₃ | H | F | CH₂CH₂CH₃ | - |
| 445 | CH₃ | H | F | CH(CH₃)₂ | - |
| 446 | CH₃ | H | F | CH₂CH₂CH₂CH₃ | - |
| 447 | CH₃ | H | F | CH(CH₃)CH₂CH₃ | - |
| 448 | CH₃ | H | F | CH₂CH(CH₃)₂ | - |
| 449 | CH₃ | H | F | CN | - |
| 450 | CH₃ | H | F | c-C₃H₅ | - |
| 451 | CH₃ | H | F | c-C₄H₇ | - |
| 452 | CH₃ | H | F | c-C₅H₉ | - |
| 453 | CH₃ | H | F | c-C₆H₁₁ | - |
| 454 | CH₃ | H | Cl | Cl | - |
| 455 | CH₃ | H | Cl | Br | - |
| 456 | CH₃ | H | Cl | CH₃ | - |
| 457 | CH₃ | H | Cl | CH₂CH₃ | - |
| 458 | CH₃ | H | Cl | CH₂CH=CH₂ | - |
| 459 | CH₃ | H | Cl | CH₂CH₂CH₃ | - |
| 460 | CH₃ | H | Cl | CH(CH₃)₂ | - |
| 461 | CH₃ | H | Cl | CH₂CH₂CH₂CH₃ | - |
| 462 | CH₃ | H | Cl | CH(CH₃)CH₂CH₃ | - |
| 463 | CH₃ | H | Cl | CH₂CH(CH₃)₂ | - |
| 464 | CH₃ | H | Cl | CN | - |
| 465 | CH₃ | H | Cl | c-C₃H₅ | - |
| 466 | CH₃ | H | Cl | c-C₄H₇ | - |
| 467 | CH₃ | H | Cl | c-C₅H₉ | - |
| 468 | CH₃ | H | Cl | c-C₆H₁₁ | - |
| 469 | CH₃ | H | Br | Br | - |
| 470 | CH₃ | H | Br | CH₃ | - |
| 471 | CH₃ | H | Br | CH₂CH₃ | - |
| 472 | CH₃ | H | Br | CH₂CH=CH₂ | - |
| 473 | CH₃ | H | Br | CH₂CH₂CH₃ | - |
| 474 | CH₃ | H | Br | CH(CH₃)₂ | - |
| 475 | CH₃ | H | Br | CH₂CH₂CH₂CH₃ | - |
| 476 | CH₃ | H | Br | CH(CH₃)CH₂CH₃ | - |
| 477 | CH₃ | H | Br | CH₂CH(CH₃)₂ | - |
| 478 | CH₃ | H | Br | CN | - |
| 479 | CH₃ | H | Br | c-C₃H₅ | - |
| 480 | CH₃ | H | Br | c-C₄H₇ | - |
| 481 | CH₃ | H | Br | c-C₅H₉ | - |
| 482 | CH₃ | H | Br | c-C₆H₁₁ | - |
| 483 | CH₃ | H | CH₃ | CH₃ | - |
| 484 | CH₃ | H | CH₃ | CH₂CH₃ | - |
| 485 | CH₃ | H | CH₃ | CH₂CH=CH₂ | - |
| 486 | CH₃ | H | CH₃ | CH₂CH₂CH₃ | - |
| 487 | CH₃ | H | CH₃ | CH(CH₃)₂ | - |
| 488 | CH₃ | H | CH₃ | CH₂CH₂CH₂CH₃ | - |
| 489 | CH₃ | H | CH₃ | CH(CH₃)CH₂CH₃ | - |
| 490 | CH₃ | H | CH₃ | CH₂CH(CH₃)₂ | - |
| 491 | CH₃ | H | CH₃ | CN | - |
| 492 | CH₃ | H | CH₃ | c-C₃H₅ | - |
| 493 | CH₃ | H | CH₃ | c-C₄H₇ | - |
| 494 | CH₃ | H | CH₃ | c-C₅H₉ | - |
| 495 | CH₃ | H | CH₃ | c-C₆H₁₁ | - |
| 496 | CH₃ | H | CH₂CH₃ | CH₂CH₃ | - |
| 497 | CH₃ | H | CH₂CH₃ | CH₂CH=CH₂ | - |
| 498 | CH₃ | H | CH₂CH₃ | CH₂CH₂CH₃ | - |
| 499 | CH₃ | H | CH₂CH₃ | CH(CH₃)₂ | - |
| 500 | CH₃ | H | CH₂CH₃ | CH₂CH₂CH₂CH₃ | - |
| 501 | CH₃ | H | CH₂CH₃ | CH(CH₃)CH₂CH₃ | - |
| 502 | CH₃ | H | CH₂CH₃ | CH₂CH(CH₃)₂ | - |
| 503 | CH₃ | H | CH₂CH₃ | CN | - |
| 504 | CH₃ | H | CH₂CH₃ | c-C₃H₅ | - |
| 505 | CH₃ | H | CH₂CH₃ | c-C₄H₇ | - |
| 506 | CH₃ | H | CH₂CH₃ | c-C₅H₉ | - |
| 507 | CH₃ | H | CH₂CH₃ | c-C₆H₁₁ | - |
| 508 | CH₃ | H | CH₂CH=CH₂ | CH₂CH=CH₂ | - |
| 509 | CH₃ | H | CH₂CH=CH₂ | CH₂CH₂CH₃ | - |
| 510 | CH₃ | H | CH₂CH=CH₂ | CH(CH₃)₂ | - |
| 511 | CH₃ | H | CH₂CH=CH₂ | CH₂CH₂CH₂CH₃ | - |
| 512 | CH₃ | H | CH₂CH=CH₂ | CH(CH₃)CH₂CH₃ | - |
| 513 | CH₃ | H | CH₂CH=CH₂ | CH₂CH(CH₃)₂ | - |
| 514 | CH₃ | H | CH₂CH=CH₂ | CN | - |
| 515 | CH₃ | H | CH₂CH=CH₂ | c-C₃H₅ | - |
| 516 | CH₃ | H | CH₂CH=CH₂ | c-C₄H₇ | - |
| 517 | CH₃ | H | CH₂CH=CH₂ | c-C₅H₉ | - |
| 518 | CH₃ | H | CH₂CH=CH₂ | c-C₆H₁₁ | - |
| 519 | CH₃ | H | H | F | CH₂ |
| 520 | CH₃ | H | H | Cl | CH₂ |
| 521 | CH₃ | H | H | Br | CH₂ |
| 522 | CH₃ | H | H | CH₂CH=CH₂ | CH₂ |
| 523 | CH₃ | H | H | CH₂CH₂CH₂CH₃ | CH₂ |
| 524 | CH₃ | H | H | CH(CH₃)CH₂CH₃ | CH₂ |
| 525 | CH₃ | H | H | CH₂CH(CH₃)₂ | CH₂ |
| 526 | CH₃ | H | H | CN | CH₂ |
| 527 | CH₃ | H | H | c-C₃H₅ | CH₂ |
| 528 | CH₃ | H | H | c-C₄H₇ | CH₂ |
| 529 | CH₃ | H | H | c-C₅H₉ | CH₂ |
| 530 | CH₃ | H | H | c-C₆H₁₁ | CH₂ |
| 531 | CH₃ | H | F | F | CH₂ |
| 532 | CH₃ | H | F | Cl | CH₂ |
| 533 | CH₃ | H | F | Br | CH₂ |
| 534 | CH₃ | H | F | CH₂CH=CH₂ | CH₂ |
| 535 | CH₃ | H | F | CH₂CH₂CH₂CH₃ | CH₂ |
| 536 | CH₃ | H | F | CH(CH₃)CH₂CH₃ | CH₂ |
| 537 | CH₃ | H | F | CH₂CH(CH₃)₂ | CH₂ |
| 538 | CH₃ | H | F | CN | CH₂ |
| 539 | CH₃ | H | F | c-C₃H₅ | CH₂ |
| 540 | CH₃ | H | F | c-C₄H₇ | CH₂ |
| 541 | CH₃ | H | F | c-C₅H₉ | CH₂ |
| 542 | CH₃ | H | F | c-C₆H₁₁ | CH₂ |
| 543 | CH₃ | H | Cl | F | CH₂ |
| 544 | CH₃ | H | Cl | Cl | CH₂ |
| 545 | CH₃ | H | Cl | Br | CH₂ |
| 546 | CH₃ | H | Cl | CH₂CH=CH₂ | CH₂ |
| 547 | CH₃ | H | Cl | CH₂CH₂CH₂CH₃ | CH₂ |
| 548 | CH₃ | H | Cl | CH(CH₃)CH₂CH₃ | CH₂ |
| 549 | CH₃ | H | Cl | CH₂CH(CH₃)₂ | CH₂ |
| 550 | CH₃ | H | Cl | CN | CH₂ |
| 551 | CH₃ | H | Cl | c-C₃H₅ | CH₂ |
| 552 | CH₃ | H | Cl | c-C₄H₇ | CH₂ |
| 553 | CH₃ | H | Cl | c-C₅H₉ | CH₂ |
| 554 | CH₃ | H | Cl | c-C₆H₁₁ | CH₂ |
| 555 | CH₃ | H | Br | F | CH₂ |
| 556 | CH₃ | H | Br | Cl | CH₂ |
| 557 | CH₃ | H | Br | Br | CH₂ |
| 558 | CH₃ | H | Br | CH₂CH=CH₂ | CH₂ |
| 559 | CH₃ | H | Br | CH₂CH₂CH₂CH₃ | CH₂ |
| 560 | CH₃ | H | Br | CH(CH₃)CH₂CH₃ | CH₂ |
| 561 | CH₃ | H | Br | CH₂CH(CH₃)₂ | CH₂ |
| 562 | CH₃ | H | Br | CN | CH₂ |
| 563 | CH₃ | H | Br | c-C₃H₅ | CH₂ |
| 564 | CH₃ | H | Br | c-C₄H₇ | CH₂ |
| 565 | CH₃ | H | Br | c-C₅H₉ | CH₂ |
| 566 | CH₃ | H | Br | c-C₆H₁₁ | CH₂ |
| 567 | CH₃ | H | CH₃ | F | CH₂ |
| 568 | CH₃ | H | CH₃ | Cl | CH₂ |
| 569 | CH₃ | H | CH₃ | Br | CH₂ |
| 570 | CH₃ | H | CH₃ | CH₂CH=CH₂ | CH₂ |
| 571 | CH₃ | H | CH₃ | CH₂CH₂CH₂CH₃ | CH₂ |
| 572 | CH₃ | H | CH₃ | CH(CH₃)CH₂CH₃ | CH₂ |
| 573 | CH₃ | H | CH₃ | CH₂CH(CH₃)₂ | CH₂ |
| 574 | CH₃ | H | CH₃ | CN | CH₂ |
| 575 | CH₃ | H | CH₃ | c-C₃H₅ | CH₂ |
| 576 | CH₃ | H | CH₃ | c-C₄H₇ | CH₂ |
| 577 | CH₃ | H | CH₃ | c-C₅H₉ | CH₂ |
| 578 | CH₃ | H | CH₃ | c-C₆H₁₁ | CH₂ |
| 579 | CH₃ | H | CH₂CH₃ | F | CH₂ |
| 580 | CH₃ | H | CH₂CH₃ | Cl | CH₂ |
| 581 | CH₃ | H | CH₂CH₃ | Br | CH₂ |
| 582 | CH₃ | H | CH₂CH₃ | CH₂CH=CH₂ | CH₂ |
| 583 | CH₃ | H | CH₂CH₃ | CH₂CH₂CH₂CH₃ | CH₂ |
| 584 | CH₃ | H | CH₂CH₃ | CH(CH₃)CH₂CH₃ | CH₂ |
| 585 | CH₃ | H | CH₂CH₃ | CH₂CH(CH₃)₂ | CH₂ |
| 586 | CH₃ | H | CH₂CH₃ | CN | CH₂ |
| 587 | CH₃ | H | CH₂CH₃ | c-C₃H₅ | CH₂ |
| 588 | CH₃ | H | CH₂CH₃ | c-C₄H₇ | CH₂ |
| 589 | CH₃ | H | CH₂CH₃ | c-C₅H₉ | CH₂ |
| 590 | CH₃ | H | CH₂CH₃ | c-C₆H₁₁ | CH₂ |
| 591 | CH₃ | H | CH₂CH=CH₂ | F | CH₂ |
| 592 | CH₃ | H | CH₂CH=CH₂ | Cl | CH₂ |
| 593 | CH₃ | H | CH₂CH=CH₂ | Br | CH₂ |
| 594 | CH₃ | H | CH₂CH=CH₂ | CH₂CH=CH₂ | CH₂ |
| 595 | CH₃ | H | CH₂CH=CH₂ | CH₂CH₂CH₂CH₃ | CH₂ |
| 596 | CH₃ | H | CH₂CH=CH₂ | CH(CH₃)CH₂CH₃ | CH₂ |
| 597 | CH₃ | H | CH₂CH=CH₂ | CH₂CH(CH₃)₂ | CH₂ |
| 598 | CH₃ | H | CH₂CH=CH₂ | CN | CH₂ |
| 599 | CH₃ | H | CH₂CH=CH₂ | c-C₃H₅ | CH₂ |
| 600 | CH₃ | H | CH₂CH=CH₂ | c-C₄H₇ | CH₂ |
| 601 | CH₃ | H | CH₂CH=CH₂ | c-C₅H₉ | CH₂ |
| 602 | CH₃ | H | CH₂CH=CH₂ | c-C₆H₁₁ | CH₂ |
| 603 | CH₃ | H | H | H | C=O |
| 604 | CH₃ | H | H | F | C=O |
| 605 | CH₃ | H | H | Cl | C=O |
| 606 | CH₃ | H | H | Br | C=O |
| 607 | CH₃ | H | H | CH₃ | C=O |
| 608 | CH₃ | H | H | CH₂CH₃ | C=O |
| 609 | CH₃ | H | H | CH₂CH=CH₂ | C=O |
| 610 | CH₃ | H | H | CH₂CH₂CH₃ | C=O |
| 611 | CH₃ | H | H | CH(CH₃)₂ | C=O |
| 612 | CH₃ | H | H | CH₂CH₂CH₂CH₃ | C=O |
| 613 | CH₃ | H | H | CH(CH₃)CH₂CH₃ | C=O |
| 614 | CH₃ | H | H | CH₂CH(CH₃)₂ | C=O |
| 615 | CH₃ | H | H | CN | C=O |
| 616 | CH₃ | H | H | c-C₃H₅ | C=O |
| 617 | CH₃ | H | H | c-C₄H₇ | C=O |
| 618 | CH₃ | H | H | c-C₅H₉ | C=O |
| 619 | CH₃ | H | H | c-C₆H₁₁ | C=O |
| 620 | CH₃ | H | F | H | C=O |
| 621 | CH₃ | H | F | F | C=O |
| 622 | CH₃ | H | F | Cl | C=O |
| 623 | CH₃ | H | F | Br | C=O |
| 624 | CH₃ | H | F | CH₃ | C=O |
| 625 | CH₃ | H | F | CH₂CH₃ | C=O |
| 626 | CH₃ | H | F | CH₂CH=CH₂ | C=O |
| 627 | CH₃ | H | F | CH₂CH₂CH₃ | C=O |
| 628 | CH₃ | H | F | CH(CH₃)₂ | C=O |
| 629 | CH₃ | H | F | CH₂CH₂CH₂CH₃ | C=O |
| 630 | CH₃ | H | F | CH(CH₃)CH₂CH₃ | C=O |
| 631 | CH₃ | H | F | CH₂CH(CH₃)₂ | C=O |
| 632 | CH₃ | H | F | CN | C=O |
| 633 | CH₃ | H | F | c-C₃H₅ | C=O |
| 634 | CH₃ | H | F | c-C₄H₇ | C=O |
| 635 | CH₃ | H | F | c-C₅H₉ | C=O |
| 636 | CH₃ | H | F | c-C₆H₁₁ | C=O |
| 637 | CH₃ | H | Cl | H | C=O |
| 638 | CH₃ | H | Cl | F | C=O |
| 639 | CH₃ | H | Cl | Cl | C=O |
| 640 | CH₃ | H | Cl | Br | C=O |
| 641 | CH₃ | H | Cl | CH₃ | C=O |
| 642 | CH₃ | H | Cl | CH₂CH₃ | C=O |
| 643 | CH₃ | H | Cl | CH₂CH=CH₂ | C=O |
| 644 | CH₃ | H | Cl | CH₂CH₂CH₃ | C=O |
| 645 | CH₃ | H | Cl | CH(CH₃)₂ | C=O |
| 646 | CH₃ | H | Cl | CH₂CH₂CH₂CH₃ | C=O |
| 647 | CH₃ | H | Cl | CH(CH₃)CH₂CH₃ | C=O |
| 648 | CH₃ | H | Cl | CH₂CH(CH₃)₂ | C=O |
| 649 | CH₃ | H | Cl | CN | C=O |
| 650 | CH₃ | H | Cl | c-C₃H₅ | C=O |
| 651 | CH₃ | H | Cl | c-C₄H₇ | C=O |
| 652 | CH₃ | H | Cl | c-C₅H₉ | C=O |
| 653 | CH₃ | H | Cl | c-C₆H₁₁ | C=O |
| 654 | CH₃ | H | Br | H | C=O |
| 655 | CH₃ | H | Br | F | C=O |
| 656 | CH₃ | H | Br | Cl | C=O |
| 657 | CH₃ | H | Br | Br | C=O |
| 658 | CH₃ | H | Br | CH₃ | C=O |
| 659 | CH₃ | H | Br | CH₂CH₃ | C=O |
| 660 | CH₃ | H | Br | CH₂CH=CH₂ | C=O |
| 661 | CH₃ | H | Br | CH₂CH₂CH₃ | C=O |
| 662 | CH₃ | H | Br | CH(CH₃)₂ | C=O |
| 663 | CH₃ | H | Br | CH₂CH₂CH₂CH₃ | C=O |
| 664 | CH₃ | H | Br | CH(CH₃)CH₂CH₃ | C=O |
| 665 | CH₃ | H | Br | CH₂CH(CH₃)₂ | C=O |
| 666 | CH₃ | H | Br | CN | C=O |
| 667 | CH₃ | H | Br | c-C₃H₅ | C=O |
| 668 | CH₃ | H | Br | c-C₄H₇ | C=O |
| 669 | CH₃ | H | Br | c-C₅H₉ | C=O |
| 670 | CH₃ | H | Br | c-C₆H₁₁ | C=O |
| 671 | CH₃ | H | CH₃ | H | C=O |
| 672 | CH₃ | H | CH₃ | F | C=O |
| 673 | CH₃ | H | CH₃ | Cl | C=O |
| 674 | CH₃ | H | CH₃ | Br | C=O |
| 675 | CH₃ | H | CH₃ | CH₃ | C=O |
| 676 | CH₃ | H | CH₃ | CH₂CH₃ | C=O |
| 677 | CH₃ | H | CH₃ | CH₂CH=CH₂ | C=O |
| 678 | CH₃ | H | CH₃ | CH₂CH₂CH₃ | C=O |
| 679 | CH₃ | H | CH₃ | CH(CH₃)₂ | C=O |
| 680 | CH₃ | H | CH₃ | CH₂CH₂CH₂CH₃ | C=O |
| 681 | CH₃ | H | CH₃ | CH(CH₃)CH₂CH₃ | C=O |
| 682 | CH₃ | H | CH₃ | CH₂CH(CH₃)₂ | C=O |
| 683 | CH₃ | H | CH₃ | CN | C=O |
| 684 | CH₃ | H | CH₃ | c-C₃H₅ | C=O |
| 685 | CH₃ | H | CH₃ | c-C₄H₇ | C=O |
| 686 | CH₃ | H | CH₃ | c-C₅H₉ | C=O |
| 687 | CH₃ | H | CH₃ | c-C₆H₁₁ | C=O |
| 688 | CH₃ | H | CH₂CH₃ | H | C=O |
| 689 | CH₃ | H | CH₂CH₃ | F | C=O |
| 690 | CH₃ | H | CH₂CH₃ | Cl | C=O |
| 691 | CH₃ | H | CH₂CH₃ | Br | C=O |
| 692 | CH₃ | H | CH₂CH₃ | CH₃ | C=O |
| 693 | CH₃ | H | CH₂CH₃ | CH₂CH₃ | C=O |
| 694 | CH₃ | H | CH₂CH₃ | CH₂CH=CH₂ | C=O |
| 695 | CH₃ | H | CH₂CH₃ | CH₂CH₂CH₃ | C=O |
| 696 | CH₃ | H | CH₂CH₃ | CH(CH₃)₂ | C=O |
| 697 | CH₃ | H | CH₂CH₃ | CH₂CH₂CH₂CH₃ | C=O |
| 698 | CH₃ | H | CH₂CH₃ | CH(CH₃)CH₂CH₃ | C=O |
| 699 | CH₃ | H | CH₂CH₃ | CH₂CH(CH₃)₂ | C=O |
| 700 | CH₃ | H | CH₂CH₃ | CN | C=O |
| 701 | CH₃ | H | CH₂CH₃ | c-C₃H₅ | C=O |
| 702 | CH₃ | H | CH₂CH₃ | c-C₄H₇ | C=O |
| 703 | CH₃ | H | CH₂CH₃ | c-C₅H₉ | C=O |
| 704 | CH₃ | H | CH₂CH₃ | c-C₆H₁₁ | C=O |
| 705 | CH₃ | H | CH₂CH=CH₂ | H | C=O |
| 706 | CH₃ | H | CH₂CH=CH₂ | F | C=O |
| 707 | CH₃ | H | CH₂CH=CH₂ | Cl | C=O |
| 708 | CH₃ | H | CH₂CH=CH₂ | Br | C=O |
| 709 | CH₃ | H | CH₂CH=CH₂ | CH₃ | C=O |
| 710 | CH₃ | H | CH₂CH=CH₂ | CH₂CH₃ | C=O |
| 711 | CH₃ | H | CH₂CH=CH₂ | CH₂CH=CH₂ | C=O |
| 712 | CH₃ | H | CH₂CH=CH₂ | CH₂CH₂CH₃ | C=O |
| 713 | CH₃ | H | CH₂CH=CH₂ | CH(CH₃)₂ | C=O |
| 714 | CH₃ | H | CH₂CH=CH₂ | CH₂CH₂CH₂CH₃ | C=O |
| 715 | CH₃ | H | CH₂CH=CH₂ | CH(CH₃)CH₂CH₃ | C=O |
| 716 | CH₃ | H | CH₂CH=CH₂ | CH₂CH(CH₃)₂ | C=O |
| 717 | CH₃ | H | CH₂CH=CH₂ | CN | C=O |
| 718 | CH₃ | H | CH₂CH=CH₂ | c-C₃H₅ | C=O |
| 719 | CH₃ | H | CH₂CH=CH₂ | c-C₄H₇ | C=O |
| 720 | CH₃ | H | CH₂CH=CH₂ | c-C₅H₉ | C=O |
| 721 | CH₃ | H | CH₂CH=CH₂ | c-C₆H₁₁ | C=O |
| 722 | CH₃ | H | H | H | CH(CH₃) |
| 723 | CH₃ | H | H | F | CH(CH₃) |
| 724 | CH₃ | H | H | Cl | CH(CH₃) |
| 725 | CH₃ | H | H | Br | CH(CH₃) |
| 726 | CH₃ | H | H | CH₃ | CH(CH₃) |
| 727 | CH₃ | H | H | CH₂CH₃ | CH(CH₃) |
| 728 | CH₃ | H | H | CH₂CH=CH₂ | CH(CH₃) |
| 729 | CH₃ | H | H | CH₂CH₂CH₃ | CH(CH₃) |
| 730 | CH₃ | H | H | CH(CH₃)₂ | CH(CH₃) |
| 731 | CH₃ | H | H | CH₂CH₂CH₂CH₃ | CH(CH₃) |
| 732 | CH₃ | H | H | CH(CH₃)CH₂CH₃ | CH(CH₃) |
| 733 | CH₃ | H | H | CH₂CH(CH₃)₂ | CH(CH₃) |
| 734 | CH₃ | H | H | CN | CH(CH₃) |
| 735 | CH₃ | H | H | c-C₃H₅ | CH(CH₃) |
| 736 | CH₃ | H | H | c-C₄H₇ | CH(CH₃) |
| 737 | CH₃ | H | H | c-C₅H₉ | CH(CH₃) |
| 738 | CH₃ | H | H | c-C₆H₁₁ | CH(CH₃) |
| 739 | CH₃ | H | F | H | CH(CH₃) |
| 740 | CH₃ | H | F | F | CH(CH₃) |
| 741 | CH₃ | H | F | Cl | CH(CH₃) |
| 742 | CH₃ | H | F | Br | CH(CH₃) |
| 743 | CH₃ | H | F | CH₃ | CH(CH₃) |
| 744 | CH₃ | H | F | CH₂CH₃ | CH(CH₃) |
| 745 | CH₃ | H | F | CH₂CH=CH₂ | CH(CH₃) |
| 746 | CH₃ | H | F | CH₂CH₂CH₃ | CH(CH₃) |
| 747 | CH₃ | H | F | CH(CH₃)₂ | CH(CH₃) |
| 748 | CH₃ | H | F | CH₂CH₂CH₂CH₃ | CH(CH₃) |
| 749 | CH₃ | H | F | CH(CH₃)CH₂CH₃ | CH(CH₃) |
| 750 | CH₃ | H | F | CH₂CH(CH₃)₂ | CH(CH₃) |
| 751 | CH₃ | H | F | CN | CH(CH₃) |
| 752 | CH₃ | H | F | c-C₃H₅ | CH(CH₃) |
| 753 | CH₃ | H | F | c-C₄H₇ | CH(CH₃) |
| 754 | CH₃ | H | F | c-C₅H₉ | CH(CH₃) |
| 755 | CH₃ | H | F | c-C₆H₁₁ | CH(CH₃) |
| 756 | CH₃ | H | Cl | H | CH(CH₃) |
| 757 | CH₃ | H | Cl | F | CH(CH₃) |
| 758 | CH₃ | H | Cl | Cl | CH(CH₃) |
| 759 | CH₃ | H | Cl | Br | CH(CH₃) |
| 760 | CH₃ | H | Cl | CH₃ | CH(CH₃) |
| 761 | CH₃ | H | Cl | CH₂CH₃ | CH(CH₃) |
| 762 | CH₃ | H | Cl | CH₂CH=CH₂ | CH(CH₃) |
| 763 | CH₃ | H | Cl | CH₂CH₂CH₃ | CH(CH₃) |
| 764 | CH₃ | H | Cl | CH(CH₃)₂ | CH(CH₃) |
| 765 | CH₃ | H | Cl | CH₂CH₂CH₂CH₃ | CH(CH₃) |
| 766 | CH₃ | H | Cl | CH(CH₃)CH₂CH₃ | CH(CH₃) |
| 767 | CH₃ | H | Cl | CH₂CH(CH₃)₂ | CH(CH₃) |
| 768 | CH₃ | H | Cl | CN | CH(CH₃) |
| 769 | CH₃ | H | Cl | c-C₃H₅ | CH(CH₃) |
| 770 | CH₃ | H | Cl | c-C₄H₇ | CH(CH₃) |
| 771 | CH₃ | H | Cl | c-C₅H₉ | CH(CH₃) |
| 772 | CH₃ | H | Cl | c-C₆H₁₁ | CH(CH₃) |
| 773 | CH₃ | H | Br | H | CH(CH₃) |
| 774 | CH₃ | H | Br | F | CH(CH₃) |
| 775 | CH₃ | H | Br | Cl | CH(CH₃) |
| 776 | CH₃ | H | Br | Br | CH(CH₃) |
| 777 | CH₃ | H | Br | CH₃ | CH(CH₃) |
| 778 | CH₃ | H | Br | CH₂CH₃ | CH(CH₃) |
| 779 | CH₃ | H | Br | CH₂CH=CH₂ | CH(CH₃) |
| 780 | CH₃ | H | Br | CH₂CH₂CH₃ | CH(CH₃) |
| 781 | CH₃ | H | Br | CH(CH₃)₂ | CH(CH₃) |
| 782 | CH₃ | H | Br | CH₂CH₂CH₂CH₃ | CH(CH₃) |
| 783 | CH₃ | H | Br | CH(CH₃)CH₂CH₃ | CH(CH₃) |
| 784 | CH₃ | H | Br | CH₂CH(CH₃)₂ | CH(CH₃) |
| 785 | CH₃ | H | Br | CN | CH(CH₃) |
| 786 | CH₃ | H | Br | c-C₃H₅ | CH(CH₃) |
| 787 | CH₃ | H | Br | c-C₄H₇ | CH(CH₃) |
| 788 | CH₃ | H | Br | c-C₅H₉ | CH(CH₃) |
| 789 | CH₃ | H | Br | c-C₆H₁₁ | CH(CH₃) |
| 790 | CH₃ | H | CH₃ | H | CH(CH₃) |
| 791 | CH₃ | H | CH₃ | F | CH(CH₃) |
| 792 | CH₃ | H | CH₃ | Cl | CH(CH₃) |
| 793 | CH₃ | H | CH₃ | Br | CH(CH₃) |
| 794 | CH₃ | H | CH₃ | CH₃ | CH(CH₃) |
| 795 | CH₃ | H | CH₃ | CH₂CH₃ | CH(CH₃) |
| 796 | CH₃ | H | CH₃ | CH₂CH=CH₂ | CH(CH₃) |
| 797 | CH₃ | H | CH₃ | CH₂CH₂CH₃ | CH(CH₃) |
| 798 | CH₃ | H | CH₃ | CH(CH₃)₂ | CH(CH₃) |
| 799 | CH₃ | H | CH₃ | CH₂CH₂CH₂CH₃ | CH(CH₃) |
| 800 | CH₃ | H | CH₃ | CH(CH₃)CH₂CH₃ | CH(CH₃) |
| 801 | CH₃ | H | CH₃ | CH₂CH(CH₃)₂ | CH(CH₃) |
| 802 | CH₃ | H | CH₃ | CN | CH(CH₃) |
| 803 | CH₃ | H | CH₃ | c-C₃H₅ | CH(CH₃) |
| 804 | CH₃ | H | CH₃ | c-C₄H₇ | CH(CH₃) |
| 805 | CH₃ | H | CH₃ | c-C₅H₉ | CH(CH₃) |
| 806 | CH₃ | H | CH₃ | c-C₆H₁₁ | CH(CH₃) |
| 807 | CH₃ | H | CH₂CH₃ | H | CH(CH₃) |
| 808 | CH₃ | H | CH₂CH₃ | F | CH(CH₃) |
| 809 | CH₃ | H | CH₂CH₃ | Cl | CH(CH₃) |
| 810 | CH₃ | H | CH₂CH₃ | Br | CH(CH₃) |
| 811 | CH₃ | H | CH₂CH₃ | CH₃ | CH(CH₃) |
| 812 | CH₃ | H | CH₂CH₃ | CH₂CH₃ | CH(CH₃) |
| 813 | CH₃ | H | CH₂CH₃ | CH₂CH=CH₂ | CH(CH₃) |
| 814 | CH₃ | H | CH₂CH₃ | CH₂CH₂CH₃ | CH(CH₃) |
| 815 | CH₃ | H | CH₂CH₃ | CH(CH₃)₂ | CH(CH₃) |
| 816 | CH₃ | H | CH₂CH₃ | CH₂CH₂CH₂CH₃ | CH(CH₃) |
| 817 | CH₃ | H | CH₂CH₃ | CH(CH₃)CH₂CH₃ | CH(CH₃) |
| 818 | CH₃ | H | CH₂CH₃ | CH₂CH(CH₃)₂ | CH(CH₃) |
| 819 | CH₃ | H | CH₂CH₃ | CN | CH(CH₃) |
| 820 | CH₃ | H | CH₂CH₃ | c-C₃H₅ | CH(CH₃) |
| 821 | CH₃ | H | CH₂CH₃ | c-C₄H₇ | CH(CH₃) |
| 822 | CH₃ | H | CH₂CH₃ | c-C₅H₉ | CH(CH₃) |
| 823 | CH₃ | H | CH₂CH₃ | c-C₆H₁₁ | CH(CH₃) |
| 824 | CH₃ | H | CH₂CH=CH₂ | H | CH(CH₃) |
| 825 | CH₃ | H | CH₂CH=CH₂ | F | CH(CH₃) |
| 826 | CH₃ | H | CH₂CH=CH₂ | Cl | CH(CH₃) |
| 827 | CH₃ | H | CH₂CH=CH₂ | Br | CH(CH₃) |
| 828 | CH₃ | H | CH₂CH=CH₂ | CH₃ | CH(CH₃) |
| 829 | CH₃ | H | CH₂CH=CH₂ | CH₂CH₃ | CH(CH₃) |
| 830 | CH₃ | H | CH₂CH=CH₂ | CH₂CH=CH₂ | CH(CH₃) |
| 831 | CH₃ | H | CH₂CH=CH₂ | CH₂CH₂CH₃ | CH(CH₃) |
| 832 | CH₃ | H | CH₂CH=CH₂ | CH(CH₃)₂ | CH(CH₃) |
| 833 | CH₃ | H | CH₂CH=CH₂ | CH₂CH₂CH₂CH₃ | CH(CH₃) |
| 834 | CH₃ | H | CH₂CH=CH₂ | CH(CH₃)CH₂CH₃ | CH(CH₃) |
| 835 | CH₃ | H | CH₂CH=CH₂ | CH₂CH(CH₃)₂ | CH(CH₃) |
| 836 | CH₃ | H | CH₂CH=CH₂ | CN | CH(CH₃) |
| 837 | CH₃ | H | CH₂CH=CH₂ | c-C₃H₅ | CH(CH₃) |
| 838 | CH₃ | H | CH₂CH=CH₂ | c-C₄H₇ | CH(CH₃) |
| 839 | CH₃ | H | CH₂CH=CH₂ | c-C₅H₉ | CH(CH₃) |
| 840 | CH₃ | H | CH₂CH=CH₂ | c-C₆H₁₁ | CH(CH₃) |
| 841 | H | H | CH₂-CH₂ | | - |
| 842 | H | H | CHF-CHF | | - |
| 843 | H | H | CF₂-CF₂ | | - |
| 844 | H | H | CHCl-CHCl | | - |
| 845 | H | H | CCl₂-CCl₂ | | - |
| 846 | H | H | CH(CH₃)-CH(CH₃) | | - |
| 847 | H | H | C(CH₃)₂-C(CH₃)₂ | | - |
| 848 | H | H | CH₂-CH₂-CH₂ | | - |
| 849 | H | H | CH₂-CHF-CH₂ | | - |
| 850 | H | H | CHF-CH₂-CHF | | - |
| 851 | H | H | CHF-CHF-CHF | | - |
| 852 | H | H | CH₂-CF₂-CH₂ | | - |
| 853 | H | H | CH₂-CHCl-CH₂ | | - |
| 854 | H | H | CH₂-CCl₂-CH₂ | | - |
| 855 | H | H | CHCl-CH₂-CHCl | | - |
| 856 | H | H | CHCl-CHCl-CHCl | | - |
| 857 | H | H | CCl₂-CH₂-CCl₂ | | - |
| 858 | H | H | CCl₂-CCl₂-CCl₂ | | - |
| 859 | H | H | CH(CH₃)-CH₂-CH(CH₃) | | - |
| 860 | H | H | CH(CH₃)-CH(CH₃)-CH(CH₃) | | - |
| 861 | H | H | C(CH₃)₂-CH₂-C(CH₃)₂ | | - |
| 862 | H | H | C(CH₃)₂-C(CH₃)₂-C(CH₃)₂ | | - |
| 863 | H | H | CH₂-CH(CH₃)-CH₂ | | - |
| 864 | H | H | CH₂-C(CH₃)₂-CH₂ | | - |
| 865 | H | H | CHF-CH₂ | | - |
| 866 | H | H | CF₂-CH₂ | | - |
| 867 | H | H | CF₂-CHF | | - |
| 868 | H | H | CHCl-CH₂ | | - |
| 869 | H | H | CCl₂-CH₂ | | - |
| 870 | H | H | CCl₂-CHCl | | - |
| 871 | H | H | CH(CH₃)-CH₂ | | - |
| 872 | H | H | C(CH₃)₂-CH₂ | | - |
| 873 | H | H | C(CH₃)₂-CH(CH₃) | | - |
| 874 | H | H | CH(OCH₃)-CH₂ | | - |
| 875 | H | H | CH(CH₂CH₃)-CH₂ | | - |
| 876 | H | H | CH(CH₂CH₂CH₃)-CH₂ | | - |
| 877 | H | H | CH(CH₂CH₂CH₂CH₃)-CH₂ | | - |
| 878 | H | H | CH(C₆H₅)-CH₂ | | - |
| 879 | H | H | CH(2-Cl-C₆H₄)-CH₂ | | - |
| 880 | H | H | CH(2-F-C₆H₄)-CH₂ | | - |
| 881 | H | H | CH(2-CN-C₆H₄)-CH₂ | | - |
| 882 | H | H | CH(2-CH₃-C₆H₄)-CH₂ | | - |
| 883 | H | H | CH(2-OCH₃-C₆H₄)-CH₂ | | - |
| 884 | H | H | CH(3-Cl-C₆H₄)-CH₂ | | - |
| 885 | H | H | CH(3-F-C₆H₄)-CH₂ | | - |
| 886 | H | H | CH(3-CN-C₆H₄)-CH₂ | | - |
| 887 | H | H | CH(3-CH₃-C₆H₄)-CH₂ | | - |
| 888 | H | H | CH(3-OCH₃-C₆H₄)-CH₂ | | - |
| 889 | H | H | CH(4-Cl-C₆H₄)-CH₂ | | - |
| 890 | H | H | CH(4-F-C₆H₄)-CH₂ | | - |
| 891 | H | H | CH(4-CN-C₆H₄)-CH₂ | | - |
| 892 | H | H | CH(4-CH₃-C₆H₄)-CH₂ | | - |
| 893 | H | H | CH(4-OCH₃-C₆H₄)-CH₂ | | - |
| 894 | H | H | CH(CH₃)-CH₂-CH₂ | | - |
| 895 | H | H | C(CH₃)₂-CH₂-CH₂ | | - |
| 896 | H | H | CH(CH₃)-CH(CH₃)-CH₂ | | - |
| 897 | H | H | C(CH₃)₂-CH(CH₃)-CH₂ | | - |
| 898 | H | H | CH(CH₃)-C(CH₃)₂-CH₂ | | - |
| 899 | H | H | C(CH₃)₂-C(CH₃)₂-CH₂ | | - |
| 900 | H | H | CHF-CH₂-CH₂ | | - |
| 901 | H | H | CF₂-CH₂-CH₂ | | - |
| 902 | H | H | CHF-CHF-CH₂ | | - |
| 903 | H | H | CF₂-CHF-CH₂ | | - |
| 904 | H | H | CHF-CF₂-CH₂ | | - |
| 905 | H | H | CF₂-CH₂-CF₂ | | - |
| 906 | H | H | CF₂-CF₂-CH₂ | | - |
| 907 | H | H | CF₂-CF₂-CF₂ | | - |
| 908 | H | H | CHCl-CH₂-CH₂ | | - |
| 909 | H | H | CCl₂-CH₂-CH₂ | | - |
| 910 | H | H | CHCl-CHCl-CH₂ | | - |
| 911 | H | H | CCl₂-CHCl-CH₂ | | - |
| 912 | H | H | CHCl-CCl₂-CH₂ | | - |
| 913 | H | H | CCl₂-CCl₂-CH₂ | | - |
| 914 | H | H | CH₂O | | - |
| 915 | H | H | CH(CH₃)-O | | - |
| 916 | H | H | (CH₃)₂-O | | - |
| 917 | H | H | CH₂-O-CH₂ | | - |
| 918 | H | H | CH₂-CH₂-O | | - |
| 919 | CH₃ | H | CH₂-CH₂ | | - |
| 920 | CH₃ | H | CHF-CHF | | - |
| 921 | CH₃ | H | CF₂-CF₂ | | - |
| 922 | CH₃ | H | CHCl-CHCl | | - |
| 923 | CH₃ | H | CCl₂-CCl₂ | | - |
| 924 | CH₃ | H | CH(CH₃)-CH(CH₃) | | - |
| 925 | CH₃ | H | C(CH₃)₂-C(CH₃)₂ | | - |
| 926 | CH₃ | H | CH₂-CH₂-CH₂ | | - |
| 927 | CH₃ | H | CH₂-CHF-CH₂ | | - |
| 928 | CH₃ | H | CHF-CH₂-CHF | | - |
| 929 | CH₃ | H | CHF-CHF-CHF | | - |
| 930 | CH₃ | H | CH₂-CF₂-CH₂ | | - |
| 931 | CH₃ | H | CH₂-CHCl-CH₂ | | - |
| 932 | CH₃ | H | CH₂-CCl₂-CH₂ | | - |
| 933 | CH₃ | H | CHCl-CH₂-CHCl | | - |
| 934 | CH₃ | H | CHCl-CHCl-CHCl | | - |
| 935 | CH₃ | H | CCl₂-CH₂-CCl₂ | | - |
| 936 | CH₃ | H | CCl₂-CCl₂-CCl₂ | | - |
| 937 | CH₃ | H | CH(CH₃)-CH₂-CH(CH₃) | | - |
| 938 | CH₃ | H | CH(CH₃)-CH(CH₃)-CH(CH₃) | | - |
| 939 | CH₃ | H | C(CH₃)₂-CH₂-C(CH₃)₂ | | - |
| 940 | CH₃ | H | C(CH₃)₂-C(CH₃)₂-C(CH₃)₂ | | - |
| 941 | CH₃ | H | CH₂-CH(CH₃)-CH₂ | | - |
| 942 | CH₃ | H | CH₂-C(CH₃)₂-CH₂ | | - |
| 943 | CH₃ | H | CHF-CH₂ | | - |
| 944 | CH₃ | H | CF₂-CH₂ | | - |
| 945 | CH₃ | H | CF₂-CHF | | - |
| 946 | CH₃ | H | CHCl-CH₂ | | - |
| 947 | CH₃ | H | CCl₂-CH₂ | | - |
| 948 | CH₃ | H | CCl₂-CHCl | | - |
| 949 | CH₃ | H | CH(CH₃)-CH₂ | | - |
| 950 | CH₃ | H | C(CH₃)₂-CH₂ | | - |
| 951 | CH₃ | H | C(CH₃)₂-CH(CH₃) | | - |
| 952 | CH₃ | H | CH(OCH₃)-CH₂ | | - |
| 953 | CH₃ | H | CH(CH₂CH₃)-CH₂ | | - |
| 954 | CH₃ | H | CH(CH₂CH₂CH₃)-CH₂ | | - |
| 955 | CH₃ | H | CH(CH₂CH₂CH₂CH₃)-CH₂ | | - |
| 956 | CH₃ | H | CH(C₆H₅)-CH₂ | | - |
| 957 | CH₃ | H | CH(2-Cl-C₆H₄)-CH₂ | | - |
| 958 | CH₃ | H | CH(2-F-C₆H₄)-CH₂ | | - |
| 959 | CH₃ | H | CH(2-CN-C₆H₄)-CH₂ | | - |
| 960 | CH₃ | H | CH(2-CH₃-C₆H₄)-CH₂ | | - |
| 961 | CH₃ | H | CH(2-OCH₃-C₆H₄)-CH₂ | | - |
| 962 | CH₃ | H | CH(3-Cl-C₆H₄)-CH₂ | | - |
| 963 | CH₃ | H | CH(3-F-C₆H₄)-CH₂ | | - |
| 964 | CH₃ | H | CH(3-CN-C₆H₄)-CH₂ | | - |
| 965 | CH₃ | H | CH(3-CH₃-C₆H₄)-CH₂ | | - |
| 966 | CH₃ | H | CH(3-OCH₃-C₆H₄)-CH₂ | | - |
| 967 | CH₃ | H | CH(4-Cl-C₆H₄)-CH₂ | | - |
| 968 | CH₃ | H | CH(4-F-C₆H₄)-CH₂ | | - |
| 969 | CH₃ | H | CH(4-CN-C₆H₄)-CH₂ | | - |
| 970 | CH₃ | H | CH(4-CH₃-C₆H₄)-CH₂ | | - |
| 971 | CH₃ | H | CH(4-OCH₃-C₆H₄)-CH₂ | | - |
| 972 | CH₃ | H | CH(CH₃)-CH₂-CH₂ | | - |
| 973 | CH₃ | H | C(CH₃)₂-CH₂-CH₂ | | - |
| 974 | CH₃ | H | CH(CH₃)-CH(CH₃)-CH₂ | | - |
| 975 | CH₃ | H | C(CH₃)₂-CH(CH₃)-CH₂ | | - |
| 976 | CH₃ | H | CH(CH₃)-C(CH₃)₂-CH₂ | | - |
| 977 | CH₃ | H | C(CH₃)₂-C(CH₃)₂-CH₂ | | - |
| 978 | CH₃ | H | CHF-CH₂-CH₂ | | - |
| 979 | CH₃ | H | CF₂-CH₂-CH₂ | | - |
| 980 | CH₃ | H | CHF-CHF-CH₂ | | - |
| 981 | CH₃ | H | CF₂-CHF-CH₂ | | - |
| 982 | CH₃ | H | CHF-CF₂-CH₂ | | - |
| 983 | CH₃ | H | CF₂-CH₂-CF₂ | | - |
| 984 | CH₃ | H | CF₂-CF₂-CH₂ | | - |
| 985 | CH₃ | H | CF₂-CF₂-CF₂ | | - |
| 986 | CH₃ | H | CHCl-CH₂-CH₂ | | - |
| 987 | CH₃ | H | CCl₂-CH₂-CH₂ | | - |
| 988 | CH₃ | H | CHCl-CHCl-CH₂ | | - |
| 989 | CH₃ | H | CCl₂-CHCl-CH₂ | | - |
| 990 | CH₃ | H | CHCl-CCl₂-CH₂ | | - |
| 991 | CH₃ | H | CCl₂-CCl₂-CH₂ | | - |
| 992 | CH₃ | H | CH₂O | | - |
| 993 | CH₃ | H | CH(CH₃)-O | | - |
| 994 | CH₃ | H | (CH₃)₂-O | | - |
| 995 | CH₃ | H | CH₂-O-CH₂ | | - |
| 996 | CH₃ | H | CH₂-CH₂-O | | - |

**Table B:**

| m=1 and R³ and R^{4a} form both together and together with the existing bond a double bond between the adjacent carbon atoms. | | | | |
|---|---|---|---|---|
| # | R¹ | R² | R^{4b} | R⁵ |
| 1 | H | H | H | H |
| 2 | H | H | H | CH₃ |
| 3 | H | H | H | CH₂CH₃ |
| 4 | H | H | H | CH₂CH₂CH₃ |
| 5 | H | H | H | CH(CH₃)₂ |

| # | R¹ | R² | R^{4b} | R⁵ |
|---|---|---|---|---|
| 6 | H | H | H | CH₂CH₂CH₂CH₃ |
| 7 | H | H | H | CH(CH₃)CH₂CH₃ |
| 8 | H | H | H | CH₂CH(CH₃)₂ |
| 9 | H | H | H | CH₂CN |
| 10 | H | H | H | O-CH₃ |
| 11 | H | H | H | O-CH₂CH₃ |
| 12 | H | H | H | O-CH₂CH₂CH₃ |
| 13 | H | H | H | O-CH(CH₃)₂ |
| 14 | H | H | H | O-CH₂CH₂CH₂CH₃ |
| 15 | H | H | H | O-CH(CH₃)CH₂CH₃ |
| 16 | H | H | H | O-CH₂CH(CH₃)₂ |
| 17 | H | H | H | O-CH₂CN |
| 18 | H | H | H | S-CH₃ |
| 19 | H | H | H | S-CH₂CH₃ |
| 20 | H | H | H | S-CH₂CH₂CH₃ |
| 21 | H | H | H | S-CH(CH₃)₂ |
| 22 | H | H | H | S-CH₂CH₂CH₂CH₃ |
| 23 | H | H | H | S-CH(CH₃)CH₂CH₃ |
| 24 | H | H | H | S-CH₂CH(CH₃)₂ |
| 25 | H | H | H | S-CH₂CN |
| 26 | H | H | H | C₆H₅ |
| 27 | H | H | H | 4-Cl-C₆H₄ |
| 28 | H | H | H | 4-F-C₆H₄ |
| 29 | H | H | H | 4-Br-C₆H₄ |
| 30 | H | H | H | 3-Cl-C₆H₄ |
| 31 | H | H | H | 3-F-C₆H₄ |
| 32 | H | H | H | 3-Br-C₆H₄ |
| 33 | H | H | H | 3,4-Cl₂-C₆H₃ |
| 34 | H | H | H | 3,4-F₂-C₆H₃ |
| 35 | H | H | H | 3-Cl-4-F-C₆H₃ |
| 36 | H | H | H | 4-Cl-3-F-C₆H₃ |
| 37 | H | H | H | 3-Br-4-F-C₆H₃ |
| 38 | H | H | H | 4-Br-3-F-C₆H₃ |
| 39 | H | H | H | 3-Br-4-Cl-C₆H₃ |
| 40 | H | H | H | 4-Br-3-Cl-C₆H₃ |
| 41 | H | H | H | NH-CH₃ |
| 42 | H | H | H | NH-CH₂CH₃ |
| 43 | H | H | H | NH-CH₂CH₂CH₃ |
| 44 | H | H | H | NH-CH(CH₃)₂ |
| 45 | H | H | H | NH-CH₂CH₂CH₂CH₃ |
| 46 | H | H | H | NH-CH(CH₃)CH₂CH₃ |
| 47 | H | H | H | NH-CH₂CH(CH₃)₂ |
| 48 | H | H | H | N(CH₃)-CH₃ |
| 49 | H | H | H | N(CH₃)-CH₂CH₃ |
| 50 | H | H | H | N(CH₃)-CH₂CH₂CH₃ |
| 51 | H | H | H | N(CH₃)-CH(CH₃)₂ |
| 52 | H | H | H | N(CH₃)-CH₂CH₂CH₂CH₃ |
| 53 | H | H | H | N(CH₃)-CH(CH₃)CH₂CH₃ |
| 54 | H | H | H | N(CH₃)-CH₂CH(CH₃)₂ |
| 55 | H | H | H | NH-CH₂C₆H₅ |
| 56 | H | H | H | NH-CH₂(4-Cl-C₆H₄) |
| 57 | H | H | H | NH-CH₂(4-F-C₆H₄) |
| 58 | H | H | H | NH-CH₂(4-Br-C₆H₄) |
| 59 | H | H | H | NH-CH₂(3-Cl-C₆H₄) |
| 60 | H | H | H | NH-CH₂(3-F-C₆H₄) |
| 61 | H | H | H | NH-CH₂(3-Br-C₆H₄) |
| 62 | H | H | H | NH-CH₂(3,4-Cl₂-C₆H₃) |
| 63 | H | H | H | NH-CH₂(3,4-F₂-C₆H₃) |
| 64 | H | H | H | NH-CH₂(3-Cl-4-F-C₆H₃) |
| 65 | H | H | H | NH-CH₂(4-Cl-3-F-C₆H₃) |
| 66 | H | H | H | NH-CH₂(3-Br-4-F-C₆H₃) |
| 67 | H | H | H | NH-CH₂(4-Br-3-F-C₆H₃) |
| 68 | H | H | H | NH-CH₂(3-Br-4-Cl-C₆H₃) |
| 69 | H | H | H | NH-CH₂(4-Br-3-Cl-C₆H₃) |
| 70 | H | H | H | N(CH₃)-CH₂C₆H₅ |
| 71 | H | H | H | N(CH₃)-CH₂(4-Cl-C₆H₄) |
| 72 | H | H | H | N(CH₃)-CH₂(4-F-C₆H₄) |
| 73 | H | H | H | N(CH₃)-CH₂(4-Br-C₆H₄) |
| 74 | H | H | H | N(CH₃)-CH₂(3-Cl-C₆H₄) |
| 75 | H | H | H | N(CH₃)-CH₂(3-F-C₆H₄) |
| 76 | H | H | H | N(CH₃)-CH₂(3-Br-C₆H₄) |
| 77 | H | H | H | N(CH₃)-CH₂(3,4-Cl₂-C₆H₃) |
| 78 | H | H | H | N(CH₃)-CH₂(3,4-F₂-C₆H₃) |
| 79 | H | H | H | N(CH₃)-CH₂(3-Cl-4-F-C₆H₃) |
| 80 | H | H | H | N(CH₃)-CH₂(4-Cl-3-F-C₆H₃) |
| 81 | H | H | H | N(CH₃)-CH₂(3-Br-4-F-C₆H₃) |
| 82 | H | H | H | N(CH₃)-CH₂(4-Br-3-F-C₆H₃) |
| 83 | H | H | H | N(CH₃)-CH₂(3-Br-4-Cl-C₆H₃) |
| 84 | H | H | H | N(CH₃)-CH₂(4-Br-3-Cl-C₆H₃) |
| 85 | H | H | H | NH-C₆H₅ |
| 86 | H | H | H | NH-(4-Cl-C₆H₄) |
| 87 | H | H | H | NH-(4-F-C₆H₄) |
| 88 | H | H | H | NH-(4-Br-C₆H₄) |
| 89 | H | H | H | NH-(3-Cl-C₆H₄) |
| 90 | H | H | H | NH-(3-F-C₆H₄) |
| 91 | H | H | H | NH-(3-Br-C₆H₄) |
| 92 | H | H | H | NH-(3,4-Cl₂-C₆H₃) |
| 93 | H | H | H | NH-(3,4-F₂-C₆H₃) |
| 94 | H | H | H | NH-(3-Cl-4-F-C₆H₃) |
| 95 | H | H | H | NH-(4-Cl-3-F-C₆H₃) |
| 96 | H | H | H | NH-(3-Br-4-F-C₆H₃) |
| 97 | H | H | H | NH-(4-Br-3-F-C₆H₃) |
| 98 | H | H | H | NH-(3-Br-4-Cl-C₆H₃) |
| 99 | H | H | H | NH-(4-Br-3-Cl-C₆H₃) |
| 100 | H | H | H | N(CH₃)-C₆H₅ |
| 101 | H | H | H | N(CH₃)-(4-Cl-C₆H₄) |
| 102 | H | H | H | N(CH₃)-(4-F-C₆H₄) |
| 103 | H | H | H | N(CH₃)-(4-Br-C₆H₄) |
| 104 | H | H | H | N(CH₃)-(3-Cl-C₆H₄) |
| 105 | H | H | H | N(CH₃)-(3-F-C₆H₄) |
| 106 | H | H | H | N(CH₃)-(3-Br-C₆H₄) |
| 107 | H | H | H | N(CH₃)-(3,4-Cl₂-C₆H₃) |
| 108 | H | H | H | N(CH₃)-(3,4-F₂-C₆H₃) |
| 109 | H | H | H | N(CH₃)-(3-Cl-4-F-C₆H₃) |
| 110 | H | H | H | N(CH₃)-(4-Cl-3-F-C₆H₃) |
| 111 | H | H | H | N(CH₃)-(3-Br-4-F-C₆H₃) |
| 112 | H | H | H | N(CH₃)-(4-Br-3-F-C₆H₃) |
| 113 | H | H | H | N(CH₃)-(3-Br-4-Cl-C₆H₃) |
| 114 | H | H | H | N(CH₃)-(4-Br-3-Cl-C₆H₃) |
| 115 | H | H | F | F |
| 116 | H | H | F | CH₃ |
| 117 | H | H | F | CH₂CH₃ |
| 118 | H | H | F | CH₂CH₂CH₃ |
| 119 | H | H | F | CH(CH₃)₂ |
| 120 | H | H | F | CH₂CH₂CH₂CH₃ |
| 121 | H | H | F | CH(CH₃)CH₂CH₃ |
| 122 | H | H | F | CH₂CH(CH₃)₂ |
| 123 | H | H | F | CH₂CN |
| 124 | H | H | F | O-CH₃ |
| 125 | H | H | F | O-CH₂CH₃ |
| 126 | H | H | F | O-CH₂CH₂CH₃ |
| 127 | H | H | F | O-CH(CH₃)₂ |
| 128 | H | H | F | O-CH₂CH₂CH₂CH₃ |
| 129 | H | H | F | O-CH(CH₃)CH₂CH₃ |
| 130 | H | H | F | O-CH₂CH(CH₃)₂ |
| 131 | H | H | F | O-CH₂CN |
| 132 | H | H | F | S-CH₃ |
| 133 | H | H | F | S-CH₂CH₃ |
| 134 | H | H | F | S-CH₂CH₂CH₃ |
| 135 | H | H | F | S-CH(CH₃)₂ |
| 136 | H | H | F | S-CH₂CH₂CH₂CH₃ |
| 137 | H | H | F | S-CH(CH₃)CH₂CH₃ |
| 138 | H | H | F | S-CH₂CH(CH₃)₂ |
| 139 | H | H | F | S-CH₂CN |
| 140 | H | H | F | C₆H₅ |
| 141 | H | H | F | 4-Cl-C₆H₄ |
| 142 | H | H | F | 4-F-C₆H₄ |
| 143 | H | H | F | 4-Br-C₆H₄ |
| 144 | H | H | F | 3-Cl-C₆H₄ |
| 145 | H | H | F | 3-F-C₆H₄ |
| 146 | H | H | F | 3-Br-C₆H₄ |
| 147 | H | H | F | 3,4-Cl₂-C₆H₃ |
| 148 | H | H | F | 3,4-F₂-C₆H₃ |
| 149 | H | H | F | 3-Cl-4-F-C₆H₃ |
| 150 | H | H | F | 4-Cl-3-F-C₆H₃ |
| 151 | H | H | F | 3-Br-4-F-C₆H₃ |
| 152 | H | H | F | 4-Br-3-F-C₆H₃ |
| 153 | H | H | F | 3-Br-4-Cl-C₆H₃ |
| 154 | H | H | F | 4-Br-3-Cl-C₆H₃ |
| 155 | H | H | F | NH-CH₃ |
| 156 | H | H | F | NH-CH₂CH₃ |
| 157 | H | H | F | NH-CH₂CH₂CH₃ |
| 158 | H | H | F | NH-CH(CH₃)₂ |
| 159 | H | H | F | NH-CH₂CH₂CH₂CH₃ |
| 160 | H | H | F | NH-CH(CH₃)CH₂CH₃ |
| 161 | H | H | F | NH-CH₂CH(CH₃)₂ |
| 162 | H | H | F | N(CH₃)-CH₃ |
| 163 | H | H | F | N(CH₃)-CH₂CH₃ |
| 164 | H | H | F | N(CH₃)-CH₂CH₂CH₃ |
| 165 | H | H | F | N(CH₃)-CH(CH₃)₂ |
| 166 | H | H | F | N(CH₃)-CH₂CH₂CH₂CH₃ |
| 167 | H | H | F | N(CH₃)-CH(CH₃)CH₂CH₃ |
| 168 | H | H | F | N(CH₃)-CH₂CH(CH₃)₂ |
| 169 | H | H | F | NH-CH₂C₆H₅ |
| 170 | H | H | F | NH-CH₂(4-Cl-C₆H₄) |
| 171 | H | H | F | NH-CH₂(4-F-C₆H₄) |
| 172 | H | H | F | NH-CH₂(4-Br-C₆H₄) |
| 173 | H | H | F | NH-CH₂(3-Cl-C₆H₄) |
| 174 | H | H | F | NH-CH₂(3-F-C₆H₄) |
| 175 | H | H | F | NH-CH₂(3-Br-C₆H₄) |
| 176 | H | H | F | NH-CH₂(3,4-Cl₂-C₆H₃) |
| 177 | H | H | F | NH-CH₂(3,4-F₂-C₆H₃) |
| 178 | H | H | F | NH-CH₂(3-Cl-4-F-C₆H₃) |
| 179 | H | H | F | NH-CH₂(4-Cl-3-F-C₆H₃) |
| 180 | H | H | F | NH-CH₂(3-Br-4-F-C₆H₃) |
| 181 | H | H | F | NH-CH₂(4-Br-3-F-C₆H₃) |
| 182 | H | H | F | NH-CH₂(3-Br-4-Cl-C₆H₃) |
| 183 | H | H | F | NH-CH₂(4-Br-3-Cl-C₆H₃) |
| 184 | H | H | F | N(CH₃)-CH₂C₆H₅ |
| 185 | H | H | F | N(CH₃)-CH₂(4-Cl-C₆H₄) |
| 186 | H | H | F | N(CH₃)-CH₂(4-F-C₆H₄) |
| 187 | H | H | F | N(CH₃)-CH₂(4-Br-C₆H₄) |
| 188 | H | H | F | N(CH₃)-CH₂(3-Cl-C₆H₄) |
| 189 | H | H | F | N(CH₃)-CH₂(3-F-C₆H₄) |
| 190 | H | H | F | N(CH₃)-CH₂(3-Br-C₆H₄) |
| 191 | H | H | F | N(CH₃)-CH₂(3,4-Cl₂-C₆H₃) |
| 192 | H | H | F | N(CH₃)-CH₂(3,4-F₂-C₆H₃) |
| 193 | H | H | F | N(CH₃)-CH₂(3-Cl-4-F-C₆H₃) |
| 194 | H | H | F | N(CH₃)-CH₂(4-Cl-3-F-C₆H₃) |
| 195 | H | H | F | N(CH₃)-CH₂(3-Br-4-F-C₆H₃) |
| 196 | H | H | F | N(CH₃)-CH₂(4-Br-3-F-C₆H₃) |
| 197 | H | H | F | N(CH₃)-CH₂(3-Br-4-Cl-C₆H₃) |
| 198 | H | H | F | N(CH₃)-CH₂(4-Br-3-Cl-C₆H₃) |
| 199 | H | H | F | NH-C₆H₅ |
| 200 | H | H | F | NH-(4-Cl-C₆H₄) |
| 201 | H | H | F | NH-(4-F-C₆H₄) |
| 202 | H | H | F | NH-(4-Br-C₆H₄) |
| 203 | H | H | F | NH-(3-Cl-C₆H₄) |
| 204 | H | H | F | NH-(3-F-C₆H₄) |
| 205 | H | H | F | NH-(3-Br-C₆H₄) |
| 206 | H | H | F | NH-(3,4-Cl₂-C₆H₃) |
| 207 | H | H | F | NH-(3,4-F₂-C₆H₃) |
| 208 | H | H | F | NH-(3-Cl-4-F-C₆H₃) |
| 209 | H | H | F | NH-(4-Cl-3-F-C₆H₃) |
| 210 | H | H | F | NH-(3-Br-4-F-C₆H₃) |
| 211 | H | H | F | NH-(4-Br-3-F-C₆H₃) |
| 212 | H | H | F | NH-(3-Br-4-Cl-C₆H₃) |
| 213 | H | H | F | NH-(4-Br-3-Cl-C₆H₃) |
| 214 | H | H | F | N(CH₃)-C₆H₅ |
| 215 | H | H | F | N(CH₃)-(4-Cl-C₆H₄) |
| 216 | H | H | F | N(CH₃)-(4-F-C₆H₄) |
| 217 | H | H | F | N(CH₃)-(4-Br-C₆H₄) |
| 218 | H | H | F | N(CH₃)-(3-Cl-C₆H₄) |
| 219 | H | H | F | N(CH₃)-(3-F-C₆H₄) |
| 220 | H | H | F | N(CH₃)-(3-Br-C₆H₄) |
| 221 | H | H | F | N(CH₃)-(3,4-Cl₂-C₆H₃) |
| 222 | H | H | F | N(CH₃)-(3,4-F₂-C₆H₃) |
| 223 | H | H | F | N(CH₃)-(3-Cl-4-F-C₆H₃) |
| 224 | H | H | F | N(CH₃)-(4-Cl-3-F-C₆H₃) |
| 225 | H | H | F | N(CH₃)-(3-Br-4-F-C₆H₃) |
| 226 | H | H | F | N(CH₃)-(4-Br-3-F-C₆H₃) |
| 227 | H | H | F | N(CH₃)-(3-Br-4-Cl-C₆H₃) |
| 228 | H | H | F | N(CH₃)-(4-Br-3-Cl-C₆H₃) |
| 229 | CH₃ | H | H | H |
| 230 | CH₃ | H | H | CH₃ |
| 231 | CH₃ | H | H | CH₂CH₃ |
| 232 | CH₃ | H | H | CH₂CH₂CH₃ |
| 233 | CH₃ | H | H | CH(CH₃)₂ |
| 234 | CH₃ | H | H | CH₂CH₂CH₂CH₃ |
| 235 | CH₃ | H | H | CH(CH₃)CH₂CH₃ |
| 236 | CH₃ | H | H | CH₂CH(CH₃)₂ |
| 237 | CH₃ | H | H | CH₂CN |
| 238 | CH₃ | H | H | O-CH₃ |
| 239 | CH₃ | H | H | O-CH₂CH₃ |
| 240 | CH₃ | H | H | O-CH₂CH₂CH₃ |
| 241 | CH₃ | H | H | O-CH(CH₃)₂ |
| 242 | CH₃ | H | H | O-CH₂CH₂CH₂CH₃ |
| 243 | CH₃ | H | H | O-CH(CH₃)CH₂CH₃ |
| 244 | CH₃ | H | H | O-CH₂CH(CH₃)₂ |
| 245 | CH₃ | H | H | O-CH₂CN |
| 246 | CH₃ | H | H | S-CH₃ |
| 247 | CH₃ | H | H | S-CH₂CH₃ |
| 248 | CH₃ | H | H | S-CH₂CH₂CH₃ |
| 249 | CH₃ | H | H | S-CH(CH₃)₂ |
| 250 | CH₃ | H | H | S-CH₂CH₂CH₂CH₃ |
| 251 | CH₃ | H | H | S-CH(CH₃)CH₂CH₃ |
| 252 | CH₃ | H | H | S-CH₂CH(CH₃)₂ |
| 253 | CH₃ | H | H | S-CH₂CN |
| 254 | CH₃ | H | H | C₆H₅ |
| 255 | CH₃ | H | H | 4-Cl-C₆H₄ |
| 256 | CH₃ | H | H | 4-F-C₆H₄ |
| 257 | CH₃ | H | H | 4-Br-C₆H₄ |
| 258 | CH₃ | H | H | 3-Cl-C₆H₄ |
| 259 | CH₃ | H | H | 3-F-C₆H₄ |
| 260 | CH₃ | H | H | 3-Br-C₆H₄ |
| 261 | CH₃ | H | H | 3,4-Cl₂-C₆H₃ |
| 262 | CH₃ | H | H | 3,4-F₂-C₆H₃ |
| 263 | CH₃ | H | H | 3-Cl-4-F-C₆H₃ |
| 264 | CH₃ | H | H | 4-Cl-3-F-C₆H₃ |
| 265 | CH₃ | H | H | 3-Br-4-F-C₆H₃ |
| 266 | CH₃ | H | H | 4-Br-3-F-C₆H₃ |
| 267 | CH₃ | H | H | 3-Br-4-Cl-C₆H₃ |
| 268 | CH₃ | H | H | 4-Br-3-Cl-C₆H₃ |
| 269 | CH₃ | H | H | NH-CH₃ |
| 270 | CH₃ | H | H | NH-CH₂CH₃ |
| 271 | CH₃ | H | H | NH-CH₂CH₂CH₃ |
| 272 | CH₃ | H | H | NH-CH(CH₃)₂ |
| 273 | CH₃ | H | H | NH-CH₂CH₂CH₂CH₃ |
| 274 | CH₃ | H | H | NH-CH(CH₃)CH₂CH₃ |
| 275 | CH₃ | H | H | NH-CH₂CH(CH₃)₂ |
| 276 | CH₃ | H | H | N(CH₃)-CH₃ |
| 277 | CH₃ | H | H | N(CH₃)-CH₂CH₃ |
| 278 | CH₃ | H | H | N(CH₃)-CH₂CH₂CH₃ |
| 279 | CH₃ | H | H | N(CH₃)-CH(CH₃)₂ |
| 280 | CH₃ | H | H | N(CH₃)-CH₂CH₂CH₂CH₃ |
| 281 | CH₃ | H | H | N(CH₃)-CH(CH₃)CH₂CH₃ |
| 282 | CH₃ | H | H | N(CH₃)-CH₂CH(CH₃)₂ |
| 283 | CH₃ | H | H | NH-CH₂C₆H₅ |
| 284 | CH₃ | H | H | NH-CH₂(4-Cl-C₆H₄) |
| 285 | CH₃ | H | H | NH-CH₂(4-F-C₆H₄) |
| 286 | CH₃ | H | H | NH-CH₂(4-Br-C₆H₄) |
| 287 | CH₃ | H | H | NH-CH₂(3-Cl-C₆H₄) |
| 288 | CH₃ | H | H | NH-CH₂(3-F-C₆H₄) |
| 289 | CH₃ | H | H | NH-CH₂(3-Br-C₆H₄) |
| 290 | CH₃ | H | H | NH-CH₂(3,4-Cl₂-C₆H₃) |
| 291 | CH₃ | H | H | NH-CH₂(3,4-F₂-C₆H₃) |
| 292 | CH₃ | H | H | NH-CH₂(3-Cl-4-F-C₆H₃) |
| 293 | CH₃ | H | H | NH-CH₂(4-Cl-3-F-C₆H₃) |
| 294 | CH₃ | H | H | NH-CH₂(3-Br-4-F-C₆H₃) |
| 295 | CH₃ | H | H | NH-CH₂(4-Br-3-F-C₆H₃) |
| 296 | CH₃ | H | H | NH-CH₂(3-Br-4-Cl-C₆H₃) |
| 297 | CH₃ | H | H | NH-CH₂(4-Br-3-Cl-C₆H₃) |
| 298 | CH₃ | H | H | N(CH₃)-CH₂C₆H₅ |
| 299 | CH₃ | H | H | N(CH₃)-CH₂(4-Cl-C₆H₄) |
| 300 | CH₃ | H | H | N(CH₃)-CH₂(4-F-C₆H₄) |
| 301 | CH₃ | H | H | N(CH₃)-CH₂(4-Br-C₆H₄) |
| 302 | CH₃ | H | H | N(CH₃)-CH₂(3-Cl-C₆H₄) |
| 303 | CH₃ | H | H | N(CH₃)-CH₂(3-F-C₆H₄) |
| 304 | CH₃ | H | H | N(CH₃)-CH₂(3-Br-C₆H₄) |
| 305 | CH₃ | H | H | N(CH₃)-CH₂(3,4-Cl₂-C₆H₃) |
| 306 | CH₃ | H | H | N(CH₃)-CH₂(3,4-F₂-C₆H₃) |
| 307 | CH₃ | H | H | N(CH₃)-CH₂(3-Cl-4-F-C₆H₃) |
| 308 | CH₃ | H | H | N(CH₃)-CH₂(4-Cl-3-F-C₆H₃) |
| 309 | CH₃ | H | H | N(CH₃)-CH₂(3-Br-4-F-C₆H₃) |
| 310 | CH₃ | H | H | N(CH₃)-CH₂(4-Br-3-F-C₆H₃) |
| 311 | CH₃ | H | H | N(CH₃)-CH₂(3-Br-4-Cl-C₆H₃) |
| 312 | CH₃ | H | H | N(CH₃)-CH₂(4-Br-3-Cl-C₆H₃) |
| 313 | CH₃ | H | H | NH-C₆H₅ |
| 314 | CH₃ | H | H | NH-(4-Cl-C₆H₄) |
| 315 | CH₃ | H | H | NH-(4-F-C₆H₄) |
| 316 | CH₃ | H | H | NH-(4-Br-C₆H₄) |
| 317 | CH₃ | H | H | NH-(3-Cl-C₆H₄) |
| 318 | CH₃ | H | H | NH-(3-F-C₆H₄) |
| 319 | CH₃ | H | H | NH-(3-Br-C₆H₄) |
| 320 | CH₃ | H | H | NH-(3,4-Cl₂-C₆H₃) |
| 321 | CH₃ | H | H | NH-(3,4-F₂-C₆H₃) |
| 322 | CH₃ | H | H | NH-(3-Cl-4-F-C₆H₃) |
| 323 | CH₃ | H | H | NH-(4-Cl-3-F-C₆H₃) |
| 324 | CH₃ | H | H | NH-(3-Br-4-F-C₆H₃) |
| 325 | CH₃ | H | H | NH-(4-Br-3-F-C₆H₃) |
| 326 | CH₃ | H | H | NH-(3-Br-4-Cl-C₆H₃) |
| 327 | CH₃ | H | H | NH-(4-Br-3-Cl-C₆H₃) |
| 328 | CH₃ | H | H | N(CH₃)-C₆H₅ |
| 329 | CH₃ | H | H | N(CH₃)-(4-Cl-C₆H₄) |
| 330 | CH₃ | H | H | N(CH₃)-(4-F-C₆H₄) |
| 331 | CH₃ | H | H | N(CH₃)-(4-Br-C₆H₄) |
| 332 | CH₃ | H | H | N(CH₃)-(3-Cl-C₆H₄) |
| 333 | CH₃ | H | H | N(CH₃)-(3-F-C₆H₄) |
| 334 | CH₃ | H | H | N(CH₃)-(3-Br-C₆H₄) |
| 335 | CH₃ | H | H | N(CH₃)-(3,4-Cl₂-C₆H₃) |
| 336 | CH₃ | H | H | N(CH₃)-(3,4-F₂-C₆H₃) |
| 337 | CH₃ | H | H | N(CH₃)-(3-Cl-4-F-C₆H₃) |
| 338 | CH₃ | H | H | N(CH₃)-(4-Cl-3-F-C₆H₃) |
| 339 | CH₃ | H | H | N(CH₃)-(3-Br-4-F-C₆H₃) |
| 340 | CH₃ | H | H | N(CH₃)-(4-Br-3-F-C₆H₃) |
| 341 | CH₃ | H | H | N(CH₃)-(3-Br-4-Cl-C₆H₃) |
| 342 | CH₃ | H | H | N(CH₃)-(4-Br-3-Cl-C₆H₃) |
| 343 | CH₃ | H | F | F |
| 344 | CH₃ | H | F | CH₃ |
| 345 | CH₃ | H | F | CH₂CH₃ |
| 346 | CH₃ | H | F | CH₂CH₂CH₃ |
| 347 | CH₃ | H | F | CH(CH₃)₂ |
| 348 | CH₃ | H | F | CH₂CH₂CH₂CH₃ |
| 349 | CH₃ | H | F | CH(CH₃)CH₂CH₃ |
| 350 | CH₃ | H | F | CH₂CH(CH₃)₂ |
| 351 | CH₃ | H | F | CH₂CN |
| 352 | CH₃ | H | F | C₆H₅ |
| 353 | CH₃ | H | F | 4-Cl-C₆H₄ |
| 354 | CH₃ | H | F | 4-F-C₆H₄ |
| 355 | CH₃ | H | F | 4-Br-C₆H₄ |
| 356 | CH₃ | H | F | 3-Cl-C₆H₄ |
| 357 | CH₃ | H | F | 3-F-C₆H₄ |
| 358 | CH₃ | H | F | 3-Br-C₆H₄ |
| 359 | CH₃ | H | F | 3,4-Cl₂-C₆H₃ |
| 360 | CH₃ | H | F | 3,4-F₂-C₆H₃ |
| 361 | CH₃ | H | F | 3-Cl-4-F-C₆H₃ |
| 362 | CH₃ | H | F | 4-Cl-3-F-C₆H₃ |
| 363 | CH₃ | H | F | 3-Br-4-F-C₆H₃ |
| 364 | CH₃ | H | F | 4-Br-3-F-C₆H₃ |
| 365 | CH₃ | H | F | 3-Br-4-Cl-C₆H₃ |
| 366 | CH₃ | H | F | 4-Br-3-Cl-C₆H₃ |
| 367 | H | H | -S-CH₂-CH₂-S- | |
| 368 | H | H | -NH-CH₂-CH₂-NH- | |
| 369 | H | H | -S-CH₂-CH₂-N H- | |
| 370 | H | H | -N(CH₃)-CH₂-CH₂-N(CH₃)- | |
| 371 | H | H | -S-CH₂-CH₂-N(CH₃)- | |
| 372 | CH₃ | H | -S-CH₂-CH₂-S- | |
| 373 | CH₃ | H | -NH-CH₂-CH₂-NH- | |
| 374 | CH₃ | H | -S-CH₂-CH₂-N H- | |
| 375 | CH₃ | H | -N(CH₃)-CH₂-CH₂-N(CH₃)- | |
| 376 | CH₃ | H | -S-CH₂-CH₂-N(CH₃)- | |

In tables A and B, the following abbreviations are used:
- c-C₃H₅:: cyclopropyl
- c-C₄H₇:: cyclobutyl
- c-C₅H₉:: cyclopentyl
- c-C₆H₁₁:: cyclohexyl
- C₆H₅:: phenyl
- 4-Cl-C₆H₄:: 4-chlorophenyl
- 4-F-C₆H₄:: 4-fluorophenyl
- 4-Br-C₆H₄:: 4-bromophenyl
- 3-Cl-C₆H₄:: 3-chlorophenyl
- 3-F-C₆H₄:: 3-fluorophenyl
- 3-Br-C₆H₄:: 3-bromophenyl
- 3,4-Cl₂-C₆H₃:: 3,4-dichlorophenyl
- 3,4-F₂-C₆H₃:: 3,4-difluorophenyl
- 3-Cl-4-F-C₆H₃:: 3-chloro-4-fluorophenyl
- 4-Cl-3-F-C₆H₃:: 4-chloro-3-fluorophenyl
- 3-Br-4-F-C₆H₃:: 3-bromo-4-fluorophenyl
- 4-Br-3-F-C₆H₃:: 4-bromo-3-fluorophenyl
- 3-Br-4-Cl-C₆H₃:: 3-bromo-4-chlorophenyl
- 4-Br-3-Cl-C₆H₃:: 4-bromo-3-chlorophenyl
- 4-CH₃-C₆H₄:: 4-methylphenyl

In the last column of table A "-" indicates that m = 0, i.e. [CR^{4a}R^{4b}] is absent.

Compounds of formula (I) according to the present invention can be prepared e.g. according to the preparation methods and preparation schemes as described below.

Compounds of formula (I) according to the present invention can be prepared by standard methods of organic chemistry e.g. by the preparation methods and preparation schemes as described below and in the experimental part of this application. The definition of **m**, **Het**, **X**, **W¹**, **W²**, **W³**, **W⁴**, **R¹**, **R²**, **R³**, **R^{4a}**, **R^{4b}** and **R⁵** of the molecular structures given in the schemes below are as defined above. Room temperature means a temperature range between about 20 and 25°C.

An example of a general method for the preparation of compounds of formula (I) is shown below in Scheme A. Thus, construction of the heterocyclic element **3** present in compounds of formula (I) can be achieved, for example, by alkylation of the appropriate 2-amino heterocycle precursor **1** with the appropriate reagent of formula **2.** The transformation is preferably carried out in polar solvents such as acetonitrile, acetone, dichloromethane, 1,4-dioxane, tetrahydrofuran, N,N-dimethylformamide, N,N-dimethylacetamide, N-methylpyrolidinone or a C₁-C₆ alcohol at a temperature ranging between room temperature and the reflux temperature of the solvent. Representative reaction conditions for the alkylation of compounds analogous to formula **1** are given in Tett. Lett. 2011, 52(23), 3033-3037. The synthesis of compounds of formula **5** can be achieved by acylation of the amine functionality in compounds of formula **3** using carboxylic acid derivatives **4** which are activated *in situ.* The transformation is preferably carried out in polar solvents such as acetonitrile, acetone, 1,4-dioxane, tetrahydrofuran, N,N-dimethylformamide, N,N-dimethylacetamide, N-methylpyrolidinone or in an inert solvent such as dichloromethane, 1,2-dichloroethane, or 1,2-dimethoxyethane at temperatures ranging between room temperature and the reflux temperature of the solvent. A representative procedure condition for the acylation is given in Journal of Medicinal Chemistry, 2012, 55, 7378-7391. Examples of suitable leaving groups (LG) in formula **2** include, but are not limited to: halogen, alkyl sulfonate, haloalkyl sulfonate, aryl sulfonate, alkyl phosphonate. Examples of suitable leaving groups (LG²) in formula **4** include, but are not limited to: halogen, alkyl sulfonate, haloalkyl sulfonate, aryl sulfonate, alkyl phosphonate and various activated esters derived from the reaction of a free carboxylic acid with a peptide coupling reagent in the presence of an amine base (Chem. Rev., 2011, 111 (11), 6557-6602). A reversal of the order of these two steps would also result in an acceptable synthesis of the desired compounds. Compounds of the formula (IV), wherein where m is 0 and R³ and R⁵ together with the carbon atom, to which they are bound form a cyclopropane ring can be prepared by reacting a 2-nitroacetic acid ester such as NO₂-CH₂-COO-C₁-C₄-alkyl, with an alkene in the presence of rhodium(II) acetate dimer and (diacetoxyiodo)benzene followed by treatment of the obtained carboxylic ester with an aqueous alkali metal or earth alkali metal hydroxide solution to obtain the free carboxylic acid or the salt thereof.

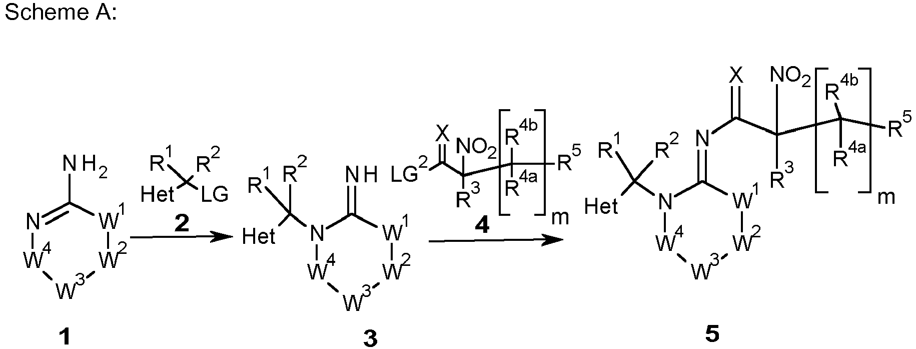

Compounds of formula (I) can also be prepared using an alternative strategy to install the nitro group as is shown below in Scheme B. Thus, construction of intermediate **3** proceeds as described in Scheme A, and amine functionality present in intermediates of type **3** can then be acylated with carboxylic acid derivatives **6**, which are activated *in situ.* The transformation is preferably carried out in polar solvents such as acetonitrile, acetone, tetrahydrofuran, 1,4-dioxane, N,N-dimethylformamide, N,N-dimethylacetamide, N-methylpyrolidinone or in an inert solvent such as dichloromethane, 1,2-dichloroethane, or 1,2-dimethoxyethane at temperatures ranging between room temperature and the reflux temperature of the solvent. Representative procedure conditions for the acylation of **3** are given in Journal of Medicinal Chemistry, 2012, 55, 7378-7391. In reagent **6**, suitable examples of substitutent **Z** include, but are not limited to: halogen, alkyl sulfate, and alkyl sulfonate. Examples of suitable leaving groups (LG³) in formula **6** include, but are not limited to: halogen, alkyl sulfonate or haloalkyl sulfonate, alkyl phosphonate, and various activated esters derived from the reaction of the free carbonic acid with a peptide coupling reagent in the presence of an amine base (Chem. Rev., 2011, 111 (11), 6557-6602). In the next step, **Z** is displaced through the action of a nitro containing compound (**8**) to afford the desired structure **5.** The transformation is preferably carried out in polar solvents such as acetonitrile, acetone, tetrahydrofuran, 1,4-dioxane, N,N-dimethylformamide, N,N-dimethylacetamide, N-methylpyrolidinone, a C₁-C₆ alcohol or in an inert solvent such as dichloromethane, 1,2-dichloroethane, 1,2-dimethoxyethane, benzene, toluene, mesitylene, cymenes, or xylenes at temperatures ranging between room temperature and the reflux temperature of the solvent. Examples of suitable nitro containing compounds (**8**) include, but are not limited to: sodium nitrite, potassium nitrite, silver nitrite, and tetraalkylammonium nitrites. Representative procedure conditions for such a reaction are given in Journal of Organic Chemistry 1982, 47, 4534-4538. Prior to the transformation through the action of the nitro containing compound (**8**), **Z** may optionally be replaced by a more suitable (more reactive) alternative **Z** (e.g. where **Z** is Cl, it can be replaced by I). This transformation is preferably carried out in polar solvents such as acetonitrile, acetone, tetrahydrofuran, 1,4-dioxane, N,N-dimethylformamide, N,N-dimethylacetamide, N-methylpyrolidinone, a C₁-C₆ alcohol or in an inert solvent such as dichloromethane, 1,2-dichloroethane, 1,2-dimethoxyethane, benzene, toluene, mesitylene, cymene, or xylenes at temperatures ranging between room temperature and the reflux temperature of the solvent. A representative procedure conditions for this exchange reaction is given in Journal of American Chemical Society 1949, 71, 1292-1297. A reversal of the order of these steps would also result in an acceptable synthesis of the desired compounds of formula (I).

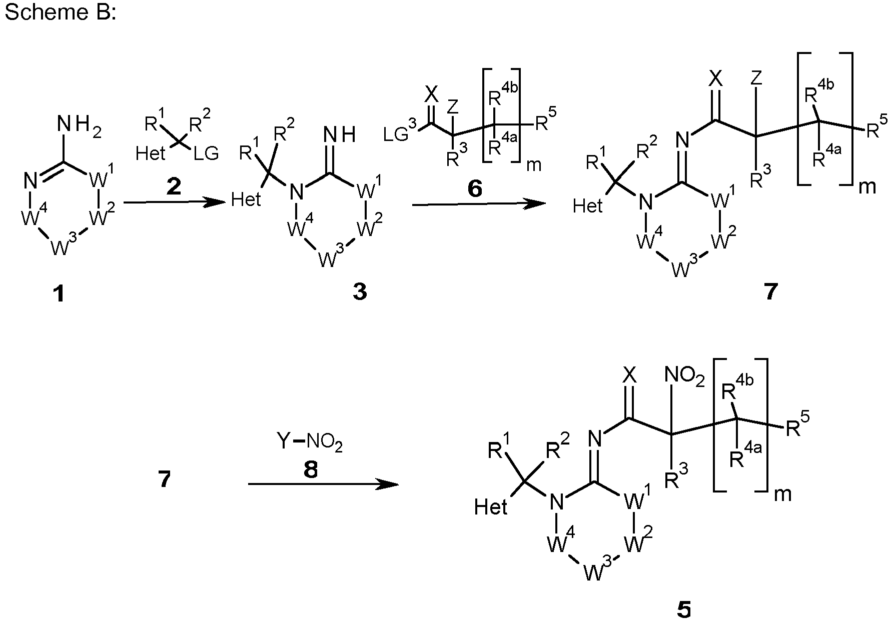

In cases where **X** is a sulfur atom, the sulfur atom is best installed in a subsequent step from the compound where **X** is an oxygen atom as detailed in Scheme C.

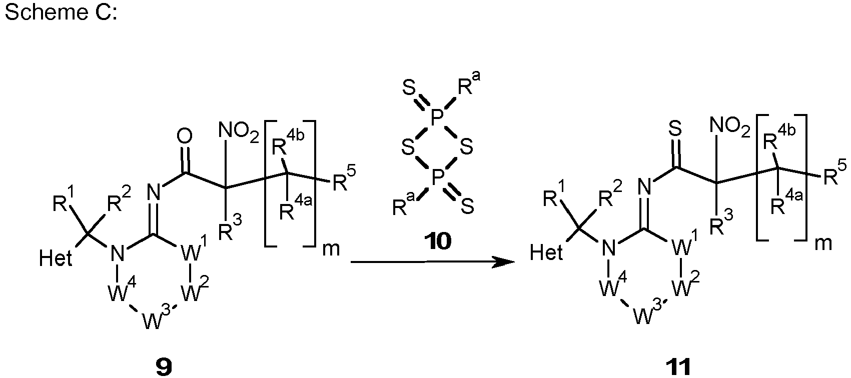

Here C=O containing compound **9** is transformed into C=S containing compound **11.** The transformation is preferably carried out using a reagent of substructure **10** in polar solvents such as acetonitrile, acetone, tetrahydrofuran, N,N-dimethylformamide, or in an inert solvent such as dichloromethane, 1,2-dichloroethane, or 1,2-dimethoxyethane at temperatures ranging between room temperature and the reflux temperature of the solvent. Suitable **R^{a}** groups in compounds of formula **10** are: thio, alkyl, aryl or substituted aryl. Representative reaction conditions for thionation analogous substrates are given in European Journal of Organic Chemistry, 2000, 3273-3278.

In cases where m = 0, and R³ or R⁵ is H, or R³ and R⁵ are both H compound **5** can be futher derivatisized as shown in Scheme D.

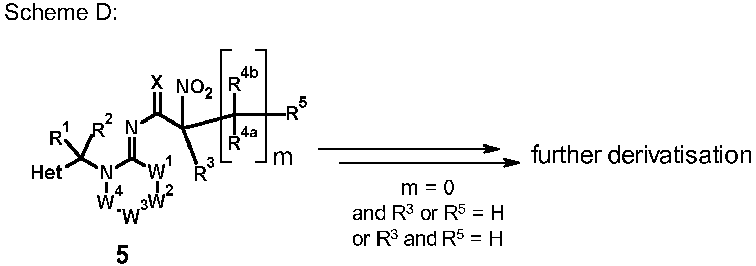

Examples for such derivatisations to obtain compounds of formula (I) and their salts are shown in Scheme E and F, below.

Reaction of the compound **15** with dihalides **16** will result in compounds, where m is 0 and where R³ and R⁵ together with the carbon atom, to which they are bound form a 3- to 6-membered carbocycle. In scheme E the radicals Hal are identical or different and suitable leaving groups such as halogen, e.g. chlorine, bromine or iodine, and the group A is C₂-C₆-alkylene, which is unsubstituted or carries 1 or 2 radicals R^{7b} as defined above. The reaction sequence is depicted in scheme E.

Reaction of the compound **15** with ketones or aldehydes **18** will result in compound of the formula (I), where m is 1 and R³ and R^{4a} form both together and together with the existing bond a double bond between the adjacent carbon atoms. The reaction sequence is depicted in scheme F.

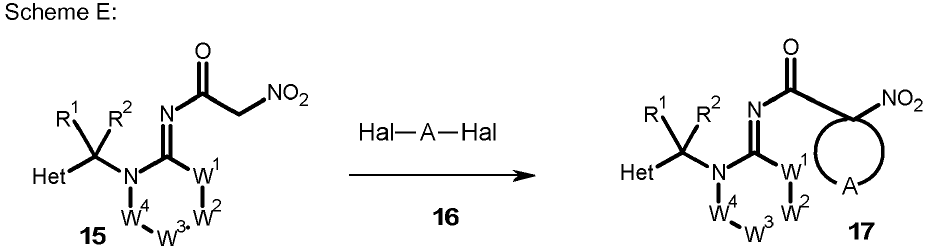

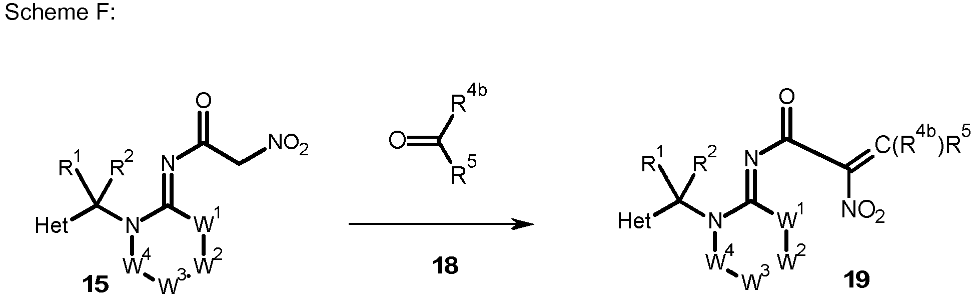

Compound **19** may be subjected to further derivatization, such as hydrogenation of the C=C-bond, epoxidation or cyclopropanation of the C=C-bond.

The compounds of the formula (I), and their salts are in particular suitable for efficiently controlling arthropodal pests such as arachnids, myriapedes and insects as well as nematodes.

The compounds of the formula (I) are especially suitable for efficiently combating insects, in particular the following pests:
Insects from the order of the **lepidopterans** *(**Lepidoptera**),* for example *Acronicta major, Adoxophyes orana, Aedia leucomelas,* Agrotis spp. such as *Agrotis fucosa, Agrotis segetum, Agrotis ypsilon; Alabama argillacea, Anticarsia gemmatalis, Anticarsia spp., Argyresthia conjugella, Autographa gamma, Barathra brassicae, Bucculatrix thurberiella, Bupalus piniarius, Cacoecia murinana, Cacoecia podana, Capua reticulana, Carpocapsa pomonella, Cheimatobia brumata,* Chilo spp. such as *Chilo suppressalis; Choristoneura fumiferana, Choristoneura occidentalis, Cirphis unipuncta*, *Clysia ambiguella, Cnaphalocerus spp., Cydia pomonella, Dendrolimus pini, Diaphania nitidalis, Diatraea grandiosella, Earias insulana, Elasmopalpus lignosellus, Ephestia cautella, Ephestia kuehniella, Eupoecilia ambiguella, Euproctis chrysorrhoea, Euxoa spp., Evetria bouliana,* Feltia spp. such as *Feltia subterranean; Galleria mellonella, Grapholitha funebrana, Grapholitha molesta,* Helicoverpa spp. such as *Helicoverpa armigera*, *Helicoverpa zea;* Heliothis spp. such as *Heliothis armigera*, *Heliothis virescens, Heliothis zea; Hellula undalis, Hibernia defoliaria, Hofmannophila pseudospretella, Homona magnanima, Hyphantria cunea, Hyponomeuta padella, Hyponomeuta malinellus, Keiferia lycopersicella, Lambdina fiscellaria,* Laphygma spp. such as *Laphygma exigua; Leucoptera coffeella, Leucoptera scitella, Lithocolletis blancardella, Lithophane antennata, Lobesia botrana, Loxagrotis albicosta, Loxostege sticticalis,* Lymantria spp. such as *Lymantria dispar, Lymantria monacha; Lyonetia clerkella, Malacosoma neustria,* Mamestra spp. such as *Mamestra brassicae; Mocis repanda, Mythimna separata, Orgyia pseudotsugata, Oria spp.,* Ostrinia spp. such as *Ostrinia nubilalis; Oulema oryzae, Panolis flammea,* Pectinophora spp. such as *Pectinophora gossypiella; Peridroma saucia, Phalera bucephala,* Phthorimaea spp. such as *Phthorimaea operculella; Phyllocnistis citrella,* Pieris spp. such as *Pieris brassicae, Pieris rapae; Plathypena scabra, Plutella maculipennis, Plutella xylostella, Prodenia spp., Pseudaletia spp., Pseudoplusia includens, Pyrausta nubilalis, Rhyacionia frustrana, Scrobipalpula absoluta, Sitotroga cerealella, Sparganothis pilleriana,* Spodoptera spp. such as *Spodoptera frugiperda, Spodoptera littoralis, Spodoptera litura; Thaumatopoea pityocampa, Thermesia gemmatalis, Tinea pellionella, Tineola bisselliella, Tortrix viridana,* Trichoplusia spp. such as *Trichoplusia ni; Tuta absoluta, and Zeiraphera canadensis;*
**Beetles** *(**Coleoptera**),* for example *Acanthoscehdes obtectus, Adoretus spp., Agelastica alni, Agrilus sinuatus,* Agriotes spp. such as *Agriotes fuscicollis, Agriotes lineatus, Agriotes obscurus; Amphimallus solstitialis, Anisandrus dispar, Anobium punctatum, Anomala rufocuprea,* Anoplophora spp. such as *Anoplophora glabripennis;* Anthonomus spp. such as *Anthonomus grandis, Anthonomus pomorum; Anthrenus spp., Aphthona euphoridae, Apogonia spp., Athous haemorrhoidalis,* Atomaria spp. such as *Atomaria linearis; Attagenus spp., Aulacophora femoralis, Blastophagus piniperda, Blitophaga undata, Bruchidius obtectus,* Bruchus spp. such as *Bruchus lentis, Bruchus pisorum, Bruchus rufimanus; Byctiscus betulae, Callosobruchus chinensis, Cassida nebulosa, Cerotoma trifurcata, Cetonia aurata,* Ceuthorhynchus spp. such as *Ceuthorrhynchus assimilis, Ceuthorrhynchus napi; Chaetocnema tibialis, Cleonus mendicus,* Conoderus spp. such as *Conoderus vespertinus; Cosmopolites spp., Costelytra zealandica, Crioceris asparagi, Cryptorhynchus lapathi,* Ctenicera ssp. such as *Ctenicera destructor; Curculio spp., Dectes texanus, Dermestes spp.,* Diabrotica spp. such as *Diabrotica 12-punctata Diabrotica speciosa, Diabrotica longicornis, Diabrotica semipunctata, Diabrotica virgifera;* Epilachna spp. such as *Epilachna varivestis, Epilachna vigintioctomaculata;* Epitrix spp. such as *Epitrix hirtipennis; Eutinobothrus brasiliensis, Faustinus cubae, Gibbium psylloides, Heteronychus arator, Hylamorpha elegans, Hylobius abietis, Hylotrupes bajulus, Hypera brunneipennis, Hypera postica, Hypothenemus spp., Ips typographus, Lachnosterna consanguinea, Lema bilineata, Lema melanopus,* Leptinotarsa spp. such as *Leptinotarsa decemlineata; Limonius californicus, Lissorhoptrus oryzophilus, Lissorhoptrus oryzophilus, Lixus spp*., Lyctus spp. such as *Lyctus bruneus; Melanotus communis,* Meligethes spp. such as *Meligethes aeneus; Melolontha hippocastani, Melolontha melolontha, Migdolus spp.,* Monochamus spp. such as *Monochamus alternatus; Naupactus xanthographus, Niptus hololeucus, Oryctes rhinoceros, Oryzaephilus surinamensis, Otiorrhynchus sulcatus, Otiorrhynchus ovatus, Otiorrhynchus sulcatus, Oulema oryzae, Oxycetonia jucunda, Phaedon cochleariae, Phyllobius pyri, Phyllopertha horticola, Phyllophaga spp.,* Phyllotreta spp. such as *Phyllotreta chrysocephala, Phyllotreta nemorum, Phyllotreta striolata; Phyllophaga spp., Phyllopertha horticola, Popillia japonica, Premnotrypes spp., Psylliodes chrysocephala, Ptinus spp., Rhizobius ventralis, Rhizopertha dominica, Sitona lineatus,* Sitophilus spp. such as *Sitophilus granaria, Sitophilus zeamais;* Sphenophorus spp. such as *Sphenophorus levis;* Sternechus spp. such as *Sternechus subsignatus; Symphyletes spp., Tenebrio molitor,* Tribolium spp. such as *Tribolium castaneum; Trogoderma spp., Tychius spp., Xylotrechus spp., and Zabrus spp. such as Zabrus tenebrioides;*
**Flies, mosquitoes** (***Diptera***)*,* e.g. Aedes spp. such as *Aedes aegypti, Aedes albopictus, Aedes vexans; Anastrepha ludens,* Anopheles spp. such as *Anopheles albimanus, Anopheles crucians, Anopheles freeborni, Anopheles gambiae, Anopheles leucosphyrus, Anopheles maculipennis, Anopheles minimus, Anopheles quadrimaculatus, Anopheles sinensis; Bibio hortulanus, Calliphora erythrocephala, Calliphora vicina, Cerafitis capitata, Ceratitis capitata,* Chrysomyia spp. such as *Chrysomya bezziana, Chrysomya hominivorax, Chrysomya macellaria; Chrysops atlanticus, Chrysops discalis, Chrysops silacea,* Cochliomyia spp. such as *Cochliomyia hominivorax;* Contarinia spp. such as *Contarinia sorghicola; Cordylobia anthropophaga,* Culex spp. such as *Culex nigripalpus, Culex pipiens, Culex quinquefasciatus, Culex tarsalis, Culex tritaeniorhynchus; Culicoides furens, Culiseta inornata, Culiseta melanura, Cuterebra spp., Dacus cucurbitae, Dacus oleae, Dasineura brassicae,* Delia spp. such as *Delia antique, Delia coarctata, Delia platura, Delia radicum; Dermatobia hominis, Drosophila spp.,* Fannia spp. such as *Fannia canicularis;* Gastraphilus spp. such as *Gasterophilus intestinalis; Geomyza Tripunctata, Glossina fuscipes, Glossina morsitans, Glossina palpalis, Glossina tachinoides, Haematobia irritans, Haplodiplosis equestris, Hippelates spp.,* Hylemyia spp. such as *Hylemyia platura;* Hypoderma spp. such as *Hypoderma lineata; Hyppobosca spp., Leptoconops torrens,* Liriomyza spp. such as *Liriomyza sativae, Liriomyza trifolii;* Lucilia spp. such as *Lucilia caprina, Lucilia cuprina, Lucilia sericata; Lycoria pectoralis, Mansonia titillanus,* Mayetiola spp. such as *Mayetiola destructor;* Musca spp. such as *Musca autumnalis, Musca domestica; Muscina stabulans,* Oestrus spp. such as *Oestrus ovis; Opomyza florum,* Oscinella spp. such as *Oscinella frit; Pegomya hysocyami, Phlebotomus argentipes,* Phorbia spp. such as *Phorbia antiqua, Phorbia brassicae, Phorbia coarctata; Prosimulium mixtum, Psila rosae, Psorophora columbiae, Psorophora discolor, Rhagoletis cerasi, Rhagoletis pomonella,* Sarcophaga spp. such as *Sarcophaga haemorrhoidalis; Simulium vittatum,* Stomoxys spp. such as *Stomoxys calcitrans;* Tabanus spp. such as *Tabanus atratus, Tabanus bovinus, Tabanus lineola, Tabanus similis; Tannia spp., Tipula oleracea, Tipula paludosa, and Wohlfahrtia spp.;*
**Thrips** (***Thysanoptera***)*,* e.g. *Baliothrips biformis, Dichromothrips corbetti, Dichromothrips ssp., Enneothrips flavens,* Frankliniella spp. such as *Frankliniella fusca, Frankliniella occidentalis, Frankliniella tritici; Heliothrips spp., Hercinothrips femoralis, Kakothrips spp., Rhipiphorothrips cruentatus,* Scirtothrips spp. such as *Scirtothrips citri; Taeniothrips cardamoni,* Thrips spp. such as *Thrips oryzae, Thrips palmi, Thrips tabaci;*
**Termites** (**Isoptera**), e.g. *Calotermes flavicollis, Coptotermes formosanus, Heterotermes aureus, Heterotermes longiceps, Heterotermes tenuis, Leucotermes flavipes, Odontotermes* spp., Reticulitermes spp. such as *Reticulitermes speratus, Reticulitermes flavipes, Reticulitermes grassei, Reticulitermes lucifugus, Reticulitermes santonensis, Reticulitermes virginicus; Termes natalensis;*
**Cockroaches** (**Blattaria - Blattodea**), e.g. *Acheta domesticus, Blatta orientalis, Blattella asahinae, Blattella germanica, Gryllotalpa spp., Leucophaea maderae, Locusta spp., Melanoplus spp., Periplaneta americana, Periplaneta australasiae, Periplaneta brunnea, Periplaneta fuligginosa, Periplaneta japonica;*
**Bugs, aphids, leafhoppers, whiteflies, scale insects, cicadas** (***Hemiptera***)*,* e.g. Acrosternum spp. such as *Acrosternum hilare;* Acyrthosipon spp. such as *Acyrthosiphon onobrychis, Acyrthosiphon pisum; Adelges laricis, Aeneolamia spp., Agonoscena spp., Aleurodes spp., Aleurolobus barodensis, Aleurothrixus spp., Amrasca spp., Anasa tristis, Antestiopsis spp., Anuraphis cardui, Aonidiella spp., Aphanostigma piri, Aphidula nasturtii,* Aphis spp. such as *Aphis fabae, Aphis forbesi, Aphis gossypii, Aphis grossulariae, Aphis pomi, Aphis sambuci, Aphis schneideri, Aphis spiraecola; Arboridia apicalis, Arilus critatus, Aspidiella spp., Aspidiotus spp., Atanus spp., Aulacorthum solani,* Bemisia spp. such as *Bemisia argentifolii, Bemisia tabaci;* Blissus spp. such as *Blissus leucopterus; Brachycaudus cardui, Brachycaudus helichrysi, Brachycaudus persicae, Brachycaudus prunicola, Brachycolus spp., Brevicoryne brassicae, Calligypona marginata, Calocoris spp., Campylomma livida, Capitophorus horni, Carneocephala fulgida, Cavelerius spp., Ceraplastes spp., Ceratovacuna lanigera, Cercopidae, Cerosipha gossypii, Chaetosiphon fragaefolii, Chionaspis tegalensis, Chlorita onukii, Chromaphis juglandicola, Chrysomphalus ficus, Cicadulina mbila,* Cimex spp. such as *Cimex hemipterus, Cimex lectularius; Coccomytilus halli, Coccus spp., Creontiades dilutus, Cryptomyzus ribis, Cryptomyzus ribis, Cyrtopeltis notatus, Dalbulus spp., Dasynus piperis, Dialeurades spp., Diaphorina spp., Diaspis spp., Dichelops furcatus, Diconocoris hewetti, Doralis spp., Dreyfusia nordmannianae, Dreyfusia piceae, Drosicha spp.,* Dysaphis spp. such as *Dysaphis plantaginea, Dysaphis pyri, Dysaphis radicola; Dysaulacorthum pseudosolani,* Dysdercus spp. such as *Dysdercus cingulatus, Dysdercus intermedius; Dysmicoccus spp.,* Empoasca spp. such as *Empoasca fabae, Empoasca solana; Eriosoma spp., Erythroneura spp.,* Eurygaster spp. such as *Eurygaster integriceps; Euscelis bilobatus, Euschistus spp. such as Euschistuos heros, Euschistus impictiventris, Euschistus servus; Geococcus coffeae,* Halyomorpha spp. such as *Halyomorpha halys; Heliopeltis spp., Homalodisca coagulata, Horcias nobilellus, Hyalopterus pruni, Hyperomyzus lactucae, Icerya spp., Idiocerus spp., Idioscopus spp., Laodelphax striatellus, Lecanium spp., Lepidosaphes spp., Leptocorisa spp., Leptoglossus phyllopus, Lipaphis erysimi,* Lygus spp. such as *Lygus hesperus, Lygus lineolaris, Lygus pratensis; Macropes excavatus,* Macrosiphum spp. such as *Macrosiphum rosae, Macrosiphum avenae, Macrosiphum euphorbiae; Mahanarva fimbriolata, Megacopta cribraria, Megoura viciae, Melanaphis pyrarius, Melanaphis sacchari, Metcafiella spp., Metopolophium dirhodum, Miridae spp., Monellia costalis, Monelliopsis pecanis,* Myzus spp. such as *Myzus ascalonicus, Myzus cerasi, Myzus persicae, Myzus varians; Nasonovia ribis-nigri,* Nephotettix spp. such as *Nephotettix malayanus, Nephotettix nigropictus, Nephotettix parvus, Nephotettix virescens;* Nezara spp. such as *Nezara viridula; Nilaparvata lugens, Oebalus spp., Oncometopia spp., Orthezia praelonga, Parabemisia myricae, Paratrioza spp., Parlatoria spp.,* Pemphigus spp. such as *Pemphigus bursarius; Pentomidae, Peregrinus maidis, Perkinsiella saccharicida, Phenacoccus spp., Phloeomyzus passerinii, Phorodon humuli, Phylloxera spp., Piesma quadrata,* Piezodorus spp. such as *Piezodorus guildinii, Pinnaspis aspidistrae, Planococcus spp., Protopulvinaria pyriformis, Psallus seriatus, Pseudacysta persea, Pseudaulacaspis pentagona,* Pseudococcus spp. such as *Pseudococcus comstocki;* Psylla spp. such as *Psylla mali, Psylla piri; Pteromalus spp., Pyrilla spp., Quadraspidiotus spp., Quesada gigas, Rastrococcus spp., Reduvius senilis, Rhodnius spp., Rhopalomyzus ascalonicus,* Rhopalosiphum spp. such as *Rhopalosiphum pseudobrassicas, Rhopalosiphum insertum, Rhopalosiphum maidis, Rhopalosiphum padi; Sagatodes spp., Sahlbergella singularis, Saissetia spp., Sappaphis mala, Sappaphis mali, Scaphoides titanus, Schizaphis graminum, Schizoneura lanuginosa, Scotinophora spp., Selenaspidus articulatus, Sitobion avenae, Sogata spp., Sogatella furcifera, Solubea insularis, Stephanitis nashi, Stictocephala festina, Tenalaphara malayensis,* Thyanta spp. such as *Thyanta perditor; Tibraca spp., Tinocallis caryaefoliae, Tomaspis spp.,* Toxoptera spp. such as *Toxoptera aurantii;* Trialeurodes spp. such as *Trialeurodes vaporariorum; Triatoma spp., Trioza spp., Typhlocyba spp.,* Unaspis spp. such as *Unaspis yanonensis; and Viteus vitifolii;*
**Ants, bees, wasps, sawflies** (**Hymenoptera**), e.g. *Athalia rosae, Atta capiguara, Atta cephalotes, Atta cephalotes, Atta laevigata, Atta robusta, Atta sexdens, Atta texana, Bombus spp., Camponotus floridanus, Crematogaster spp., Dasymutilla occidentalis, Diprion spp., Dolichovespula maculata,* Hoplocampa spp. such as *Hoplocampa minuta, Hoplocampa testudinea;* Lasius spp. such as *Lasius niger, Linepithema humile, Monomorium pharaonis, Paravespula germanica, Paravespula pennsylvanica, Paravespula vulgaris, Pheidole megacephala, Pogonomyrmex barbatus, Pogonomyrmex californicus, Polistes rubiginosa, Solenopsis geminata, Solenopsis invicta, Solenopsis richteri, Solenopsis xyloni,* Vespa spp. such as *Vespa crabro, and Vespula squamosal;*
**Crickets, grasshoppers, locusts** (**Orthoptera**), e.g. *Acheta domestica, Calliptamus italicus, Chortoicetes terminifera, Dociostaurus maroccanus, Gryllotalpa africana, Gryllotalpa gryllotalpa, Hieroglyphus daganensis, Kraussaria angulifera, Locusta migratoria, Locustana pardalina, Melanoplus bivittatus, Melanoplus femurrubrum, Melanoplus mexicanus, Melanoplus sanguinipes, Melanoplus spretus, Nomadacris septemfasciata, Oedaleus senegalensis, Schistocerca americana, Schistocerca gregaria, Tachycines asynamorus, and Zonozerus variegatus;*
**Earwigs** (***Dermaptera**),* e.g. *forficula auricularia,*
**Lice** (***Phthiraptera***)*,* e.g. *Damalinia spp.,* Pediculus spp. such as *Pediculus humanus capitis, Pediculus humanus corporis; Pthirus pubis,* Haematopinus spp. such as *Haematopinus eurysternus, Haematopinus suis;* Linognathus spp. such as *Linognathus vituli; Bovicola bovis, Menopon gallinae, Menacanthus stramineus* and *Solenopotes capillatus, Trichodectes spp.;*
**Fleas** (***Siphonaptera***), e.g. *Ceratophyllus spp., Ctenocephalides felis, Ctenocephalides canis, Xenopsylla cheopis, Pulex irritans, Tunga penetrans,* and *Nosopsyllus fasciatus.*

The compounds of the formula (I) are also suitable for efficiently combating arthropd pests different from insects such as, in particular the following pests:
**arachnids** (***Arachnida***), such as acari,e.g. of the families Argasidae, Ixodidae and Sarcoptidae, such as Amblyomma spp. (e.g. *Amblyomma americanum, Amblyomma variegatum, Amblyomma maculatum),* Argas spp. (e.g. *Argas persicus),* Boophilus spp. (e.g. *Boophilus annulatus, Boophilus decoloratus, Boophilus microplus), Dermacentor silvarum, Dermacentor andersoni, Dermacentor variabilis,* Hyalomma spp. (e.g. *Hyalomma truncatum),* Ixodes spp. (e.g. *Ixodes ricinus, Ixodes rubicundus, Ixodes scapularis, Ixodes holocyclus, Ixodes pacificus),* Ornithodorus spp. (e.g. *Ornithodorus moubata, Ornithodorus hermsi, Ornithodorus turicata), Ornithonyssus bacoti, Otobius megnini, Dermanyssus gallinae,* Psoroptes spp. (e.g. *Psoroptes ovis),* Rhipicephalus spp. (e.g. *Rhipicephalus sanguineus, Rhipicephalus appendiculatus, Rhipicephalus evertsi), Rhizoglyphus spp.,* Sarcoptes spp. (e.g. *Sarcoptes scabies*), and **Eriophyidae** spp. such as *Acaria sheldoni,* Aculops spp. (e.g. *Aculops pelekassi*) Aculus spp. (e.g. *Aculus schlechtendali*), *Epitrimerus pyri, Phyllocoptruta oleivora* and Eriophyes spp. (e.g. *Eriophyes sheldom*); Tarsonemidae spp. such as *Hemitarsonemus spp., Phytonemus pallidus and Polyphagotarsonemus latus, Stenotarsonemus spp.;* Tenuipalpidae spp. such as Brevipalpus spp. (e.g. *Brevipalpus phoenicis);* Tetranychidae spp. such as *Eotetranychus spp., Eutetranychus spp., Oligonychus spp., Tetranychus cinnabarinus, Tetranychus kanzawai, Tetranychus pacificus, Tetranychus telarius* and *Tetranychus* urticae; *Bryobia praetiosa,* Panonychus spp. (e.g. *Panonychus ulmi, Panonychus citri*), *Metatetranychus spp.* and Oligonychus spp. (e.g. *Oligonychus pratensis), Vasates lycopersici;* Araneida, e.g. *Latrodectus mactans,* and *Loxosceles reclusa.* And *Acarus siro, Chorioptes spp., Scorpio maurus;*
**Silverfish, firebrat** (***Thysanura***), e.g. *Lepisma saccharina* and *Thermobia domestica;*
**Centipedes** (***Chilopoda***), e.g. *Geophilus spp.,* Scutigera spp. such as *Scutigera coleoptrata;*
**Millipedes** (***Diplopoda***), e.g. *Blaniulus guttulatus, Narceus spp.,*
**Springtails** (***Collembola***), *e.g.* Onychiurus ssp. such as *Onychiurus armatus,*

They are also suitable for controlling **nematodes:** plant parasitic nematodes such as root knot nematodes, *Meloidogyne hapla, Meloidogyne incognita, Meloidogyne javanica,* and other Meloidogyne species; cyst-forming nematodes, *Globodera rostochiensis* and other Globodera species; *Heterodera avenae, Heterodera glycines, Heterodera schachtii, Heterodera trifolii,* and other Heterodera species; Seed gall nematodes, Anguina species; Stem and foliar nematodes, Aphelenchoides species such as *Aphelenchoides besseyi*; Sting nematodes, *Belonolaimus longicaudatus* and other Belonolaimus species; Pine nematodes, *Bursaphelenchus lignicolus Mamiya et Kiyohara, Bursaphelenchus xylophilus* and other Bursaphelenchus species; Ring nematodes, Criconema species, Criconemella species, Criconemoides species, Mesocriconema species; Stem and bulb nematodes, *Ditylenchus destructor, Ditylenchus dipsaci* and other Ditylenchus species; Awl nematodes, Dolichodorus species; Spiral nematodes, *Heliocotylenchus multicinctus* and other Helicotylenchus species; Sheath and sheathoid nematodes, Hemicycliophora species and Hemicriconemoides species; Hirshmanniella species; Lance nematodes, Hoploaimus species; false rootknot nematodes, Nacobbus species; Needle nematodes, *Longidorus elongatus* and other Longidorus species; Lesion nematodes, *Pratylenchus brachyurus, Pratylenchus neglectus, Pratylenchus penetrans, Pratylenchus curvitatus, Pratylenchus goodeyi* and other Pratylenchus species; Burrowing nematodes, *Radopholus similis* and other Radopholus species; Reniform nematodes, *Rotylenchus robustus, Rotylenchus reniformis* and other Rotylenchus species; Scutellonema species; Stubby root nematodes, *Trichodorus primitivus* and other Trichodorus species, Paratrichodorus species; Stunt nematodes, *Tylenchorhynchus claytoni, Tylenchorhynchus dubius* and other Tylenchorhynchus species; Citrus nematodes, Tylenchulus species such as *Tylenchulus semipenetrans;* Dagger nematodes, Xiphinema species; and other plant parasitic nematode species.

Examples of further pest species which may be controlled by compounds of fomula (I) include: from the class of the *Bivalva,* for example, *Dreissena spp*.; from the class of the *Gastropoda,* for example, *Arion spp., Biomphalaria spp., Bulinus spp., Deroceras spp., Galba spp., Lymnaea spp., Oncomelania spp., Succinea spp.;* from the class of the *helminths,* for example, *Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp., Dictyocaulus filaria, Diphyllobothrium latum, Dracunculus medinensis, Echinococcus granulosus, Echinococcus multilocularis, Enterobius vermicularis, Faciola spp.,* Haemonchus spp. such as *Haemonchus contortus; Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp., Strongyloides fuelleborni, Strongyloides stercora lis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria*, *Wuchereria bancrofti;* from the order of the *Isopoda,* for example, *Armadillidium vulgare, Oniscus asellus, Porcellio scaber;* from the order of the *Symphyla,* for example, *Scutigerella immaculata.*

Further examples of pest species which may be controlled by compounds of formula (I) include: *Anisoplia austriaca, Apamea spp., Austroasca viridigrisea, Baliothrips biformis, Caenorhabditis elegans, Cephus spp., Ceutorhynchus napi, Chaetocnema aridula, Chilo auricilius, Chilo indicus*, *Chilo polychrysus, Chortiocetes terminifera, Cnaphalocroci medinalis, Cnaphalocrosis spp., Colias eurytheme, Collops spp., Cornitermes cumulans, Creontiades spp., Cyclocephala spp., Dalbulus maidis, Deraceras reticulatum* , *Diatrea saccharalis, Dichelops furcatus, Dicladispa armigera*, Diloboderus spp. such as *Diloboderus abderus; Edessa spp., Epinotia spp., Formicidae, Geocoris spp., Globitermes sulfureus, Gryllotalpidae, Halotydeus destructor, Hipnodes bicolor, Hydrellia philippina, Julus spp., Laodelphax spp., Leptocorsia acuta, Leptocorsia oratorius*, *Liogenys fuscus, Lucillia spp., Lyogenys fuscus, Mahanarva spp., Maladera matrida, Marasmia spp., Mastotermes spp., Mealybugs, Megascelis ssp, Metamasius hemipterus*, *Microtheca spp., Mocis latipes, Murgantia spp., Mythemina separata , Neocapritermes opacus, Neocapritermes parvus, Neomegalotomus spp., Neotermes spp., Nymphula depunctalis, Oebalus pugnax,* Orseolia spp. such as *Orseolia oryzae; Oxycaraenus hyalinipennis, Plusia spp., Pomacea canaliculata, Procornitermes ssp, Procornitermes triacifer, Psylloides spp., Rachiplusia spp., Rhodopholus spp., Scaptocoris castanea, Scaptocoris spp.,* Scirpophaga spp. such as *Scirpophaga incertulas*, *Scirpophaga innotata;* Scotinophara spp. such as *Scotinophara coarctata;* Sesamia spp. such as *Sesamia inferens, Sogaella frucifera, Solenapsis geminata, Spissistilus spp., Stalk borer, Stenchaetothrips biformis, Steneotarsonemus spinki, Sylepta derogata, Telehin licus, Trichostrongylus spp..*

Compounds of the formula (I) are particularly useful for controlling insects of the orders Hemiptera and Thysanoptera.

For use in a method according to the present invention, the compounds of the formula (I) can be converted into the customary formulations, e.g. solutions, emulsions, suspensions, dusts, powders, pastes, granules and directly sprayable solutions. The use form depends on the particular purpose and application method. Formulations and application methods are chosen to ensure in each case a fine and uniform distribution of the compound of the formula (I) according to the present invention.

The formulations are prepared in a known manner (see e.g. for review US 3,060,084, EP-A 707 445 (for liquid concentrates), Browning, "Agglomeration", Chemical Engineering, Dec. 4, 1967, 147-48, Perry's Chemical Engineer's Handbook, 4th Ed., McGraw-Hill, New York, 1963, pages 8-57 and et seq. WO 91/13546, US 4,172,714, US 4,144,050, US 3,920,442, US 5,180,587, US 5,232,701, US 5,208,030, GB 2,095,558, US 3,299,566, Klingman, Weed Control as a Science, John Wiley and Sons, Inc., New York, 1961, Hance et al., Weed Control Handbook, 8th Ed., Blackwell Scientific Publications, Oxford, 1989 and Mollet, H., Grubemann, A., Formulation technology, Wiley VCH Verlag GmbH, Weinheim (Germany), 2001, 2. D. A. Knowles, Chemistry and Technology of Agrochemical Formulations, Kluwer Academic Publishers, Dordrecht, 1998 (ISBN 0-7514-0443-8), for example by extending the active compound with auxiliaries suitable for the formulation of agrochemicals, such as solvents and/or carriers, if desired emulsifiers, surfactants and dispersants, preservatives, antifoaming agents, anti-freezing agents, for seed treatment formulation also optionally colorants and/or binders and/or gelling agents.

Solvents/carriers, which are suitable, are e.g.:
- solvents such as water, aromatic solvents (for example Solvesso products, xylene and the like), paraffins (for example mineral fractions), alcohols (for example methanol, butanol, pentanol, benzyl alcohol), ketones (for example cyclohexanone, gamma-butyrolactone), pyrrolidones (N-metyhl-pyrrolidone (NMP),N-octylpyrrolidone NOP), acetates (glycol diacetate), alkyl lactates, lactones such as g-butyrolactone, glycols, fatty acid dimethylamides, fatty acids and fatty acid esters, triglycerides, oils of vegetable or animal origin and modified oils such as alkylated plant oils. In principle, solvent mixtures may also be used.
- carriers such as ground natural minerals and ground synthetic minerals, such as silica gels, finely divided silicic acid, silicates, talc, kaolin, attaclay, limestone, lime, chalk, bole, loess, clay, dolomite, diatomaceous earth, calcium sulfate and magnesium sulfate, magnesium oxide, ground synthetic materials, fertilizers, such as, for example, ammonium sulfate, ammonium phosphate, ammonium nitrate, ureas and products of vegetable origin, such as cereal meal, tree bark meal, wood meal and nutshell meal, cellulose powders and other solid carriers.

Suitable emulsifiers are nonionic and anionic emulsifiers, for example polyoxyethylene fatty alcohol ethers, alkylsulfonates and arylsulfonates.

Examples of dispersants are lignin-sulfite waste liquors and methylcellulose.

Suitable surfactants are alkali metal, alkaline earth metal and ammonium salts of lignosulfonic acid, naphthalenesulfonic acid, phenolsulfonic acid, dibutylnaphthalenesulfonic acid, alkylarylsulfonates, alkyl sulfates, alkylsulfonates, fatty alcohol sulfates, fatty acids and sulfated fatty alcohol glycol ethers, furthermore condensates of sulfonated naphthalene and naphthalene derivatives with formaldehyde, condensates of naphthalene or of naphthalenesulfonic acid with phenol and formaldehyde, polyoxyethylene octylphenyl ether, ethoxylated isooctylphenol, octylphenol, nonylphenol, alkylphenyl polyglycol ethers, tributylphenyl polyglycol ether, tristearylphenyl polyglycol ether, alkylaryl polyether alcohols, alcohol and fatty alcohol/ethylene oxide condensates, ethoxylated castor oil, polyoxyethylene alkyl ethers, ethoxylated polyoxypropylene, lauryl alcohol polyglycol ether acetal, sorbitol esters,

Also anti-freezing agents such as glycerin, ethylene glycol, propylene glycol and bactericides such as can be added to the formulation.

Suitable antifoaming agents are for example antifoaming agents based on silicon or magnesium stearate.

Suitable preservatives are for example dichlorophen und benzyl alcohol hemiformal

Suitable thickeners are compounds which confer a pseudoplastic flow behavior to the formulation, i.e. high viscosity at rest and low viscosity in the agitated stage. Mention may be made, in this context, for example, of commercial thickeners based on polysaccharides, such as Xanthan Gum® (Kelzan® from Kelco), Rhodopol®23 (Rhone Poulenc) or Veegum® (from R.T. Vanderbilt), or organic phyllosilicates, such as Attaclay® (from Engelhardt). Antifoam agents suitable for the dispersions according to the invention are, for example, silicone emulsions (such as, for example, Silikon® SRE, Wacker or Rhodorsil® from Rhodia), long-chain alcohols, fatty acids, organofluorine compounds and mixtures thereof. Biocides can be added to stabilize the compositions according to the invention against attack by microorganisms. Suitable biocides are, for example, based on isothiazolones such as the compounds marketed under the trademarks Proxel® from Avecia (or Arch) or Acticide® RS from Thor Chemie and Kathon® MK from Rohm & Haas. Suitable antifreeze agents are organic polyols, for example ethylene glycol, propylene glycol or glycerol. These are usually employed in amounts of not more than 10% by weight, based on the total weight of the active compound composition. If appropriate, the active compound compositions according to the invention may comprise 1 to 5% by weight of buffer, based on the total amount of the formulation prepared, to regulate the pH, the amount and type of the buffer used depending on the chemical properties of the active compound or the active compounds. Examples of buffers are alkali metal salts of weak inorganic or organic acids, such as, for example, phosphoric acid, boronic acid, acetic acid, propionic acid, citric acid, fumaric acid, tartaric acid, oxalic acid and succinic acid.

Substances which are suitable for the preparation of directly sprayable solutions, emulsions, pastes or oil dispersions are mineral oil fractions of medium to high boiling point, such as kerosene or diesel oil, furthermore coal tar oils and oils of vegetable or animal origin, aliphatic, cyclic and aromatic hydrocarbons, for example toluene, xylene, paraffin, tetrahydronaphthalene, alkylated naphthalenes or their derivatives, methanol, ethanol, propanol, butanol, cyclohexanol, cyclohexanone, isophorone, strongly polar solvents, for example dimethyl sulfoxide, N-methylpyrrolidone and water.

Powders, materials for spreading and dusts can be prepared by mixing or concomitantly grinding the active substances with a solid carrier.

Granules, for example coated granules, impregnated granules and homogeneous granules, can be prepared by binding the active ingredients to solid carriers. Examples of solid carriers are mineral earths such as silica gels, silicates, talc, kaolin, attaclay, limestone, lime, chalk, bole, loess, clay, dolomite, diatomaceous earth, calcium sulfate, magnesium sulfate, magnesium oxide, ground synthetic materials, fertilizers, such as, for example, ammonium sulfate, ammonium phosphate, ammonium nitrate, ureas, and products of vegetable origin, such as cereal meal, tree bark meal, wood meal and nutshell meal, cellulose powders and other solid carriers.

In general, the formulations comprise from 0.01 to 95% by weight, preferably from 0.1 to 90% by weight, of the active ingredient. The active ingredients are employed in a purity of from 90% to 100%, preferably 95% to 100% (according to NMR spectrum).

For seed treatment purposes, respective formulations can be diluted 2-10 fold leading to concentrations in the ready to use preparations of 0,01 to 60% by weight active compound by weight, preferably 0,1 to 40% by weight.

The compound of formula (I) can be used as such, in the form of their formulations or the use forms prepared therefrom, for example in the form of directly sprayable solutions, powders, suspensions or dispersions, emulsions, oil dispersions, pastes, dustable products, materials for spreading, or granules, by means of spraying, atomizing, dusting, spreading or pouring. The use forms depend entirely on the intended purposes; they are intended to ensure in each case the finest possible distribution of the active compounds according to the invention.

The following are examples of formulations:
1. Products for dilution with water. For seed treatment purposes, such products may be applied to the seed diluted or undiluted.
   A) Water-soluble concentrates (SL, LS)
      10 parts by weight of the active compound is dissolved in 90 parts by weight of water or a water-soluble solvent. As an alternative, wetters or other auxiliaries are added. The active compound dissolves upon dilution with water, whereby a formulation with 10 % (w/w) of active compound is obtained.
   B) Dispersible concentrates (DC)
      20 parts by weight of the active compound is dissolved in 70 parts by weight of cyclohexanone with addition of 10 parts by weight of a dispersant, for example polyvinylpyrrolidone. Dilution with water gives a dispersion, whereby a formulation with 20% (w/w) of active compounds is obtained.
   C) Emulsifiable concentrates (EC)
      15 parts by weight of the active compounds is dissolved in 7 parts by weight of xylene with addition of calcium dodecylbenzenesulfonate and castor oil ethoxylate (in each case 5 parts by weight). Dilution with water gives an emulsion, whereby a formulation with 15% (w/w) of active compounds is obtained.
   D) Emulsions (EW, EO, ES)
      25 parts by weight of the active compound is dissolved in 35 parts by weight of xylene with addition of calcium dodecylbenzenesulfonate and castor oil ethoxylate (in each case 5 parts by weight). This mixture is introduced into 30 parts by weight of water by means of an emulsifier machine (e.g. Ultraturrax) and made into a homogeneous emulsion. Dilution with water gives an emulsion, whereby a formulation with 25% (w/w) of active compound is obtained.
   E) Suspensions (SC, OD, FS)
      In an agitated ball mill, 20 parts by weight of the active compound is comminuted with addition of 10 parts by weight of dispersants, wetters and 70 parts by weight of water or of an organic solvent to give a fine active compound suspension. Dilution with water gives a stable suspension of the active compound, whereby a formulation with 20% (w/w) of active compound is obtained.
   F) Water-dispersible granules and water-soluble granules (WG, SG)
      50 parts by weight of the active compound is ground finely with addition of 50 parts by weight of dispersants and wetters and made as water-dispersible or water-soluble granules by means of technical appliances (for example extrusion, spray tower, fluidized bed). Dilution with water gives a stable dispersion or solution of the active compound, whereby a formulation with 50% (w/w) of active compound is obtained.
   G) Water-dispersible powders and water-soluble powders (WP, SP, SS, WS)
      75 parts by weight of the active compound are ground in a rotor-stator mill with addition of 25 parts by weight of dispersants, wetters and silica gel. Dilution with water gives a stable dispersion or solution of the active compound, whereby a formulation with 75% (w/w) of active compound is obtained.
   H) Gel-Formulation (GF)

   In an agitated ball mill, 20 parts by weight of the active compound is comminuted with addition of 10 parts by weight of dispersants, 1 part by weight of a gelling agent wetters and 70 parts by weight of water or of an organic solvent to give a fine active compound suspension. Dilution with water gives a stable suspension of the active compound, whereby a formulation with 20% (w/w) of active compound is obtained.
2. Products to be applied undiluted for foliar applications. For seed treatment purposes, such products may be applied to the seed diluted or undiluted.
   I) Dustable powders (DP, DS)
      5 parts by weight of the active compound are ground finely and mixed intimately with 95 parts by weight of finely divided kaolin. This gives a dustable product having 5% (w/w) of active compound.
   J) Granules (GR, FG, GG, MG)
      0.5 part by weight of the active compound is ground finely and associated with 95.5 parts by weight of carriers, whereby a formulation with 0.5% (w/w) of active compound is obtained. Current methods are extrusion, spray-drying or the fluidized bed. This gives granules to be applied undiluted for foliar use.
   K) ULV solutions (UL)
      10 parts by weight of the active compound is dissolved in 90 parts by weight of an organic solvent, for example xylene. This gives a product having 10% (w/w) of active compound, which is applied undiluted for foliar use.

Aqueous use forms can be prepared from emulsion concentrates, pastes or wettable powders (sprayable powders, oil dispersions) by adding water. To prepare emulsions, pastes or oil dispersions, the substances, as such or dissolved in an oil or solvent, can be homogenized in water by means of a wetter, tackifier, dispersant or emulsifier. Alternatively, it is possible to prepare concentrates composed of active substance, wetter, tackifier, dispersant or emulsifier and, if appropriate, solvent or oil, and such concentrates are suitable for dilution with water.

The active ingredient concentrations in the ready-to-use products can be varied within relatively wide ranges. In general, they are from 0.0001 to 10%, preferably from 0.01 to 1 %.

The active ingredients may also be used successfully in the ultra-low-volume process (ULV), it being possible to apply formulations comprising over 95% by weight of active ingredient, or even to apply the active ingredient without additives.

In the method of this invention compounds of formula (I) may be applied with other active ingredients, for example with other pesticides, insecticides, herbicides, fertilizers such as ammonium nitrate, urea, potash, and superphosphate, phytotoxicants and plant growth regulators, safeners and nematicides. These additional ingredients may be used sequentially or in combination with the above-described compositions, if appropriate also added only immediately prior to use (tank mix). For example, the plant(s) may be sprayed with a composition of this invention either before or after being treated with other active ingredients.
M.1 Acetylcholine esterase (AChE) inhibitors from the class of
M.1A carbamates, for example aldicarb, alanycarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, metolcarb, oxamyl, pirimicarb, propoxur, thiodicarb, thiofanox, trimethacarb, XMC, xylylcarb and triazamate; or from the class of
M.1B organophosphates, for example acephate, azamethiphos, azinphos-ethyl, azinphosmethyl, cadusafos, chlorethoxyfos, chlorfenvinphos, chlormephos, chlorpyrifos, chlorpyrifos-methyl, coumaphos, cyanophos, demeton-S-methyl, diazinon, dichlorvos/ DDVP, dicrotophos, dimethoate, dimethylvinphos, disulfoton, EPN, ethion, ethoprophos, famphur, fenamiphos, fenitrothion, fenthion, fosthiazate, heptenophos, imicyafos, isofenphos, isopropyl O-(methoxyaminothio-phosphoryl) salicylate, isoxathion, malathion, mecarbam, methamidophos, methidathion, mevinphos, monocrotophos, naled, omethoate, oxydemeton-methyl, parathion, parathion-methyl, phenthoate, phorate, phosalone, phosmet, phosphamidon, phoxim, pirimiphos- methyl, profenofos, propetamphos, prothiofos, pyraclofos, pyridaphenthion, quinalphos, sulfotep, tebupirimfos, temephos, terbufos, tetrachlorvinphos, thiometon, triazophos, trichlorfon and vamidothion;
M.2. GABA-gated chloride channel antagonists such as:
M.2A cyclodiene organochlorine compounds, as for example endosulfan or chlordane; or
M.2B fiproles (phenylpyrazoles), as for example ethiprole, fipronil, flufiprole, pyrafluprole and pyriprole;
M.3 Sodium channel modulators from the class of
M.3A pyrethroids, for example acrinathrin, allethrin, d-cis-trans allethrin, d-trans allethrin, bifenthrin, bioallethrin, bioallethrin S-cylclopentenyl, bioresmethrin, cycloprothrin, cyfluthrin, beta-cyfluthrin, cyhalothrin, lambda-cyhalothrin, gamma-cyhalothrin, cypermethrin, alpha-cypermethrin, beta-cypermethrin, theta-cypermethrin, zeta-cypermethrin, cyphenothrin, deltamethrin, empenthrin, esfenvalerate, etofenprox, fenpropathrin, fenvalerate, flucythrinate, flumethrin, tau-fluvalinate, halfenprox, imiprothrin, meperfluthrin,metofluthrin, momfluorothrin, permethrin, phenothrin, prallethrin, profluthrin, pyrethrin (pyrethrum), resmethrin, silafluofen, tefluthrin, tetramethylfluthrin, tetramethrin, tralomethrin and transfluthrin; or
M.3B sodium channel modulators such as DDT or methoxychlor;
M.4 Nicotinic acetylcholine receptor agonists (nAChR) from the class of
M.4A neonicotinoids, for example acteamiprid, chlothianidin, dinotefuran, imidacloprid, nitenpyram, thiacloprid and thiamethoxam; or the compounds
M.4A.1: 1-[(6-chloro-3-pyridinyl)methyl]-2,3,5,6,7,8-hexahydro-9-nitro-(5S,8R)-5,8-Epoxy-1H-imidazo[1,2-a]azepine; or
M.4A.2: 1-[(6-chloro-3-pyridyl)methyl]-2-nitro-1-[(E)-pentylideneamino]guanidine; or
M4.A.3: 1-[(6-chloro-3-pyridyl)methyl]-7-methyl-8-nitro-5-propoxy-3,5,6,7-tetrahydro-2H-imidazo[1,2-a]pyridine;
   or M.4B nicotine.
M.5 Nicotinic acetylcholine receptor allosteric activators from the class of spinosyns,
   for example spinosad or spinetoram;
M.6 Chloride channel activators from the class of avermectins and milbemycins, for example abamectin, emamectin benzoate, ivermectin, lepimectin or milbemectin;
M.7 Juvenile hormone mimics, such as
M.7A juvenile hormone analogues as hydroprene, kinoprene and methoprene; or others as M.7B fenoxycarb or M.7C pyriproxyfen;
M.8 miscellaneous non-specific (multi-site) inhibitors, for example
M.8A alkyl halides as methyl bromide and other alkyl halides, or
M.8B chloropicrin, or M.8C sulfuryl fluoride, or M.8D borax, or M.8E tartar emetic;
M.9 Selective homopteran feeding blockers, for example
M.9B pymetrozine, or M.9C flonicamid;
M.10 Mite growth inhibitors, for example
M.10A clofentezine, hexythiazox and diflovidazin, or M.10B etoxazole;
M.11 Microbial disruptors of insect midgut membranes, for example *bacillus thuringiensis* or *bacillus sphaericus* and the insecticdal proteins they produce such as *bacillus thuringiensis subsp. israelensis*, *bacillus sphaericus, bacillus thuringiensis subsp. aizawai, bacillus thuringiensis subsp. kurstaki* and *bacillus thuringiensis subsp. tenebrionis,* or the Bt crop proteins: Cry1Ab, Cry1Ac, Cry1Fa, Cry2Ab, mCry3A, Cry3Ab, Cry3Bb and Cry34/35Ab1;
M.12 Inhibitors of mitochondrial ATP synthase, for example
M.12A diafenthiuron, or
M.12B organotin miticides such as azocyclotin, cyhexatin or fenbutatin oxide, or M.12C propargite, or M.12D tetradifon;
M.13 Uncouplers of oxidative phosphorylation via disruption of the proton gradient, for example chlorfenapyr, DNOC or sulfluramid;
M.14 Nicotinic acetylcholine receptor (nAChR) channel blockers, for example nereistoxin analogues as bensultap, cartap hydrochloride, thiocyclam or thiosultap sodium;
M.15 Inhibitors of the chitin biosynthesis type 0, such as benzoylureas as for example bistrifluron, chlorfluazuron, diflubenzuron, flucycloxuron, flufenoxuron, hexaflumuron, lufenuron, novaluron, noviflumuron, teflubenzuron or triflumuron;
M.16 Inhibitors of the chitin biosynthesis type 1, as for example buprofezin;
M.17 Moulting disruptors, Dipteran, as for example cyromazine;
M.18 Ecdyson receptor agonists such as diacylhydrazines, for example methoxyfenozide, tebufenozide, halofenozide, fufenozide or chromafenozide;
M.19 Octopamin receptor agonists, as for example amitraz;
M.20 Mitochondrial complex III electron transport inhibitors, for example
M.20A hydramethylnon, or M.20B acequinocyl, or M.20C fluacrypyrim;
M.21 Mitochondrial complex I electron transport inhibitors, for example
M.21A METI acaricides and insecticides such as fenazaquin, fenpyroximate, pyrimidifen, pyridaben, tebufenpyrad or tolfenpyrad, or M.21B rotenone;
M.22 Voltage-dependent sodium channel blockers, for example
M.22A indoxacarb, or M.22B metaflumizone, or M.22C 1-[(E)-[2-(4-cyanophenyl)-1-[3-(trifluoromethyl)phenyl]ethylidene]amino]-3-[4-(difluoromethoxy)phenyl]urea;
M.23 Inhibitors of the of acetyl CoA carboxylase, such as Tetronic and Tetramic acid derivatives, for example spirodiclofen, spiromesifen or spirotetramat;
M.24 Mitochondrial complex IV electron transport inhibitors, for example
M.24A phosphine such as aluminium phosphide, calcium phosphide, phosphine or
   zinc phosphide, or M.24B cyanide.
M.25 Mitochondrial complex II electron transport inhibitors, such as beta-ketonitrile derivatives, for example cyenopyrafen or cyflumetofen;
M.28 Ryanodine receptor-modulators from the class of diamides, as for example flubendiamide, chlorantraniliprole (rynaxypyr®), cyantraniliprole (cyazypyr®), or the phthalamide compounds
M.28.1: (R)-3-Chlor-N1-{2-methyl-4-[1,2,2,2-tetrafluor-1-(trifluormethyl)ethyl]phenyl}-N2-(1-methyl-2-methylsulfonylethyl)phthalamid and
M.28.2: (S)-3-Chlor-N1-{2-methyl-4-[1,2,2,2-tetrafluor-1-(trifluormethyl)ethyl]phenyl}-N2-(1-methyl-2-methylsulfonylethyl)phthalamid, or the compound
M.28.3: 3-bromo-N-{2-bromo-4-chloro-6-[(1-cyclopropylethyl)carbamoyl]phenyl}-1 -(3-chlorpyridin-2-yl)-1H-pyrazole-5-carboxamide (proposed ISO name: cyclaniliprole), or the compound
M.28.4: methyl-2-[3,5-dibromo-2-({[3-bromo-1-(3-chlorpyridin-2-yl)-1H-pyrazol-5-yl]carbonyl}amino)benzoyl]-1,2-dimethylhydrazinecarboxylate; or a compound selected from M.28.5a) to M.28.5l):
M.28.5a) N-[4,6-dichloro-2-[(diethyl-lambda-4-sulfanylidene)carbamoyl]-phenyl]-2-(3-chloro-2-pyridyl)-5-(trifluoromethyl)pyrazole-3-carboxamide;
M.28.5b) N-[4-chloro-2-[(diethyl-lambda-4-sulfanylidene)carbamoyl]-6-methyl-phenyl]-2-(3-chloro-2-pyridyl)-5-(trifluoromethyl)pyrazole-3-carboxamide;
M.28.5c) N-[4-chloro-2-[(di-2-propyl-lambda-4-sulfanylidene)carbamoyl]-6-methyl-phenyl]-2-(3-chloro-2-pyridyl)-5-(trifluoromethyl)pyrazole-3-carboxamide;
M.28.5d) N-[4,6-dichloro-2-[(di-2-propyl-lambda-4-sulfanylidene)carbamoyl]-phenyl]-2-(3-chloro-2-pyridyl)-5-(trifluoromethyl)pyrazole-3-carboxamide;
M.28.5e) N-[4,6-dichloro-2-[(diethyl-lambda-4-sulfanylidene)carbamoyl]-phenyl]-2-(3-chloro-2-pyridyl)-5-(difluoromethyl)pyrazole-3-carboxamide;
M.28.5f) N-[4,6-dibromo-2-[(di-2-propyl-lambda-4-sulfanylidene)carbamoyl]-phenyl]-2-(3-chloro-2-pyridyl)-5-(trifluoromethyl)pyrazole-3-carboxamide;
M.28.5g) N-[4-chloro-2-[(di-2-propyl-lambda-4-sulfanylidene)carbamoyl]-6-cyano-phenyl]-2-(3-chloro-2-pyridyl)-5-(trifluoromethyl)pyrazole-3-carboxamide;
M.28.5h) N-[4,6-dibromo-2-[(diethyl-lambda-4-sulfanylidene)carbamoyl]-phenyl]-2-(3-chloro-2-pyridyl)-5-(trifluoromethyl)pyrazole-3-carboxamide;
M.28.5i) N-[2-(5-amino-1,3,4-thiadiazol-2-yl)-4-chloro-6-methyl-phenyl]-5-bromo-2-(3-chloro-2-pyridyl)pyrazole-3-carboxamide;
M.28.5j) 5-chloro-2-(3-chloro-2-pyridyl)-N-[2,4-dichloro-6-[(1-cyano-1-methylethyl)carbamoyl]phenyl]pyrazole-3-carboxamide;
M.28.5k) 5-bromo-N-[2,4-dichloro-6-(methylcarbamoyl)phenyl]-2-(3,5-dichloro-2-pyridyl)pyrazole-3-carboxamide;
M.28.5l) N-[2-(tert-butylcarbamoyl)-4-chloro-6-methyl-phenyl]-2-(3-chloro-2-pyridyl)-5-(fluoromethoxy)pyrazole-3-carboxamide; or a compound selected from
M.28.6 N2-(1-cyano-1-methyl-ethyl)-N1-(2,4-dimethylphenyl)-3-iodo-phthalamide; or
M.28.7 3-chloro-N2-(1-cyano-1-methyl-ethyl)-N1-(2,4-dimethylphenyl)phthalamide;
M.UN.X insecticidal active compounds of unknown or uncertain mode of action, as for example afidopyropen, azadirachtin, amidoflumet, benzoximate, bifenazate, bromopropylate, chinomethionat, cryolite, dicofol, flufenerim, flometoquin, fluensulfone, flupyradifurone, piperonyl butoxide, pyridalyl, pyrifluquinazon, sulfoxaflor, pyflubumide or the compounds
M.UN.X.1: 4-[5-(3,5-Dichloro-phenyl)-5-trifluoromethyl-4,5-dihydro-isoxazol-3-yl]-2-methyl-N-[(2,2,2-trifluoro-ethylcarbamoyl)-methyl]-benzamide, or the compound
M.UN.X.2: 4-[5-[3-chloro-5-(trifluoromethyl)phenyl]-5-(trifluoromethyl)-4H-isoxazol-3-yl]-N-[2-oxo-2-(2,2,2-trifluoroethylamino)ethyl]naphthalene-1-carboxamide, or the compound
M.UN.X.3: 11-(4-chloro-2,6-dimethylphenyl)-12-hydroxy-1,4-dioxa-9-azadispiro[4.2.4.2]-tetradec-11-en-10-one, or the compound
M.UN.X.4: 3-(4'-fluoro-2,4-dimethylbiphenyl-3-yl)-4-hydroxy-8-oxa-1-azaspiro[4.5]dec-3-en-2-one, or the compound
M.UN.X.5: 1-[2-fluoro-4-methyl-5-[(2,2,2-trifluoroethyl)sulfinyl]phenyl]-3-(trifluoromethyl)-1H-1,2,4-triazole-5-amine, or actives on basis of *bacillus firmus* (Votivo, I-1582); or
M.UN.X.6; a compound selected from the group of
M.UN.X.6a) (E/Z)-N-[1-[(6-chloro-3-pyridyl)methyl]-2-pyridylidene]-2,2,2-trifluoroacetamide;
M.UN.X.6b) (E/Z)-N-[1-[(6-chloro-5-fluoro-3-pyridyl)methyl]-2-pyridylidene]-2,2,2-trifluoroacetamide;
M.UN.X.6c) (E/Z)-2,2,2-trifluoro-N-[1-[(6-fluoro-3-pyridyl)methyl]-2-pyridylidene]acetamide;
M.UN.X.6d) (E/Z)-N-[1-[(6-bromo-3-pyridyl)methyl]-2-pyridylidene]-2,2,2-trifluoroacetamide;
M.UN.X.6e) (E/Z)-N-[1-[1-(6-chloro-3-pyridyl)ethyl]-2-pyridylidene]-2,2,2-trifluoroacetamide;
M.UN.X.6f) (E/Z)-N-[1-[(6-chloro-3-pyridyl)methyl]-2-pyridylidene]-2,2-difluoro-acetamide;
M.UN.X.6g) (E/Z)-2-chloro-N-[1-[(6-chloro-3-pyridyl)methyl]-2-pyridylidene]-2,2-difluoro-acetamide;
M.UN.X.6h) (E/Z)-N-[1-[(2-chloropyrimidin-5-yl)methyl]-2-pyridylidene]-2,2,2-trifluoroacetamide and
M.UN.X.6i) (E/Z)-N-[1-[(6-chloro-3-pyridyl)methyl]-2-pyridylidene]-2,2,3,3,3-pentafluoro-propanamide.); or of the compounds
M.UN.X.7: 3-[3-chloro-5-(trifluoromethyl)phenyl]-4-oxo-1-(pyrimidin-5-ylmethyl)pyrido[1,2-a]pyrimidin-1-ium-2-olate; or
M.UN.X.8: 8-chloro-N-[2-chloro-5-methoxyphenyl)sulfonyl]-6-trifluoromethyl)-imidazo[1,2-a]pyridine-2-carboxamide; or
M.UN.X.9: 4-[5-(3,5-dichlorophenyl)-5-(trifluoromethyl)-4H-isoxazol-3-yl]-2-methyl-N-(1-oxothietan-3-yl)benzamide; or
M.UN.X.10: 5-[3-[2,6-dichloro-4-(3,3-dichloroallyloxy)phenoxy]propoxy]-1H-pyrazole.

The commercially available compounds of the group M listed above may be found in The Pesticide Manual, 15th Edition, C. D. S. Tomlin, British Crop Protection Council (2011) among other publications.

The quinoline derivative flometoquin is shown in WO 2006/013896. The aminofuranone compound flupyradifurone is known from WO 2007/115644. The sulfoximine compound sulfoxaflor is known from WO 2007/149134. The pyrethroid momfluorothrin is known from US 6908945. The pyrazole acaricide pyflubumide is known from WO 2007/020986. The isoxazoline compounds have been described likewise M.UN.X.1 in WO 2005/085216, M.UN.X2. in WO 2009/002809 and in WO 2011/149749 and the isoxazoline M.UN.X.9 in WO 2013/050317. The pyripyropene derivative afidopyropen has been described in WO 2006/129714. The spiroketal-substituted cyclic ketoenol derivative M.UN.X.3 is known from WO 2006/089633 and the biphenyl-substituted spirocyclic ketoenol derivative M.UN.X.4 from WO 2008/067911. Finally triazoylphenylsulfide like M.UN.X.5 have been described in WO 2006/043635 and biological control agents on basis of *bacillus firmus* in WO 2009/124707. The neonicotionids 4A.1 is known from WO 20120/069266 and WO 2011/06946, the M.4.A.2 from WO 2013/003977, the M4.A.3.from WO 2010/069266.

The Metaflumizone analogue M.22C is described in CN 10171577. The phthalamides M.28.1 and M.28.2 are both known from WO 2007/101540. The anthranilamide M.28.3 has been described in WO 2005/077934. The hydrazide compound M.28.4 has been described in WO 2007/043677. The anthranilamides M.28.5a) to M.28.5h) can be prepared as described in WO 2007/006670, WO 2013/024009 and WO 2013/024010, the anthranilamide M.28.5i) is described in WO 2011/085575, the M.28.5j) in WO 2008/134969, the M.28.5k) in US 2011/046186 and the M.28.5l) in WO 2012/034403. The diamide compounds M.28.6 and M.28.7 can be found in CN 102613183.

The compounds M.UN.X.6a) to M.UN.X.6i) listed in M.UN.X.6 have been described in WO 2012/029672. The mesoionic compound M.UN.X.7 was described in WO 2012/092115, the nematicide M.UN.X.8 in WO 2013/055584 and the Pyridalyl-type analogue M.UN.X.10 in WO 2010/060379.

In another embodiment of the invention, the compounds of formula (I), or their stereoisomers, salts, tautomers and N-oxides, may also be applied with fungicides as compound II.

The following list F of active substances, in conjunction with which the compounds according to the invention can be used, is intended to illustrate the possible combinations but does not limit them:
F.I) Respiration Inhibitors
F.I-1) Inhibitors of complex III at Qo site:
   strobilurins: azoxystrobin, coumethoxystrobin, coumoxystrobin, dimoxystrobin, enestroburin, fluoxastrobin, kresoxim-methyl, metominostrobin, orysastrobin, picoxystrobin, pyraclostrobin, pyrametostrobin, pyraoxystrobin, pyribencarb, triclopyricarb/chlorodincarb, trifloxystrobin, 2-[2-(2,5-dimethyl-phenoxymethyl)-phenyl]-3-methoxy-acrylic acid methyl ester and 2(2-(3-(2,6-dichlorophenyl)-1-methyl-allylideneaminooxymethyl)-phenyl)-2-methoxyimino-N methyl-acetamide;
   oxazolidinediones and imidazolinones: famoxadone, fenamidone;
F.I-2) Inhibitors of complex II (e.g. carboxamides):
   carboxanilides: benodanil, benzovindiflupyr, bixafen, boscalid, carboxin, fenfuram, fenhexamid, fluopyram, flutolanil, furametpyr, isopyrazam, isotianil, mepronil, oxycarboxin, penflufen, penthiopyrad, sedaxane, tecloftalam, thifluzamide, tiadinil, 2-amino-4 methyl-thiazole-5-carboxanilide, N-(3',4',5' trifluorobiphenyl-2yl)-3-difluoromethyl-1-methyl-1H-pyrazole-4 carboxamide (fluxapyroxad), N-(4'-trifluoromethylthiobiphenyl-2-yl)-3 difluoromethyl-1-methyl-1H pyrazole-4-carboxamide, N-(2-(1,3,3-trimethyl-butyl)-phenyl)-1,3-dimethyl-5fluoro-1H-pyrazole-4 carboxamide, 3-(difluoromethyl)-1-methyl-N-(1,1,3-trimethylindan-4-yl)pyrazole-4-carboxamide, 3-(trifluoromethyl)-1-methyl-N-(1,1,3-trimethylindan-4-yl)pyrazole-4-carboxamide, 1,3-dimethyl-N-(1,1,3-trimethylindan-4-yl)pyrazole-4-carboxamide, 3-(trifluoromethyl)-1,5-dimethyl-N-(1,1,3-trimethylindan-4-yl)pyrazole-4-carboxamide, 3-(difluoromethyl)-1,5-dimethyl-N-(1,1,3-trimethylindan-4-yl)pyrazole-4-carboxamide, 1,3,5-trimethyl-N-(1,1,3-trimethylindan-4-yl)pyrazole-4-carboxamide, 3-(difluoromethyl)-1-methyl-N-(1,1,3-trimethylindan-4-yl)pyrazole-4-carboxamide, 3-(trifluoromethyl)-1-methyl-N-(1,1,3-trimethylindan-4-yl)pyrazole-4-carboxamide, 1,3-dimethyl-N-(1,1,3-trimethylindan-4-yl)pyrazole-4-carboxamide, 3-(trifluoromethyl)-1,5-dimethyl-N-(1,1,3-trimethylindan-4-yl)pyrazole-4-carboxamide, 3-(difluoromethyl)-1,5-dimethyl-N-(1,1,3-trimethylindan-4-yl)pyrazole-4-carboxamide, 1,3,5-trimethyl-N-(1,1,3-trimethylindan-4-yl)pyrazole-4-carboxamide;
F.I-3) Inhibitors of complex III at Qi site: cyazofamid, amisulbrom, [(3S,6S,7R,8R)-8-benzyl-3-[(3-acetoxy-4-methoxy-pyridine-2-carbonyl)amino]-6-methyl-4,9-dioxo-1,5-dioxonan-7-yl] 2-methylpropanoate, [(3S,6S,7R,8R)-8-benzyl-3-[[3-(acetoxymethoxy)-4-methoxy-pyridine-2-carbonyl]amino]-6-methyl-4,9-dioxo-1,5-dioxonan-7-yl] 2-methylpropanoate, [(3S,6S,7R,8R)-8-benzyl-3-[(3-isobutoxycarbonyloxy-4-methoxy-pyridine-2-carbonyl)amino]-6-methyl-4,9-dioxo-1,5-dioxonan-7-yl] 2-methylpropanoate, [(3S,6S,7R,8R)-8-benzyl-3-[[3-(1,3-benzodioxol-5-ylmethoxy)-4-methoxy-pyridine-2-carbonyl]amino]-6-methyl-4,9-dioxo-1,5-dioxonan-7-yl] 2-methylpropanoate, 3S,6*S*,7*R*,8*R*)-3-[[(3-hydroxy-4-methoxy-2-pyridinyl)carbonyl]amino]-6-methyl-4,9-dioxo-8-(phenylmethyl)-1,5-dioxonan-7-yl 2-methylpropanoate;
F.I-4) Other respiration inhibitors (complex I, uncouplers) diflumetorim; (5,8-difluoroquinazolin-4-yl)-{2-[2-fluoro-4-(4-trifluoromethylpyridin-2-yloxy)-phenyl]-ethyl}-amine; tecnazen;ametoctradin; silthiofam; nitrophenyl derivates: binapacryl, dinobuton, dinocap, fluazinam, ferimzone, nitrthal-isopropyl,
   and including organometal compounds: fentin salts, such as fentin-acetate, fentin chloride or fentin hydroxide;
F.II) Sterol biosynthesis inhibitors (SBI fungicides)
F.II-1) C14 demethylase inhibitors (DMI fungicides, e.g. triazoles, imidazoles)
   triazoles: azaconazole, bitertanol, bromuconazole, cyproconazole, difenoconazole, diniconazole, diniconazole-M, epoxiconazole, fenbuconazole, fluquinconazole, flusilazole, flutriafol, hexaconazole, imibenconazole, ipconazole, metconazole, myclobutanil, paclobutrazole, penconazole, propiconazole, prothioconazole, simeconazole, tebuconazole, tetraconazole, triadimefon, triadimenol, triticonazole, uniconazole, 1-[*rel*-(2*S*;3*R*)-3-(2-chlorophenyl)-2-(2,4-difluorophenyl)-oxiranylmethyl]-5-thiocyanato-1H-[1,2,4]triazole,2-[*rel*-(2*S*;3*R*)-3-(2-chlorophenyl)-2-(2,4-difluorophenyl)-oxiranylmethyl]-2H-[1,2,4]triazole-3-thiol;
   imidazoles: imazalil, pefurazoate, oxpoconazole, prochloraz, triflumizole;
   pyrimidines, pyridines and piperazines: fenarimol, nuarimol, pyrifenox, triforine, 1-[rel-(2*S*;3*R*)-3-(2-chlorophenyl)-2-(2,4-difluorophenyl)-oxiranylmethyl]-5-thiocyanato-1H-[1,2,4]triazole, 2-[rel-(2S;3R)-3-(2-chlorophenyl)-2-(2,4-difluorophenyl)-oxiranylmethyl]-2H-[1,2,4]triazole-3-thiol;
F.II-2) Delta14-reductase inhitors (Amines, e.g. morpholines, piperidines)
   morpholines: aldimorph, dodemorph, dodemorph-acetate, fenpropimorph, tridemorph;
   piperidines: fenpropidin, piperalin; spiroketalamines: spiroxamine;
F.II-3) Inhibitors of 3-keto reductase: hydroxyanilides: fenhexamid;
F.III) Nucleic acid synthesis inhibitors
F.III-1) RNA, DNA synthesis
   phenylamides or acyl amino acid fungicides: benalaxyl, benalaxyl-M, kiralaxyl, metalaxyl, metalaxyl-M (mefenoxam), ofurace, oxadixyl;
   isoxazoles and iosothiazolones: hymexazole, octhilinone;
F.III-2) DNA topisomerase inhibitors: oxolinic acid;
F.III-3) Nucleotide metabolism (e.g. adenosin-deaminase), hydroxy (2-amino)-pyrimidines: bupirimate;
F.IV) Inhibitors of cell division and or cytoskeleton
F.IV-1) Tubulin inhibitors: benzimidazoles and thiophanates: benomyl, carbendazim, fuberidazole, thiabendazole, thiophanate-methyl;
   triazolopyrimidines: 5-chloro-7 (4-methylpiperidin-1-yl)-6-(2,4,6-trifluorophenyl)-[1,2,4]triazolo[1,5 a]pyrimidine;
F.IV-2) Other cell division inhibitors
   benzamides and phenyl acetamides: diethofencarb, ethaboxam, pencycuron, fluopicolide, zoxamide;
F.IV-3) Actin inhibitors: benzophenones: metrafenone; pyriofenone;
F.V) Inhibitors of amino acid and protein synthesis
F.V-1) Methionine synthesis inhibitors (anilino-pyrimidines)
   anilino-pyrimidines: cyprodinil, mepanipyrim, nitrapyrin, pyrimethanil;
F.V-2) Protein synthesis inhibitors (anilino-pyrimidines)
   antibiotics: blasticidin-S, kasugamycin, kasugamycin hydrochloride-hydrate, mildiomycin, streptomycin, oxytetracyclin, polyoxine, validamycin A;
F.VI) Signal transduction inhibitors
F.VI-1) MAP / Histidine kinase inhibitors (e.g. anilino-pyrimidines)
   dicarboximides: fluoroimid, iprodione, procymidone, vinclozolin;
   phenylpyrroles: fenpiclonil, fludioxonil;
F.VI-2) G protein inhibitors: quinolines: quinoxyfen;
F.VII) Lipid and membrane synthesis inhibitors
F.VII-1) Phospholipid biosynthesis inhibitors
   organophosphorus compounds: edifenphos, iprobenfos, pyrazophos;
   dithiolanes: isoprothiolane;
F.VII-2) Lipid peroxidation: aromatic hydrocarbons: dicloran, quintozene, tecnazene, tolclofos-methyl, biphenyl, chloroneb, etridiazole;
F.VII-3) Carboxyl acid amides (CAA fungicides)
   cinnamic or mandelic acid amides: dimethomorph, flumorph, mandiproamid, pyrimorph;
   valinamide carbamates: benthiavalicarb, iprovalicarb, pyribencarb, valifenalate and N-(1-(1-(4-cyano-phenyl)ethanesulfonyl)-but-2-yl) carbamic acid-(4-fluorophenyl) ester;
F.VII-4) Compounds affecting cell membrane permeability and fatty acids:
   1-[4-[4-[5-(2,6-difluorophenyl)-4,5-dihydro-3-isoxazolyl]-2-thiazolyl]-1-piperidinyl]-2-[5-methyl-3-(trifluoromethyl)-1H-pyrazol-1-yl]ethanone, carbamates: propamocarb, propamocarb-hydrochlorid,
F.VII-5) fatty acid amide hydrolase inhibitors: 1-[4-[4-[5-(2,6-difluorophenyl)-4,5-dihydro-3-isoxazolyl]-2-thiazolyl]-1-piperidinyl]-2-[5-methyl-3-(trifluoromethyl)-1H-pyrazol-1-yl]ethanone;
F.VIII) Inhibitors with Multi Site Action
F.VIII-1) Inorganic active substances: Bordeaux mixture, copper acetate, copper hydroxide, copper oxychloride, basic copper sulfate, sulfur;
F.VIII-2) Thio- and dithiocarbamates: ferbam, mancozeb, maneb, metam, methasulphocarb, metiram, propineb, thiram, zineb, ziram;
F.VIII-3) Organochlorine compounds (e.g. phthalimides, sulfamides, chloronitriles):
   anilazine, chlorothalonil, captafol, captan, folpet, dichlofluanid, dichlorophen, flusulfamide, hexachlorobenzene, pentachlorphenole and its salts, phthalide, tolylfluanid, N-(4-chloro-2-nitrophenyl)-N-ethyl-4-methyl-benzenesulfonamide;
F.VIII-4) Guanidines and other: guanidine, dodine, dodine free base, guazatine, guazatine-acetate, iminoctadine, iminoctadine-triacetate, iminoctadine-tris(albesilate), 2,6-dimethyl-1H,5H-[1,4]dithiino[2,3-c:5,6-c']dipyrrole-1,3,5,7(2H,6H)-tetraone;
F.VIII-5) Ahtraquinones: dithianon;
F.IX) Cell wall synthesis inhibitors
F.IX-1) Inhibitors of glucan synthesis: validamycin, polyoxin B;
F.IX-2) Melanin synthesis inhibitors: pyroquilon, tricyclazole, carpropamide, dicyclomet, fenoxanil;
F.X) Plant defence inducers
F.X-1) Salicylic acid pathway: acibenzolar-S-methyl;
F.X-2) Others: probenazole, isotianil, tiadinil, prohexadione-calcium;
   phosphonates: fosetyl, fosetyl-aluminum, phosphorous acid and its salts;
F.XI) Unknown mode of action:bronopol, chinomethionat, cyflufenamid, cymoxanil, dazomet, debacarb, diclomezine, difenzoquat, difenzoquat-methylsulfate, diphenylamin, fenpyrazamine, flumetover, flusulfamide, flutianil, methasulfocarb, nitrapyrin, nitrothal-isopropyl, oxathiapiprolin, oxin-copper, proquinazid, tebufloquin, tecloftalam, triazoxide, 2-butoxy-6-iodo-3-propylchromen-4-one, N-(cyclopropylmethoxyimino-(6-difluoro-methoxy-2,3-difluoro-phenyl)-methyl)-2-phenyl acetamide, N'-(4-(4-chloro-3-trifluoromethyl-phenoxy)-2,5-dimethyl-phenyl)-N-ethyl-N methyl formamidine, N' (4-(4-fluoro-3-trifluoromethyl-phenoxy)-2,5-dimethyl-phenyl)-N-ethyl-N-methyl formamidine, N'-(2-methyl-5-trifluoromethyl-4-(3-trimethylsilanyl-propoxy)-phenyl)-N-ethyl-N-methyl formamidine, N'-(5-difluoromethyl-2 methyl-4-(3-trimethylsilanyl-propoxy)-phenyl)-N-ethyl-N-methyl formamidine, 2-{1-[2-(5-methyl-3-trifluoromethyl-pyrazole-1-yl)-acetyl]-piperidin-4-yl}-thiazole-4-carboxylic acid methyl-(1,2,3,4-tetrahydro-naphthalen-1-yl)-amide, 2-{1-[2-(5-methyl-3-trifluoromethyl-pyrazole-1-yl)-acetyl]-piperidin-4-yl}-thiazole-4-carboxylic acid methyl-(*R*)-1,2,3,4-tetrahydro-naphthalen-1-yl-amide, methoxy-acetic acid 6-tert-butyl-8-fluoro-2,3-dimethyl-quinolin-4-yl ester and N-Methyl-2-{1-[(5-methyl-3-trifluoromethyl-1H-pyrazol-1-yl)-acetyl]-piperidin-4-yl}-N-[(1*R*)-1,2,3,4-tetrahydronaphthalen-1-yl]-4-thiazolecarboxamide, 3-[5-(4-chloro-phenyl)-2,3-dimethyl-isoxazolidin-3 yl]-pyridine, pyrisoxazole, 5-amino-2-isopropyl-3-oxo-4-ortho-tolyl-2,3-dihydro-pyrazole-1 carbothioic acid *S-*allyl ester, N-(6-methoxy-pyridin-3-yl) cyclopropanecarboxylic acid amide, 5-chloro-1 (4,6-dimethoxy-pyrimidin-2-yl)-2-methyl-1H-benzoimidazole, 2-(4-chloro-phenyl)-N-[4-(3,4-dimethoxy-phenyl)-isoxazol-5-yl]-2-prop-2-ynyloxyacetamide;
F.XI) Growth regulators: abscisic acid, amidochlor, ancymidol, 6-benzylaminopurine, brassinolide, butralin, chlormequat (chlormequat chloride), choline chloride, cyclanilide, daminozide, dikegulac, dimethipin, 2,6-dimethylpuridine, ethephon, flumetralin, flurprimidol, fluthiacet, forchlorfenuron, gibberellic acid, inabenfide, indole-3-acetic acid, maleic hydrazide, mefluidide, mepiquat (mepiquat chloride), naphthaleneacetic acid, N 6-benzyladenine, paclobutrazol, prohexadione (prohexadione-calcium), prohydrojasmon, thidiazuron, triapenthenol, tributyl phosphorotrithioate, 2,3,5 tri iodobenzoic acid, trinexapac-ethyl and uniconazole;
F.XII) Biological control agents

*Ampelomyces quisqualis* (e.g. AQ 10® from Intrachem Bio GmbH & Co. KG, Germany), *Aspergillus flavus* (e.g. AFLAGUARD® from Syngenta, CH), *Aureobasidium pullulans* (e.g. BOTECTOR® from bio-ferm GmbH, Germany), *Bacillus pumilus* (e.g. NRRL Accession No. B-30087 in SONATA® and BALLAD® Plus from AgraQuest Inc., USA), *Bacillus subtilis* (e.g. isolate NRRL-Nr. B-21661 in RHAPSODY®, SERENADE® MAX and SERENADE® ASO from AgraQuest Inc., USA), *Bacillus subtilis* var. *amyloliquefaciens* FZB24 (e.g. TAEGRO® from Novozyme Biologicals, Inc., USA), *Candida oleophila* I-82 (e.g. ASPIRE® from Ecogen Inc., USA), *Candida saitoana* (e.g. BIOCURE® (in mixture with lysozyme) and BIOCOAT® from Micro Flo Company, USA (BASF SE) and Arysta), Chitosan (e.g. ARMOUR-ZEN from BotriZen Ltd., NZ), *Clonostachys rosea* f. *catenulata,* also named *Gliocladium catenulatum* (e.g. isolate J1446: PRESTOP® from Verdera, Finland), *Coniothyrium minitans* (e.g. CONTANS® from Prophyta, Germany), *Cryphonectria parasitica* (e.g. *Endothia parasitica* from CNICM, France), *Cryptococcus albidus* (e.g. YIELD PLUS® from Anchor Bio-Technologies, South Africa), *Fusarium oxysporum* (e.g. BIOFOX® from S.I.A.P.A., Italy, FUSACLEAN® from Natural Plant Protection, France), *Metschnikowia fructicola* (e.g. SHEMER® from Agrogreen, Israel), *Microdochium dimerum* (e.g. ANTIBOT® from Agrauxine, France), *Phlebiopsis gigantea* (e.g. ROTSOP®from Verdera, Finland), *Pseudozyma flocculosa* (e.g. SPORODEX® from Plant Products Co. Ltd., Canada), *Pythium oligandrum* DV74 (e.g. POLYVERSUM® from Remeslo SSRO, Biopreparaty, Czech Rep.), *Reynoutria sachlinensis* (e.g. REGALIA® from Marrone Biolnnovations, USA), *Talaromyces flavus* V117b (e.g. PROTUS® from Prophyta, Germany), *Trichoderma asperellum* SKT-1 (e.g. ECO-HOPE® from Kumiai Chemical Industry Co., Ltd., Japan), *T. atroviride* LC52 (e.g. SENTINEL® from Agrimm Technologies Ltd, NZ), *T. harzianum* T-22 (e.g. PLANTSHIELD® der Firma BioWorks Inc., USA), *T. harzianum* TH 35 (e.g. ROOT PRO® from Mycontrol Ltd., Israel), *T. harzianum* T-39 (e.g. TRICHODEX® and TRICHODERMA 2000® from Mycontrol Ltd., Israel and Makhteshim Ltd., Israel), *T. harzianum* and *T. viride* (e.g. TRICHOPEL from Agrimm Technologies Ltd, NZ), *T. harzianum* ICC012 and *T. viride* ICC080 (e.g. REMEDIER® WP from Isagro Ricerca, Italy), *T. polysporum* and *T. harzianum* (e.g. BINAB® from BINAB Bio-Innovation AB, Sweden), *T. stromaticum* (e.g. TRICOVAB® from C.E.P.L.A.C., Brazil), *T. virens* GL-21 (e.g. SOILGARD® from Certis LLC, USA), *T. viride* (e.g. TRIECO® from Ecosense Labs. (India) Pvt. Ltd., Indien, BIO-CURE® F from T. Stanes & Co. Ltd., Indien), *T. viride* TV1 (e.g. T. viride TV1 from Agribiotec srl, Italy), *Ulocladium oudemansii* HRU3 (e.g. BOTRY-ZEN® from Botry-Zen Ltd, NZ).

The commercially available compounds II of the group F listed above may be found in The Pesticide Manual, 15th Edition, C. D. S. Tomlin, British Crop Protection Council (2011) among other publications. Their preparation and their activity against harmful fungi is known (cf.: http://www.alanwood.net/pesticides/); these substances are commercially available. The compounds described by IUPAC nomenclature, their preparation and their fungicidal activity are also known (cf. Can. J. Plant Sci. 48(6), 587-94, 1968; EP A 141 317; EP-A 152 031; EP A 226 917; EP A 243 970; EP A 256 503; EP-A 428 941; EP-A 532 022; EP-A 1 028 125; EP-A 1 035 122; EP A 1 201 648; EP A 1 122 244, JP 2002316902; DE 19650197; DE 10021412; DE 102005009458; US 3,296,272; US 3,325,503; WO 98/46608; WO 99/14187; WO 99/24413; WO 99/27783; WO 00/29404; WO 00/46148; WO 00/65913; WO 01/54501; WO 01/56358; WO 02/22583; WO 02/40431; WO 03/10149; WO 03/11853; WO 03/14103; WO 03/16286; WO 03/53145; WO 03/61388; WO 03/66609; WO 03/74491; WO 04/49804; WO 04/83193; WO 05/120234; WO 05/123689; WO 05/123690; WO 05/63721; WO 05/87772; WO 05/87773; WO 06/15866; WO 06/87325; WO 06/87343; WO 07/82098; WO 07/90624, WO 11/028657).

The invertebrate pest, e.g. the insects, arachnids and nematodes, the plant, soil or water in which the plant is growing can be contacted with the present compounds of formula (I), including their stereoisomers and tautomers, as well the salts thereof, or composition(s) containing them by any application method known in the art. As such, "contacting" includes both direct contact (applying the compounds/compositions directly on the animal pest or plant - typically to the foliage, stem or roots of the plant) and indirect contact (applying the compounds/compositions to the locus of the animal pest or plant).

The compounds of formula (I), including their stereoisomers and tautomers, as well the salts thereof, or the pesticidal compositions comprising them may be used to protect growing plants and crops from attack or infestation by animal pests, especially insects, acaridae or arachnids by contacting the plant/crop with a pesticidally effective amount of compounds of formula (I). The term "crop" refers both to growing and harvested crops.

The compounds of the present invention and the compositions comprising them are particularly important in the control of a multitude of insects on various cultivated plants, such as cereal, root crops, oil crops, vegetables, spices, ornamentals, for example seed of durum and other wheat, barley, oats, rye, maize (fodder maize and sugar maize / sweet and field corn), soybeans, oil crops, crucifers, cotton, sunflowers, bananas, rice, oilseed rape, turnip rape, sugarbeet, fodder beet, eggplants, potatoes, grass, lawn, turf, fodder grass, tomatoes, leeks, pumpkin/squash, cabbage, iceberg lettuce, pepper, cucumbers, melons, Brassica species, melons, beans, peas, garlic, onions, carrots, tuberous plants such as potatoes, sugar cane, tobacco, grapes, petunias, geranium/pelargoniums, pansies and impatiens.

The compounds of the present invention are employed as such or in form of compositions by treating the insects or the plants, plant propagation materials, such as seeds, soil, surfaces, materials or rooms to be protected from insecticidal attack with a insecticidally effective amount of the active compounds. The application can be carried out both before and after the infection of the plants, plant propagation materials, such as seeds, soil, surfaces, materials or rooms by the insects.

The present invention also includes a method of combating animal pests which comprises contacting the animal pests, their habit, breeding ground, food supply, cultivated plants, seed, soil, area, material or environment in which the animal pests are growing or may grow, or the materials, plants, seeds, soils, surfaces or spaces to be protected from animal attack or infestation with a pesticidally effective amount of at least one active compound of the formula (I), a stereoisomers, a tautomere or a salt thereof.
Moreover, animal pests may be controlled by contacting the target pest, its food supply, habitat, breeding ground or its locus with a pesticidally effective amount of compounds of formula (I), a stereoisomer, a tautomere or a salt thereof. As such, the application may be carried out before or after the infection of the locus, growing crops, or harvested crops by the pest.

The compounds of the invention can also be applied preventively to places at which occurrence of the pests is expected.

The compounds of formula (I), including their stereoisomers and their tautomers, as well as their salts may be also used to protect growing plants from attack or infestation by pests. The use includes contacting the plant with a pesticidally effective amount of compounds of formula (I), a stereoisomer, a tautomere or a salt thereof. As such, "contacting" includes both direct contact, i.e. applying the compounds/compositions directly on the pest and/or plant - typically to the foliage, stem or roots of the plant, and indirect contact, i.e. applying the compounds/compositions to the locus of the pest and/or plant.

"Locus" means a habitat, breeding ground, plant, seed, soil, area, material or environment in which a pest or parasite is growing or may grow.

The term "plant propagation material" is to be understood to denote all the generative parts of the plant such as seeds and vegetative plant material such as cuttings and tubers (e. g. potatoes), which can be used for the multiplication of the plant. This includes seeds, roots, fruits, tubers, bulbs, rhizomes, shoots, sprouts and other parts of plants. Seedlings and young plants, which are to be transplanted after germination or after emergence from soil, may also be included. These plant propagation materials may be treated prophylactically with a plant protection compound either at or before planting or transplanting.

The term "cultivated plants" is to be understood as including plants which have been modified by breeding, mutagenesis or genetic engineering. Genetically modified plants are plants, which genetic material has been so modified by the use of recombinant DNA techniques that under natural circumstances cannot readily be obtained by cross breeding, mutations or natural recombination. Typically, one or more genes have been integrated into the genetic material of a genetically modified plant in order to improve certain properties of the plant. Such genetic modifications also include but are not limited to targeted post-transtional modification of protein(s) (oligo- or polypeptides) poly for example by glycosylation or polymer additions such as prenylated, acetylated or farnesylated moieties or PEG moieties(e.g. as disclosed in Biotechnol Prog. 2001 Jul-Aug;17(4):720-8., Protein Eng Des Sel. 2004 Jan;17(1):57-66, Nat Protoc. 2007;2(5):1225-35., Curr Opin Chem Biol. 2006 Oct;10(5):487-91. Epub 2006 Aug 28., Biomaterials. 2001 Mar;22(5):405-17, Bioconjug Chem. 2005 Jan-Feb;16(1):113-21).

The term "cultivated plants" is to be understood also including plants that have been rendered tolerant to applications of specific classes of herbicides, such as hydroxy-phenylpyruvate dioxygenase (HPPD) inhibitors; acetolactate synthase (ALS) inhibitors, such as sulfonyl ureas (see e. g. US 6,222,100, WO 01/82685, WO 00/26390, WO 97/41218, WO 98/02526, WO 98/02527, WO 04/106529, WO 05/20673, WO 03/14357, WO 03/13225, WO 03/14356, WO 04/16073) or imidazolinones (see e. g. US 6,222,100, WO 01/82685, WO 00/26390, WO 97/41218, WO 98/02526, WO 98/02527, WO 04/106529, WO 05/20673, WO 03/14357, WO 03/13225, WO 03/14356, WO 04/16073); enolpyruvylshikimate-3-phosphate synthase (EPSPS) inhibitors, such as glyphosate (see e. g. WO 92/00377); glutamine synthetase (GS) inhibitors, such as glufosinate (see e. g. EP-A-0242236, EP-A-242246) or oxynil herbicides (see e. g. US 5,559,024) as a result of conventional methods of breeding or genetic engineering. Several cultivated plants have been rendered tolerant to herbicides by conventional methods of breeding (mutagenesis), for example Clearfield® summer rape (Canola) being tolerant to imidazolinones, e. g. imazamox. Genetic engineering methods have been used to render cultivated plants, such as soybean, cotton, corn, beets and rape, tolerant to herbicides, such as glyphosate and glufosinate, some of which are commercially available under the trade names RoundupReady® (glyphosate) and LibertyLink® (glufosinate).

The term "cultivated plants" is to be understood also including plants that are by the use of recombinant DNA techniques capable to synthesize one or more insecticidal proteins, especially those known from the bacterial genus Bacillus, particularly from Bacillus thuringiensis, such as ä-endotoxins, e. g. CryIA(b), CryIA(c), CryIF, CryIF(a2), CryIIA(b), CryIIIA, CryIIIB(b1) or Cry9c; vegetative insecticidal proteins (VIP), e. g. VIP1, VIP2, VIP3 or VIP3A; insecticidal proteins of bacteria colonizing nematodes, for example Photorhabdus spp. or Xenorhabdus spp.; toxins produced by animals, such as scorpion toxins, arachnid toxins, wasp toxins, or other insect-specific neurotoxins; toxins produced by fungi, such Streptomycetes toxins, plant lectins, such as pea or barley lectins; agglutinins; proteinase inhibitors, such as trypsin inhibitors, serine protease inhibitors, patatin, cystatin or papain inhibitors; ribosome-inactivating proteins (RIP), such as ricin, maize-RIP, abrin, luffin, saporin or bryodin; steroid metabolism enzymes, such as 3-hydroxysteroid oxidase, ecdysteroid-IDP-glycosyl-transferase, cholesterol oxidases, ecdysone inhibitors or HMG-CoA-reductase; ion channel blockers, such as blockers of sodium or calcium channels; juvenile hormone esterase; diuretic hormone receptors (helicokinin receptors); stilben synthase, bibenzyl synthase, chitinases or glucanases. In the context of the present invention these insecticidal proteins or toxins are to be understood expressly also as pre-toxins, hybrid proteins, truncated or otherwise modified proteins. Hybrid proteins are characterized by a new combination of protein domains, (see, for example WO 02/015701). Further examples of such toxins or genetically-modified plants capable of synthesizing such toxins are dis-closed, for example, in EP-A 374 753, WO 93/007278, WO 95/34656, EP-A 427 529, EP-A 451 878, WO 03/018810 und WO 03/052073. The methods for producing such genetically modified plants are generally known to the person skilled in the art and are described, for example, in the publications mentioned above. These insecticidal proteins contained in the genetically modified plants impart to the plants producing these proteins protection from harmful pests from certain taxonomic groups of arthropods, particularly to beetles (Coleoptera), flies (Diptera), and butterflies and moths (Lepidoptera) and to plant parasitic nematodes (Nematoda).

The term "cultivated plants" is to be understood also including plants that are, e.g. by the use of recombinant DNA techniques, capable of synthesizing one or more proteins in order to increase the resistance or tolerance of those plants to bacterial, viral or fungal pathogens. Examples of such proteins are the so-called "pathogenesis-related proteins", also termed PR proteins - see, for example EP-A 0 392 225 -, or plant disease resistance genes - for example potato cultivars, which express resistance genes acting against Phytophthora infestans derived from the mexican wild potato Solanum bulbocastanum - or T4-lysozym - e. g. potato cultivars capable of synthesizing these proteins with increased resistance against bacteria such as Erwinia amylvora. The methods for producing such genetically modified plants are generally known to the person skilled in the art and are described, for example, in the publications mentioned above.

The term "cultivated plants" is to be understood also including plants that are, e.g. by the use of recombinant DNA techniques, capable of synthesizing one or more proteins to increase the productivity, e. g. bio mass production, grain yield, starch content, oil content or protein content, or to improve tolerance to drought, salinity or other growth-limiting environmental factors or tolerance to pests and fungal, bacterial or viral pathogens of those plants.

The term "cultivated plants" is to be understood also including plants that contain by the use of recombinant DNA techniques a modified amount of substances of content or new substances of content, specifically to improve human or animal nutrition, for ex-ample oil crops that produce health-promoting long-chain omega-3 fatty acids or unsaturated omega-9 fatty acids (e. g. Nexera® rape).

The term "cultivated plants" is to be understood also including plants that contain by the use of recombinant DNA techniques a modified amount of substances of content or new substances of content, specifically to improve raw material production, for example potatoes that produce increased amounts of amylopectin (e. g. Amflora® potato).

In general, "pesticidally effective amount" means the amount of active ingredient needed to achieve an observable effect on growth, including the effects of necrosis, death, retardation, prevention, and removal, destruction, or otherwise diminishing the occurrence and activity of the target organism. The pesticidally effective amount can vary for the various compounds/compositions used in the invention. A pesticidally effective amount of the compositions will also vary according to the prevailing conditions such as desired pesticidal effect and duration, weather, target species, locus, mode of application, and the like.

In the case of soil treatment or of application to the pests dwelling place or nest, the quantity of active ingredient ranges from 0.0001 to 500 g per 100 m², preferably from 0.001 to 20 g per 100 m².

Customary application rates in the protection of materials are, for example, from 0.01 g to 1000 g of active compound per m² treated material, desirably from 0.1 g to 50 g per m².

Insecticidal compositions for use in the impregnation of materials typically contain from 0.001 to 95 weight %, preferably from 0.1 to 45 weight %, and more preferably from 1 to 25 weight % of at least one repellent and/or insecticide.

For use in treating crop plants, the rate of application of the active ingredients of this invention may be in the range of 0.1 g to 4000 g per hectare, desirably from 1 g to 500 g per hectare, more desirably from 10 g to 250 g per hectare.

The compounds of formula (I), including the tautomers and stereoisomers, as well as their salts, are effective through both contact, e.g. via soil, glass, wall, bed net, carpet, plant parts or animal parts, and ingestion, e.g. via ingestion of bait or plant part.

The compounds of the invention may also be applied against non-crop insect pests, such as ants, termites, wasps, flies, mosquitos, crickets, or cockroaches. For use against said non-crop pests, compounds of formula (I), including the tautomers and stereoisomers, as well as their salts, are preferably used in a bait composition.

The bait can be a liquid, a solid or a semisolid preparation (e.g. a gel). Solid baits can be formed into various shapes and forms suitable to the respective application e.g. granules, blocks, sticks, disks. Liquid baits can be filled into various devices to ensure proper application, e.g. open containers, spray devices, droplet sources, or evaporation sources. Gels can be based on aqueous or oily matrices and can be formulated to particular necessities in terms of stickyness, moisture retention or aging characteristics.

The bait employed in the composition is a product, which is sufficiently attractive to incite insects such as ants, termites, wasps, flies, mosquitos, crickets etc. or cockroaches to eat it. The attractiveness can be manipulated by using feeding stimulants or sex pheromones. Food stimulants are chosen, for example, but not exclusively, from animal and/or plant proteins (meat-, fish- or blood meal, insect parts, egg yolk), from fats and oils of animal and/or plant origin, or mono-, oligo- or polyorganosaccharides, especially from sucrose, lactose, fructose, dextrose, glucose, starch, pectin or even molasses or honey. Fresh or decaying parts of fruits, crops, plants, animals, insects or specific parts thereof can also serve as a feeding stimulant. Sex pheromones are known to be more insect specific. Specific pheromones are described in the literature and are known to those skilled in the art.

For use in bait compositions, the typical content of active ingredient is from 0.001 weight % to 15 weight %, desirably from 0.001 weight % to 5% weight % of active compound.

Formulations of compounds of formula (I), including the tautomers and stereoisomers, as well as their salts, as aerosols, e.g in spray cans, oil sprays or pump sprays are highly suitable for the non-professional user for controlling pests such as flies, fleas, ticks, mosquitos or cockroaches. Aerosol recipes are preferably composed of the active compound, solvents such as lower alcohols, e.g. methanol, ethanol, propanol or butanol, ketones, e.g. acetone, methyl ethyl ketone, paraffin hydrocarbons, e.g. kerosenes or mineral oils, having boiling ranges of approximately 50 to 250°C, dimethylformamide, N-methylpyrrolidone, dimethyl sulfoxide, aromatic hydrocarbons such as toluene, xylene, water, furthermore auxiliaries such as emulsifiers such as sorbitol monooleate, oleyl ethoxylate having 3-7 mol of ethylene oxide, fatty alcohol ethoxylate, perfume oils such as ethereal oils, esters of medium fatty acids with lower alcohols, aromatic carbonyl compounds, if appropriate stabilizers such as sodium benzoate, amphoteric surfactants, lower epoxides, triethyl orthoformate and, if required, propellants such as propane, butane, nitrogen, compressed air, dimethyl ether, carbon dioxide, nitrous oxide, or mixtures of these gases.

The oil spray formulations differ from the aerosol recipes in that no propellants are used.

For use in spray compositions, the content of active ingredient is from 0.001 to 80 weights %, preferably from 0.01 to 50 weight % and most preferably from 0.01 to 15 weight %.

The compounds of formula (I), including the tautomers and stereoisomers, as well as their salts, and their respective compositions can also be used in mosquito and fumigating coils, smoke cartridges, vaporizer plates or long-term vaporizers and also in moth papers, moth pads or other heat-independent vaporizer systems.

Methods to control infectious diseases transmitted by insects, such as malaria, dengue and yellow fever, lymphatic filariasis, and leishmaniasis, with compounds of formula (I) or the stereoisomers, tautomers or salts thereof, and with their respective compositions also comprise treating surfaces of huts and houses, air spraying and impregnation of curtains, tents, clothing items, bed nets, tsetse-fly trap or the like. Insecticidal compositions for application to fibers, fabric, knitgoods, nonwovens, netting material or foils and tarpaulins preferably comprise a mixture including the insecticide, optionally a repellent and at least one binder. Suitable repellents for example are N,N-Diethyl-meta-toluamide (DEET), N,N-diethylphenylacetamide (DEPA), 1-(3-cyclohexan-1-yl-carbonyl)-2-methylpiperine, (2-hydroxymethylcyclohexyl) acetic acid lactone, 2-ethyl-1,3-hexandiol, indalone, Methylneodecanamide (MNDA), a pyrethroid not used for insect control such as {(+/-)-3-allyl-2-methyl-4-oxocyclopent-2-(+)-enyl-(+)-trans-chrysantemate (Esbiothrin), a repellent derived from or identical with plant extracts like limonene, eugenol, (+)-Eucamalol (1), (-)-1-epi-eucamalol or crude plant extracts from plants like Eucalyptus maculata, Vitex rotundifolia, Cymbopogan martinii, Cymbopogan citratus (lemon grass), Cymopogan nartdus (citronella). Suitable binders are selected for example from polymers and copolymers of vinyl esters of aliphatic acids (such as such as vinyl acetate and vinyl versatate), acrylic and methacrylic esters of alcohols, such as butyl acrylate, 2-ethylhexylacrylate, and methyl acrylate, mono- and di-ethylenically unsaturated hydrocarbons, such as styrene, and aliphatic diens, such as butadiene.

The impregnation of curtains and bednets is done in general by dipping the textile material into emulsions or dispersions of the insecticide or spraying them onto the nets.

The compounds of formula (I), including the tautomers and stereoisomers, as well as their salts, and their compositions can be used for protecting wooden materials such as trees, board fences, sleepers, etc. and buildings such as houses, outhouses, factories, but also construction materials, furniture, leathers, fibers, vinyl articles, electric wires and cables etc. from ants and/or termites, and for controlling ants and termites from doing harm to crops or human being, e.g. when the pests invade into houses and public facilities. The compounds of formula (I), their stereoisomers, their tautomers or their salts are applied not only to the surrounding soil surface or into the under-floor soil in order to protect wooden materials but it can also be applied to lumbered articles such as surfaces of the under-floor concrete, alcove posts, beams, plywoods, furniture, etc., wooden articles such as particle boards, half boards, etc. and vinyl articles such as coated electric wires, vinyl sheets, heat insulating material such as styrene foams, etc. In case of application against ants doing harm to crops or human beings, the ant controller of the present invention is applied to the crops or the surrounding soil, or is directly applied to the nest of ants or the like.

The compounds of formula (I), including the tautomers and stereoisomers, as well as their salts, are also suitable for the treatment of seeds in order to protect the seed from insect pest, in particular from soil-living insect pests and the resulting plant's roots and shoots against soil pests and foliar insects.

The compounds of formula (I), including the tautomers and stereoisomers, as well as their salts, are particularly useful for the protection of the seed from soil pests and the resulting plant's roots and shoots against soil pests and foliar insects. The protection of the resulting plant's roots and shoots is preferred. More preferred is the protection of resulting plant's shoots from piercing and sucking insects, wherein the protection from aphids is most preferred.

The present invention therefore comprises a method for the protection of seeds from insects, in particular from soil insects and of the seedling's roots and shoots from insects, in particular from soil and foliar insects, said method comprising contacting the seeds before sowing and/or after pregermination with a compound of the general formula (I), a tautomer, a stereosiomer or a salt thereof. Particularly preferred is a method, wherein the plant's roots and shoots are protected, more preferably a method, wherein the plants shoots are protected form piercing and sucking insects, most preferably a method, wherein the plants shoots are protected from aphids.

The term seed includes seeds and plant propagules of all kinds including but not limited to true seeds, seed pieces, suckers, corms, bulbs, fruit, tubers, grains, cuttings, cut shoots and the like and means in a preferred embodiment true seeds.

The term seed treatment includes all suitable seed treatment techniques known in the art, such as seed dressing, seed coating, seed dusting, seed soaking and seed pelleting.

The present disclosure also relates to seeds coated with or containing the active compound of the present invention, i.e. containing a compound of formula (I), a stereoisomer, a tautomer or a salt thereof.

The term "coated with and/or containing" generally signifies that the active ingredient is for the most part on the surface of the propagation product at the time of application, although a greater or lesser part of the ingredient may penetrate into the propagation product, depending on the method of application. When the said propagation product is (re)planted, it may absorb the active ingredient.

Suitable seed is seed of cereals, root crops, oil crops, vegetables, spices, ornamentals, for example seed of durum and other wheat, barley, oats, rye, maize (fodder maize and sugar maize / sweet and field corn), soybeans, oil crops, crucifers, cotton, sunflowers, bananas, rice, oilseed rape, turnip rape, sugarbeet, fodder beet, eggplants, potatoes, grass, lawn, turf, fodder grass, tomatoes, leeks, pumpkin/squash, cabbage, iceberg lettuce, pepper, cucumbers, melons, Brassica species, melons, beans, peas, garlic, onions, carrots, tuberous plants such as potatoes, sugar cane, tobacco, grapes, petunias, geranium/pelargoniums, pansies and impatiens.

In addition, the compounds of formula (I), including the tautomers and stereoisomers, as well as their salts, may also be used for the treatment seeds from plants, which tolerate the action of herbicides or fungicides or insecticides owing to breeding, including genetic engineering methods.

For example, the compounds of formula (I), including the tautomers and stereoisomers, as well as their salts, can be employed in treatment of seeds from plants, which are resistant to herbicides from the group consisting of the sulfonylureas, imidazolinones, glufosinate-ammonium or glyphosate-isopropylammonium and analogous active substances (see for example, EP-A-0242236, EP-A-242246) (WO 92/00377) (EP-A-0257993, U.S. Pat. No. 5,013,659) or in transgenic crop plants, for example cotton, with the capability of producing Bacillus thuringiensis toxins (Bt toxins) which make the plants resistant to certain pests (EP-A-0142924, EP-A-0193259),

Furthermore, the compounds of formula (I), including the tautomers and stereoisomers, as well as their salts, can be used also for the treatment of seeds from plants, which have modified characteristics in comparison with existing plants consist, which can be generated for example by traditional breeding methods and/or the generation of mutants, or by recombinant procedures). For example, a number of cases have been described of recombinant modifications of crop plants for the purpose of modifying the starch synthesized in the plants (e.g. WO 92/11376, WO 92/14827, WO 91/19806) or of transgenic crop plants having a modified fatty acid composition (WO 91/13972).

The seed treatment application of the active compound is carried out by spraying or by dusting the seeds before sowing of the plants and before emergence of the plants.

Compositions which are especially useful for seed treatment are e.g.:
- A: Soluble concentrates (SL, LS)
- D: Emulsions (EW, EO, ES)
- E: Suspensions (SC, OD, FS)
- F: Water-dispersible granules and water-soluble granules (WG, SG)
- G: Water-dispersible powders and water-soluble powders (WP, SP, WS)
- H: Gel-Formulations (GF)
- I: Dustable powders (DP, DS)

Conventional seed treatment formulations include for example flowable concentrates FS, solutions LS, powders for dry treatment DS, water dispersible powders for slurry treatment WS, water-soluble powders SS and emulsion ES and EC and gel formulation GF. These formulations can be applied to the seed diluted or undiluted. Application to the seeds is carried out before sowing, either directly on the seeds or after having pregerminated the latter

In a preferred embodiment a FS formulation is used for seed treatment. Typcially, a FS formulation may comprise 1-800 g/l of active ingredient, 1-200 g/l Surfactant, 0 to 200 g/l antifreezing agent, 0 to 400 g/l of binder, 0 to 200 g/l of a pigment and up to 1 liter of a solvent, preferably water.

Especially preferred FS formulations of a compound of formula (I), a stereoisomer, a tautomer or a salt, for seed treatment usually comprise from 0.1 to 80% by weight (1 to 800 g/l) of the the compound of formula (I), including its tautomers and stereoisomers, or a salt thereof, from 0.1 to 20 % by weight (1 to 200 g/l) of at least one surfactant, e.g. 0.05 to 5 % by weight of a wetter and from 0.5 to 15 % by weight of a dispersing agent, up to 20 % by weight, e.g. from 5 to 20 % of an anti-freeze agent, from 0 to 15 % by weight, e.g. 1 to 15 % by weight of a pigment and/or a dye, from 0 to 40 % by weight, e.g. 1 to 40 % by weight of a binder (sticker /adhesion agent), optionally up to 5 % by weight, e.g. from 0.1 to 5 % by weight of a thickener, optionally from 0.1 to 2 % of an anti-foam agent, and optionally a preservative such as a biocide, antioxidant or the like, e.g. in an amount from 0.01 to 1 % by weight and a filler/vehicle up to 100 % by weight.

Seed Treatment formulations may additionally also comprise binders and optionally colorants.

Binders can be added to improve the adhesion of the active materials on the seeds after treatment. Suitable binders are homo- and copolymers from alkylene oxides like ethylene oxide or propylene oxide, polyvinylacetate, polyvinylalcohols, polyvinylpyrrolidones, and copolymers thereof, ethylene-vinyl acetate copolymers, acrylic homo- and copolymers, polyethyleneamines, polyethyleneamides and polyethyleneimines, polysaccharides like celluloses, tylose and starch, polyolefin homo- and copolymers like olefin/maleic anhydride copolymers, polyurethanes, polyesters, polystyrene homo and copolymers

Optionally, also colorants can be included in the formulation. Suitable colorants or dyes for seed treatment formulations are Rhodamin B, C.I. Pigment Red 112, C.I. Solvent Red 1, pigment blue 15:4, pigment blue 15:3, pigment blue 15:2, pigment blue 15:1, pigment blue 80, pigment yellow 1, pigment yellow 13, pigment red 112, pigment red 48:2, pigment red 48:1, pigment red 57:1, pigment red 53:1, pigment orange 43, pigment orange 34, pigment orange 5, pigment green 36, pigment green 7, pigment white 6, pigment brown 25, basic violet 10, basic violet 49, acid red 51, acid red 52, acid red 14, acid blue 9, acid yellow 23, basic red 10, basic red 108.

### Examples of a gelling agent is carrageen (Satiagel®)

In the treatment of seed, the application rates of the compounds of formula (I) are generally from 0.1 g to 10 kg per 100 kg of seed, preferably from 1 g to 5 kg per 100 kg of seed, more preferably from 1 g to 1000 g per 100 kg of seed and in particular from 1 g to 200 g per 100 kg of seed.

The disclosure therefore also relates to seed comprising a compound of the formula (I), a tautomer, a stereoisomer or an agriculturally useful salt thereof, as defined herein. The amount of the compound of the formula (I) or the agriculturally useful salt thereof will in general vary from 0.1 g to 10 kg per 100 kg of seed, preferably from 1 g to 5 kg per 100 kg of seed, in particular from 1 g to 1000 g per 100 kg of seed. For specific crops such as lettuce the rate can be higher.

The compounds of formula (I), including their stereoisomers and their tautomers, and the veterinarily acceptable salts thereof are in particular also suitable for being used for combating parasites in and on animals.

An object of the present invention is therefore also to provide compounds of formula (I) for use in methods to control parasites in and on animals. Another object of the invention is to provide safer pesticides for animals. Another object of the invention is further to provide pesticides for animals that may be used in lower doses than existing pesticides. And another object of the invention is to provide pesticides for animals, which provide a long residual control of the parasites.

The invention also relates to compositions containing a parasiticidally effective amount of a compound of formula (I) or a stereoisomer or a tautomer or a veterinarily acceptable salt thereof and an acceptable carrier, for combating parasites in and on animals.

The present invention also provides compounds of formula (I) for use in a method for treating, controlling, preventing and protecting animals against infestation and infection by parasites, which comprises orally, topically or parenterally administering or applying to the animals a parasiticidally effective amount of a compound of formula (I) or a stereoisomer or a tautomer or a veterinarily acceptable salt thereof or a composition comprising it.

The invention also provides a process for the preparation of a composition for treating, controlling, preventing or protecting animals against infestation or infection by parasites which comprises a parasiticidally effective amount of a compound of formula (I) or a stereoisomer or a tautomer or a veterinarily acceptable salt thereof or a composition comprising it.

Activity of compounds against agricultural pests does not suggest their suitability for control of endo- and ectoparasites in and on animals which requires, for example, low, non-emetic dosages in the case of oral application, metabolic compatibility with the animal, low toxicity, and a safe handling.

Surprisingly it has now been found that compounds of formula (I), including their stereoisomers and tautomers, and the salts thereof, are suitable for combating endo- and ectoparasites in and on animals.

Compounds of formula (I), including their stereoisomers and their tautomers, and the veterinarily acceptable salts thereof, and compositions comprising them are preferably used for controlling and preventing infestations and infections animals including warm-blooded animals, including humans, and fish. They are for example suitable for controlling and preventing infestations and infections in mammals such as cattle, sheep, swine, camels, deer, horses, pigs, poultry, rabbits, goats, dogs and cats, water buffalo, donkeys, fallow deer and reindeer, and also in fur-bearing animals such as mink, chinchilla and raccoon, birds such as hens, geese, turkeys and ducks and fish such as fresh- and salt-water fish such as trout, carp and eels.

Compounds of formula (I), including their stereoisomers and their tautomers, and the veterinarily acceptable salts thereof and compositions comprising them are preferably used for controlling and preventing infestations and infections in domestic animals, such as dogs or cats.

Infestations in warm-blooded animals and fish include, but are not limited to, lice, biting lice, ticks, nasal bots, keds, biting flies, muscoid flies, flies, myiasitic fly larvae, chiggers, gnats, mosquitoes and fleas.

The compounds of formula (I), including their stereoisomers and their tautomers, and the veterinarily acceptable salts thereof and compositions comprising them are suitable for systemic and/or non-systemic control of ecto- and/or endoparasites. They are active against all or some stages of development.

The compounds of formula (I) including their stereoisomers and their tautomers, and the veterinarily acceptable salts thereof are especially useful for combating ectoparasites.

The compounds of formula (I), including their stereoisomers and their tautomers, and the veterinarily acceptable salts thereof are especially useful for combating parasites of the following orders and species, respectively:
fleas (Siphonaptera), e.g. *Ctenocephalides felis, Ctenocephalides canis, Xenopsylla cheopis, Pulex irritans*, *Tunga penetrans,* and *Nosopsyllus fasciatus;*
cockroaches (Blattaria - Blattodea), e.g. *Blattella germanica, Blattella asahinae, Periplaneta americana, Periplaneta japonica, Periplaneta brunnea, Periplaneta fuligginosa, Periplaneta australasiae,* and *Blatta orientalis;*
flies, mosquitoes (Diptera), e.g. *Aedes aegypti, Aedes albopictus, Aedes vexans, Anastrepha ludens, Anopheles maculipennis, Anopheles crucians, Anopheles albimanus, Anopheles gambiae, Anopheles freeborni, Anopheles leucosphyrus, Anopheles minimus, Anopheles quadrimaculatus, Calliphora vicina, Chrysomya bezziana, Chrysomya hominivorax, Chrysomya macellaria, Chrysops discalis, Chrysops silacea, Chrysops atlanticus, Cochliomyia hominivorax, Cordylobia anthropophaga, Culicoides furens, Culex pipiens, Culex nigripalpus, Culex quinquefasciatus, Culex tarsalis, Culiseta inornata, Culiseta melanura, Dermatobia hominis, Fannia canicularis, Gasterophilus intestinalis, Glossina morsitans, Glossina palpalis, Glossina fuscipes, Glossina tachinoides, Haematobia irritans, Haplodiplosis equestris, Hippelates spp., Hypoderma lineata, Leptoconops torrens, Lucilia caprina, Lucilia cuprina, Lucilia sericata, Lycoria pectoralis, Mansonia spp., Musca domestica, Muscina stabulans, Oestrus ovis, Phlebotomus argentipes, Psorophora columbiae, Psorophora discolor, Prosimulium mixtum, Sarcophaga haemorrhoidalis, Sarcophaga sp., Simulium vittatum, Stomoxys calcitrans, Tabanus bovinus, Tabanus atratus, Tabanus lineola,* and *Tabanus similis;*
lice (Phthiraptera), e.g. *Pediculus humanus capitis, Pediculus humanus corporis, Pthirus pubis, Haematopinus eurysternus, Haematopinus suis, Linognathus vituli, Bovicola bovis, Menopon gallinae, Menacanthus stramineus* and *Solenopotes capillatus;*
ticks and parasitic mites (Parasitiformes): ticks (Ixodida), e.g. *Ixodes scapularis, Ixodes holocyclus, Ixodes pacificus, Rhiphicephalus sanguineus, Dermacentor andersoni, Dermacentor variabilis, Amblyomma americanum, Ambryomma maculatum, Ornithodorus hermsi, Ornithodorus turicata* and parasitic mites (Mesostigmata), e.g. *Ornithonyssus bacoti* and *Dermanyssus gallinae;*
Actinedida (Prostigmata) und Acaridida (Astigmata) e.g. *Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp.,Knemidocoptes spp., Cytodites spp.,* and *Laminosioptes spp;*
Bugs (Heteropterida): *Cimex lectularius, Cimex hemipterus, Reduvius senilis, Triatoma spp., Rhodnius ssp., Panstrongylus ssp.* and *Arilus critatus;*
Anoplurida, e.g. *Haematopinus spp., Linognathus spp., Pediculus spp., Phtirus spp.,* and *Solenopotes spp.;*
Mallophagida (suborders Arnblycerina and Ischnocerina), e.g. *Trimenopon spp., Menopon spp., Trinoton spp., Bovicola spp., Werneckiella spp., Lepikentron spp., Trichodectes spp.,* and *Felicola spp;*
Roundworms Nematoda:
   Wipeworms and Trichinosis (Trichosyringida), e.g. Trichinellidae *(Trichinella spp.), (*Trichuridae*) Trichuris spp., Capillaria spp.;*
   Rhabditida, e.g. *Rhabditis spp, Strongyloides spp., Helicephalobus spp;*
   Strongylida, e.g. *Strongylus spp., Ancylostoma spp., Necator americanus, Bunostomum spp. (Hookworm), Trichostrongylus spp., Haemonchus contortus., Ostertagia spp., Cooperia spp., Nematodirus spp., Dictyocaulus spp., Cyathostoma spp., Oesophagostomum spp., Stephanurus dentatus, Ollulanus spp., Chabertia spp., Stephanurus dentatus, Syngamus trachea, Ancylostoma spp., Uncinaria spp., Globocephalus spp., Necator spp., Metastrongylus spp., Muellerius capillaris, Protostrongylus spp., Angiostrongylus spp., Parelaphostrongylus spp. Aleurostrongylus abstrusus,* and *Dioctophyma renale;*
   Intestinal roundworms (Ascaridida), e.g. *Ascaris lumbricoides, Ascaris suum, Ascaridia galli, Parascaris equorum, Enterobius vermicularis (Threadworm), Toxocara canis, Toxascaris leonine, Skrjabinema spp.,* and *Oxyuris equi;*
   Camallanida, e.g. *Dracunculus medinensis* (guinea worm);
   Spirurida, e.g. *Thelazia spp. Wuchereria spp., Brugia spp., Onchocerca spp., Dirofilari spp.a, Dipetalonema spp., Setaria spp., Elaeophora spp., Spirocerca lupi,* and *Habronema spp.;*
   Thorny headed worms (Acanthocephala), e.g. *Acanthocephalus spp., Macracanthorhynchus hirudinaceus* and *Oncicola spp;*
Planarians (Plathelminthes):
   Flukes (Trematoda), e.g. *Faciola spp., Fascioloides magna, Paragonimus spp., Dicrocoelium spp., Fasciolopsis buski, Clonorchis sinensis, Schistosoma spp., Trichobilharzia spp., Alaria alata, Paragonimus spp.,* and *Nanocyetes spp;*
   Cercomeromorpha, in particular Cestoda (Tapeworms), e.g. *Diphyllobothrium spp., Tenia spp., Echinococcus spp., Dipylidium caninum, Multiceps spp., Hymenolepis spp., Mesocestoides spp., Vampirolepis spp., Moniezia spp., Anoplocephala spp., Sirometra spp., Anoplocephala spp.,* and *Hymenolepis spp.*

The compounds of formula (I), including their stereoisomers and their tautomers, and the salts thereof and compositions containing them are particularly useful for the control of pests from the orders Diptera, Siphonaptera and Ixodida.

Moreover, the compounds of formula (I), including their stereoisomers and their tautomers, and the salts thereof and compositions containing them for combating mosquitoes is especially preferred.

The compounds of formula (I), including their stereoisomers and their tautomers, and the salts thereof and compositions containing them for combating flies is a further preferred embodiment of the present invention.

Furthermore, the compounds of formula (I), including their stereoisomers and their tautomers, and the salts thereof and compositions containing them for combating fleas is especially preferred.

The compounds of formula (I), including their stereoisomers and their tautomers, and the salts thereof and compositions containing them for combating ticks is a further preferred embodiment of the present invention.

The compounds of formula (I), including their stereoisomers and their tautomers, and the salts thereof also are especially useful for combating endoparasites (roundworms nematoda, thorny headed worms and planarians).

Administration can be carried out both prophylactically and therapeutically.

Administration of the active compounds is carried out directly or in the form of suitable preparations, orally, topically/dermally or parenterally.

For oral administration to warm-blooded animals, the compounds of the present invention may be formulated as animal feeds, animal feed premixes, animal feed concentrates, pills, solutions, pastes, suspensions, drenches, gels, tablets, boluses and capsules. In addition, the compounds of the present invention may be administered to the animals in their drinking water. For oral administration, the dosage form chosen should provide the animal with 0.01 mg/kg to 100 mg/kg of animal body weight per day of the formula (I) compound, preferably with 0.5 mg/kg to 100 mg/kg of animal body weight per day.

Alternatively, the compounds of the present invention may be administered to animals parenterally, for example, by intraruminal, intramuscular, intravenous or subcutaneous injection. The compounds of the present invention may be dispersed or dissolved in a physiologically acceptable carrier for subcutaneous injection. Alternatively, the compounds of the present invention may be formulated into an implant for subcutaneous administration. In addition the compounds of the present invention may be transdermally administered to animals. For parenteral administration, the dosage form chosen should provide the animal with 0.01 mg/kg to 100 mg/kg of animal body weight per day of a compound of the present invention.

The compounds of the present invention may also be applied topically to the animals in the form of dips, dusts, powders, collars, medallions, sprays, shampoos, spot-on and pour-on formulations and in ointments or oil-in-water or water-in-oil emulsions. For topical application, dips and sprays usually contain 0.5 ppm to 5,000 ppm and preferably 1 ppm to 3,000 ppm of the compounds of the present invention. In addition, the compounds of the present invention may be formulated as ear tags for animals, particularly quadrupeds such as cattle and sheep.

Suitable preparations are:
- Solutions such as oral solutions, concentrates for oral administration after dilution, solutions for use on the skin or in body cavities, pouring-on formulations, gels;
- Emulsions and suspensions for oral or dermal administration; semi-solid preparations;
- Formulations in which the active compound is processed in an ointment base or in an oil-in-water or water-in-oil emulsion base;
- Solid preparations such as powders, premixes or concentrates, granules, pellets, tablets, boluses, capsules; aerosols and inhalants, and active compound-containing shaped articles.

Compositions suitable for injection are prepared by dissolving the active ingredient in a suitable solvent and optionally adding further ingredients such as acids, bases, buffer salts, preservatives, and solubilizers. The solutions are filtered and filled sterile.

Suitable solvents are physiologically tolerable solvents such as water, alkanols such as ethanol, butanol, benzyl alcohol, glycerol, propylene glycol, polyethylene glycols, N-methylpyrrolidone, 2-pyrrolidone, and mixtures thereof.

The compounds of the present invention can optionally be dissolved in physiologically tolerable vegetable or synthetic oils which are suitable for injection.

Suitable solubilizers are solvents which promote the dissolution of the active compound in the main solvent or prevent its precipitation. Examples are polyvinylpyrrolidone, polyvinyl alcohol, polyoxyethylated castor oil, and polyoxyethylated sorbitan ester.

Suitable preservatives are benzyl alcohol, trichlorobutanol, p-hydroxybenzoic acid esters, and n-butanol.

Oral solutions are administered directly. Concentrates are administered orally after prior dilution to the use concentration. Oral solutions and concentrates are prepared according to the state of the art and as described above for injection solutions, sterile procedures not being necessary.

Solutions for use on the skin are trickled on, spread on, rubbed in, sprinkled on or sprayed on.

Solutions for use on the skin are prepared according to the state of the art and according to what is described above for injection solutions, sterile procedures not being necessary.

Further suitable solvents are polypropylene glycol, phenyl ethanol, phenoxy ethanol, ester such as ethyl or butyl acetate, benzyl benzoate, ethers such as alkyleneglycol alkylether, e.g. dipropylenglycol monomethylether, ketons such as acetone, methylethylketone, aromatic hydrocarbons, vegetable and synthetic oils, dimethylformamide, dimethylacetamide, transcutol, solketal, propylencarbonate, and mixtures thereof.

It may be advantageous to add thickeners during preparation. Suitable thickeners are inorganic thickeners such as bentonites, colloidal silicic acid, aluminium monostearate, organic thickeners such as cellulose derivatives, polyvinyl alcohols and their copolymers, acrylates and methacrylates.

Gels are applied to or spread on the skin or introduced into body cavities. Gels are prepared by treating solutions which have been prepared as described in the case of the injection solutions with sufficient thickener that a clear material having an ointment-like consistency results. The thickeners employed are the thickeners given above.

Pour-on formulations are poured or sprayed onto limited areas of the skin, the active compound penetrating the skin and acting systemically.

Pour-on formulations are prepared by dissolving, suspending or emulsifying the active compound in suitable skin-compatible solvents or solvent mixtures. If appropriate, other auxiliaries such as colorants, bioabsorption-promoting substances, antioxidants, light stabilizers, adhesives are added.

Suitable solvents are, for example, water, alkanols, glycols, polyethylene glycols, polypropylene glycols, glycerol, aromatic alcohols such as benzyl alcohol, phenylethanol, phenoxyethanol, esters such as ethyl acetate, butyl acetate, benzyl benzoate, ethers such as alkylene glycol alkyl ethers such as dipropylene glycol monomethyl ether, diethylene glycol mono-butyl ether, ketones such as acetone, methyl ethyl ketone, cyclic carbonates such as propylene carbonate, ethylene carbonate, aromatic and/or aliphatic hydrocarbons, vegetable or synthetic oils, DMF, dimethylacetamide, n-alkylpyrrolidones such as methylpyrrolidone, n-butylpyrrolidone or n-octylpyrrolidone, N-methylpyrrolidone, 2-pyrrolidone, 2,2-dimethyl-4-oxymethylene-1,3-diox- olane and glycerol formal.

Suitable colorants are all colorants permitted for use on animals and which can be dissolved or suspended.

Suitable absorption-promoting substances are, for example, DMSO, spreading oils such as isopropyl myristate, dipropylene glycol pelargonate, silicone oils and copolymers thereof with polyethers, fatty acid esters, triglycerides, fatty alcohols.

Suitable antioxidants are, for example, sulfites or metabisulfites such as potassium metabisulfite, ascorbic acid, butylhydroxytoluene, butylhydroxyanisole, tocopherol.

Suitable light stabilizers are, for example, novantisolic acid.

Suitable adhesives are, for example, cellulose derivatives, starch derivatives, polyacrylates, natural polymers such as alginates, gelatin.

Emulsions can be administered orally, dermally or as injections.

Emulsions are either of the water-in-oil type or of the oil-in-water type.

They are prepared by dissolving the active compound either in the hydrophobic or in the hydrophilic phase and homogenizing this with the solvent of the other phase with the aid of suitable emulsifiers and, if appropriate, other auxiliaries such as colorants, absorption-promoting substances, preservatives, antioxidants, light stabilizers, viscosity-enhancing substances.

Suitable hydrophobic phases (oils) are, for example: liquid paraffins, silicone oils, natural vegetable oils such as sesame oil, almond oil, castor oil, synthetic triglycerides such as caprylic/capric biglyceride, triglyceride mixture with vegetable fatty acids of the chain length C₈-C₁₂ or other specially selected natural fatty acids, partial glyceride mixtures of saturated or unsaturated fatty acids possibly also containing hydroxyl groups, mono- and diglycerides of the C₈-C₁₀ fatty acids, fatty acid esters such as ethyl stearate, di-n-butyryl adipate, hexyl laurate, dipropylene glycol perlargonate, esters of a branched fatty acid of medium chain length with saturated fatty alcohols of chain length C₁₆-C₁₈, isopropyl myristate, isopropyl palmitate, caprylic/capric acid esters of saturated fatty alcohols of chain length C₁₂-C₁₈, isopropyl stearate, oleyl oleate, decyl oleate, ethyl oleate, ethyl lactate, waxy fatty acid esters such as synthetic duck coccygeal gland fat, dibutyl phthalate, diisopropyl adipate, and ester mixtures related to the latter, fatty alcohols such as isotridecyl alcohol, 2-octyldodecanol, cetylstearyl alcohol, oleyl alcohol, and fatty acids such as oleic acid and mixtures thereof.

Suitable hydrophilic phases are, for example, water, alcohols such as propylene glycol, glycerol, sorbitol and mixtures thereof.

Suitable emulsifiers are, for example,
- non-ionic surfactants, e.g. polyethoxylated castor oil, polyethoxylated sorbitan monooleate, sorbitan monostearate, glycerol monostearate, polyoxyethyl stearate, alkylphenol polyglycol ether;
- ampholytic surfactants such as di-sodium N-lauryl-p-iminodipropionate or lecithin;
- anionic surfactants, such as sodium lauryl sulfate, fatty alcohol ether sulfates, mono/dialkyl polyglycol ether orthophosphoric acid ester monoethanolamine salt;
- cation-active surfactants, such as cetyltrimethylammonium chloride.

Suitable further auxiliaries are substances which enhance the viscosity and stabilize the emulsion, such as carboxymethylcellulose, methylcellulose and other cellulose and starch derivatives, polyacrylates, alginates, gelatin, gum arabic, polyvinylpyrrolidone, polyvinyl alcohol, copolymers of methyl vinyl ether and maleic anhydride, polyethylene glycols, waxes, colloidal silicic acid or mixtures of the substances mentioned.

Suspensions can be administered orally or topically/dermally. They are prepared by suspending the active compound in a suspending agent, if appropriate with addition of other auxiliaries such as wetting agents, colorants, bioabsorption-promoting substances, preservatives, antioxidants, light stabilizers.

Liquid suspending agents are all homogeneous solvents and solvent mixtures.

Suitable wetting agents (dispersants) are the emulsifiers given above.

Other auxiliaries, which may be mentioned, are those given above.

Semi-solid preparations can be administered orally or topically/dermally. They differ from the suspensions and emulsions described above only by their higher viscosity.

For the production of solid preparations, the active compound is mixed with suitable excipients, if appropriate with addition of auxiliaries, and brought into the desired form.

Suitable excipients are all physiologically tolerable solid inert substances. Those used are inorganic and organic substances. Inorganic substances are, for example, sodium chloride, carbonates such as calcium carbonate, hydrogencarbonates, aluminium oxides, titanium oxide, silicic acids, argillaceous earths, precipitated or colloidal silica, or phosphates. Organic substances are, for example, sugar, cellulose, foodstuffs and feeds such as milk powder, animal meal, grain meals and shreds, starches.

Suitable auxiliaries are preservatives, antioxidants, and/or colorants which have been mentioned above.

Other suitable auxiliaries are lubricants and glidants such as magnesium stearate, stearic acid, talc, bentonites, disintegration-promoting substances such as starch or crosslinked polyvinylpyrrolidone, binders such as starch, gelatin or linear polyvinylpyrrolidone, and dry binders such as microcrystalline cellulose.

In general, "parasiticidally effective amount" means the amount of active ingredient needed to achieve an observable effect on growth, including the effects of necrosis, death, retardation, prevention, and removal, destruction, or otherwise diminishing the occurrence and activity of the target organism. The parasiticidally effective amount can vary for the various compounds/compositions used in the invention. A parasiticidally effective amount of the compositions will also vary according to the prevailing conditions such as desired parasiticidal effect and duration, target species, mode of application, and the like.

The compositions which can be used in the invention can comprise generally from about 0.001 to 95% of a compound of formula (I), a stereoisomer, a tautomer or a salt thereof.

Generally it is favorable to apply the compounds of the present invention in total amounts of 0.5 mg/kg to 100 mg/kg per day, preferably 1 mg/kg to 50 mg/kg per day.

Ready-to-use preparations contain the compounds acting against parasites, preferably ectoparasites, in concentrations of 10 ppm to 80 per cent by weight, preferably from 0.1 to 65 per cent by weight, more preferably from 1 to 50 per cent by weight, most preferably from 5 to 40 per cent by weight.

Preparations which are diluted before use contain the compounds acting against ectoparasites in concentrations of 0.5 to 90 per cent by weight, preferably of 1 to 50 per cent by weight.

Furthermore, the preparations for controlling endoparasites comprise a compound of the present invention usually in concentrations of 10 ppm to 2 per cent by weight, preferably of 0.05 to 0.9 per cent by weight, very particularly preferably of 0.005 to 0.25 per cent by weight.

In a preferred embodiment of the present invention, the compositions comprising the a compound of the present invention are applied dermally / topically.

In a further preferred embodiment, the topical application is conducted in the form of compound-containing shaped articles such as collars, medallions, ear tags, bands for fixing at body parts, and adhesive strips and foils.

Generally it is favorable to apply solid formulations which release compounds of the present invention in total amounts of 10 mg/kg to 300 mg/kg, preferably 20 mg/kg to 200 mg/kg, most preferably 25 mg/kg to 160 mg/kg body weight of the treated animal in the course of three weeks.

For the preparation of the shaped articles, thermoplastic and flexible plastics as well as elastomers and thermoplastic elastomers are used. Suitable plastics and elastomers are polyvinyl resins, polyurethane, polyacrylate, epoxy resins, cellulose, cellulose derivatives, polyamides and polyester which are sufficiently compatible with the compounds of the present invention. A detailed list of plastics and elastomers as well as preparation procedures for the shaped articles is given e.g. in WO 03/086075.

The present invention is now illustrated in further details by the following examples, without imposing any limitation thereto.

The following abbreviations are used:
TFA: trifluoroacetic acid
EtOAc: ethyl acetate
HPLC: High Performance Liquid Chromatography
MS: Mass spectrometry
MeOH: Methanol
THF: Tetrahydrofuran
t_{R}: retention time

The Compound examples were characterized by coupled High Performance Liquid Chromatography with mass spectrometry (HPLC/MS) or by their melting point.

Analytical UPLC column: Phenomenex Kinetex 1,7 µm XB-C18 100A; 50 x 2.1 mm; mobile phase: A: water; B: acetonitrile; gradient: 5-100% B in 1.50 minutes; 100% B 0.20 min; flow: 0,8-1,0mL/min in 1,50 minutes at 60°C.
MS-method: ESI positive.

### A. Preparation examples

### Example 1: (Compound example E1.4; compound of formula I, wherein Het is 6-chloropyridin-3-yl, -W¹-W²-W³-W⁴- represent -CH=CH-CH=CH-, X is O, R³ is H, R⁵ is H and m is 0)

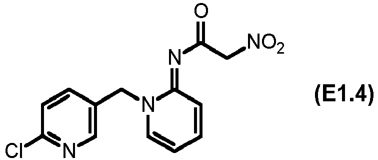

### 1.1 Synthesis of amine salt E1.1

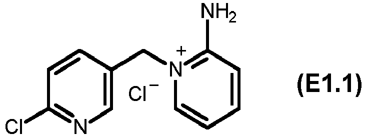

A solution of 2-chloro-5-chloromethylpyridine (16.20 g, 100 mmol) and 2-amino-pyridine (9.60 g, 102 mmol) in ethanol (100 mL) was refluxed for 24 hours. The reaction was then cooled to room temperature, and concentrated in vacuo. Then 100 mL of toluene were added to the residue, and the mixture was concentrated in vacuo. Then, 75 mL of CH₂Cl₂ were added to the residue and the mixture was rapidly stirred for 15 minutes, during which time a precipitate forms. The precipitate was then filtered, and washed with CH₂Cl₂ (50 mL), diethyl ether (50 mL), and dried under vacuum to afford the title product as a pale yellow solid (14.0 g, 55% yield).
LC-MS: mass calculated for C₁₁H₁₁ClN₃ [M]⁺ 220.1, found 220.1; t_{R}= 0.529 min (t_{R}: retention time)

### 1.2 Synthesis of compound E1.2

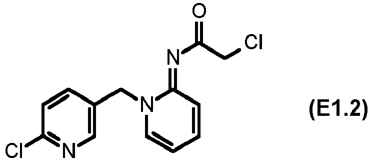

To a suspension of amine salt **E1.1** (20 g, 78.1 mmol), triethylamine (23.7 g, 234 mmol), and DMAP (1.91 g, 0.2 mmol) in dichloromethane (300 mL) at 5°C was added 2-chloroacetyl chloride (13.2 g, 117 mmol). The reaction was then stirred at room temperature for 14 h. The reaction was diluted with dichloromethane (200 mL), washed with saturated aqueous NaHCO₃ (75 mL) and water (2 x 30mL). The organic layer was dried over Na₂SO₄ and then concentrated in vacuo to afford a residue, which was purified using column chromatography over silica gel (20→45% cyclohexane/acetone) to afford the title product as a beige solid (8.5 g, 37% yield). LC-MS: mass calculated for C₁₃H₁₁N₃OCl₂ [M+H]⁺ 296.0, found 295.7; t_{R}= 0.643 min (t_{R}: retention time).

### 1.3 Synthesis of compound E1.3

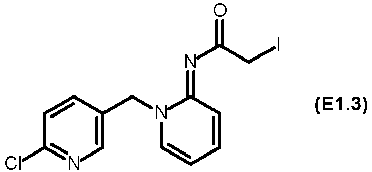

To a solution **of E1.2** (4 g, 13.5 mmol) in acetone (80 mL) was added sodium iodide (10.1 g, 67.5 mmol). The reaction was then stirred at room temperature for 3 d, followed by concentration in vacuo to afford a residue. The residue was diluted with dichloromethane (200 mL), washed with water (3 x 30 mL) and the aqueous layer was extracted with dichloromethane (100 mL). The combined organic layers were dried over Na₂SO₄ and then concentrated in vacuo to afford a beige solid (5.1 g, 98%), which was used without further purification in the next step.
LC-MS: mass calculated for C₁₃H₁₁N₃OICl [M+H]⁺ 388.0, found 387.7; t_{R}= 0.679 min.

### 1.4 Synthesis of compound E1.4

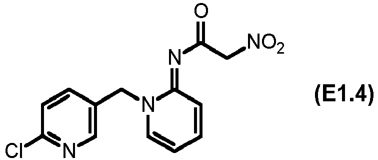

To a solution of **E1.3** (2.65 g, 6.84 mmol) in THF (160 mL) and diethylether (40 mL) was added silver nitrite (1.58 g, 10.3 mmol). The reaction was then stirred at room temperature for 4 h, filtered through celite (eluent ethylacetate/acetone, 8:2, 100 mL) and then concentrated in vacuo to afford a residue, which was purified using column chromatography over silica gel (20→40% cyclohexane/acetone) to afford the title product as a yellow solid (1.3 g, 62% yield). LC-MS: mass calculated for C₁₃H₁₁N₄O₃Cl [M+H]⁺ 307.1, found 307.3; t_{R}= 0.735 min.

### Example 2: (Compound example E2.3; compound of formula I, wherein Het is 6-chloropyridin-3-yl, -W¹-W²-W³-W⁴- represent -CH=CH-CH=CH-, X is O, m = 0, R³ and R⁵ form together and with the carbon they are bound to 2-phenyl-cyclopropyl)

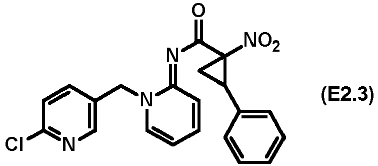

### 2.1 Synthesis of E2.1

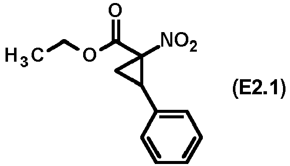

To a solution of ethyl 2-nitroacetate (1.0 g, 7.51 mmol) in styrene (3.91 g, 37.6 mmol) was added rhodium(II) acetate dimer (33 mg, 0.08 mmol), followed by the addition of (diacetoxyiodo)benzene (2.66 g, 8.26 mmol). After stirring the reaction mixture for 24 h at room temperature, the mixture was concentrated in vacuo to afford a residue, which was purified using column chromatography over silica gel (0→5% cyclohexane/ethyl acetate) to afford the title product as colourless liquid (1.0 g, 57% yield).
LC-MS: mass calculated for C₁₂H₁₄NO₄ [M+H]⁺ 236.2, found 236.4; t_{R}= 1.185 min

### 2.2 Synthesis of compound E2.2

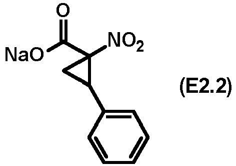

To a solution of **E2.1** (0.75 g, 3.19 mmol) in ethanol (30 mL) was added sodium hydroxide (6.4 mL of a 2 N aqueous solution). After stirring the reaction mixture for 16 h at room temperature, the mixture was concentrated in vacuo to afford a residue, which was used without further purification in the next step.

### 2.4 Synthesis of compound E2.3

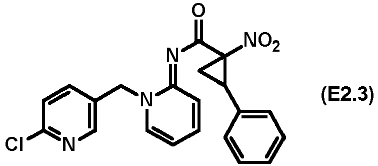

To a solution of **E2.2** in dichloromethane (15 mL) was added bromotripyrrolidinophosphonium hexafluorophosphate (1.97 g, 4.23 mmol), followed by **E1.1** (902 mg, 3.52 mmol) and *N*,*N*-diisopropylethylamine (1.82 g, 14.1 mmol). After stirring the reaction mixture for 4 h at room temperature, the reaction mixture was washed with saturated aqueous Na₂CO₃ (20 mL). The organic layer was dried over Na₂SO₄ and then concentrated in vacuo to afford a residue, which was purified using column chromatography over silica gel (20→40% cyclohexane/acetone) to afford the title product as a white solid (1.0 g, 77% yield).
LC-MS: mass calculated for C₂₁H₁₈N₄O₃Cl [M+H]⁺ 409.8, found 408.8; t_{R}= 1.013 min

### Example 3: (Compound example E3.1; compound of formula I, wherein Het is 6-chloropyridin-3-yl, -W¹-W²-W³-W⁴- represent -CH=CH-CH=CH-, X is O, m is 1, R³ and R^{4a} with the existing bond form both together a double bond, R^{4b} is H and R⁵ is isopropyl)

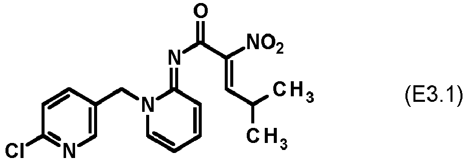

### 3.1 Synthesis of E3.1

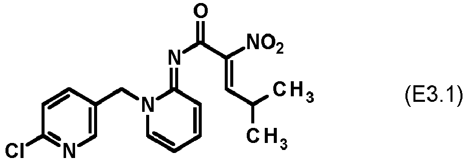

A solution of titanium tetrachloride (3.26 mL, 3.26 mmol) in dry THF (40 mL) under argon was cooled to 0°C. To this solution was added a solution of **E1.4** (500 mg, 1.63 mmol) and isobutyraldehyde (588 mg, 8.15 mmol) in THF/dichloromethane (2 mL each). After stirring for 10 min triethylamine (660 mg, 6.52 mmol) in THF (7 mL) was added dropwise over a period of 2 h to the reaction mixture. After stirring the reaction mixture for 72 h at room temperature, a saturated aqueous potassium sodium tartrate solution (10 mL) was added. The mixture was stirred for 30 minutes before the mixture was extracted with ethyl acetate (4x20 mL). The combined organic layers were dried over Na₂SO₄ and then concentrated in vacuo to afford a residue, which was purified using column chromatography over silica gel (10→30% cyclohexane/acetone) to afford the title product as yellow solid (239 mg, 33% yield, 80% purity).
LC-MS: mass calculated for C₁₇H₁₈N₄O₃Cl [M+H]⁺ 361.8, found 361.4; t_{R}= 1.002 min.

### B. Biological examples

The biological activity of the compounds of formula I of the present invention may be evaluated in biological tests as described in the following.

General conditions: If not otherwise specified, most test solutions are to be prepared as follows:The active compound is to be dissolved at the desired concentration in a mixture of 1:1 (vol:vol) distilled water:acteon. Further, the test solutions are to be prepared at the day of use (and, if not otherwised specified, in general at concentrations wt/vol).

### B.1 Boll weevil (Anthonomus grandis)

For evaluating control of boll weevil (*Anthonomus grandis*) the test unit consisted of 96-well-microtiter plates containing an insect diet and 5-10 *A*. *grandis* eggs.

The compounds were formulated using a solution containing 75% v/v water and 25% v/v DMSO. Different concentrations of formulated compounds were sprayed onto the insect diet at 5 µl, using a custom built micro atomizer, at two replications.

After application, microtiter plates were incubated at about 25 ± 1°C and about 75 ± 5 % relative humidity for 5 days. Egg and larval mortality was then visually assessed.

In this test, compound E1.4 at 2500 ppm showed at least 75 % mortality in comparison with untreated controls.

### B.2 Mediterranean fruitfly (Ceratitis capitata)

For evaluating control of Mediterranean fruitfly (*Ceratitis capitata*) the test unit consisted of microtiter plates containing an insect diet and 50-80 *C. capitata* eggs.
The compounds were formulated using a solution containing 75% v/v water and 25% v/v DMSO. Different concentrations of formulated compounds were sprayed onto the insect diet at 5 µl, using a custom built micro atomizer, at two replications.
After application, microtiter plates were incubated at about 28 ± 1°C and about 80 ± 5 % relative humidity for 5 days. Egg and larval mortality was then visually assessed.

In this test, compound E1.4 and E3.1 at 2500 ppm showed at least 75 % mortality in comparison with untreated controls.

### B.3 Vetch aphid (Megoura viciae)

For evaluating control of vetch aphid (*Megoura viciae*) through contact or systemic means the test unit consisted of 24-well-microtiter plates containing broad bean leaf disks.

The compounds were formulated using a solution containing 75% v/v water and 25% v/v DMSO. Different concentrations of formulated compounds were sprayed onto the leaf disks at 2.5 µl, using a custom built micro atomizer, at two replications.

After application, the leaf disks were air-dried and 5 - 8 adult aphids were placed on the leaf disks inside the microtiter plate wells. The aphids were then allowed to suck on the treated leaf disks and were incubated at about 23 ± 1°C and about 50 ± 5% relative humidity for 5 days. Aphid mortality and fecundity was then visually assessed.

In this test, compound E1.4, E2.3, and E3.1 at 2500 ppm showed at least 75% mortality in comparison with untreated controls.

### B.4 Green Peach Aphid (Myzus persicae)

For evaluating control of green peach aphid (*Myzus persicae*) through systemic means, the test unit consists of 96-well-microtiter plates containing liquid artificial diet under an artificial membrane.

The compounds are formulated using a solution containing 75% v/v water and 25% v/v DMSO. Different concentrations of formulated compounds are pipetted into the aphid diet, using a custom built pipetter, at two replications.

After application, 5 - 8 adult aphids are placed on the artificial membrane inside the microtiter plate wells. The aphids are then allowed to suck on the treated aphid diet and incubated at about 23 + 1°C and about 50 + 5% relative humidity for 3 days. Aphid mortality and fecundity is then visually assessed.

In this test, compound E1.4, E2.3, and E3.1 at 2500 ppm showed at least 75% mortality in comparison with untreated controls.

### B.5 Orchid thrips (dichromothrips corbetti)

*Dichromothrips corbetti* adults used for bioassay are obtained from a colony maintained continuously under laboratory conditions. For testing purposes, the test compound is diluted in a 1:1 mixture of acetone:water (vol:vol), plus 0.01% vol/vol Alkamuls® EL 620 surfactant.

Thrips potency of each compound is evaluated by using a floral-immersion technique. Plastic petri dishes are used as test arenas. All petals of individual, intact orchid flowers are dipped into treatment solution and allowed to dry. Treated flowers are placed into individual petri dishes along with about 20 adult thrips. The petri dishes are then covered with lids. All test arenas are held under continuous light and a temperature of about 28°C for duration of the assay. After 3 days, the numbers of live thrips are counted on each flower, and along inner walls of each petri dish. The percent mortality is recorded 72 hours after treatment.

In this test, compounds E1.4 and E3.1 at 500 ppm showed at least 75% mortality in comparison with untreated controls.

### B.6 Cowpea aphid (Aphis craccivora)

The active compound is dissolved at the desired concentration in a mixture of 1:1 (vol:vol) distilled water: acetone. Surfactant (Alkamuls® EL 620) is added at a rate of 0.1% (vol/vol). The test solution is prepared at the day of use.

Potted cowpea plants are colonized with approximately 50 - 100 aphids of various stages by manually transferring a leaf tissue cut from infested plant 24 hours before application. Plants are sprayed after the pest population has been recorded. Treated plants are maintained on light carts at about 28°C. Percent mortality is assessed after 72 hours.

In this test, compounds E1.4, E2.3, and E3.1 at 500 ppm showed at least 75% mortality in comparison with untreated controls.

### B.7 Rice green leafhopper (Nephotettix virescens)

Rice seedlings are cleaned and washed 24 hours before spraying. The active compounds are formulated in 50:50 acetone:water (vol:vol), and 0.1% vol/vol surfactant (EL 620) is added. Potted rice seedlings are sprayed with 5 ml test solution, air dried, placed in cages and inoculated with 10 adults. Treated rice plants are kept at about 28-29°C and relative humidity of about 50-60%. Percent mortality is recorded after 72 hours.

In this test, compounds E1.4 and E3.1 at 500 ppm showed at least 75% mortality in comparison with untreated controls.

### B.8 Green Soldier Stink Bug (Nezara viridula)

The active compound is dissolved at the desired concentration in a mixture of 1:1 (vol:vol) distilled water: aceteone. Surfactant (Kinetic HV) is added at a rate of 0.01% (vol/vol).The test solution is prepared at the day of use.

Soybean pods were placed in microwavable plastic cups lined with moist filter paper and inoculated with ten 3rd instar *N. viridula.* Using a hand atomizer, approximately 2 ml solution is sprayed into each cup. Treated cups were kept at about 28-29°C and relative humidity of about 50-60%. Percent mortality was recorded after 5 days.

In this test, compound E1.4 at 500 ppm showed over 75% mortality in comparison with untreated controls.

### B.9 Rice brown plant hopper (Nilaparvata lugens)

Rice seedlings are cleaned and washed 24 hours before spraying. The active compounds is formulated in 50:50 acetone:water (vol:vol) and 0.1% vol/vol surfactant (EL 620) was added. Potted rice seedlings are sprayed with 5 ml test solution, air dried, placed in cages and inoculated with 10 adults. Treated rice plants are kept at about 28-29°C and relative humidity of about 50-60%. Percent mortality is recorded after 72 hours.

In this test, compounds E1.4 at 500 ppm showed at least 75% mortality in comparison with untreated controls.

### B.10 Diamond back moth (Plutella xylostella)

The active compound is dissolved at the desired concentration in a mixture of 1:1 (vol:vol) distilled water: aceteone. Surfactant (Alkamuls® EL 620) is added at a rate of 0.1% (vol/vol).The test solution is prepared at the day of use.

Leaves of cabbage were dipped in test solution and air-dried. Treated leaves were placed in petri dish eslined with moist filter paper and inoculated with ten 3^{rd} instar larvae. Mortality was recorded 72 hours after treatment. Feeding damages were also recorded using a scale of 0-100%.

In this test, compound E1.4 at 500 ppm showed over 75% mortality in comparison with untreated controls.

### B.11 Cotton aphid (Aphis gossypii)

The active compound is dissolved at the desired concentration in a mixture of 1:1 (vol:vol) distilled water: acetone. Surfactant (Alkamuls® EL 620) is added at a rate of 0.1% (vol/vol). The test solution is prepared at the day of use.

Potted cowpea plants were colonized with approximately 50 - 100 aphids of various stages by manually transferring a leaf tissue cut from infested plant 24 hours before application. Plants were sprayed after the pest population has been recorded. Treated plants are maintained on light carts at about 28°C. Percent mortality was assessed after 72 hours.

In this test, compounds E1.4 and E3.1 at 500 ppm showed over 75% mortality in comparison with untreated controls.

### B.12 Silverleaf whitefly (bemisia argentifolii)

The active compounds are formulated in cyclohexanone as a 10,000 ppm solution supplied in tubes. The tubes are inserted into an automated electrostatic sprayer equipped with an atomizing nozzle and they serve as stock solutions for which lower dilutions are made in 50% acetone:50% water (v/v). A nonionic surfactant (Kinetic®) is included in the solution at a volume of 0.01% (v/v).

Cotton plants at the cotyledon stage (one plant per pot) are sprayed by an automated electrostatic plant sprayer equipped with an atomizing spray nozzle. The plants are dried in the sprayer fume hood and then removed from the sprayer. Each pot is placed into a plastic cup and about 10 to 12 whitefly adults (approximately 3-5 days old) are introduced. The insects are collected using an aspirator and a nontoxic Tygon® tubing connected to a barrier pipette tip. The tip, containing the collected insects, is then gently inserted into the soil containing the treated plant, allowing insects to crawl out of the tip to reach the foliage for feeding. Cups are covered with a reusable screened lid. Test plants are maintained in a growth room at about 25°C and about 20-40% relative humidity for 3 days, avoiding direct exposure to fluorescent light (24 hour photoperiod) to prevent trapping of heat inside the cup. Mortality is assessed 3 days after treatment, compared to untreated control plants.

In this test, compound E1.4 and E3.1 at 500 ppm showed at least 75% mortality in comparison with untreated controls.

### B.13 Southern armyworm (Spodoptera eridania),

The active compounds were formulated in cyclohexanone as a 10,000 ppm solution supplied in tubes. The tubes were inserted into an automated electrostatic sprayer equipped with an atomizing nozzle and they served as stock solutions for which lower dilutions were made in 50% acetone:50% water (v/v). A nonionic surfactant (Kinetic®) was included in the solution at a volume of 0.01% (v/v).

Lima bean plants (variety Sieva) were grown 2 plants to a pot and selected for treatment at the 1^{st} true leaf stage. Test solutions were sprayed onto the foliage by an automated electrostatic plant sprayer equipped with an atomizing spray nozzle. The plants were dried in the sprayer fume hood and then removed from the sprayer. Each pot was placed into perforated plastic bags with a zip closure. About 10 to 11 armyworm larvae were placed into the bag and the bags zipped closed. Test plants were maintained in a growth room at about 25°C and about 20-40% relative humidity for 4 days, avoiding direct exposure to fluorescent light (24 hour photoperiod) to prevent trapping of heat inside the bags. Mortality and reduced feeding were assessed 4 days after treatment, compared to untreated control plants.

In this test, compound E1.4 at 500 ppm showed at least 75% mortality in comparison with untreated controls.

## Claims

1. An N-acylimino compound of formula (I): 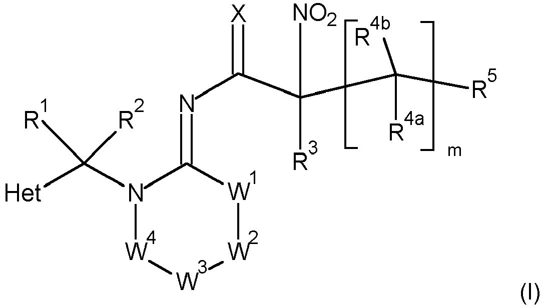 wherein
m is an integer selected from 0, 1, 2, 3, 4, 5 or 6;
X is O or S;
Het is a 5- or 6-membered carbon-bound or nitrogen-bound heterocyclic or heteroaromatic ring, comprising 1, 2, 3, 4 or 5 carbon atoms and 1, 2 or 3 heteroatoms as ring members, which are independently selected from sulfur, oxygen or nitrogen, wherein the carbon, sulfur and nitrogen ring members can independently be partly or completely oxidized, and wherein each ring is optionally substituted by k identical or different substituents R⁶, wherein k is an integer selected from 0, 1, 2, 3 or 4;
W¹-W²-W³-W⁴ represents a carbon chain group connected to N and C=N, and thus forming a saturated, unsaturated, or partially unsaturated 5- or 6-membered nitrogen containing heterocycle, wherein
W¹, W², W³ and W⁴ each individually represent CR^{v}R^{w}, wherein
each R^{w} independently from each other, is selected from the group consisting hydrogen, halogen, cyano, azido, nitro, SCN, SF₅, C₁-C₁₀-alkyl, C₃-C₈-cycloalkyl, C₂-C₁₀-alkenyl, C₂-C₁₀-alkynyl,
wherein each of the four last mentioned radicals may be unsubstituted, partly or completely halogenated and/or may optionally be substituted with 1, 2 or 3 identical or different radicals R7,
OR⁸, NR^{9a}R^{9b}, S(O)ₙR⁸, S(O)ₙNR^{9a}R^{9b}, C(=O)R^{7a}, C(=O)NR^{9a}R^{9b}, C(=O)OR⁸, C(=S)R^{7a}, C(=S)NR^{9a}R^{9b}, C(=S)OR⁸, C(=S)SR⁸, C(=NR¹⁷)R^{7a}, C(=NR¹⁷)NR^{9a}R^{9b} and Si(R¹¹)₂R¹²,
each R^{v} independently from each other, are selected from the group consisting of hydrogen, halogen, cyano, C₁-C₁₀-alkyl, C₃-C₈-cycloalkyl, C₂-C₁₀-alkenyl and C₂-C₁₀-alkynyl,
wherein each of the four last mentioned radicals may be unsubstituted or may be partly or completely halogenated or may optionally be further substituted with 1, 2 or 3 identical or different radicals R⁷; or
R^{v} and R^{w} present in one of the groups may together form =O, =CR¹³R¹⁴, =S, =NR¹⁷, =NOR¹⁶ or =NNR^{9a}R^{9b}, or
two R^{w} of adjacent carbon atoms, may form both together and together with the existing bond a double bond between the adjacent carbon atoms;
and wherein one of W² or W³ may optionally represent a single or a double bond between the adjacent carbon atoms;
R¹ and R² are independently from each other selected from the group consisting of hydrogen, halogen, CN, SCN, nitro, C₁-C₆-alkyl, C₃-C₆-cycloalkyl,
wherein each of the two aforementioned radicals is unsubstituted, partly or completely halogenated or may carry 1, 2 or 3 identical or different radicals R⁷,
Si(R¹¹)₂R¹², OR⁸, OSO₂R⁸, S(O)ₙR⁸, S(O)ₙNR^{9a}R^{9b}, NR^{9a}R^{9b}, C(=O)NR^{9a}R^{9b}, C(=S)NR^{9a}R^{9b}, C(=O)OR⁸, C(=O)R^{7a}, C(=S)R^{7a}, phenyl, benzyl,
wherein the phenyl ring in the last two radicals is unsubstituted or optionally substituted with 1, 2, 3, 4 or 5 identical or different substituents R¹⁰,
a 3-, 4-, 5-, 6- or 7- membered saturated, partly saturated or unsaturated aromatic heterocyclic ring comprising 1, 2 or 3 identical or different heteroatoms as ring members, which are selected from oxygen, nitrogen and sulfur,
wherein the heterocyclic ring is optionally substituted with 1, 2, 3 or 4 identical or different substituents R¹⁰, and wherein the nitrogen and/or the sulfur atom(s) of the heterocyclic ring may optionally be oxidized,
or
R¹ and R² form, together with the carbon atom, which they attached to, a 3-, 4-, 5- or 6-membered saturated or partly unsaturated carbocyclic or heterocyclic ring,
wherein each of the carbon atoms of said cycle are unsubstituted or may carry 1 or 2 identical or different radicals R⁷,
or
R¹ and R² may together be =O, =CR¹³R¹⁴, =S, =NR¹⁷, =NOR¹⁶ or =NNR^{9a}R^{9b};
R³ is selected from the group consisting of hydrogen, halogen, NO₂, C₁-C₆-alkyl, C₂-C₆-alkenyl, C₂-C₆-alkynyl, C₃-C₈-cycloalkyl and C₃-C₈-cycloalkenyl,
wherein each of the five last mentioned radicals is unsubstituted, partly or completely halogenated or carries 1 or 2 identical or different radicals R⁷, it being also possible for cycloalkyl and cycloalkenyl to carry 1, 2, 3, 4, 5 or 6 identical or different C₁-C₄-alkyl groups,
S(O)ₙNR^{9a}R^{9b}, NR^{9a}R^{9b}, C(=O)OR⁸, C(=O)NR^{9a}R^{9b}, C(=S)NR^{9a}R^{9b}, C(=O)R^{7a}, C(=S)R^{7a}, NR^{9a}-C(=O)R^{7a}, NR^{9a}-C(=S)R^{7a}, NR^{9a}-S(O)ₙR^{8a}, phenyl, benzyl,
wherein the phenyl ring in the last two mentioned radicals is unsubstituted or may be substituted with one or more, e.g. 1, 2, 3, 4 or 5 identical or different substituents R¹⁰,
and a 3-, 4-, 5-, 6- or 7- membered saturated, partly saturated or unsaturated aromatic heterocyclic ring comprising 1, 2 or 3 heteroatoms as ring members, which are identical or different and selected from oxygen, nitrogen and sulfur,
wherein the heterocyclic ring is optionally substituted with 1, 2, 3 or 4 identical or different substituents R¹⁰, and wherein the nitrogen and/or the sulfur atom(s) of the heterocyclic ring may optionally be oxidized;
R^{4a} and R^{4b} are selected each independently from one another and independently from the integer of m from the group consisting of hydrogen, halogen, CN, C₁-C₆-alkyl, C₁-C₆-alkoxy, di-(C₁-C₆-alkyl)amino, C₁-C₆-alkylthio, and C₃-C₈-cycloalkyl,
wherein each of the five last mentioned radicals is unsubstituted, partly or completely halogenated,
it being also possible for R^{4b} to be selected from
C₁-C₆-alkylsulfinyl, C₁-C₆-alkylsulfonyl,
wherein each of the two last mentioned radicals is unsubstituted, partly or completely halogenated,
C₁-C₆-alkyl or C₃-C₈-cycloalkyl,
wherein each of the two last mentioned radicals carries 1, 2 or 3 identical or different radicals R⁷,
SH, S(O)ₙNR^{9a}R^{9b}, NR^{9a}R^{9b}, C(=O)OR⁸, C(=O)NR^{9a}R^{9b}, C(=S)NR^{9a}R^{9b}, C(=O)R^{7a}, C(=S)R^{7a},
a moiety Q¹-phenyl,
wherein the phenyl ring is optionally substituted with 1, 2, 3, 4 or 5 identical or different substituents R¹⁰,
and a moiety Q¹-Het^{#},
wherein
Q¹ is a single bond, NR^{9a}, C₁-C₄-alkylene or NR^{9a}-C₁-C₄-alkylene,
wherein the heteroatom in the last moiety is bound to the carbon atom of CR^{4a}R^{4b};
or
R^{4a} and R^{4b} may form together with the carbon atom they are bound to, a 3-, 4-, 5- or 6-membered aliphatic ring,
wherein each of the carbon atoms of the ring may be unsubstituted, partly or completely halogenated, and/or may carry 1 or 2 identical or different radicals selected from C₁-C₄-alkyl, C₁-C₄-haloalkyl, C₁-C₄-alkoxy and C₁-C₄-haloalkoxy;
or
R^{4a} and R^{4b} may together form =O, =CR¹³R¹⁴, =S, =NR¹⁷, =NOR¹⁶ or =NNR^{9a}R^{9b};
R⁵ is selected from the group consisting of
hydrogen, halogen, CN, C₁-C₆-alkyl, C₁-C₆-alkoxy, C₁-C₆-alkylthio, C₁-C₆-alkylsulfinyl, C₁-C₆-alkylsulfonyl, C₂-C₆-alkenyl, C₂-C₆-alkenylthio, C₂-C₆-alkenylsulfinyl, C₂-C₆-alkenylsulfonyl, C₂-C₆-alkynyl, C₂-C₆-alkynylthio, C₂-C₆-alkynylsulfinyl, C₂-C₆-alkynylsulfonyl, C₃-C₈-cycloalkyl, C₃-C₈-cycloalkoxy, C₃-C₈-cycloalkylthio, C₃-C₈-cycloalkylsulfinyl and C₃-C₈-cycloalkylsulfonyl,
wherein each of the 18 last mentioned radicals is unsubstituted, partly or completely halogenated and/or carries 1, 2 or 3 identical or different
radicals R⁷, it being also possible for cycloalkyl radicals to carry 1, 2, 3, 4, 5 or 6 identical or different C₁-C₄-alkyl groups,
S(O)ₙNR^{9a}R^{9b}, O-NR^{9a}R^{9b}, NR^{9a}R^{9b}, NR^{9a}NR^{9a}R^{9b}, C(=O)OR⁸, C(=O)NR^{9a}R^{9b}, C(=S)NR^{9a}R^{9b}, C(=O)R^{7a}, C(=S)R^{7a}, NR^{9a}-C(=O)R^{7a}, NR^{9a}-C(=S)R^{7a}, NR^{9a}C(=O)NR^{9a}R^{9b}, NR^{9a}C(=S)NR^{9a}R^{9b}, NR^{9a}-S(O)ₙR^{8a}, a moiety Q-phenyl,
wherein the phenyl ring is optionally substituted with 1, 2, 3, 4 or 5 identical or different substituents R¹⁰,
a moiety Y-R^{5a},
and a moiety Q-Het^{#},
wherein
Y is O, S, S(=O) or S(=O)₂;
R^{5a} is selected from the group consisting of C(=O)NR^{9a}R^{9b}, C(=S)NR^{9a}R^{9b}, C(=O)R⁷, C(=S)R⁷;
phenyl or CH₂-phenyl,
optionally substituted with one or more, e.g. 1, 2, 3, 4 or 5 identical or different substituents R¹⁰;
a 3-, 4-, 5-, 6- or 7- membered saturated, partly saturated or unsaturated aromatic heterocyclic ring comprising 1, 2 or 3 heteroatoms selected from oxygen, nitrogen and/or sulfur, optionally substituted with 1, 2, 3 or 4 identical or different substituents R^{y},
wherein the nitrogen and/or the sulfur atom(s) of the heterocyclic ring may optionally be oxidized, and
R^{y} is selected from the group consisting of hydrogen, halogen, cyano, nitro, C₁-C₁₀-alkyl, C₂-C₁₀-alkenyl,
wherein the two last mentioned radicals may optionally be substituted with one or more identical or different radical R¹⁸,
phenyl,
optionally substituted with halogen, cyano, nitro, C₁-C₆-alkyl, C₁-C₆-haloalkyl, C₁-C₆-alkoxy or C₁-C₆-haloalkoxy, C₁-C₄ alkyl and C₁-C₄ alkoxy, wherein both of the last two mentioned aliphatic radicals may be unsubstituted, partly or completely halogenated, OR¹⁹, -S(O)ₙR¹⁹, S(O)ₙNR^{20a}R^{20b}, NR^{20a}R^{20b}, C(=O)R¹⁸, C(=O)OR¹⁹, -C(=NR^{20a})R¹⁸, C(=O)NR^{20a}R^{20b}, C(=S)NR^{20a}R^{20b},
or
two R^{y} present together on one atom of a partly saturated heterocyclic may be =O, =CR^{21a}R^{21b}; =NR^{20a}, =NOR¹⁹ or =NNR^{20a};
or
two R^{y} on adjacent carbon atoms may be a bridge selected from CH₂CH₂CH₂CH₂, CH=CH-CH=CH, N=CH-CH=CH, CH=N-CH=CH, N=CH-N=CH, CH₂CH₂CH₂, CH=CHCH₂, CH₂CH₂NR^{17a}, CH₂CH=N, CH=CH-NR^{17a}, and form together with the carbon atoms to which the two R^{y} are bonded a 5-membered or 6-membered fused partly saturated or unsaturated, aromatic carbocyclic or heterocyclic ring, wherein the ring may optionally be substituted with one or two substituents selected from =O, OH, CH₃, OCH₃, halogen, cyano, C₁-C₄-alkoxy, C₁-C₄-haloalkoxy, C₁-C₄-alkyl and C₁-C₄-haloalkyl;
Q irrespectively of its occurrence, is a single bond, CH₂, S, O, S(=O), S(=O)₂, NR^{9a}, -O-S(=O)₂-, -NR^{9a}-S(=O)₂-, -O-C(=O)-, -NR^{9a}-C(=O)-, -O-C₁-C₄-alkylene, -S-C₁-C₄-alkylene, -NR^{9a}-C₁-C₄-alkylene, -O-C(=O)-C₁-C₄-alkylene or -NR^{9a}-C(=O)-C₁-C₄-alkylene,
wherein the heteroatom in the last 7 moieties is bound to the carbon atom of C(NO₂)R³ or CR^{4a}R^{4b}, respectively;
or, if m is 1,
R³ and R^{4a} may also form both together and together with the existing bond a double bond between the adjacent carbon atoms;
or, if m is 1,
one of R^{4a} or R^{4b} together with R⁵ may also form with the carbon atom they are bound to, a 3-, 4-, 5- or 6-membered saturated or partially unsaturated carbocycle or heterocycle,
wherein each of the carbon atoms of the carbocycle or heterocycle may be unsubstituted or may carry 1 or 2 identical or different radicals R^{7b}, and wherein the heterocylce has 1 or 2 non-adjacent identical or different heteroatoms or heteroatom moieties as ring members, which are selected from O, S, N, and N-R^{17c}; or, if m = 0,
R³ and R⁵ may also form together and with the carbon atom they are bound to a 3-, 4-, 5-or 6-membered saturated or partially unsaturated carbocycle or heterocycle,
wherein each of the carbon atoms of the carbocycle or heterocycle may be unsubstituted or may carry 1 or 2 identical or different radicals R^{7b}, and wherein the heterocylce has 1 or 2 non-adjacent identical or different heteroatoms or heteroatom moieties as ring members, which are selected from O, S, N, and N-R^{17c}, or
R³ and R⁵ may together form =O or =S;
where, independently of their occurrence,
n is 0, 1 or 2;
R⁶ is selected from the group consisting of
halogen, cyano, azido, nitro, SCN, SF₅,
C₁-C₁₀-alkyl, C₃-C₈-cycloalkyl, C₂-C₁₀-alkenyl, C₂-C₁₀-alkynyl,
wherein each of the four last mentioned radicals may be partially or completely halogenated and/or may optionally be substituted independently from one another with 1, 2 or 3 identical or different radicals R⁷,
OR⁸, NR^{17a}R^{17b}, S(O)ₙR⁸, S(O)ₙNR^{17a}R^{17b}, C(=O)R^{7a}, C(=O)NR^{17a}R^{17b}, C(=O)OR⁸, C(=S)R^{7a}, C(=S)NR^{17a}R^{17b}, C(=S)OR⁸, C(=S)SR⁸, C(=NR¹⁷)R^{7a}, C(=NR¹⁷)NR^{17a}R^{17b}, Si(R¹¹)₂R¹²,
phenyl, optionally substituted with 1, 2, 3, 4 or 5 identical or different substituents R¹⁰,
a 3-, 4-, 5-, 6- or 7- membered saturated, partly saturated or unsaturated aromatic heterocyclic ring comprising 1, 2 or 3 heteroatoms as ring members, which are identical or different and selected from oxygen, nitrogen and sulfur,
wherein the heterocyclic ring is optionally substituted with 1, 2, 3 or 4 identical or different substituents R¹⁰, and wherein the nitrogen and/or the sulfur atom(s) of the heterocyclic ring may optionally be oxidized;
or two of R⁶ present on one ring carbon may together form =O, =CR¹³R¹⁴, =S, =NR¹⁷, =NOR¹⁶, =NNR^{9a}R^{9b};
or two R⁶ together form a linear C₂-C₇ alkylene chain, thus forming, together with the ring atom(s) to which they are bound, a 3-, 4-, 5-, 6-, 7- or 8-membered ring,
wherein 1 or 2 CH₂ moieties of the alkylene chain may be replaced by 1 or 2 heteroatom moieties selected from O, S and NR^{17c} and/or
1 or 2 of the CH₂ groups of the alkylene chain may be replaced by a group C=O, C=S and/or C=NR¹⁷; and
wherein the alkylene chain is unsubstituted or may be substituted with 1, 2, 3, 4, 5 or 6 identical or different radicals selected from the group consisting of
halogen, C₁-C₆-haloalkyl, C₁-C₆-alkoxy, C₁-C₆-haloalkoxy, C₁-C₆-alkylthio, C₁-C₆-haloalkylthio, C₃-C₈-cycloalkyl, C₃-C₈-halocycloalkyl, C₂-C₆-alkenyl, C₂-C₆-haloalkenyl, C₂-C₆-alkynyl, C₂-C₆-haloalkynyl,
phenyl which may be substituted with 1, 2, 3, 4 or 5 identical or different radicals R¹⁰, and
a 3-, 4-, 5-, 6- or 7-membered saturated, partially unsaturated or aromatic heterocyclic ring containing 1, 2 or 3 heteroatoms or heteroatom groups selected from N, O, S, NO, SO and SO₂, as ring members, wherein the heterocyclic ring may be substituted with 1, 2, 3, 4 or 5 identical or different radicals R¹⁰;
R⁷ independently of its occurrence, is selected from the group consisting of cyano, azido, nitro, -OH, -SH, -SCN, SF₅, C₁-C₆-alkyl, C₁-C₆-haloalkyl, C₃-C₈-cycloalkyl, C₃-C₈-halocycloalkyl, C₂-C₆-alkenyl, C₂-C₆-haloalkenyl, C₂-C₆-alkynyl, C₂-C₆-haloalkynyl, C₁-C₆-alkoxy, C₁-C₆-haloalkoxy, C₁-C₆-alkylthio, C₁-C₆-alkylsulfinyl, C₁-C₆-alkylsulfonyl, C₁-C₆-haloalkylthio, Si(R¹¹)₂R¹², OR⁸, OSO₂R⁸, S(O)ₙR⁸, S(O)ₙNR^{17a}R^{17b}, NR^{17a}R^{17b}, C(=O)NR^{17a}R^{17b}, C(=S)NR^{17a}R^{17b}, C(=O)OR⁸, C(=O)R¹⁵, C(=S)R¹⁵, C(=NR¹⁷)R¹⁵,
phenyl, phenyl-C₁-C₄-alkyl, phenoxy,
wherein the phenyl ring in the last three radicals is unsubstituted or may be substituted with 1, 2, 3, 4 or 5 identical or different radicals selected from halogen, C₁-C₆-alkyl, cyano, azido, nitro, C₁-C₁₀-alkyl, C₁-C₆-haloalkyl, C₁-C₆-alkoxy, C₁-C₆ haloalkoxy, (C₁-C₆-alkoxy)carbonyl, (C₁-C₆-alkyl)amino and di-(C₁-C₆-alkyl)amino, and
a 3-, 4-, 5-, 6- or 7- membered saturated, partly saturated or unsaturated aromatic heterocyclic ring comprising 1, 2 or 3 heteroatoms as ring members, which are identical or different and selected from oxygen, nitrogen and sulfur,
where the heterocyclic ring is unsubstituted or may be substituted with 1, 2, 3 or 4 identical or different radicals selected from halogen, C₁-C₆-alkyl, cyano, azido, nitro, C₁-C₁₀-alkyl, C₁-C₆-haloalkyl, C₁-C₆-alkoxy, C₁-C₆ haloalkoxy, (C₁-C₆-alkoxy)carbonyl, (C₁-C₆-alkyl)amino and di-(C₁-C₆-alkyl)amino, and
wherein the nitrogen and/or the sulfur atom(s) of the heterocyclic ring may optionally be oxidized,
or
two R⁷ present on one carbon atom may together form =O, =CR¹³R¹⁴, =S, =NR¹⁷, =NOR¹⁶, =NNR^{9a}R^{9b},
or
two R⁷ may form a 3-, 4-, 5-, 6-, 7- or 8-membered saturated or partly unsaturated carbocyclic or heterocyclic ring together with the carbon atoms to which the two R⁷ are bonded, wherein the heterocyclic ring comprises 1, 2 or 3 heteroatoms as ring members, which are identical or different and selected from oxygen, nitrogen and sulfur,
wherein the heterocyclic ring is unsubstituted or may be substituted with 1, 2, 3 or 4 identical or different radicals selected from halogen, C₁-C₆-alkyl, cyano, azido, nitro, C₁-C₁₀-alkyl, C₁-C₆-haloalkyl, C₁-C₆-alkoxy, C₁-C₆ haloalkoxy, (C₁-C₆-alkoxy)carbonyl, (C₁-C₆-alkyl)amino and di-(C₁-C₆-alkyl)amino;
R^{7a} independently of its occurrence, is selected from the group consisting of hydrogen, C₁-C₆-alkyl, C₁-C₆-haloalkyl, C₁-C₆-alkoxy, C₁-C₆-haloalkoxy, C₁-C₆-alkylthio, C₁-C₆-alkylsulfinyl, C₁-C₆-alkylsulfonyl, C₁-C₆-haloalkylthio, C₃-C₈-cycloalkyl, C₃-C₈-halocycloalkyl, C₃-C₆-cycloalkyl-C₁-C₄-alkyl, C₂-C₆-alkenyl, C₂-C₆-haloalkenyl, C₂-C₆-alkynyl, C₂-C₆-haloalkynyl,
phenyl, optionally substituted with 1, 2, 3, 4 or 5 identical or different substituents R¹⁰,
benzyl, wherein the phenyl moiety of benzyl is unsubstituted or substituted by 1, 2 or 3 identical or different radicals selected from the group consisting of halogen, CN, C₁-C₄-alkyl, C₁-C₄-haloalkyl, C₁-C₄-alkoxy and C₁-C₄-haloalkoxy, and
a 3-, 4-, 5-, 6- or 7- membered saturated, partly saturated or unsaturated aromatic heterocyclic ring comprising 1, 2 or 3 heteroatoms as ring members, which are identical or different and selected from oxygen, nitrogen and sulfur,
wherein the heterocyclic ring is optionally substituted with 1, 2, 3 or 4 identical or different substituents R¹⁰, and
wherein the nitrogen and/or the sulfur atom(s) of the heterocyclic ring may optionally be oxidized;
R^{7b} is selected from the group consisting of halogen, cyano, C₁-C₁₀-alkyl, C₃-C₈-cycloalkyl, C₂-C₁₀-alkenyl, C₂-C₁₀-alkynyl,
wherein each of the four last mentioned radicals is unsubstituted, partly or completely halogenated, and/or may be substituted with 1, 2 or 3 identical or different radicals R⁷,
OR⁸, NR^{17a}R^{17b}, S(O)ₙR⁸, S(O)ₙNR^{17a}R^{17b}, C(=O)R^{7a}, C(=O)NR^{17a}R^{17b}, C(=O)OR⁸, C(=S)R^{7a}, C(=S)NR^{17a}R^{17b}, C(=S)OR⁸, C(NR¹⁷)R^{7b}, C(NR¹⁷)NR^{17a}NR^{17b}, Si(R¹¹)₂R¹²; phenyl, optionally substituted with 1, 2, 3, 4 or 5 identical or different substituents R¹⁰,
a 3-, 4-, 5-, 6- or 7- membered saturated, partly saturated or unsaturated aromatic heterocyclic ring comprising 1, 2 or 3 heteroatoms as ring members, which are identical or different and selected from oxygen, nitrogen and sulfur,
wherein the heterocyclic ring is optionally substituted with 1, 2, 3 or 4 identical or different substituents R¹⁰, and wherein the nitrogen and/or the sulfur atom(s) of the heterocyclic ring may optionally be oxidized,
or
two of R^{7b} present on one ring carbon may together form =O, =S or =CR¹³R¹⁴,
or
two R^{7b} together form a linear C₂-C₇ alkylene chain, thus forming, together with the ring atom(s) to which they are bound, a 3-, 4-, 5-, 6-, 7- or 8-membered ring,
wherein 1 or 2 CH₂ moieties of the alkylene chain may be replaced by 1 or 2 heteroatom moieties selected from O, S and NR^{17c} and/or
1 or 2 of the CH₂ groups of the alkylene chain may be replaced by a group C=O, C=S and/or C=NR¹⁷; and
wherein the alkylene chain is unsubstituted or may be substituted with 1, 2, 3, 4, 5 or 6 identical or different radicals selected from the group consisting of
halogen, C₁-C₄-alkyl, C₁-C₆-haloalkyl, C₁-C₆-alkoxy, C₁-C₆-haloalkoxy, C₁-C₆-alkylthio, C₁-C₆-haloalkylthio, C₃-C₈-cycloalkyl, C₃-C₈-halocycloalkyl, C₂-C₆-alkenyl, C₂-C₆-haloalkenyl, C₂-C₆-alkynyl, C₂-C₆-haloalkynyl, phenyl which may be substituted with 1, 2, 3, 4 or 5 identical or different radicals R¹⁰, and
a 3-, 4-, 5-, 6- or 7-membered saturated, partially unsaturated or aromatic heterocyclic ring containing 1, 2 or 3 heteroatoms or heteroatom groups selected from N, O, S, NO, SO and SO₂, as ring members, wherein the heterocyclic ring may be substituted with 1, 2, 3, 4 or 5 identical or different radicals R¹⁰;
R⁸ independently of its occurrence, is selected from the group consisting of hydrogen, C₁-C₆-alkyl, C₁-C₆-haloalkyl, C₃-C₈-cycloalkyl, C₃-C₈-cycloalkyl-C₁-C₄-alkyl, C₃-C₈-halocycloalkyl, C₂-C₆-alkenyl, C₂-C₆-haloalkenyl, C₂-C₆-alkynyl, C₂-C₆ haloalkynyl, C(=O)R¹⁵, C(=O)NR^{17a}R^{17b}, C(=S)NR^{17a}R^{17b}, C(=O)OR¹⁶, phenyl, phenyl-C₁-C₄-alkyl, wherein the phenyl ring in the last two mentioned radicals is unsubstituted or substituted with 1, 2, 3, 4 or 5 identical or different substituents R¹⁰, and
a 3-, 4-, 5-, 6- or 7- membered saturated, partly saturated or unsaturated aromatic heterocyclic ring comprising 1, 2 or 3 heteroatoms as ring members, which are identical or different and selected from oxygen, nitrogen and sulfur,
wherein the heterocyclic ring is optionally substituted with 1, 2, 3 or 4 identical or different substituents R¹⁰, and
wherein the nitrogen and/or the sulfur atom(s) of the heterocyclic ring may optionally be oxidized,
R^{8a} independently of its occurrence, is selected from the group consisting of C₁-C₆-alkyl, C₃-C₈-cycloalkyl, C₃-C₈-cycloalkyl-C₁-C₄-alkyl, C₂-C₆-alkenyl, C₂-C₆-alkynyl,
wherein each of the five last mentioned radicals is unsubstituted or partly or completely halogenated,
phenyl, benzyl,
wherein the phenyl ring in the last two mentioned radicals is unsubstituted or may be substituted with 1, 2, 3, 4 or 5 identical or different substituents R¹⁰,
and a 3-, 4-, 5-, 6- or 7- membered saturated, partly saturated or unsaturated aromatic heterocyclic ring comprising 1, 2 or 3 heteroatoms as ring members, which are identical or different and selected from oxygen, nitrogen and sulfur,
wherein the heterocyclic ring is optionally substituted with 1, 2, 3 or 4 identical or different substituents R¹⁰, and
wherein the nitrogen and/or the sulfur atom(s) of the heterocyclic ring may optionally be oxidized,
R⁹ independently of its occurrence, is selected from the group consisting of hydrogen, C₁-C₆-alkyl, C₁-C₆-haloalkyl, C₁-C₆-alkoxy, C₁-C₆-haloalkoxy, C₃-C₈-cycloalkyl, C₃-C₈-cycloalkyl-C₁-C₄-alkyl, C₃-C₈-halocycloalkyl, C₂-C₆-alkenyl, C₂-C₆-haloalkenyl, C₂-C₆-alkynyl, C₂-C₆ haloalkynyl,
phenyl, optionally substituted with 1, 2, 3, 4 or 5 identical or different substituents R¹⁰;
a 3-, 4-, 5-, 6- or 7- membered saturated, partly saturated or unsaturated aromatic C-bound heterocyclic ring comprising 1, 2 or 3 heteroatoms as ring members, which are identical or different and selected from oxygen, nitrogen and sulfur,
wherein the heterocyclic ring is optionally substituted with 1, 2, 3 or 4 identical or different substituents R¹⁰, and
wherein the nitrogen and/or the sulfur atom(s) of the heterocyclic ring may optionally be oxidized,
R^{9a} and R^{9b} are each independently from one another selected from the group consisting of
hydrogen, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₃-C₈-cycloalkyl, C₃-C₈-halocycloalkyl, C₃-C₈-cycloalkyl-C₁-C₄-alkyl, C₂-C₆-alkenyl, C₂-C₆-haloalkenyl, C₂-C₆-alkynyl, C₂-C₆ haloalkynyl,
S(O)ₙR¹⁶, -S(O)ₙNR^{17a}R^{17b}, C(=O)R¹⁵, C(=O)OR¹⁶, C(=O)NR^{17a}R^{17b}, C(=S)R¹⁵; C(=S)SR¹⁶, C(=S)NR^{17a}R^{17b}, C(=NR¹⁷)R¹⁵;
phenyl, benzyl, 1-phenethyl or 2-phenethyl,
wherein the phenyl ring in the last four mentioned radicals is unsubstituted or may be substituted with 1, 2, 3, 4 or 5 identical or different substituents R¹⁰; and
a 3-, 4-, 5-, 6- or 7- membered saturated, partly saturated or unsaturated aromatic C-bound heterocyclic ring comprising 1, 2 or 3 heteroatoms as ring members, which are identical or different and selected from oxygen, nitrogen and sulfur,
wherein the heterocyclic ring is optionally substituted with 1, 2, 3 or 4 identical or different substituents R¹⁰, and
wherein the nitrogen and/or the sulfur atom(s) of the heterocyclic ring may optionally be oxidized,
or,
R^{9a} and R^{9b} are together a C₂-C₇ alkylene chain and form a 3-, 4-, 5-, 6-, 7- or 8-membered saturated, partly saturated or unsaturated aromatic ring together with the nitrogen atom they are bonded to,
wherein the alkylene chain may contain one or two heteroatoms, which are, independently of each other, selected from oxygen, sulfur or nitrogen, and wherein the alkylene chain may optionally be substituted with 1, 2, 3 or 4 identical or different radicals selected from
halogen, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₃-C₈-cycloalkyl, C₃-C₈-halocycloalkyl, C₂-C₆-alkenyl, C₂-C₆-haloalkenyl, C₂-C₆-alkynyl, C₂-C₆-haloalkynyl, phenyl, optionally substituted with 1, 2, 3, 4 or 5 identical or different substituents R¹⁰, and
a 3-, 4-, 5-, 6- or 7- membered saturated, partly saturated or unsaturated aromatic C-bound heterocyclic ring comprising 1, 2 or 3 heteroatoms as ring members, which are identical or different and selected from oxygen, nitrogen and sulfur,
wherein the heterocyclic ring is optionally substituted with 1, 2, 3 or 4 identical or different substituents R¹⁰, and wherein the nitrogen and/or the sulfur atom(s) of the heterocyclic ring may optionally be oxidized,
or
R^{9a} and R^{9b} together may form =CR¹³R¹⁴, =NR¹⁷, =NOR¹⁶, =NNR^{17a}R^{17b} moiety;
R¹⁰ independently of its occurrence, is selected from the group consisting of halogen, cyano, azido, nitro, SCN, SF₅, C₁-C₁₀-alkyl, C₁-C₄-haloalkyl, C₃-C₈-cycloalkyl, C₂-C₁₀-alkenyl, C₂-C₁₀-alkynyl,
wherein each of the four last mentioned radicals may optionally be substituted with 1, 2, 3, 4 or 5 identical or different radicals R⁷,
Si(R¹¹)₂R¹², OR¹⁶, OS(O)ₙR¹⁶, -S(O)ₙR¹⁶, S(O)ₙNR^{17a}R^{17b}, NR^{17a}R^{17b}, C(=O)R¹⁵, C(=S)R¹⁵, C(=O)OR¹⁶, -C(=NR¹⁷)R¹⁵, C(=O)NR^{17a}R^{17b}, C(=S)NR^{17a}R^{17b}, phenyl,
optionally substituted with 1, 2, 3, 4 or 5 identical or different radicals selected from OH, halogen, cyano, nitro, C₁-C₆-alkyl, C₁-C₆-haloalkyl, C₁-C₆-alkoxy and C₁-C₆-haloalkoxy, and
a 3-, 4-, 5-, 6- or 7- membered saturated, partly saturated or unsaturated aromatic heterocyclic ring comprising 1, 2 or 3 heteroatoms as ring members, which are identical or different and selected from oxygen, nitrogen and sulfur,
wherein the heterocyclic ring is unsubstituted or may be substituted with 1, 2, 3, 4 or 5 identical or different substituents selected from halogen, cyano, NO₂, C₁-C₆-alkyl, C₁-C₆-haloalkyl, C₁-C₆-alkoxy and C₁-C₆-haloalkoxy, and
wherein the nitrogen and/or the sulfur atom(s) of the heterocyclic ring may optionally be oxidized;
or
two R¹⁰ present together on one carbon ring atom of a saturated or partly unsaturated heterocyclic radical may form =O, =CR¹³R¹⁴, =S, =NR¹⁷, =NOR¹⁶, =NNR^{17a}R^{17b};
or,
two R¹⁰ on adjacent carbon ring atoms may together be a bivalent radical selected from CH₂CH₂CH₂CH₂, CH=CH-CH=CH, N=CH-CH=CH, CH=N-CH=CH, N=CH-N=CH, OCH₂CH₂CH₂, OCH=CHCH₂, CH₂OCH₂CH₂, OCH₂CH₂O, OCH₂OCH₂, CH₂CH₂CH₂, CH=CHCH₂, CH₂CH₂O, CH=CHO, CH₂OCH₂, CH₂C(=O)O, C(=O)OCH₂, O(CH₂)O, SCH₂CH₂CH₂, SCH=CHCH₂, CH₂SCH₂CH₂, SCH₂CH₂S, SCH₂SCH₂, CH₂CH₂S, CH=CHS, CH₂SCH₂, CH₂C(=S)S, C(=S)SCH₂, S(CH₂)S, CH₂CH₂NR¹⁷, CH₂CH=N, CH=CH-NR¹⁷, OCH=N, SCH=N, and form together with said adjacent carbon ring atoms a 5-membered or 6-membered partly saturated or unsaturated, aromatic carbocyclic or heterocyclic ring, wherein the ring may optionally be substituted with one or two substituents independently selected from =O, OH, CH₃, OCH₃, halogen, cyano, halomethyl and halomethoxy;
R¹¹ and R¹² independently of their occurrence, are selected from the group consisting of C₁-C₆-alkyl, C₁-C₆-haloalkyl, C₁-C₆-alkoxy, C₁-C₆-alkoxy-C₁-C₄-alkyl, C₂-C₆-alkenyl, C₂-C₆-haloalkenyl, C₂-C₆-alkynyl, C₂-C₆-haloalkynyl, C₃-C₈-cycloalkyl, C₃-C₈-halocycloalkyl, C₃-C₈-cycloalkyl-C₁-C₄-alkyl, C₃-C₈-halocycloalkyl-C₁-C₄-alkyl, C₁-C₆-haloalkoxy-C₁-C₄-alkyl,
phenyl and benzyl,
wherein the phenyl ring in the two last mentioned radicals is unsubstituted or substituted with 1, 2, 3, 4 or 5 identical or different radicals selected from halogen, OH, cyano, NO₂, C₁-C₆-alkyl, C₁-C₆-haloalkyl, C₁-C₆-alkoxy and C₁-C₆-haloalkoxy;
R¹³ and R¹⁴ independently of their occurrence, are selected from the group consisting of hydrogen, halogen, CN, C₁-C₆-alkyl, C₃-C₆-cycloalkyl, C₁-C₄-alkoxy-C₁-C₄-alkyl, phenyl and benzyl;
R¹⁵ independently of its occurrence, is selected from the group consisting of hydrogen, C₁-C₆-alkyl, C₂-C₆-alkenyl, C₂-C₆-alkynyl, C₃-C₈-cycloalkyl,
wherein the four last mentioned aliphatic and cycloaliphatic radicals may be unsubstituted, partially or completely halogenated and/or oxygenated and/or may carry 1 or 2 identical or different radicals selected from C₁-C₄ alkoxy; C₃-C₆-cycloalkyl-C₁-C₄-alkyl, phenyl, benzyl and pyridyl,
wherein the last three radicals may be unsubstituted, partially or completely halogenated and/or may carry 1, 2 or 3 identical or different substituents selected from C₁-C₆-alkyl, C₁-C₆-haloalkyl, C₁-C₆-alkoxy, C₁-C₆ haloalkoxy, (C₁-C₆-alkoxy)carbonyl, (C₁-C₆-alkyl)amino and di-(C₁-C₆-alkyl)amino;
R¹⁶ independently of its occurrence, is selected from the group consisting of hydrogen, C₁-C₆-alkyl, C₂-C₆-alkenyl, C₂-C₆-alkynyl, C₃-C₈-cycloalkyl, C₃-C₈-cycloalkyl-C₁-C₄-alkyl,
wherein the five last mentioned aliphatic and cycloaliphatic radicals may be unsubstituted, partially or completely halogenated and/or oxygenated and/or may carry 1 or 2 radicals selected from C₁-C₄ alkoxy,
phenyl, benzyl and pyridyl,
wherein the last three radicals may be unsubstituted, partially or completely halogenated and/or may carry 1, 2 or 3 identical or different substituents selected from C₁-C₆-alkyl, C₁-C₆-haloalkyl, C₁-C₆-alkoxy, C₁-C₆ haloalkoxy, (C₁-C₆-alkoxy)carbonyl, (C₁-C₆-alkyl)amino and di-(C₁-C₆-alkyl)amino;
R¹⁷ independently of its occurrence, is selected from the group consisting of hydrogen, C₁-C₆-alkoxy, C₁-C₆-haloalkoxy, C₁-C₆-alkylthio, trimethylsilyl, triethylsilyl, *tert*butyldimethylsilyl, C₁-C₆-alkyl, C₂-C₆-alkenyl, C₂-C₆-alkynyl, C₃-C₈-cycloalkyl,
wherein the four last mentioned radicals may be unsubstituted, partially or completely halogenated and/or oxygenated and/or may carry 1 or 2 radicals selected from C₁-C₄-alkoxy,
C₃-C₆-cycloalkyl-C₁-C₄-alkyl, phenyl, benzyl, pyridyl, phenoxy, wherein the four last mentioned radicals may be unsubstituted, partially or completely halogenated and/or may carry 1, 2 or 3 identical or different substituents selected from C₁-C₆-alkyl, C₁-C₆-haloalkyl, C₁-C₆-alkoxy, C₁-C₆ haloalkoxy and (C₁-C₆-alkoxy)carbonyl,
R^{17a} and R^{17b} are each independently from one another selected from the group consisting of hydrogen, C₁-C₆-alkoxy, C₁-C₆-haloalkoxy, C₁-C₆-alkylthio, C₁-C₆-alkylsulfinyl, C₁-C₆-alkylsulfonyl, C₁-C₆-haloalkylthio, trimethylsilyl, triethylsilyl, *tert*butyldimethylsilyl, C₁-C₆-alkyl, C₂-C₆-alkenyl, C₂-C₆-alkynyl, C₃-C₈-cycloalkyl,
wherein the four last mentioned radicals may be unsubstituted, partially or completely halogenated and/or oxygenated and/or may carry 1 or 2 identical or different radicals selected from C₁-C₄-alkoxy,
phenyl, benzyl, pyridyl, phenoxy,
wherein the four last mentioned radicals may be unsubstituted, partially or completely halogenated and/or may carry 1, 2 or 3 identical or different substituents selected from C₁-C₆-alkyl, C₁-C₆-haloalkyl, C₁-C₆-alkoxy, C₁-C₆ haloalkoxy and (C₁-C₆-alkoxy)carbonyl,
or,
R^{17a} and R^{17b} may together be a C₂-C₆ alkylene chain forming a 3- to 7-membered saturated, partly saturated or unsaturated ring together with the nitrogen atom R^{17a} and R^{17b} are bonded to,
wherein the alkylene chain may contain 1 or 2 heteroatoms selected, independently of each other, from oxygen, sulfur and nitrogen, and may optionally be substituted with halogen, C₁-C₄-haloalkyl, C₁-C₄-alkoxy or C₁-C₄-haloalkoxy, and
wherein the nitrogen and/or the sulfur atom(s) of the heterocyclic ring may optionally be oxidized;
or
R^{17a} and R^{17b} together may form a =CR¹³R¹⁴, =NR¹⁷ or =NOR¹⁶ moiety;
R^{17c} independently of its occurrence, is selected from the group consisting of hydrogen, CN, C₁-C₆-alkyl, C₂-C₆-alkenyl, C₂-C₆-alkynyl, C₃-C₈-cycloalkyl, C₃-C₈-cycloalkyl-C₁-C₄-alkyl,
wherein the five last mentioned radicals may be unsubstituted, partially or completely halogenated and/or oxygenated and/or may carry 1 or 2 radicals independently selected from C₁-C₄ alkoxy,
phenyl, benzyl and pyridyl, wherein the last three radicals may be unsubstituted, partially or completely halogenated and/or may carry 1, 2 or 3 identical or different substituents selected from C₁-C₆-alkyl, C₁-C₆-haloalkyl, C₁-C₆-alkoxy, C₁-C₆ haloalkoxy, (C₁-C₆-alkoxy)carbonyl, (C₁-C₆-alkyl)amino and di-(C₁-C₆-alkyl)amino;
R¹⁸ is each independently from one another selected from the group consisting of hydrogen, halogen, cyano, nitro, OH, SH, SCN, SF₅, C₁-C₆-alkoxy, C₁-C₆-haloalkoxy, C₁-C₆-alkylthio, C₁-C₆-alkylsulfinyl, C₁-C₆-alkylsulfonyl, C₁-C₆-haloalkylthio, trimethylsilyl, triethylsilyl, *tert*butyldimethylsilyl, C₁-C₆-alkyl, C₂-C₆-alkenyl, C₂-C₆-alkynyl, C₃-C₈-cycloalkyl,
wherein the four last mentioned aliphatic and cyclo-aliphatic radicals may be unsubstituted, partially or completely halogenated and/or oxygenated and/or may carry 1 or 2 radicals selected from C₁-C₄ alkoxy;
phenyl, benzyl, pyridyl, phenoxy,
wherein the last four radicals may be unsubstituted, partially or completely halogenated and/or to carry 1, 2 or 3 substituents selected from C₁-C₆-alkyl, C₁-C₆-haloalkyl, C₁-C₆-alkoxy, C₁-C₆ haloalkoxy, (C₁-C₆-alkoxy)carbonyl, (C₁-C₆-alkyl)amino or di-(C₁-C₆-alkyl)amino,
or
two R¹⁸ present on the same carbon atom may together be =O, =CH(C₁-C₄), =C(C₁-C₄-alkyl)C₁-C₄-alkyl, =N(C₁-C₆-alkyl) or =NO(C₁-C₆-alkyl);
R¹⁹ is each independently from one another selected from the group consisting of hydrogen, cyano, C₁-C₆-alkoxy, C₁-C₆-haloalkoxy, C₁-C₆-alkylthio, C₁-C₆-alkylsulfinyl, C₁-C₆-alkylsulfonyl, C₁-C₆-haloalkylthio, trimethylsilyl, triethylsilyl, *tert*butyldimethylsilyl, C₁-C₆-alkyl, C₂-C₆-alkenyl, C₂-C₆-alkynyl, C₃-C₈-cycloalkyl,
wherein the four last mentioned radicals may be unsubstituted, partially or completely halogenated and/or oxygenated and/or may carry 1 or 2 radicals selected from C₁-C₄ alkoxy,
phenyl, benzyl, pyridyl, phenoxy,
wherein the last four radicals may be unsubstituted, partially or completely halogenated and/or carry 1, 2 or 3 substituents selected from C₁-C₆-alkyl, C₁-C₆-haloalkyl, C₁-C₆-alkoxy, C₁-C₆ haloalkoxy or (C₁-C₆-alkoxy)carbonyl;
R^{20a}, R^{20b} are each independently from one another selected from the group consisting of hydrogen, cyano, C₁-C₆-alkoxy, C₁-C₆-haloalkoxy, C₁-C₆-alkylthio, C₁-C₆-alkylsulfinyl, C₁-C₆-alkylsulfonyl, C₁-C₆-haloalkylthio, trimethylsilyl, triethylsilyl, *tert*butyldimethylsilyl, C₁-C₆-alkyl, C₂-C₆-alkenyl, C₂-C₆-alkynyl, C₃-C₈-cycloalkyl,
wherein the four last mentioned aliphatic and cyclo-aliphatic radicals may be unsubstituted, partially or completely halogenated and/or oxygenated and/or may carry 1 or 2 identical or different radicals selected from C₁-C₄-alkoxy,
phenyl, benzyl, pyridyl, phenoxy,
wherein the four last mentioned radicals may be unsubstituted, partially or completely halogenated and/or carry 1, 2 or 3 identical or different substituents selected from C₁-C₆-alkyl, C₁-C₆-haloalkyl, C₁-C₆-alkoxy, C₁-C₆ haloalkoxy and (C₁-C₆-alkoxy)carbonyl,
or,
R^{20a} and R^{20b} may together be a C₂-C₆ alkylene chain forming a 3- to 7-membered saturated, partly saturated or unsaturated ring together with the nitrogen atom R^{20a} and R^{20b} are bonded to, wherein the alkylene chain may contain 1 or 2 heteroatoms selected from oxygen, sulfur and nitrogen, and may optionally be substituted with halogen, C₁-C₄-haloalkyl, C₁-C₄-alkoxy or C₁-C₄-haloalkoxy, and wherein the nitrogen and/or the sulfur atom(s) of the heterocyclic ring may optionally be oxidized;
R^{21a}, R^{21b} are each independently from one another selected from the group consisting of hydrogen, C₁-C₄ alkyl, C₁-C₆ cycloalkyl, C₁-C₄ alkoxyalkyl, phenyl and benzyl;
Het^{#} represents a 3-, 4-, 5-, 6- or 7- membered saturated, partly saturated or unsaturated aromatic heterocyclic ring comprising 1, 2 or 3 heteroatoms as ring members, which are selected from oxygen, nitrogen and/or sulfur,
wherein the heterocyclic ring is optionally substituted with 1, 2, 3, 4 or 5 identical or different substituents R¹⁰, and wherein the nitrogen and/or the sulfur atom(s) of the heterocyclic ring may optionally be oxidized,
the stereoisomers, tautomers and the salts thereof.

2. The compound of claim 1, wherein
Het is selected from the group consisting of radicals of the following formula Het-1 to Het-24: 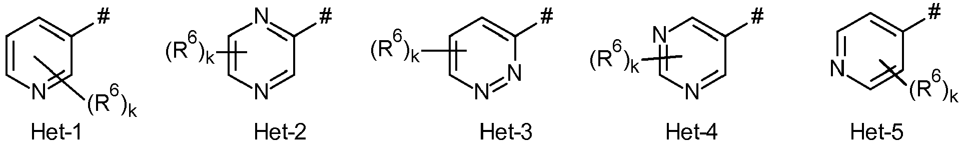 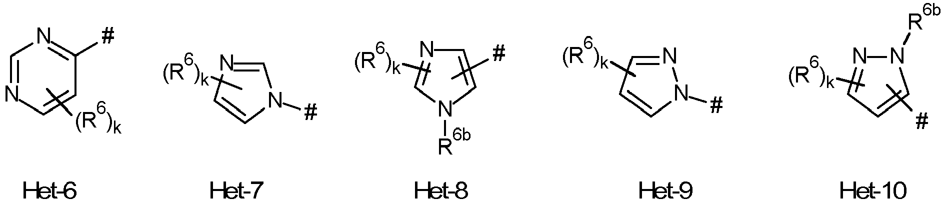 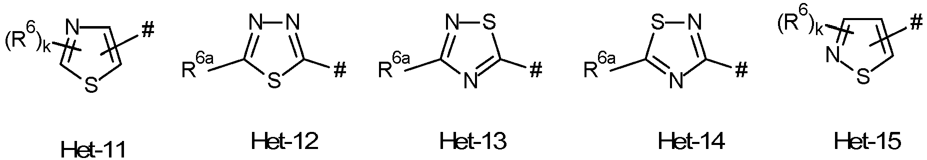 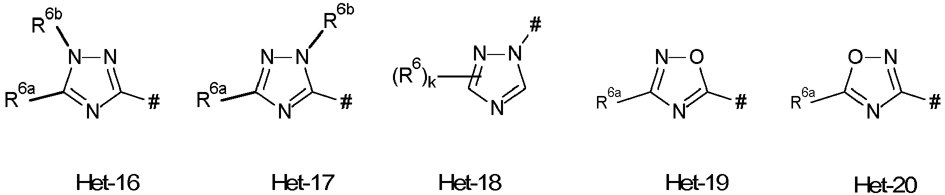 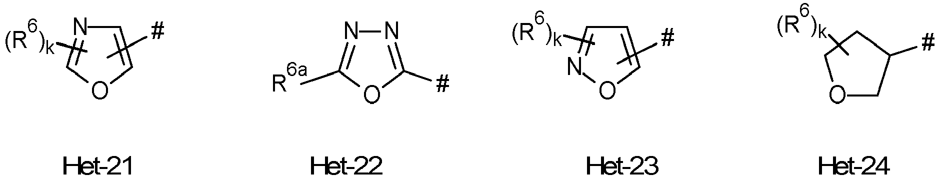
wherein # denotes the bond in formula (I), and wherein
k is 0, 1 or 2; and
R^{6a} is hydrogen or has one of the meanings given for R⁶ in claim 1 and
R^{6b} is hydrogen, C₁-C₄-alkyl or C₁-C₄-haloalkyl.

3. The compound of claim 2, wherein Het is Het-1a 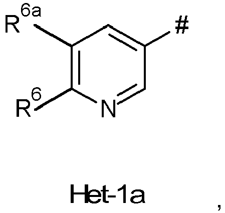 wherein # denotes the bond in formula (I),
R⁶ is selected from halogen, C₁-C₄-alkyl and C₁-C₄-haloalkyl, and
R^{6a} is hydrogen or halogen, in particular hydrogen.

4. The compound of any of the preceding claims, wherein
R¹ and R² are independently from each other selected from the group consisting of hydrogen, halogen, CN, C₁-C₆-alkyl, C₃-C₆-cycloalkyl, C₁-C₆-haloalkyl, C₃-C₆-halocycloalkyl;
or
R¹ and R² form, together with the carbon atom, which they attached to, a 3- to 5-membered saturated carbocyclic ring; and wherein in particular both R¹ and R² are hydrogen, or R¹ is methyl and R² is hydrogen.

5. The compound of any of the preceding claims, wherein
R³ is selected from the group consisting of hydrogen, halogen, C₁-C₆-alkyl, C₁-C₆-haloalkyl, C₂-C₆-alkenyl, C₂-C₆-haloalkenyl, C₃-C₆-cycloalkyl, C₃-C₆-halocycloalkyl, C₃-C₆-cycloalkenyl, NR^{9a}R^{9b} and NR^{9a}-C(=O)R^{7a}.

6. The compound of any of the preceding claims, wherein m is 0 or 1.

7. The compound of any of claims 1 to 4, wherein
m is 1, and
R³ and R^{4a} form both together and together with the existing bond a double bond between the adjacent carbon atoms.

8. The compound of any of the preceding claims, wherein
R⁵ is hydrogen, halogen, CN, NR^{9a}R^{9b}, C₁-C₆-alkyl, C₁-C₆-haloalkyl, C₂-C₆-alkenyl, C₂-C₆-haloalkenyl, C₁-C₆-alkoxy, C₁-C₆-haloalkoxy, C₃-C₆-cycloalkyl, C₃-C₆-halocycloalkyl, C₃-C₆-cycloalkoxy, C₃-C₆-halocycloalkoxy, C(=O)OR⁸, C(=O)NR^{9a}R^{9b}, C(=S)NR^{9a}R^{9b}, C(=O)R^{7a}, C(=S)R^{7a}, and Q-phenyl,
wherein phenyl is unsubstituted or substituted with 1, 2, 3, 4 or 5 identical or different substituents R¹⁰.

9. The compound of any of claims 1 to 6, wherein
m is 0, and
R³ and R⁵ together with the carbon atom, to which they are bound, form a 3-, 4-, 5- or 6-membered saturated carbocyle, a 4-, 5- or 6- membered unsaturated carbocyle, an epoxide ring or an oxetane ring,
wherein each of the carbon atoms of the saturated or unsaturated carbocycle may be unsubstituted or may carry 1 or 2 identical or different radicals R^{7b}, wherein
R^{7b} is selected from the group consisting of halogen, C₁-C₆-alkyl, C₁-C₆-alkoxy, and C₂-C₆-alkynyl,
wherein the carbon atoms of the aforementioned aliphatic radicals may optionally be partly or completely halogenated,
and one or two radicals R^{7b} may also be C₃-C₆-cycloalkyl, which is unsubstituted or carries 1, 2, 3, 4 or 5 identical or different radicals selected from
fluorine, chlorine or methyl,
phenyl, optionally substituted with 1, 2, 3, 4 or 5 identical or different substituents R^{10a}, or
a 3-, 4-, 5-, 6- or 7- membered saturated, partly saturated or unsaturated aromatic heterocyclic ring comprising 1, 2 or 3 heteroatoms as ring members, which are identical or different and selected from oxygen, nitrogen and sulfur,
wherein the heterocyclic ring is optionally substituted with 1, 2, 3 or 4 identical or different substituents R^{10a}, and
wherein the nitrogen and/or the sulfur atom(s) of the heterocyclic ring may optionally be oxidized,
or two of R^{7b} present on one ring carbon together form =O or =S,
or two R^{7b} together form a linear C₂-C₇ alkylene chain, thus forming, together with the carbon atom to which they are bound, a 3-, 4-, 5- or 6-membered ring,
wherein 1 or 2 CH₂ moieties of the alkylene chain may be replaced by 1 or 2 heteroatom moieties independently selected from O and S, and
wherein the alkylene chain is unsubstituted or may be substituted with 1, 2, 3, 4, 5 or 6 identical or different radicals selected from the group consisting of halogen, C₁-C₄-alkyl, C₁-C₄-haloalkyl, C₁-C₄-alkoxy and C₁-C₄-haloalkoxy, and
R^{10a} is selected from the group consisting of halogen, C₁-C₄-alkyl, C₁-C₄-haloalkyl, C₁-C₄-alkoxy and C₁-C₄-haloalkoxy.

10. The compound of any of the preceding claims, wherein the moiety of the formula 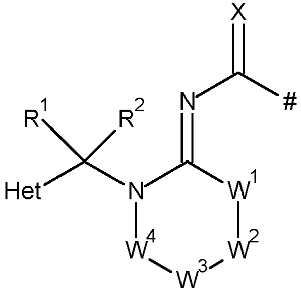 represents a radical A selected from the group consisting of W.Het-1, W.Het-2, W.Het-3, W.Het-4, W.Het-5, W.Het-6, W.Het-7, W.Het-8, W.Het-9, W.Het-10, W.Het-11 and W.Het-12: 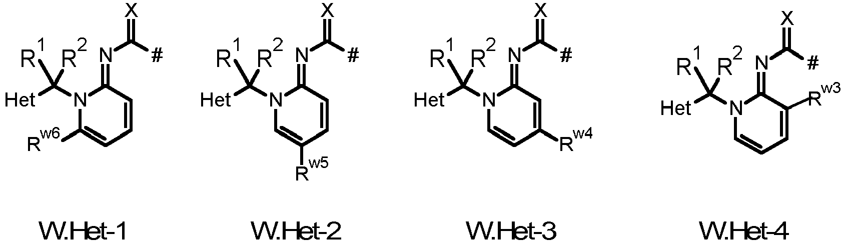 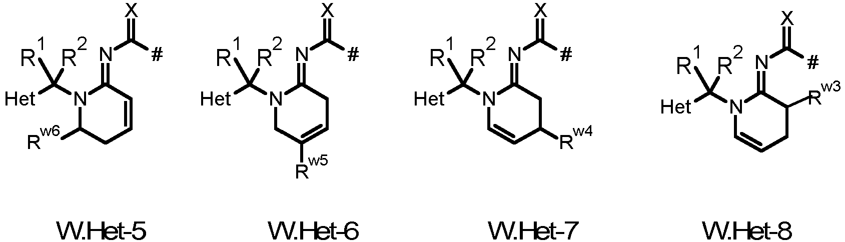 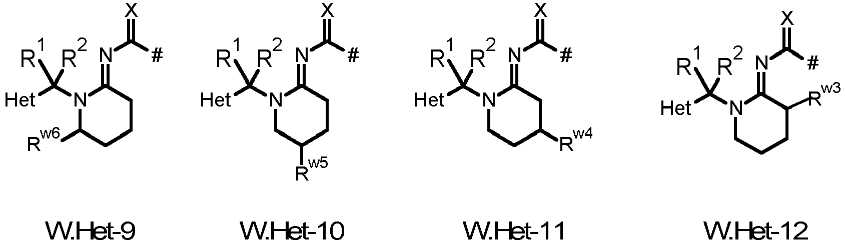 wherein # denotes the bond to the remainder of the molecule; R¹, R² and Het are as defined in any of claims 1 to 6, and wherein
R^{w3}, R^{w4}, R^{w5} and R^{w6} are, independently of each other, selected from hydrogen, halogen, C₁-C₄-alkoxy, C₁-C₄-haloalkoxy, C₁-C₄-alkyl and C₁-C₄-haloalkyl, especially hydrogen.

11. The compound of any of the preceding claims, wherein
X is O.

12. An agricultural or veterinary composition for combating animal pests comprising at least one compound as defined in any of claims 1 to 11 and at least one inert liquid and/or solid acceptable carrier and optionally, if desired, at least one surfactant.

13. The use of a compound as defined in any of claims 1 to 11 for combating or controlling invertebrate pests, for protecting growing plants from attack or infestation by invertebrate pests, for protecting plant propagation material, especially seeds, from soil insects, or for protect the seedlings roots and shoots of plants from soil and foliar insects, excluding the use on the human or animal body by therapy.

14. A non-therapeutic method for combating or controlling invertebrate pests, which method comprises contacting said pest or its food supply, habitat or breeding grounds with a pesticidally effective amount of at least one compound as defined in any one of claims 1 to 11.

15. A method for protecting growing plants from attack or infestation by invertebrate pests, which method comprises contacting a plant, or soil or water in which the plant is growing, with a pesticidally effective amount of at least one compound as defined in any of claims 1 to 11.

16. A method for the protection of plant propagation material, especially seeds, from soil insects and of the seedlings roots and shoots from soil and foliar insects comprising contacting the plant propagation material before sowing and/or after pregermination with at least one compound as defined in any one of claims 1 to 11.

17. The compound as defined in any of claims 1 to 11 for the use in the treatment of animals infested or infected by parasites, for the prevention of animals of getting infected or infested by parasites or for the protection of animals against infestation or infection by parasites.

## Patentansprüche

1. N-Acyliminoverbindung nach Formel (I): 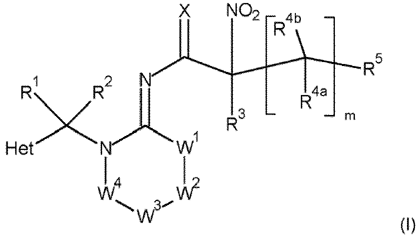 wobei
m eine ganze Zahl ist, die aus 0, 1, 2, 3, 4, 5 oder 6 ausgewählt ist;
X für O oder S steht;
Het für einen 5- oder 6-gliedrigen, an Kohlenstoff oder an Stickstoff gebundenen heterozyklischen oder heteroaromatischen Ring steht, der 1, 2, 3, 4 oder 5 Kohlenstoffatome und 1, 2 oder 3 Heteroatome als Ringglieder umfasst, die auf unabhängige Weise aus Schwefel, Sauerstoff oder Stickstoff ausgewählt sind, wobei die Kohlenstoff-, Schwefel- und Stickstoffringglieder auf unabhängige Weise teilweise oder vollständig oxidiert sein können, und wobei jeder Ring möglicherweise mit k gleichartigen oder verschiedenartigen Substituenten R⁶ versehen ist, wobei k eine ganze Zahl ist, die aus 0, 1, 2, 3 oder 4 ausgewählt ist;
W¹-W²-W³-W⁴ für eine Kohlenstoffkettengruppe steht, die an N oder C=N gebunden ist und somit einen gesättigten, ungesättigten oder teilweise ungesättigten 5- oder 6-gliedrigen stickstoffhaltigen Heterozyklus enthält, wobei
W¹, W², W³ und W⁴ jeweils einzeln für CR^{v}R^{w} stehen,
wobei
jeder R^{w} unabhängig voneinander aus der Gruppe ausgewählt ist, die aus Wasserstoff, Halogen, Cyano, Azido, Nitro, SCN, SF₅, C₁-C₁₀-Alkyl, C₃-C₈-Cycloalkyl, C₂-C₁₀-Alkenyl, C₂-C₁₀-Alkinyl,
wobei jeder der vier letztgenannten Reste unsubstituiert, teilweise oder vollständig halogeniert sein kann und/oder möglicherweise mit 1, 2 oder 3 gleichartigen oder verschiedenartigen Resten R⁷ substituiert sein kann,
aus OR⁸, NR^{9a}R^{9b}, S(O)ₙR⁸, S(O)ₙNR^{9a}R^{9b,} C(=O)R^{7a}, C(=O)NR^{9a}R^{9b}, C(=O)OR⁸, C(=S)R^{7a}, C(=S)NR^{9a}R^{9b}, C(=S)OR⁸, C(=S)SR⁸, C(=NR¹⁷) R^{7a}, C(=NR¹⁷) NR^{9a}R^{9b} und Si(R¹¹)₂R¹² besteht,
jedes R^{v} unabhängig voneinander aus der Gruppe ausgewählt ist, die aus Wasserstoff, Halogen, Cyano, C₁-C₁₀-Alkyl, C₃-C₈-Cycloalkyl, C₂-C₁₀-Alkenyl und C₂-C₁₀-Alkinyl besteht,
wobei jeder der vier letztgenannten Reste unsubstituiert, teilweise oder vollständig halogeniert sein kann oder möglicherweise weiterhin mit 1, 2 oder 3 gleichartigen oder verschiedenartigen Resten R⁷ substituiert sein kann; oder
R^{v} und R^{w}, die in einer der Gruppen vorliegen, zusammen =O, =CR¹³R¹⁴, =S, =NR¹⁷, =NOR¹⁶ oder =NNR^{9a}R^{9b} bilden können, oder
zwei R^{w} von benachbarten Kohlenstoffatomen sowohl miteinander als auch mit der bestehenden Bindung eine Doppelbildung zwischen den benachbarten Kohlenstoffatomen bilden können;
und wobei eines von W² oder W³ möglicherweise eine Einzel- oder Doppelbindung zwischen den benachbarten Kohlenstoffatomen darstellen kann;
R¹ und R² unabhängig voneinander aus der Gruppe ausgewählt sind, die aus Wasserstoff, Halogen, CN, SCN, Nitro, C₁-C₆-Alkyl, C₃-C₆-Cycloalkyl,
wobei jeder der zwei vorstehend genannten Reste unsubstituiert, teilweise oder vollständig halogeniert sein kann oder mit 1, 2 oder 3 gleichartigen oder verschiedenartigen Resten R⁷ versehen sein kann,
aus Si(R¹¹)₂R¹², OR⁸, OSO₂R⁸, S(O)ₙR⁸, S(O)ₙNR^{9a}R^{9b}, NR^{9a}R^{9b}, C(=O)NR^{9a}R^{9b}, C(=S)NR^{9a}R^{9b}, C(=O)OR⁸, C(=O)R^{7a}, C(=S)R^{7a}, aus Phenyl, Benzyl,
wobei der Phenylring in den beiden letzteren Resten unsubstituiert oder möglicherweise mit 1, 2, 3, 4 oder 5 gleichartigen oder verschiedenartigen Substituenten R¹⁰ versehen ist,
aus einem 3-, 4-, 5-, 6- oder 7-gliedrigen gesättigten, teilweise gesättigten oder ungesättigten aromatischen heterozyklischen Ring besteht, der 1, 2 oder 3 gleichartige oder verschiedenartige Heteroatome als Ringglieder umfasst, die aus Sauerstoff, Stickstoff und Schwefel ausgewählt sind,
wobei der heterozyklische Ring möglicherweise mit 1, 2, 3 oder 4 gleichartigen oder verschiedenartigen Substituenten R¹⁰ versehen ist, und wobei das/die Stickstoff- und/oder Schwefelatom(e) des heterozyklischen Rings möglicherweise oxidiert sein können,
oder
R¹ und R² zusammen mit dem Kohlenstoffatom, an welches sie gebunden sind, eine 3-, 4-, 5- oder 6-gliedrigen gesättigten oder teilweise ungesättigten carbozyklischen oder heterozyklischen Ring bilden,
wobei die Kohlenstoffatome des Zyklus jeweils unsubstituiert sind oder mit 1 oder 2 gleichartigen oder verschiedenartigen Resten R⁷ versehen sein können,
oder
R¹ und R² zusammen =O, =CR¹³R¹⁴, =S, =NR¹⁷, =NOR¹⁶ oder =NNR^{9a}R^{9b} darstellen können;
R³ aus der Gruppe ausgewählt ist, die aus Wasserstoff, Halogen, NO₂ C₁-C₆-Alkyl, C₂-C₆-Alkenyl, C₂-C₆-Alkinyl, C₃-C₈-Cycloalkyl und C₃-C₈-Cycloalkenyl,
wobei jeder der fünf letztgenannten Reste unsubstituiert, teilweise oder vollständig halogeniert ist oder mit 1 oder 2 gleichartigen oder verschiedenartigen Resten R⁷ versehen ist, wobei es für Cycloalkyl und Cycloalkenyl auch möglich ist, dass diese mit 1, 2, 3, 4, 5 oder 6 gleichartigen oder verschiedenartigen C₁-C₄-Alkylgruppen versehen sind,
aus S(O)ₙNR^{9a}R^{9b}, NR^{9a}R^{9b}, C(=O)OR⁸, C(=O)NR^{9a}R^{9b}, C(=S)NR^{9a}R^{9b}, C(=O)R^{7a}, C(=S)R^{7a}, NR^{9a}-C(=O)R^{7a}, NR^{9a}-C(=S)R^{7a}, NR^{9a}-S(O)ₙR^{8a}, aus Phenyl, Benzyl,
wobei der Phenylring in den beiden letztgenannten Resten unsubstituiert ist oder mit einem oder mehreren, z.B. 1, 2, 3, 4 oder 5 gleichartigen oder verschiedenartigen Substituenten R¹⁰ versehen sein kann,
und aus einem 3-, 4-, 5-, 6- oder 7-gliedrigen gesättigten, teilweise gesättigten oder ungesättigten aromatischen heterozyklischen Ring besteht, der 1, 2 oder 3 Heteroatome als Ringglieder umfasst, die gleichartig oder verschiedenartig sind und aus Sauerstoff, Stickstoff und Schwefel ausgewählt sind,
wobei der heterozyklische Ring möglicherweise mit 1, 2, 3 oder 4 gleichartigen oder verschiedenartigen Substituenten R¹⁰ versehen ist, und wobei das/die Stickstoff- und/oder Schwefelatom(e) des heterozyklischen Rings möglicherweise oxidiert sein kann/können;
R^{4a} und R^{4b} jeweils unabhängig voneinander und auf unabhängige Weise von der ganzen Zahl m aus der Gruppe ausgewählt sind, die aus Wasserstoff, Halogen, CN, C₁-C₆-Alkyl, C₁-C₆-Alkoxy, Di-(C₁-C₆-alkyl)amino, C₁-C₆-Alkylthio und C₃-C₈-Cycloalkyl besteht,
wobei jeder der fünf letztgenannten Reste unsubstituiert, teilweise oder vollständig halogeniert ist,
wobei es weiterhin möglich ist, dass R^{4b} aus C₁-C₆-Alkylsulfinyl, C₁-C₆-Alkylsulfonyl,
wobei jeder der beiden letztgenannten Reste unsubstituiert, teilweise oder vollständig halogeniert ist,
C₁-C₆-Alkyl oder C₃-C₈-Cycloalkyl,
wobei jeder der beiden letztgenannten Reste mit 1, 2 oder 3 gleichartigen oder verschiedenartigen Resten R⁷ versehen ist,
aus SH, S(O)ₙNR^{9a}R^{9b}, NR^{9a}R^{9b}, C(=O)OR⁸, C(=O)NR^{9a}R^{9b}, C(=S)NR^{9a}R^{9b}, C(=O)R^{7a}, C(=S)R^{7a}, aus einem Q¹-Phenyl-Baustein,
wobei der Phenylring möglicherweise mit 1, 2, 3, 4 oder 5 gleichartigen oder verschiedenartigen Substituenten R¹⁰ versehen ist,
und aus einem Q¹-Het#-Baustein ausgewählt ist, wobei
Q¹ für eine Einfachbindung, NR^{9a}, C₁-C₄-Alkylen oder NR^{9a}-C₁-C₄-Alkylen steht,
wobei das Heteroatom in dem letzteren Baustein an das Kohlenstoffatom von CR^{4a}R^{4b} gebunden ist;
oder
R^{4a} und R^{4b} zusammen mit dem Kohlenstoffatom, an welches sie gebunden sind, einen 3-, 4-, 5- oder 6-gliedrigen aliphatischen Ring bilden,
wobei die Kohlenstoffatome des Rings jeweils unsubstituiert, teilweise oder vollständig halogeniert sein können und/oder mit 1 oder 2 gleichartigen oder verschiedenartigen Resten versehen sein können, die aus C₁-C₄-Alkyl, C₁-C₄-Halogenalkyl, C₁-C₄-Alkoxy und C₁-C₄-Halogenalkoxy ausgewählt sind;
oder
R^{4a} und R^{4b} zusammen =O, =CR¹³R^{14,} =S, =NR¹⁷, =NOR¹⁶ oder =NNR^{9a}R^{9b} bilden können;
R⁵ aus der Gruppe ausgewählt ist, die aus Wasserstoff, Halogen, CN, C₁-C₆-Alkyl, C₁-C₆-Alkoxy, C₁-C₆-Alkylthio, C₁-C₆-Alkylsulfinyl, C₁-C₆-Alkylsulfonyl, C₂-C₆-Alkenyl, C₂-C₆-Alkenylthio, C₂-C₆-Alkenylsulfinyl, C₂-C₆-Alkenylsulfonyl, C₂-C₆-Alkinyl, C₂-C₆-Alkinylthio, C₂-C₆-Alkinylsulfinyl, C₂-C₆-Alkinylsulfonyl, C₃-C₈-Cycloalkyl, C₃-C₈-Cycloalkoxy, C₃-C₈-Cycloalkylthio, C₃-C₈-Cycloalkylsulfinyl und C₃-C₈-Cycloalkylsulfonyl,
wobei jeder der 18 letztgenannten Reste unsubstituiert, teilweise oder vollständig halogeniert ist und/oder mit 1, 2 oder 3 gleichartigen oder verschiedenartigen Resten R⁷ versehen ist, wobei es für Cycloalkylreste auch möglich ist, dass diese mit 1, 2, 3, 4, 5 oder 6 gleichartigen oder verschiedenartigen C₁-C₄-Alkylgruppen versehen sind,
aus S(O)ₙNR^{9a}R^{9b}, O-NR^{9a}R^{9b}, NR^{9a}R^{9b}, NR^{9a}NR^{9a}R^{9b}, C(=O)OR⁸, C(=O)NR^{9a}R^{9b}, C(=S)NR^{9a}R^{9b}, C(=O)R^{7a}, C(=S)R^{7a}, NR^{9a}-C(=O)R^{7a}, NR^{9a}-C(=S)R^{7a}, NR^{9a}C(=O)NR^{9a}R^{9b}, NR^{9a}C(=S)NR^{9a}R^{9b}, NR^{9a}-S(O)ₙR^{8a}, aus einem Q-Phenyl-Baustein,
wobei der Phenylring möglicherweise mit 1, 2, 3, 4 oder 5 gleichartigen oder verschiedenartigen Substituenten R¹⁰ versehen ist,
aus einem Y-R^{5a}-Baustein,
und aus einem Q-Het#-Baustein besteht,
wobei
Y für O, S, S(=O) oder S(=O)₂ steht;
R^{5a} aus der Gruppe ausgewählt ist, die aus C(=O)NR^{9a}R^{9b}, C(=S)NR^{9a}R^{9b}, C(=O)R⁷, C(=S)R⁷; aus Phenyl oder CH₂-Phenyl,
die möglicherweise mit einem oder mehreren, z.B. mit 1, 2, 3, 4 oder 5 gleichartigen oder verschiedenartigen Substituenten R¹⁰ versehen sind,
aus einem 3-, 4-, 5-, 6- oder 7-gliedrigen gesättigten, teilweise gesättigten oder ungesättigten aromatischen heterozyklischen Ring besteht, der 1, 2 oder 3 Heteroatome umfasst, die aus Sauerstoff, Stickstoff und/oder Schwefel ausgewählt sind, wobei sie möglicherweise mit 1, 2, 3 oder 4 gleichartigen oder verschiedenartigen Substituenten R^{y} versehen sind,
wobei das Stickstoff- und/oder das Schwefelatom des heterozyklischen Rings möglicherweise oxidiert sein kann/können, und
R^{y} aus der Gruppe ausgewählt ist, die aus Wasserstoff, Halogen, Cyano, Nitro, C₁-C₁₀-Alkyl, C₂-C₁₀-Alkenyl,
wobei die beiden letztgenannten Reste möglicherweise mit einem oder mehreren gleichartigen oder verschiedenartigen Resten R¹⁸ versehen sein können,
aus Phenyl,
welches möglicherweise mit Halogen, Cyano, Nitro, C₁-C₆-Alkyl, C₁-C₆-Halogenalkyl, C₁-C₆-Alkoxy oder C₁-C₆-Halogenalkoxy, C₁-C₄-Alkyl und C₁-C₄-Alkoxy substituiert ist, wobei beide der zwei letztgenannten aliphatischen Reste unsubstituiert, teilweise oder vollständig halogeniert sein können,
aus OR¹⁹, -S(O)ₙR¹⁹, S(O)ₙNR^{20a}R^{20b}, NR^{20a}R^{20b}, C(=O)R¹⁸, C(=O)OR¹⁹, -C(=NR^{20a})R¹⁸, C(=O)NR^{20a}R^{20b}, C(=S)NR^{20a}R^{20b} besteht,
oder
zwei R^{y}, die gemeinsam an einem Atom eines teilweise gesättigten heterozyklischen Bausteins vorliegen, =O, =CR^{21a}R^{21b}; =NR^{20a}, =NOR¹⁹ oder =NNR^{20a} darstellen können;
oder
zwei R^{y} an benachbarten Kohlenstoffatomen eine Brücke darstellen können, die aus CH₂CH₂CH₂CH₂, CH=CH-CH=CH, N=CH-CH=CH, CH=N-CH=CH, N=CH-N=CH, CH₂CH₂CH₂, CH=CHCH₂, CH₂CH₂NR^{17a}, CH₂CH=N, CH=CH-NR^{17a} ausgewählt ist, wobei sie zusammen mit dem Kohlenstoffatom, an welches die beiden R^{y} gebunden sind, einen 5-gliedrigen oder 6-gliedrigen kondensierten, teilweise gesättigten oder ungesättigten, aromatischen, carbozyklischen oder heterozyklischen Ring bilden, wobei der Ring möglicherweise mit einem oder zwei Substituenten versehen sein kann, die aus =O, OH, CH₃, OCH₃, Halogen, Cyano, C₁-C₄-Alkoxy, C₁-C₄-Halogenalkoxy, C₁-C₄-Alkyl und C₁-C₄-Halogenalkyl ausgewählt sind;
Q unabhängig davon, wo es vorkommt, für eine Einfachbindung CH₂, S, O, S(=O), S(=O)₂, NR^{9a},-O-S(=O)₂-, -NR^{9a}-S(=O)₂-, -O-C(=O)-, -NR^{9a}-C(=O)-, -O-C₁-C₄-Alkylen, -S-C₁-C₄-Alkylen, -NR^{9a}-C₁-C₄-Alkylen, -O-C(=O)-C₁-C₄-Alkylen oder -NR^{9a}-C(=O)-C₁-C₄-Alkylen steht,
wobei das Heteroatom in dem letzteren 7 Bausteinen an das Kohlenstoffatom von C(NO₂)R³ beziehungsweise CR^{4a}R^{4b} gebunden ist;
oder, wenn m gleich 1 ist,
R³ und R^{4a} weiterhin sowohl zusammen miteinander als auch zusammen mit der bestehenden Bindung eine Doppelbildung zwischen den benachbarten Kohlenstoffatomen bilden können;
oder, wenn m gleich 1 ist,
eines von R^{4a} oder R^{4b} zusammen mit R⁵ weiterhin mit dem Kohlenstoffatom, an welches sie gebunden sind, einen 3-, 4-, 5- oder 6-gliedrigen gesättigten oder teilweise ungesättigten Carbozyklus oder Heterozyklus bilden kann,
wobei die Kohlenstoffatome des Carbozyklus oder Heterozyklus jeweils unsubstituiert sein können oder mit 1 oder 2 gleichartigen oder verschiedenartigen Resten R^{7b} versehen sein können, und wobei der Heterozyklus 1 oder 2 nicht-benachbarte gleichartige oder verschiedenartige Heteroatome oder Heteroatombausteine als Ringglieder aufweist, welche aus O, S, N und N-R^{17c} ausgewählt sind;
oder, wenn m gleich 0 ist,
R³ oder R⁵ weiterhin zusammen mit dem Kohlenstoffatom, an welches sie gebunden sind, einen 3-, 4-, 5-oder 6-gliedrigen gesättigten oder teilweise ungesättigten Carbozyklus oder Heterozyklus bilden können, wobei die Kohlenstoffatome des Carbozyklus oder Heterozyklus jeweils unsubstituiert sein können oder mit 1 oder 2 gleichartigen oder verschiedenartigen Resten R^{7b} versehen sein können, und wobei der Heterozyklus 1 oder 2 nicht-benachbarte gleichartige oder verschiedenartige Heteroatome oder Heteroatombausteine als Ringglieder aufweist, welche aus O, S, N und N-R^{17c} ausgewählt sind, oder
R³ und R⁵ zusammen =O oder =S bilden können;
wobei, unabhängig davon, wo es vorkommt,
n für 0, 1 oder 2 steht,
R⁶ aus der Gruppe ausgewählt ist, die aus Halogen, Cyano, Azido, Nitro, SCN, SF₅, aus C₁-C₁₀-Alkyl, C₃-C₈-Cycloalkyl, C₂-C₁₀-Alkenyl, C₂-C₁₀-Alkinyl,
wobei die vier letztgenannten Reste jeweils teilweise oder vollständig halogeniert sein können und/oder möglicherweise unabhängig voneinander mit 1, 2 oder 3 gleichartigen oder verschiedenartigen Resten R⁷ substituiert sein können,
aus OR⁸, NR^{17a}R^{17b}, S(O)ₙR⁸, S(O)ₙNR^{17a}R^{17b}, C(=O)R^{7a}, C(=O)NR^{17a}R^{17b,} C(=O)OR⁸, C(=S)R^{7a}, C(=S)NR^{17a}R^{17b}, C(=S)OR⁸, C(=S)SR⁸, C(=NR¹⁷)R^{7a}, C(=NR¹⁷)NR^{17a}R^{17b}, Si(R¹¹)₂R¹²,
aus Phenyl, welches möglicherweise mit 1, 2, 3, 4 oder 5 gleichartigen oder verschiedenartigen Substituenten R¹⁰ versehen ist,
aus einem 3-, 4-, 5-, 6- oder 7-gliedrigen gesättigten, teilweise gesättigten oder ungesättigten aromatischen heterozyklischen Ring besteht, der 1, 2 oder 3 Heteroatome als Ringglieder umfasst, die gleichartig oder verschiedenartig sind und aus Sauerstoff, Stickstoff und Schwefel ausgewählt sind,
wobei der heterozyklische Ring möglicherweise mit 1, 2, 3 oder 4 gleichartigen oder verschiedenartigen Substituenten R¹⁰ versehen ist, und wobei das/die Stickstoff- und/oder Schwefelatom(e) des heterozyklischen Rings möglicherweise oxidiert sein kann/können;
oder zwei der R⁶, welche an ein und demselben Kohlenstoffring vorliegen, zusammen =O, =CR¹³R¹⁴, =S, =NR¹⁷, =NOR¹⁶, =NNR^{9a}R^{9b} bilden können;
oder zwei R⁶ zusammen eine geradkettige C₂-C₇-Alkylengruppe bilden, sodass sie zusammen mit dem/den Ringatom(en), an welche(s) sie gebunden sind, einen 3-, 4-, 5-, 6-, 7- oder 8-gliedrigen Ring bilden,
wobei 1 oder 2 CH₂-Bausteine der Alkylenkette durch 1 oder 2 Heteroatombausteine ersetzt sein können, welche aus O, S und NR^{17c} ausgewählt sind, und/oder
1 oder 2 der CH₂-Gruppen der Alkylenkette durch eine der Gruppen C=O, C=S und/oder C=NR¹⁷ ersetzt sein können; und
wobei die Alkylenkette unsubstituiert ist oder mit 1, 2, 3, 4, 5 oder 6 gleichartigen oder verschiedenartigen Resten substituiert sein kann, die aus der Gruppe ausgewählt sind, welche aus
Halogen, C₁-C₆-Halogenalkyl, C₁-C₆-Alkoxy, C₁-C₆-Halogenalkoxy, C₁-C₆-Alkylthio, C₁-C₆-Halogenalkylthio, C₃-C₈-Cycloalkyl, C₃-C₈-Halogencycloalkyl, C₂-C₆-Alkenyl, C₂-C₆-Halogenalkenyl, C₂-C₆-Alkinyl, C₂-C₆-Halogenalkinyl,
aus Phenyl, welches mit 1, 2, 3, 4 oder 5 gleichartigen oder verschiedenartigen Resten R¹⁰ substituiert sein kann, and aus einem 3-, 4-, 5-, 6- oder 7-gliedrigen gesättigten, teilweise ungesättigten oder aromatischen heterozyklischen Ring besteht, der 1, 2 oder 3 Heteroatome oder Heteroatomgruppen enthält, welche aus N, O, S, NO, SO und SO₂ ausgewählt sind und ringständig sind, wobei der heterozyklische Ring mit 1, 2, 3, 4 oder 5 gleichartigen oder verschiedenartigen Resten R¹⁰ substituiert sein kann;
R⁷ unabhängig davon, wo er vorkommt, aus der Gruppe ausgewählt ist, die aus Cyano, Azido, Nitro, -OH, -SH, -SCN, SF₅, C₁-C₆-Alkyl, C₁-C₆-Halogenalkyl, C₃-C₈-Cycloalkyl, C₃-C₈-Halogencycloalkyl, C₂-C₆-Alkenyl, C₂-C₆-Halogenalkenyl, C₂-C₆-Alkinyl, C₂-C₆-Halogenalkinyl, C₁-C₆-Alkoxy, C₁-C₆-Halogenalkoxy, C₁-C₆-Alkylthio, C₁-C₆-Alkylsulfinyl, C₁-C₆-Alkylsulfonyl, C₁-C₆-Halogenalkylthio, Si(R¹¹)₂R^{12,} OR⁸, OSO₂R⁸, S(O)ₙR⁸, S(O)ₙNR^{17a}R^{17b}, NR^{17a}R^{17b}, C(=O)NR^{17a}R^{17b,} C(=S)NR^{17a}R^{17b,} C(=O)OR⁸, C(=O)R¹⁵, C(=S)R¹⁵, C (=NR¹⁷) R¹⁵,
aus Phenyl, Phenyl-C₁-C₄-alkyl, Phenoxy,
wobei der Phenylring in den letzteren drei Resten unsubstituiert ist oder mit 1, 2, 3, 4 oder 5 gleichartigen oder verschiedenartigen Resten substituiert sein kann, die aus Halogen, C₁-C₆-Alkyl, Cyano, Azido, Nitro, C₁-C₁₀-Alkyl, C₁-C₆-Halogenalkyl, C₁-C₆-Alkoxy, C₁-C₆ Halogenalkoxy, (C₁-C₆-Alkoxy) carbonyl, (C₁-C₆-Alkyl) amino und di-(C₁-C₆-Alkyl) amino ausgewählt sind, und
aus einem 3-, 4-, 5-, 6- oder 7-gliedrigen gesättigten, teilweise gesättigten oder ungesättigten aromatischen heterozyklischen Ring besteht, der 1, 2 oder 3 Heteroatome als Ringglieder umfasst, die gleichartig oder verschiedenartig sind und aus Sauerstoff, Stickstoff und Schwefel ausgewählt sind,
wobei der heterozyklische Ring unsubstituiert ist oder mit 1, 2, 3 oder 4 gleichartigen oder verschiedenartigen Resten substituiert sein kann, die aus Halogen, C₁-C₆-Alkyl, Cyano, Azido, Nitro, C₁-C₁₀-Alkyl, C₁-C₆-Halogenalkyl, C₁-C₆-Alkoxy, C₁-C₆-Halogenalkoxy, (C₁-C₆-Alkoxy) carbonyl, (C₁-C₆-Alkyl) amino und di-(C₁-C₆-Alkyl) amino ausgewählt sind, und
wobei das Stickstoff- und/oder das Schwefelatom des heterozyklischen Rings möglicherweise oxidiert sein kann/können,
oder
zwei R⁷, welche an ein und demselben Kohlenstoffatom vorliegen, zusammen =O, =CR¹³R¹⁴, =S, =NR¹⁷, =NOR¹⁶, =NNR^{9a}R^{9b} bilden können;
oder
zwei R⁷ zusammen mit den Kohlenstoffatomen, an welche die beiden R⁷ gebunden sind, einen 3-, 4-, 5-, 6-, 7- oder 8-gliedrigen gesättigten oder teilweise ungesättigten carbozyklischen oder heterozyklischen Ring bilden können, wobei der heterozyklische Ring 1, 2 oder 3 Heteroatome als Ringglieder umfasst, welche gleichartig oder verschiedenartig sind und aus Sauerstoff, Stickstoff und Schwefel ausgewählt sind,
wobei der heterozyklische Ring unsubstituiert ist oder mit 1, 2, 3 oder 4 gleichartigen oder verschiedenartigen Resten substituiert sein kann, die aus Halogen, C₁-C₆-Alkyl, Cyano, Azido, Nitro, C₁-C₁₀-Alkyl, C₁-C₆-Halogenalkyl, C₁-C₆-Alkoxy, C₁-C₆ Halogenalkoxy, (C₁-C₆-Alkoxy) carbonyl, (C₁-C₆-Alkyl) amino und di-(C₁-C₆-Alkyl) amino ausgewählt sind;
R^{7a} unabhängig davon, wo er vorkommt, aus der Gruppe ausgewählt ist, die aus
Wasserstoff, C₁-C₆-Alkyl, C₁-C₆-Halogenalkyl, C₁-C₆-Alkoxy, C₁-C₆-Halogenalkoxy, C₁-C₆-Alkylthio, C₁-C₆-Alkylsulfinyl, C₁-C₆-Alkylsulfonyl, C₁-C₆-Halogenalkylthio, C₃-C₈-Cycloalkyl, C₃-C₈-Halogencycloalkyl, C₃-C₆-Cycloalkyl-C₁-C₄-alkyl, C₂-C₆-Alkenyl, C₂-C₆-Halogenalkenyl, C₂-C₆-Alkinyl, C₂-C₆-Halogenalkinyl,
aus Phenyl, welches möglicherweise mit 1, 2, 3, 4 oder 5 gleichartigen oder verschiedenartigen Substituenten R¹⁰ versehen ist,
aus Benzyl, wobei der Phenylbaustein des Benzyls unsubstituiert ist oder mit 1, 2 oder 3 gleichartigen oder verschiedenartigen Resten substituiert ist, welche aus der Gruppe ausgewählt sind, die aus Halogen, CN, C₁-C₄-Alkyl, C₁-C₄-Halogenalkyl, C₁-C₄-Alkoxy und C₁-C₄-Halogenalkoxy besteht, und
aus einem 3-, 4-, 5-, 6- oder 7-gliedrigen gesättigten, teilweise gesättigten oder ungesättigten aromatischen heterozyklischen Ring besteht, der 1, 2 oder 3 Heteroatome als Ringglieder umfasst, die gleichartig oder verschiedenartig sind und aus Sauerstoff, Stickstoff und Schwefel ausgewählt sind,
wobei der heterozyklische Ring möglicherweise mit 1, 2, 3 oder 4 gleichartigen oder verschiedenartigen Substituenten R¹⁰ versehen ist, und wobei das Stickstoff- und/oder das Schwefelatom des heterozyklischen Rings möglicherweise oxidiert sein kann/können,
R^{7b} aus der Gruppe ausgewählt ist, die aus Halogen, Cyano, C₁-C₁₀-Alkyl, C₃-C₈-Cycloalkyl, C₂-C₁₀-Alkenyl, C₂-C₁₀-Alkinyl,
wobei jeder der vier letztgenannten Reste unsubstituiert, teilweise oder vollständig halogeniert ist und/oder möglicherweise mit 1, 2 oder 3 gleichartigen oder verschiedenartigen Resten R⁷ substituiert sein kann,
aus OR⁸, NR^{17a}R^{17b,} S(O)ₙR⁸, S(O)ₙNR^{17a}R^{17b}, C(=O)R^{7a}, C(=O)NR^{17a}R^{17b}, C(=O)OR⁸, C(=S)R^{7a}, C(=S)NR^{17a}R^{17b}, C(=S)OR⁸, C(=NR¹⁷)R^{7a}, C(=NR¹⁷)NR^{17a}R^{17b}, Si(R¹¹)₂R¹²; aus Phenyl, welches möglicherweise mit 1, 2, 3, 4 oder 5 gleichartigen oder verschiedenartigen Substituenten R¹⁰ versehen ist,
aus einem 3-, 4-, 5-, 6- oder 7-gliedrigen gesättigten, teilweise gesättigten oder ungesättigten aromatischen heterozyklischen Ring besteht, der 1, 2 oder 3 Heteroatome als Ringglieder umfasst, die gleichartig oder verschiedenartig sind und aus Sauerstoff, Stickstoff und Schwefel ausgewählt sind,
wobei der heterozyklische Ring möglicherweise mit 1, 2, 3 oder 4 gleichartigen oder verschiedenartigen Substituenten R¹⁰ substituiert ist, und wobei das/die Stickstoff- und/oder Schwefelatom(e) des heterozyklischen Rings möglicherweise oxidiert sein kann/können,
oder
zwei der R^{7b}, welche an ein und demselben ringständigen Kohlenstoff vorliegen, zusammen =O, =S oder =CR¹³R¹⁴ bilden können;
oder
zwei R^{7b} zusammen eine geradkettige C₂-C₇-Alkylengruppe bilden, sodass sie zusammen mit dem/den Ringatom(en), an welche(s) sie gebunden sind, einen 3-, 4-, 5-, 6-, 7- oder 8-gliedrigen Ring bilden,
wobei 1 oder 2 CH₂-Bausteine der Alkylenkette durch 1 oder 2 Heteroatombausteine ersetzt sein können, welche aus O, S und NR^{17c} ausgewählt sind, und/oder
1 oder 2 der CH₂-Gruppen der Alkylenkette durch eine der Gruppen C=O, C=S und/oder C=NR¹⁷ ersetzt sein können; und
wobei die Alkylenkette unsubstituiert ist oder mit 1, 2, 3, 4, 5 oder 6 gleichartigen oder verschiedenartigen Resten substituiert sein kann, die aus der Gruppe ausgewählt sind, welche aus
Halogen, C₁-C₄-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₃-C₈-Cycloalkyl, C₃-C₈-Halogericycloalkyl, C₂-C₆-Alkenyl, C₂-C₆-Halogenalkenyl, C₂-C₆-Alkinyl, C₂-C₆-Halogenalkinyl,
aus Phenyl, welches möglicherweise mit 1, 2, 3, 4 oder 5 gleichartigen oder verschiedenartigen Resten R¹⁰ substituiert ist, und
aus einem 3-, 4-, 5-, 6- oder 7-gliedrigen gesättigten, teilweise ungesättigten oder aromatischen heterozyklischen Ring besteht, der 1, 2 oder 3 Heteroatome oder Heteroatomgruppen enthält, welche aus N, O, S, NO, SO und SO₂ ausgewählt sind und ringständig sind, wobei der heterozyklische Ring mit 1, 2, 3, 4 oder 5 gleichartigen oder verschiedenartigen Resten R¹⁰ substituiert sein kann;
R⁸ unabhängig davon, wo er vorkommt, aus der Gruppe ausgewählt ist, die aus Wasserstoff, C₁-C₆-Alkyl, C₁-C₆-Halogenalkyl, C₃-C₈-Cycloalkyl, C₃-C₈-Cycloalkyl-C₁-C₄-alkyl, C₃-C₈-Halogencycloalkyl, C₂-C₆-Alkenyl, C₂-C₆-Halogenalkenyl, C₂-C₆-Alkinyl, C₂-C₆ Halogenalkinyl, C(=O)R¹⁵, C(=O)NR^{17a}R^{17b}, C(=S)NR^{17a}R^{17b}, C(=O)OR¹⁶,
aus Phenyl, Phenyl-C₁-C₄-alkyl, wobei der Phenylring in den beiden letztgenannten Resten unsubstituiert oder möglicherweise mit 1, 2, 3, 4 oder 5 gleichartigen oder verschiedenartigen Substituenten R¹⁰ versehen ist, und
aus einem 3-, 4-, 5-, 6- oder 7-gliedrigen gesättigten, teilweise gesättigten oder ungesättigten aromatischen heterozyklischen Ring besteht, der 1, 2 oder 3 Heteroatome als Ringglieder umfasst, die gleichartig oder verschiedenartig sind und aus Sauerstoff, Stickstoff und Schwefel ausgewählt sind,
wobei der heterozyklische Ring möglicherweise mit 1, 2, 3 oder 4 gleichartigen oder verschiedenartigen Substituenten R¹⁰ versehen ist, und
wobei das Stickstoff- und/oder das Schwefelatom des heterozyklischen Rings möglicherweise oxidiert sein kann/können,
R^{8a} unabhängig davon, wo er vorkommt, aus der Gruppe ausgewählt ist, die aus C₁-C₆-Alkyl, C₃-C₈-Cycloalkyl, C₃-C₈-Cycloalkyl-C₁-C₄-alkyl, C₂-C₆-Alkenyl, C₂-C₆-Alkinyl besteht,
wobei jeder der fünf letztgenannten Reste unsubstituiert oder teilweise oder vollständig halogeniert ist,
aus Phenyl, Benzyl,
wobei der Phenylring in den beiden letztgenannten Resten unsubstituiert ist oder mit 1, 2, 3, 4 oder 5 gleichartigen oder verschiedenartigen Substituenten R¹⁰ versehen sein kann,
und aus einem 3-, 4-, 5-, 6- oder 7-gliedrigen gesättigten, teilweise gesättigten oder ungesättigten aromatischen heterozyklischen Ring besteht, der 1, 2 oder 3 Heteroatome als Ringglieder umfasst, die gleichartig oder verschiedenartig sind und aus Sauerstoff, Stickstoff und Schwefel ausgewählt sind,
wobei der heterozyklische Ring möglicherweise mit 1, 2, 3 oder 4 gleichartigen oder verschiedenartigen Substituenten R¹⁰ versehen ist, und wobei das Stickstoff- und/oder das Schwefelatom des heterozyklischen Rings möglicherweise oxidiert sein kann/können,
R⁹ unabhängig davon, wo er vorkommt, aus der Gruppe ausgewählt ist, die aus Wasserstoff, C₁-C₆-Alkyl, C₁-C₆-Halogenalkyl, C₁-C₆-Alkoxy, C₁-C₆-Halogenalkoxy, C₃-C₈-Cycloalkyl, C₃-C₈-Cycloalkyl-C₁-C₄-alkyl, C₃-C₈-Halogencycloalkyl, C₂-C₆-Alkenyl, C₂-C₆-Halogenalkenyl, C₂-C₆-Alkinyl, C₂-C₆ Halogenalkinyl,
aus Phenyl, welches möglicherweise mit 1, 2, 3, 4 oder 5 gleichartigen oder verschiedenartigen Substituenten R¹⁰ versehen ist;
aus einem 3-, 4-, 5-, 6- oder 7-gliedrigen gesättigten, teilweise gesättigten oder ungesättigten, aromatischen, C-gebundenen, heterozyklischen Ring besteht, der 1, 2 oder 3 Heteroatome als Ringglieder umfasst, die gleichartig oder verschiedenartig sind und aus Sauerstoff, Stickstoff und Schwefel ausgewählt sind,
wobei der heterozyklische Ring möglicherweise mit 1, 2, 3 oder 4 gleichartigen oder verschiedenartigen Substituenten R¹⁰ versehen ist, und
wobei das Stickstoff- und/oder das Schwefelatom des heterozyklischen Rings möglicherweise oxidiert sein kann/können,
R^{9a} und R^{9b} jeweils unabhängig voneinander aus der Gruppe ausgewählt sind, die aus
Wasserstoff, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₃-C₈-Cycloalkyl, C₃-C₈-Halogencycloalkyl, C₃-C₈-Cycloalkyl-C₁-C₄-alkyl, C₂-C₆-Alkenyl, C₂-C₆-Halogenalkenyl, C₂-C₆-Alkinyl, C₂-C₆ Halogenalkinyl,
aus S(O)ₙR¹⁶, -S(O)ₙNR^{17a}R^{17b}, C(=O)R¹⁵, C(=O)OR¹⁶, C(=O)NR^{17a}R^{17b}, C(=S)R¹⁵, C(=S)SR¹⁶, C(=S)NR^{17a}R^{17b}, C(=NR¹⁷)R¹⁵;
aus Phenyl, Benzyl, 1-Phenethyl oder 2-Phenethyl,
wobei der Phenylring in den vier letztgenannten Resten unsubstituiert ist oder mit 1, 2, 3, 4 oder 5 gleichartigen oder verschiedenartigen Substituenten R¹⁰ versehen sein kann; und
aus einem 3-, 4-, 5-, 6- oder 7-gliedrigen gesättigten, teilweise gesättigten oder ungesättigten, aromatischen, C-gebundenen, heterozyklischen Ring besteht, der 1, 2 oder 3 Heteroatome als Ringglieder umfasst, die gleichartig oder verschiedenartig sind und aus Sauerstoff, Stickstoff und Schwefel ausgewählt sind,
wobei der heterozyklische Ring möglicherweise mit 1, 2, 3 oder 4 gleichartigen oder verschiedenartigen Substituenten R¹⁰ versehen ist, und wobei das Stickstoff- und/oder das Schwefelatom des heterozyklischen Rings möglicherweise oxidiert sein kann/können,
oder
R^{9a} und R^{9b} zusammen eine C₂-C₇-Alkylenkette darstellen und gemeinsam mit dem Stickstoffatom, an welches sie gebunden sind, einen 3-, 4-, 5-, 6-, 7- oder 8-gliedrigen gesättigten, teilweise gesättigten oder ungesättigten aromatischen Ring bilden,
wobei die Alkylenkette ein oder zwei Heteroatome enthalten kann, die jeweils unabhängig voneinander aus Sauerstoff, Schwefel oder Stickstoff ausgewählt sind, und
wobei die Alkylenkette möglicherweise mit 1, 2, 3 oder 4 gleichartigen oder verschiedenartigen Resten substituiert sind kann, die aus
Halogen, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₃-C₈-Cycloalkyl, C₃-C₈-Halogencycloalkyl, C₂-C₆-Alkenyl, C₂-C₆-Halogenalkenyl, C₂-C₆-Alkinyl, C₂-C₆-Halogenalkinyl,
aus Phenyl, welches möglicherweise mit 1, 2, 3, 4 oder 5 gleichartigen oder verschiedenartigen Substituenten R¹⁰ versehen ist, und
aus einem 3-, 4-, 5-, 6- oder 7-gliedrigen gesättigten, teilweise gesättigten oder ungesättigten, aromatischen, C-gebundenen, heterozyklischen Ring ausgewählt sind, der 1, 2 oder 3 Heteroatome als Ringglieder umfasst, die gleichartig oder verschiedenartig sind und aus Sauerstoff, Stickstoff und Schwefel ausgewählt sind,
wobei der heterozyklische Ring möglicherweise mit 1, 2, 3 oder 4 gleichartigen oder verschiedenartigen Substituenten R¹⁰ versehen ist, und
wobei das Stickstoff- und/oder das Schwefelatom des heterozyklischen
Rings möglicherweise oxidiert sein kann/können,
oder
R^{9a} und R^{9b} gemeinsam Bausteine der Art =CR¹³R¹⁴, =NR¹⁷, =NOR¹⁶, =NNR^{17a}R^{17b} bilden können;
R¹⁰ unabhängig davon, wo er vorkommt, aus der Gruppe ausgewählt ist, die aus Halogen, Cyano, Azido, Nitro, SCN, SF₅, C₁-C₁₀-Alkyl, C₁-C₄-Halogenalkyl, C₃-C₈-Cycloalkyl, C₂-C₁₀-Alkenyl, C₂-C₁₀-Alkinyl,
wobei die vier letztgenannten Reste jeweils möglicherweise mit 1, 2, 3, 4 oder 5 gleichartigen oder verschiedenartigen Resten R⁷ substituiert sein können,
aus Si(R¹¹)₂R¹², OR¹⁶, OS(O)ₙR¹⁶, -S(O)ₙR¹⁶, S(O)ₙNR^{17a}R^{17b}, NR^{17a}R^{17b}, C(=O)R¹⁵, C(=S)R¹⁵, C(=O)OR¹⁶, -C(=NR¹⁷)R¹⁵, C(=O)NR^{17a}R^{17b}, C(=S)NR^{17a}R^{17b}, aus Phenyl,
welches möglicherweise mit 1, 2, 3, 4 oder 5 gleichartigen oder verschiedenartigen Resten substituiert ist, die aus OH, Halogen, Cyano, Nitro, C₁-C₆-Alkyl, C₁-C₆-Halogenalkyl, C₁-C₆-Alkoxy und C₁-C₆-Halogenalkoxy ausgewählt sind, und
aus einem 3-, 4-, 5-, 6- oder 7-gliedrigen gesättigten, teilweise gesättigten oder ungesättigten aromatischen heterozyklischen Ring besteht, der 1, 2 oder 3 Heteroatome als Ringglieder umfasst, die gleichartig oder verschiedenartig sind und aus Sauerstoff, Stickstoff und Schwefel ausgewählt sind,
wobei der heterozyklische Ring unsubstituiert ist oder mit 1, 2, 3, 4 oder 5 gleichartigen oder verschiedenartigen Substituenten versehen sein kann, die aus Halogen, Cyano, NO₂, C₁-C₆-Alkyl, C₁-C₆-Halogenalkyl, C₁-C₆-Alkoxy und C₁-C₆-Halogenalkoxy ausgewählt sind, und
wobei das Stickstoff- und/oder das Schwefelatom des heterozyklischen Rings möglicherweise oxidiert sein kann/können,
oder
zwei R¹⁰, die an ein und demselben ringständigen Kohlenstoffatom eines gesättigten oder teilweise ungesättigten heterozyklischen Rests vorliegen, =O, =CR¹³R¹⁴, =S, =NR¹⁷, =NOR¹⁶, =NNR^{17a}R^{17b} bilden können;
oder
zwei R¹⁰, die an benachbarten ringständigen Kohlenstoffatomen vorliegen, zusammen einen zweibindigen Rest darstellen können, der aus CH₂CH₂CH₂CH₂, CH=CH-CH=CH, N=CH-CH=CH, CH=N-CH=CH, N=CH-N=CH, OCH₂CH₂CH₂, OCH=CHCH₂, CH₂OCH₂CH₂, OCH₂CH₂O, OCH₂OCH₂, CH₂CH₂CH₂, CH=CHCH₂, CH₂CH₂O, CH=CHO, CH₂OCH₂, CH₂C(=O)O, C(=O)OCH₂, O(CH₂)O, SCH₂CH₂CH₂, SCH=CHCH₂, CH₂SCH₂CH₂, SCH₂CH₂S, SCH₂SCH₂, CH₂CH₂S, CH=CHS, CH₂SCH₂, CH₂C(=S)S, C(=S)SCH₂, S(CH₂)S, CH₂CH₂NR¹⁷, CH₂CH=N, CH=CH-NR¹⁷, OCH=N, SCH=N ausgewählt ist, und zusammen mit dem benachbarten ringständigen Kohlenstoffatomen einen 5-gliedrigen oder 6-gliedrigen teilweise gesättigten oder ungesättigten, aromatischen, carbozyklischen oder heterozyklischen Ring bilden, wobei der Ring möglicherweise mit einem oder zwei Substituenten versehen sein kann, die auf unabhängige Weise aus =O, OH, CH₃, OCH₃, Halogen, Cyano, Halogenmethyl und Halogenmethoxy ausgewählt sind;
R¹¹ und R¹², unabhängig davon, wo sie vorkommen, aus der der Gruppe ausgewählt sind, die aus C₁-C₆-Alkyl, C₁-C₆-Halogenalkyl, C₁-C₆-Alkoxy, C₁-C₆-Alkoxy-C₁-C₄-alkyl, C₂-C₆-Alkenyl, C₂-C₆-Halogenalkenyl, C₂-C₆-Alkinyl, C₂-C₆-Halogenalkinyl, C₃-C₈-Cycloalkyl, C₃-C₈-Halogencycloalkyl, C₃-C₈-Cycloalkyl-C₁-C₄-alkyl, C₃-C₈-Halogencycloalkyl-C₁-C₄-alkyl, C₁-C₆-Halogenalkoxy-C₁-C₄-alkyl,
aus Phenyl und Benzyl besteht,
wobei der Phenylring in den beiden letztgenannten Resten unsubstituiert oder mit 1, 2, 3, 4 oder 5 gleichartigen oder verschiedenartigen Resten substituiert ist, die aus Halogen, OH, Cyano, NO₂, C₁-C₆-Alkyl, C₁-C₆-Halogenalkyl, C₁-C₆-Alkoxy und C₁-C₆-Halogenalkoxy ausgewählt sind;
R¹³ und R¹⁴ unabhängig davon, wo sie vorkommen, aus der Gruppe ausgewählt sind, die aus Wasserstoff, Halogen, CN, C₁-C₆-Alkyl, C₃-C₆-Cycloalkyl, C₁-C₄-Alkoxy-C₁-C₄-Alkyl,
aus Phenyl und Benzyl besteht;
R¹⁵ unabhängig davon, wo er vorkommt, aus der Gruppe ausgewählt ist, die aus
aus Wasserstoff, C₁-C₆-Alkyl, C₂-C₆-Alkenyl, C₂-C₆-Alkinyl, C₃-C₈-Cycloalkyl besteht,
wobei die vier letztgenannten aliphatischen und cycloaliphatischen Reste unsubstituiert, teilweise oder vollständig halogeniert und/oder mit Sauerstoff umgesetzt sein können und/oder mit 1 oder 2 gleichartigen oder verschiedenartigen Resten versehen sein können, die aus C₁-C₄-Alkoxy; C₃-C₆-Cycloalkyl-C₁-C₄-alkyl, Phenyl, Benzyl und Pyridyl ausgewählt sind,
wobei die drei letzteren Reste unsubstituiert, teilweise oder vollständig halogeniert sein können und/oder mit 1, 2 oder 3 gleichartigen oder verschiedenartigen Substituenten versehen sein können, die aus C₁-C₆-Alkyl, C₁-C₆-Halogenalkyl, C₁-C₆-Alkoxy, C₁-C₆ Halogenalkoxy, (C₁-C₆-Alkoxy) carbonyl, (C₁-C₆-Alkyl) amino und di-(C₁-C₆-Alkyl) amino ausgewählt sind;
R¹⁶ unabhängig davon, wo er vorkommt, aus der Gruppe ausgewählt ist, die aus Wasserstoff, C₁-C₆-Alkyl, C₂-C₆-Alkenyl, C₂-C₆-Alkinyl, C₃-C₈-Cycloalkyl, C₃-C₈-Cycloalkyl-C₁-C₄-alkyl,
wobei die fünf letztgenannten aliphatischen und cycloaliphatischen Reste unsubstituiert, teilweise oder vollständig halogeniert und/oder mit Sauerstoff umgesetzt sein können und/oder mit 1 oder 2 Resten versehen sein können, die aus C₁-C₄-Alkoxy ausgewählt sind,
aus Phenyl, Benzyl und Pyridyl besteht,
wobei die drei letzteren Reste unsubstituiert, teilweise oder vollständig halogeniert sein können und/oder mit 1, 2 oder 3 gleichartigen oder verschiedenartigen Substituenten versehen sein können, die aus C₁-C₆-Alkyl, C₁-C₆-Halogenalkyl, C₁-C₆-Alkoxy, C₁-C₆ Halogenalkoxy, (C₁-C₆-Alkoxy) carbonyl, (C₁-C₆-Alkyl) amino und di-(C₁-C₆-Alkyl) amino ausgewählt sind;
R¹⁷ unabhängig davon, wo er vorkommt, aus der Gruppe ausgewählt ist, die aus
Wasserstoff, C₁-C₆-Alkoxy, C₁-C₆-Halogenalkoxy, C₁-C₆-Alkylthio, Trimethylsilyl, Triethylsilyl, tert.-Butyldimethylsilyl, C₁-C₆-Alkyl, C₂-C₆-Alkenyl, C₂-C₆-Alkinyl, C₃-C₈-Cycloalkyl,
wobei die vier letztgenannten Reste unsubstituiert, teilweise oder vollständig halogeniert und/oder mit Sauerstoff umgesetzt sein können und/oder mit 1 oder 2 Resten versehen sein können, die aus C₁-C₄-Alkoxy ausgewählt sind,
aus C₃-C₆-Cycloalkyl-C₁-C₄-alkyl, Phenyl, Benzyl, Pyridyl, Phenoxy besteht,
wobei die vier letztgenannten Reste unsubstituiert, teilweise oder vollständig halogeniert sein können und/oder mit 1, 2 oder 3 gleichartigen oder verschiedenartigen Substituenten versehen sein können, die aus C₁-C₆-Alkyl, C₁-C₆-Halogenalkyl, C₁-C₆-Alkoxy, C₁-C₆ Halogenalkoxy und (C₁-C₆-Alkoxy)carbonyl ausgewählt sind;
R^{17a} und R^{17b} jeweils unabhängig voneinander aus der Gruppe ausgewählt sind, die aus Wasserstoff, C₁-C₆-Alkoxy, C₁-C₆-Halogenalkoxy, C₁-C₆-Alkylthio, C₁-C₆-Alkylsulfinyl, C₁-C₆-Alkylsulfonyl, C₁-C₆-Halogenalkylthio, Trimethylsilyl, Triethylsilyl, tert.-Butyldimethylsilyl, C₁-C₆-Alkyl, C₂-C₆-Alkenyl, C₂-C₆-Alkinyl, C₃-C₈-Cycloalkyl,
wobei die vier letztgenannten Reste unsubstituiert, teilweise oder vollständig halogeniert und/oder mit Sauerstoff umgesetzt sein können und/oder mit 1 oder 2 gleichartigen oder verschiedenartigen Resten versehen sein können, die aus C₁-C₄-Alkoxy ausgewählt sind,
aus Phenyl, Benzyl, Pyridyl, Phenoxy besteht,
wobei die vier letztgenannten Reste unsubstituiert, teilweise oder vollständig halogeniert sein können und/oder mit 1, 2 oder 3 gleichartigen oder verschiedenartigen Substituenten versehen sein können, die aus C₁-C₆-Alkyl, C₁-C₆-Halogenalkyl, C₁-C₆-Alkoxy, C₁-C₆ Halogenalkoxy und (C₁-C₆-Alkoxy)carbonyl ausgewählt sind;
oder
R^{17a} und R^{17b} zusammen eine C₂-C₆-Alkylenkette darstellen können, die zusammen mit dem Stickstoffatom, an welches R^{17a} und R^{17b} gebunden sind, einen 3- bis 7-gliedrigen, gesättigten, teilweise gesättigten oder ungesättigten Ring bildet,
wobei die Alkylenkette 1 oder 2 Heteroatome enthalten kann, die jeweils unabhängig voneinander aus Sauerstoff, Schwefel und Stickstoff ausgewählt sind und möglicherweise mit Halogen, C₁-C₄-Halogenalkyl, C₁-C₄-Alkoxy oder C₁-C₄-Halogenalkoxy substituiert sein können, und wobei das Stickstoff- und/oder das Schwefelatom des heterozyklischen Rings möglicherweise oxidiert sein kann/können;
oder
R^{17a} und R^{17b} gemeinsam Bausteine der Art =CR¹³R¹⁴, =NR¹⁷ oder =NOR¹⁶ bilden können;
R^{17c} unabhängig davon, wo er vorkommt, aus der Gruppe ausgewählt ist, die aus
Wasserstoff, CN, C₁-C₆-Alkyl, C₂-C₆-Alkenyl, C₂-C₆-Alkinyl, C₃-C₈-Cycloalkyl, C₃-C₈-Cycloalkyl-C₁-C₄-alkyl,
wobei die fünf letztgenannten Reste unsubstituiert, teilweise oder vollständig halogeniert und/oder mit Sauerstoff umgesetzt sein können und/oder mit 1 oder 2 Resten versehen sein können, die aus C₁-C₄-Alkoxy ausgewählt sind,
aus Phenyl, Benzyl und Pyridyl besteht, wobei die drei letzteren Reste unsubstituiert, teilweise oder vollständig halogeniert sein können und/oder mit 1, 2 oder 3 gleichartigen oder verschiedenartigen Substituenten versehen sein können, die aus C₁-C₆-Alkyl, C₁-C₆-Halogenalkyl, C₁-C₆-Alkoxy, C₁-C₆-Halogenalkoxy, (C₁-C₆-Alkoxy)carbonyl, (C₁-C₆-Alkyl) amino und di-(C₁-C₆-Alkyl)amino ausgewählt sind;
R¹⁸ jeweils unabhängig voneinander aus der Gruppe ausgewählt ist, die aus
Wasserstoff, Halogen, Cyano, Nitro, OH, SH, SCN, SF₅, C₁-C₆-Alkoxy, C₁-C₆-Halogenalkoxy, C₁-C₆-Alkylthio, C₁-C₆-Alkylsulfinyl, C₁-C₆-Alkylsulfonyl, C₁-C₆-Halogenalkylthio, Trimethylsilyl, Triethylsilyl, *tert.-*Butyldimethylsilyl, C₁-C₆-Alkyl, C₂-C₆-Alkenyl, C₂-C₆-Alkinyl, C₃-C₈-Cycloalkyl,
wobei die vier letztgenannten aliphatischen und cycloaliphatischen Reste unsubstituiert, teilweise oder vollständig halogeniert und/oder mit Sauerstoff umgesetzt sein können und/oder mit 1 oder 2 Resten versehen sein können, die aus C₁-C₄-Alkoxy ausgewählt sind;
aus Phenyl, Benzyl, Pyridyl, Phenoxy besteht,
wobei die vier letzteren Reste unsubstituiert, teilweise oder vollständig halogeniert sein können und/oder mit 1, 2 oder 3 Substituenten versehen sein können, die aus C₁-C₆-Alkyl, C₁-C₆-Halogenalkyl, C₁-C₆-Alkoxy, C₁-C₆ Halogenalkoxy, (C₁-C₆-Alkoxy)carbonyl, (C₁-C₆-Alkyl)amino oder di-(C₁-C₆-Alkyl)amino ausgewählt sind,
oder
zwei R¹⁸, die an ein und demselben Kohlenstoffatome vorliegen, zusammen =O, =CH(C₁-C₄), =C(C₁-C₄-Alkyl)C₁-C₄-alkyl, =N(C₁-C₆-Alkyl) oder =NO(C₁-C₆-Alkyl) darstellen können;
R¹⁹ jeweils unabhängig voneinander aus der Gruppe ausgewählt ist, die aus
Wasserstoff, Cyano, C₁-C₆-Alkoxy, C₁-C₆-Halogenalkoxy, C₁-C₆-Alkylthio, C₁-C₆-Alkylsulfinyl, C₁-C₆-Alkylsulfonyl, C₁-C₆-Halogenalkylthio, Trimethylsilyl, Triethylsilyl, tert.-Butyldimethylsilyl, C₁-C₆-Alkyl, C₂-C₆-Alkenyl, C₂-C₆-Alkinyl, C₃-C₈-Cycloalkyl,
wobei die vier letztgenannten Reste unsubstituiert, teilweise oder vollständig halogeniert und/oder mit Sauerstoff umgesetzt sein können und/oder mit 1 oder 2 Resten versehen sein können, die aus C₁-C₄-Alkoxy ausgewählt sind,
aus Phenyl, Benzyl, Pyridyl, Phenoxy besteht,
wobei die vier letzteren Reste unsubstituiert, teilweise oder vollständig halogeniert sein können und/oder mit 1, 2 oder 3 Substituenten versehen sein können, die aus C₁-C₆-Alkyl, C₁-C₆-Halogenalkyl, C₁-C₆-Alkoxy, C₁-C₆ Halogenalkoxy oder (C₁-C₆-Alkoxy)carbonyl ausgewählt sind;
R^{20a}, R^{20b} jeweils unabhängig voneinander aus der Gruppe ausgewählt sind, die aus Wasserstoff, Cyano, C₁-C₆-Alkoxy, C₁-C₆-Halogenalkoxy, C₁-C₆-Alkylthio, C₁-C₆-Alkylsulfinyl, C₁-C₆-Alkylsulfonyl, C₁-C₆-Halogenalkylthio, Trimethylsilyl, Triethylsilyl, *tert.-*Butyldimethylsilyl, C₁-C₆-Alkyl, C₂-C₆-Alkenyl, C₂-C₆-Alkinyl, C₃-C₈-Cycloalkyl,
wobei die vier letztgenannten aliphatischen und cycloaliphatischen Reste unsubstituiert, teilweise oder vollständig halogeniert und/oder mit Sauerstoff umgesetzt sein können und/oder mit 1 oder 2 gleichartigen oder verschiedenartigen Resten versehen sein können, die aus C₁-C₄-Alkoxy ausgewählt sind,
aus Phenyl, Benzyl, Pyridyl, Phenoxy besteht,
wobei die vier letztgenannten Reste unsubstituiert, teilweise oder vollständig halogeniert sein können und/oder mit 1, 2 oder 3 gleichartigen oder verschiedenartigen Substituenten versehen sein können, die aus C₁-C₆-Alkyl, C₁-C₆-Halogenalkyl, C₁-C₆-Alkoxy, C₁-C₆ Halogenalkoxy und (C₁-C₆-Alkoxy)carbonyl ausgewählt sind;
oder
R^{20a} und R^{20b} zusammen eine C₂-C₆-Alkylenkette darstellen können, die zusammen mit dem Stickstoffatom, an welches R^{20a} und R^{20b} gebunden sind, einen 3- bis 7-gliedrigen, gesättigten, teilweise gesättigten oder ungesättigten Ring bildet, wobei die Alkylenkette 1 oder 2 Heteroatome enthalten kann, die aus Sauerstoff, Schwefel und Stickstoff ausgewählt sind und möglicherweise mit Halogen, C₁-C₄-Halogenalkyl, C₁-C₄-Alkoxy oder C₁-C₄-Halogenalkoxy substituiert sein können, und wobei das Stickstoff- und/oder das Sauerstoffatom des heterozyklischen Rings möglicherweise oxidiert sein kann/können;
R^{21a}, R^{21b} unabhängig voneinander aus der Gruppe ausgewählt sind, die aus Wasserstoff, C₁-C₄-Alkyl, C₁-C₆-Cycloalkyl, C₁-C₄-Alkoxyalkyl, Phenyl und Benzyl besteht;
Het# für einen 3-, 4-, 5-, 6- oder 7-gliedrigen gesättigten, teilweise gesättigten oder ungesättigten aromatischen heterozyklischen Ring steht, der 1, 2 oder 3 Heteroatome als Ringglieder umfasst, welche aus Sauerstoff, Stickstoff und/oder Schwefel ausgewählt sind,
wobei der heterozyklische Ring möglicherweise mit 1, 2, 3, 4 oder 5 gleichartigen oder verschiedenartigen Substituenten R¹⁰ versehen ist, und wobei das/die Stickstoff- und/oder Schwefelatom(e) des heterozyklischen Rings möglicherweise oxidiert sein kann/können,
deren Stereoisomere, Tautomere und Salze.

2. Verbindung nach Anspruch 1, wobei
Het aus der Gruppe ausgewählt ist, die aus den Resten der folgenden Formeln Het-1 bis Het-24 besteht: 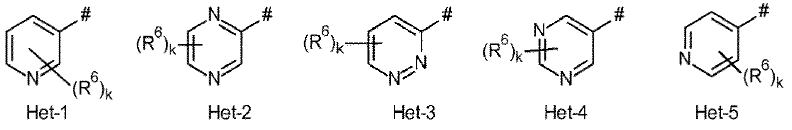 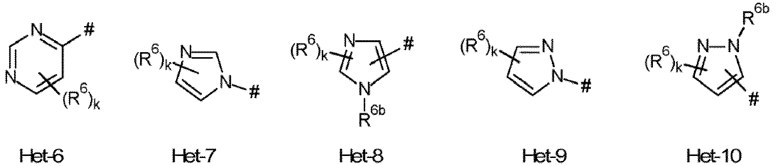 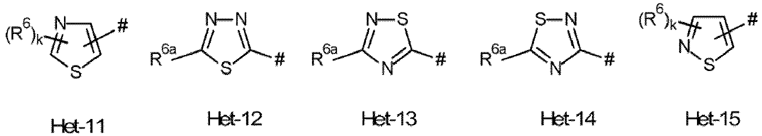 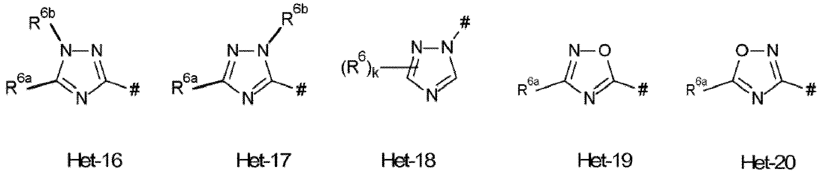 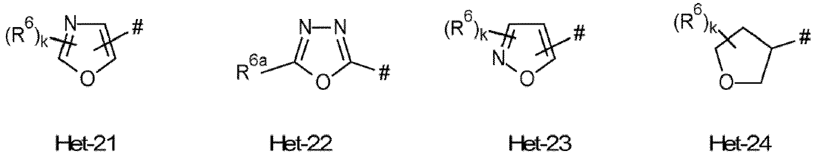
wobei # die Bindung in der Formel (I) bezeichnet, und wobei
k für 0, 1 oder 2 steht; und
R^{6a} für Wasserstoff steht und eine der Bedeutungen hat, wie sie in Anspruch 1 für R⁶ angegeben sind, und
R^{6b} für Wasserstoff, C₁-C₄-Alkyl oder C₁-C₄-Halogenalkyl steht.

3. Verbindung nach Anspruch 2, wobei Het gleich Het-1a ist 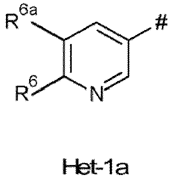 wobei # die Bindung in der Formel (I) bezeichnet,
R⁶ aus Halogen, C₁-C₄-Alkyl und C₁-C₄-Halogenalkyl ausgewählt ist, und
R^{6a} für Wasserstoff oder Halogen steht, insbesondere für Wasserstoff.

4. Verbindung nach einem beliebigen der vorhergehenden Ansprüche, wobei
R¹ und R² unabhängig voneinander aus der Gruppe ausgewählt sind, die aus
Wasserstoff, Halogen, CN, C₁-C₆-Alkyl, C₃-C₆-Cycloalkyl, C₁-C₆-Halogenalkyl, C₃-C₆-Halogencycloalkyl besteht;
oder
R¹ und R² zusammen mit dem Kohlenstoffatom, an welches sie gebunden sind, einen 3- bis 5-gliedrigen gesättigten carbozyklischen Ring bilden; und wobei insbesondere sowohl R¹ als auch R² für Wasserstoff stehen, oder R¹ für Methyl steht und R² für Wasserstoff steht.

5. Verbindung nach einem beliebigen der vorhergehenden Ansprüche, wobei
R³ aus der Gruppe ausgewählt ist, die aus Wasserstoff, Halogen, C₁-C₆-Alkyl, C₁-C₆-Halogenalkyl, C₂-C₆-Alkenyl, C₂-C₆-Halogenalkenyl, C₃-C₆-Cycloalkyl, C₃-C₆-Halogencycloalkyl, C₃-C₆-Cycloalkenyl, NR^{9a}R^{9b} und NR^{9a}-C(=O)R^{7a} besteht.

6. Verbindung nach einem beliebigen der vorhergehenden Ansprüche, wobei m für 0 oder 1 steht.

7. Verbindung nach einem beliebigen der Ansprüche 1 bis 4, wobei
m gleich 1 ist, und
R³ und R^{4a} sowohl zusammen miteinander als auch zusammen mit der bestehenden Bindung eine Doppelbildung zwischen den benachbarten Kohlenstoffatomen bilden.

8. Verbindung nach einem beliebigen der vorhergehenden Ansprüche, wobei
R⁵ für Wasserstoff, Halogen, CN, NR^{9a}R^{9b}, C₁-C₆-Alkyl, C₁-C₆-Halogenalkyl, C₂-C₆-Alkenyl, C₂-C₆-Halogenalkenyl, C₁-C₆-Alkoxy, C₁-C₆-Halogenalkoxy, C₃-C₆-Cycloalkyl, C₃-C₆-Halogencycloalkyl, C₃-C₆-Cycloalkoxy, C₃-C₆-Halogencycloalkoxy, C(=O) OR⁸, C(=O)NR^{9a}R^{9b}, C(=S)NR^{9a}R^{9b}, C(=O)R^{7a}, C(=S)R^{7a}, und Q-Phenyl steht,
wobei das Phenyl unsubstituiert oder möglicherweise mit 1, 2, 3, 4 oder 5 gleichartigen oder verschiedenartigen Substituenten R¹⁰ versehen ist.

9. Verbindung nach einem beliebigen der Ansprüche 1 bis 6, wobei
m gleich 0 ist, und
R³ und R⁵ zusammen mit dem Kohlenstoffatom, an welches sie gebunden sind, einen 3-, 4-, 5- oder 6-gliedrigen gesättigten Carbozyklus, einen 4-, 5-oder 6-gliedrigen ungesättigten Carbozyklus, einen Epoxidring oder einen Oxetanring bilden,
wobei jedes der Kohlenstoffatome des gesättigten oder ungesättigten Carbozyklus unsubstituiert sein kann oder mit 1 oder 2 gleichartigen oder verschiedenartigen Resten R^{7b} versehen sein kann, wobei
R^{7b} aus der Gruppe ausgewählt ist, die aus Halogen, C₁-C₆-Alkyl, C₁-C₆-Alkoxy und C₂-C₆-Alkinyl besteht,
wobei die Kohlenstoffatome der vorstehend genannten aliphatischen Reste möglicherweise teilweise oder vollständig halogeniert sein können,
und ein oder zwei Reste R^{7b} weiterhin C₃-C₆-Cycloalkyl darstellen können, wobei dieses unsubstituiert ist oder mit 1, 2, 3, 4 oder 5 gleichartigen oder verschiedenartigen Resten versehen ist, die aus
Fluor, Chlor oder Methyl,
aus Phenyl, welches möglicherweise mit 1, 2, 3, 4 oder 5 gleichartigen oder verschiedenartigen Substituenten R^{10a} versehen ist, oder
aus einem 3-, 4-, 5-, 6- oder 7-gliedrigen gesättigten, teilweise gesättigten oder ungesättigten aromatischen heterozyklischen Ring ausgewählt sind, der 1, 2 oder 3 Heteroatome als Ringglieder umfasst, die gleichartig oder verschiedenartig sind und aus Sauerstoff, Stickstoff und Schwefel ausgewählt sind,
wobei der heterozyklische Ring möglicherweise mit 1, 2, 3 oder 4 gleichartigen oder verschiedenartigen Substituenten R^{10a} versehen ist, und
wobei das Stickstoff- und/oder das Schwefelatom des heterozyklischen Rings möglicherweise oxidiert sein kann/können,
oder zwei der R^{7b}, welche an ein und demselben ringständigen Kohlenstoff vorliegen, zusammen =O, =S bilden,
oder zwei R^{7b} zusammen eine geradkettig C₂-C₇-Alkylengruppe bilden, sodass sie zusammen mit dem Kohlenstoffatom, an welches sie gebunden sind, einen 3-, 4-, 5- oder 6-gliedrigen Ring bilden,
wobei 1 oder 2 CH₂-Bausteine der Alkylenkette durch 1 oder 2 Heteroatombausteine ersetzt sein können, welche auf unabhängige Weise aus O und S ausgewählt sind, und
wobei die Alkylenkette unsubstituiert ist oder mit 1, 2, 3, 4, 5 oder 6 gleichartigen oder verschiedenartigen Resten substituiert sein kann, die aus der Gruppe ausgewählt sind, welche aus Halogen, C₁-C₄-Alkyl, C₁-C₄-Halogenalkyl, C₁-C₄-Alkoxy und C₁-C₄-Halogenalkoxy besteht, und
R^{10a} aus der Gruppe ausgewählt ist, die aus Halogen, C₁-C₄-Alkyl, C₁-C₄-Halogenalkyl, C₁-C₄-Alkoxy und C₁-C₄-Halogenalkoxy besteht.

10. Verbindung nach einem beliebigen der vorhergehenden Ansprüche, wobei der Baustein nach der folgenden Formel 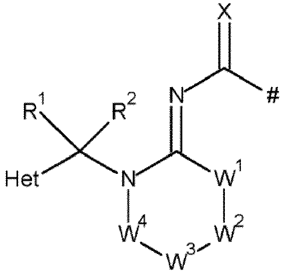 für einen Rest A steht, der aus der Gruppe ausgewählt ist, welche aus W.Het-1, W.Het-2, W.Het-3, W.Het-4, W.Het-5, W.Het-6, W.Het-7, W.Het-8, W.Het-9, W.Het-10, W.Het-11 und W.Het-12 besteht: 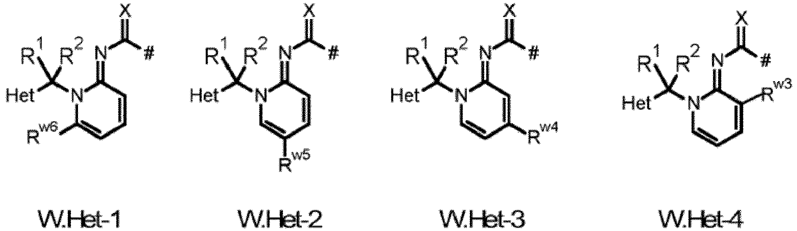 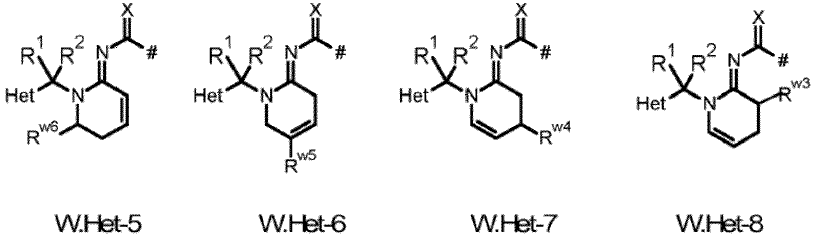 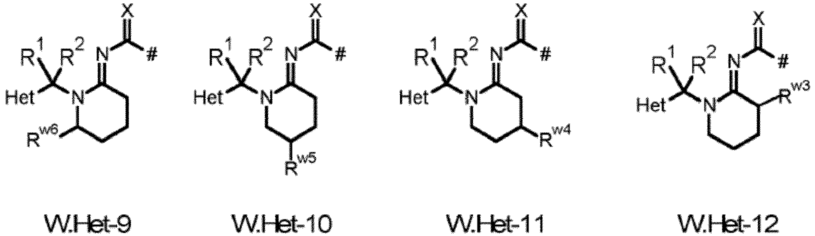 wobei # die Bindung an den verbleibenden Abschnitt des Moleküls bezeichnet; R¹, R² und Het den Begriffsbestimmungen in einem beliebigen der Ansprüche 1 bis 6 entsprechen, und wobei
R^{w3}, R^{w4}, R^{w5} und R^{w6} unabhängig voneinander aus Wasserstoff, Halogen, C₁-C₄-Alkoxy, C₁-C₄-Halogenalkoxy, C₁-C₄-Alkyl und C₁-C₄-Halogenalkyl ausgewählt sind und insbesondere für Wasserstoff stehen.

11. Verbindung nach einem beliebigen der vorhergehenden Ansprüche, wobei
X für O steht.

12. Zusammensetzung für die Landwirtschaft oder die Tiermedizin, wobei sie dafür geeignet ist, tierische Schädlinge zu bekämpfen, und mindestens eine Verbindung gemäß der Begriffsbestimmung in beliebigen der Ansprüche 1 bis 11 und mindestens einen inerten, flüssigen und/oder feststofflichen unbedenklichen Trägerstoff und möglicherweise, sofern dies gewünscht wird, mindestens ein Tensid umfasst.

13. Verwendung einer Verbindung gemäß der Begriffsbestimmung in beliebigen der Ansprüche 1 bis 11 zum Bekämpfen oder in Schach halten wirbelloser Schädlinge, zum Schutz wachsender Pflanzen vor einem Angriff oder Befall durch wirbellose Schädlinge, zum Schutz von Pflanzenvermehrungsmaterial, insbesondere Saatgut, vor Bodeninsekten, oder zum Schutz der Sämlingswurzeln und Sprossen von Pflanzen vor Boden- und Blattinsekten, wobei eine therapeutische Verwendung auf dem Körper von Menschen oder Tieren ausgeschlossen ist.

14. Nicht-therapeutisches Verfahren zum Bekämpfen oder in Schach halten wirbelloser Schädlinge, wobei das Verfahren das Inkontaktbringen des Schädlings oder von dessen Nahrungsquelle, Habitat oder Brutstätten mit einer schädlingsbekämpfend wirkenden Menge mindestens einer Verbindung gemäß der Begriffsbestimmung in beliebigen der Ansprüche 1 bis 11 umfasst.

15. Verfahren zum Schutz wachsender Pflanzen vor einem Angriff oder Befall durch wirbellose Schädlinge, wobei das Verfahren das Inkontaktbringen einer Pflanze, oder von Boden oder Wasser, in welchem die Pflanze wächst, mit einer schädlingsbekämpfend wirkenden Menge mindestens einer Verbindung gemäß den Begriffsbestimmungen in beliebigen der Ansprüche 1 bis 11 umfasst.

16. Verfahren zum Schutz von Pflanzenvermehrungsmaterial, insbesondere Saatgut, vor Bodeninsekten sowie von Sämlingswurzeln und Sprossen vor Boden- und Blattinsekten, wobei es das Inkontaktbringen des Pflanzenvermehrungsmaterials vor der Aussaat und/oder nach dem Vorkeimen mit mindestens einer Verbindung gemäß den Begriffsbestimmungen in beliebigen der Ansprüche 1 bis 11 umfasst.

17. Verbindung gemäß den Begriffsbestimmungen in beliebigen der Ansprüche 1 bis 11, zur Verwendung bei der Behandlung von Tieren, die von Parasiten befallen oder mit diesen infiziert sind, zur Verhütung eines Befalls oder einer Infektion von Tieren durch Parasiten oder zum Schutz von Tieren vor einem Befall oder einer Infektion durch Parasiten.

## Revendications

1. Composé de N-acylimino de formule (I) : 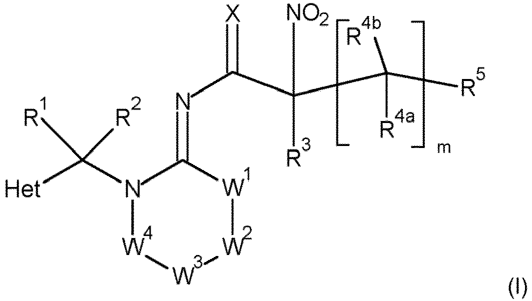 dans lequel
m est un nombre entier choisi parmi 0, 1, 2, 3, 4, 5 ou 6 ;
X est O ou S ;
Hét est un cycle hétérocyclique ou hétéroaromatique de 5 ou 6 chaînons lié en C ou lié en N, comprenant 1, 2, 3, 4 ou 5 atomes de carbone et 1, 2 ou 3 hétéroatomes comme chaînons, qui sont choisis indépendamment parmi le soufre, l'oxygène ou l'azote, où les chaînons de carbone, de soufre et d'azote peuvent indépendamment être partiellement ou complètement oxydés, et où chaque cycle est éventuellement substitué par k substituants R⁶ identiques ou différents, où k est un nombre entier choisi parmi 0, 1, 2, 3 ou 4 ;
W¹-W²-W³-W⁴ représente un groupement à chaîne carbonée relié à N et C=N, et formant ainsi un hétérocycle azoté de 5 ou 6 chaînons saturé, insaturé, ou partiellement insaturé, où
W¹, W², W³ et W⁴ représentent chacun individuellement CR^{v}R^{w}, où
chaque R^{w}, indépendamment les uns des autres, est choisi dans le groupe constitué par hydrogène, halogène, cyano, azido, nitro, SCN, SF₅, C₁-C₁₀-alkyle, C₃-C₈-cycloalkyle, C₂-C₁₀-alcényle, C₂-C₁₀-alcynyle,
où chacun parmi les quatre radicaux susmentionnés peut être non substitué, partiellement ou complètement halogéné et/ou peut éventuellement être substitué par 1, 2 ou 3 radicaux R⁷ identiques ou différents,
OR⁸, NR^{9a}R^{9b}, S(O)ₙR⁸, S(O)ₙNR^{9a}R^{9b}, C(=O)R^{7a}, C(=O)NR^{9a}R^{9b}, C(=O)OR⁸, C(=S)R^{7a}, C(=S)NR^{9a}R^{9b}, C(=S)OR⁸, C(=S)SR⁸, C(=NR¹⁷)R^{7a}, C(=NR¹⁷)NR^{9a}R^{9b} et Si(R¹¹)₂R¹²,
chaque R^{v}, indépendamment les uns des autres, est choisi dans le groupe constitué par hydrogène, halogène, cyano, C₁-C₁₀-alkyle, C₃-C₈-cycloalkyle, C₂-C₁₀-alcényle et C₂-C₁₀-alcynyle,
où chacun parmi les quatre radicaux susmentionnés peut être non substitué ou peut être partiellement ou complètement halogéné ou peut éventuellement être davantage substitué par 1, 2 ou 3 radicaux R⁷ identiques ou différents ; ou
R^{v} et R^{w} présents dans l'un des groupements peuvent former ensemble =O, =CR¹³R¹⁴, =S, =NR¹⁷, =NOR¹⁶ ou =NNR^{9a}R^{9b}, ou
deux R^{w} d'atomes de carbone adjacents, peuvent former ensemble ainsi que conjointement avec la liaison existante une double liaison entre les atomes de carbone adjacents ;
et où l'un parmi W² ou W³ peut éventuellement représenter une liaison simple ou double entre les atomes de carbone adjacents ;
R¹ et R² sont indépendamment l'un de l'autre choisis dans le groupe constitué par hydrogène, halogène, CN, SCN, nitro, C₁-C₆-alkyle, C₃-C₆-cycloalkyle,
où chacun parmi les deux radicaux susmentionnés est non substitué, partiellement ou complètement halogéné ou peut porter 1, 2 ou 3 radicaux R⁷ identiques ou différents,
Si(R¹¹)₂R¹², OR⁸, OSO₂R⁸, S(O)ₙR⁸, S(O)ₙNR^{9a}R^{9b}, NR^{9a}R^{9b}, C(=O)NR^{9a}R^{9b}, C(=S)NR^{9a}R^{9b}, C(=O)OR⁸, C(=O)R^{7a}, C(=S)R^{7a},
phényle, benzyle,
où le cycle phényle dans les deux derniers radicaux est non substitué ou éventuellement substitué par 1, 2, 3, 4 ou 5 substituants R¹⁰ identiques ou différents,
un cycle hétérocyclique de 3, 4, 5, 6 ou 7 chaînons saturé, partiellement saturé ou insaturé aromatique comprenant 1, 2 ou 3 hétéroatomes identiques ou différents comme chaînons, qui sont choisis parmi l'oxygène, l'azote et le soufre,
où le cycle hétérocyclique est éventuellement substitué par 1, 2, 3 ou 4 substituants R¹⁰ identiques ou différents, et où le ou les atomes d'azote et/ou de soufre du cycle hétérocyclique peuvent éventuellement être oxydés,
ou R¹ et R² forment, conjointement avec l'atome de carbone auquel ils sont liés, un cycle carbocyclique ou hétérocyclique de 3, 4, 5 ou 6 chaînons saturé ou partiellement insaturé,
où chacun parmi les atomes de carbone dudit cycle est non substitué ou peut porter 1 ou 2 radicaux R⁷ identiques ou différents,
ou R¹ et R² peuvent être ensemble =O, =CR¹³R¹⁴, =S, =NR¹⁷, =NOR¹⁶ ou =NNR^{9a}R^{9b} ;
R³ est choisi dans le groupe constitué par hydrogène, halogène, NO₂, C₁-C₆-alkyle, C₂-C₆-alcényle, C₂-C₆-alcynyle, C₃-C₈-cycloalkyle et C₃-C₈-cycloalcényle,
où chacun parmi les cinq radicaux susmentionnés est non substitué, partiellement ou complètement halogéné ou porte 1 ou 2 radicaux R⁷ identiques ou différents, cycloalkyle et cycloalcényle pouvant également porter 1, 2, 3, 4, 5 ou 6 groupements C₁-C₄-alkyle identiques ou différents,
S(O)ₙNR^{9a}R^{9b}, NR^{9a}R^{9b}, C(=O)OR⁸, C(=O)ₙNR^{9a}R^{9b}, C(=S)ₙNR^{9a}R^{9b}, C(=O)R^{7a}, C(=S)R^{7a}, NR^{9a}-C(=O)R^{7a}, NR^{9a}-C(=S)R^{7a}, NR^{9a}-S(O)R^{8a},
phényle, benzyle,
où le cycle phényle dans les deux radicaux susmentionnés est non substitué ou peut être substitué par un ou plusieurs, par exemple 1, 2, 3, 4 ou 5 substituants R¹⁰ identiques ou différents,
et un cycle hétérocyclique de 3, 4, 5, 6 ou 7 chaînons saturé, partiellement saturé ou insaturé aromatique comprenant 1, 2 ou 3 hétéroatomes comme chaînons, qui sont identiques ou différents et choisis parmi l'oxygène, l'azote et le soufre,
où le cycle hétérocyclique est éventuellement substitué par 1, 2, 3 ou 4 substituants R¹⁰ identiques ou différents, et où le ou les atomes d'azote et/ou de soufre du cycle hétérocyclique peuvent éventuellement être oxydés ;
R^{4a} et R^{4b} sont choisis chacun indépendamment l'un de l'autre et indépendamment du nombre entier m dans le groupe constitué par hydrogène, halogène, CN, C₁-C₆-alkyle, C₁-C₆-alcoxy, di-(C₁-C₆-alkyl) amino, C₁-C₆-alkylthio, et C₃-C₈-cycloalkyle,
où chacun parmi les cinq radicaux susmentionnés est non substitué, partiellement ou complètement halogéné,
R^{4b} pouvant également être choisi parmi
C₁-C₆-alkylsulfinyle, C₁-C₆-alkylsulfonyle,
où chacun parmi les deux radicaux susmentionnés est non substitué, partiellement ou complètement halogéné,
C₁-C₆-alkyle ou C₃-C₈-cycloalkyle,
où chacun parmi les deux radicaux susmentionnés porte 1, 2 ou 3 radicaux R⁷ identiques ou différents,
SH, S(O)ₙNR^{9a}R^{9b}, NR^{9a}R^{9b}, C(=O)OR⁸, C(=O)ₙNR^{9a}R^{9b}, C(=S)NR^{9a}R^{9b}, C(=O)R^{7a}, C(=S)R^{7a},
un motif Q¹-phényle,
où le cycle phényle est éventuellement substitué par 1, 2, 3, 4 ou 5 substituants R¹⁰ identiques ou différents,
et un motif Q¹-Hét^{#}, où
Q¹ est une liaison simple, NR^{9a}, C₁-C₄-alkylène ou NR^{9a}-C₁-C₄-alkylène,
où l'hétéroatome dans le dernier motif est lié à l'atome de carbone de CR^{4a}R^{4b} ;
ou R^{4a} et R^{4b} peuvent former, conjointement avec l'atome de carbone auquel ils sont liés, un cycle aliphatique de 3, 4, 5 ou 6 chaînons,
où chacun parmi les atomes de carbone du cycle peut être non substitué, partiellement ou complètement halogéné, et/ou peut porter 1 ou 2 radicaux identiques ou différents choisis parmi C₁-C₄-alkyle, C₁-C₄-halogénoalkyle, C₁-C₄-alcoxy et C₁-C₄-halogénoalcoxy ;
ou R^{4a} et R^{4b} peuvent former ensemble =O, =CR¹³R¹⁴, =S, =NR¹⁷, =NOR¹⁶ ou =NNR^{9a}R^{9b} ;
R⁵ est choisi dans le groupe constitué par
hydrogène, halogène, CN, C₁-C₆-alkyle, C₁-C₆-alcoxy, C₁-C₆-alkylthio, C₁-C₆-alkylsulfinyle, C₁-C₆-alkyl-sulfonyle, C₂-C₆-alcényle, C₂-C₆-alcénylthio, C₂-C₆-alcénylsulfinyle, C₂-C₆-alcénylsulfonyle, C₂-C₆-alcynyle, C₂-C₆-alcynylthio, C₂-C₆-alcynylsulfinyle, C₂-C₆-alcynyl-sulfonyle, C₃-C₈-cycloalkyle, C₃-C₈-cycloalcoxy, C₃-C₈-cycloalkylthio, C₃-C₈-cycloalkylsulfinyle et C₃-C₈-cycloalkylsulfonyle,
où chacun parmi les 18 radicaux susmentionnés est non substitué, partiellement ou complètement halogéné et/ou porte 1, 2 ou 3 radicaux R⁷ identiques ou différents, les radicaux cycloalkyle pouvant également porter 1, 2, 3, 4, 5 ou 6 groupements C₁-C₄-alkyle identiques ou différents,
S(O)ₙNR^{9a}R^{9b}, O-NR^{9a}R^{9b}, NR^{9a}R^{9b}, NR^{9a}NR^{9a}R^{9b}, C(=O)OR⁸, C(=O)NR^{9a}R^{9b}, C(=S)NR^{9a}R^{9b}, C(=O)R^{7a}, C(=S)R^{7a}, NR^{9a}-C(=O)R^{7a}, NR^{9a}-C(=S)R^{7a}, NR^{9a}C(=O)NR^{9a}R^{9b}, NR^{9a}C(=S)NR^{9a}R^{9b}, NR^{9a}-S(O)ₙR^{8a},
un motif Q-phényle,
où le cycle phényle est éventuellement substitué par 1, 2, 3, 4 ou 5 substituants R¹⁰ identiques ou différents,
un motif Y-R^{5a},
et un motif Q-Hét^{#}, où
Y est O, S, S(=O) ou S(=O)₂ ;
R^{5a} est choisi dans le groupe constitué par C(=O)NR^{9a}R^{9b}, C(=S)NR^{9a}R^{9b}, C(=O)R⁷, C(=S)R⁷ ;
phényle ou CH₂-phényle,
éventuellement substitué par un ou plusieurs, par exemple 1, 2, 3, 4 ou 5 substituants R¹⁰ identiques ou différents ;
un cycle hétérocyclique de 3, 4, 5, 6 ou 7 chaînons saturé, partiellement saturé ou insaturé aromatique comprenant 1, 2 ou 3 hétéroatomes choisis parmi l'oxygène, l'azote et/ou le soufre, éventuellement substitué par 1, 2, 3 ou 4 substituants R^{y} identiques ou différents,
où le ou les atomes d'azote et/ou de soufre du cycle hétérocyclique peuvent éventuellement être oxydés, et
R^{y} est choisi dans le groupe constitué par
hydrogène, halogène, cyano, nitro, C₁-C₁₀-alkyle, C₂-C₁₀-alcényle,
où les deux radicaux susmentionnés peuvent éventuellement être substitués par un ou plusieurs radicaux R¹⁸ identiques ou différents,
phényle,
éventuellement substitué par halogène, cyano, nitro, C₁-C₆-alkyle, C₁-C₆-halogénoalkyle, C₁-C₆-alcoxy ou C₁-C₆-halogénoalcoxy, C₁-C₄-alkyle et C₁-C₄-alcoxy, où les deux parmi les deux radicaux aliphatiques susmentionnés peuvent être non substitués, partiellement ou complètement halogénés,
OR¹⁹, -S(O)ₙR¹⁹, S(O)ₙNR^{20a}R^{20b}, NR^{20a}R^{20b}, C(=O)R⁸, C(=O) OR¹⁹, -C(=NR^{20a})R¹⁸, C(=O)NR^{20a}R^{20b}, C(=S)NR^{20a}R^{20b} ou
deux R^{y} présents ensemble sur un atome d'un cycle hétérocyclique partiellement saturé peuvent être =O, =CR^{21a}R^{21b} ; =NR^{20a}, =NOR¹⁹ ou =NNR^{20a} ;
ou deux R^{y} sur des atomes de carbone adjacents peuvent être un pont choisi parmi CH₂CH₂CH₂CH₂, CH=CH-CH=CH, N=CH-CH=CH, CH=N-CH=CH, N=CH-N=CH, CH₂CH₂CH₂, CH=CHCH₂, CH₂CH₂NR^{17a}, CH₂CH=N, CH=CH-NR^{17a}, et forment, conjointement avec les atomes de carbone auxquels les deux R^{y} sont liés, un cycle carbocyclique ou hétérocyclique de 5 chaînons ou 6 chaînons condensé et partiellement saturé ou insaturé, aromatique, où le cycle peut éventuellement être substitué par un ou deux substituants choisis parmi =O, OH, CH₃, OCH₃, halogène, cyano, C₁-C₄-alcoxy, C₁-C₄-halogénoalcoxy, C₁-C₄-alkyle et C₁-C₄-halogénoalkyle ;
Q indépendamment de son occurrence, est une liaison simple, CH₂, S, O, S(=O), S(=O)₂, NR^{9a}, -O-S(=O)₂-, -NR^{9a}-S(=O)₂-, -O-C(=O)-, -NR^{9a}-C(=O)-, -O-C₁-C₄-alkylène, -S-C₁-C₄-alkylène, -NR^{9a}-C₁-C₄-alkylène, -O-C(=O)-C₁-C₄-alkylène ou -NR^{9a}-C(=O)-C₁-C₄-alkylène,
où l'hétéroatome dans les 7 derniers motifs est lié à l'atome de carbone de C(NO₂)R³ ou CR^{4a}R^{4b}, respectivement ;
ou, si m vaut 1,
R³ et R^{4a} peuvent également former, ensemble ainsi que conjointement avec la liaison existante, une double liaison entre les atomes de carbone adjacents ;
ou, si m vaut 1,
l'un parmi R^{4a} ou R^{4b} conjointement avec R⁵ peuvent également former avec l'atome de carbone auquel ils sont liés, un carbocycle ou hétérocycle de 3, 4, 5 ou 6 chaînons saturé ou partiellement insaturé,
où chacun parmi les atomes de carbone du carbocycle ou de l'hétérocycle peut être non substitué ou peut porter 1 ou 2 radicaux R^{7b} identiques ou différents, et où l'hétérocycle possède 1 ou 2 hétéroatomes ou motifs d'hétéroatomes non adjacents identiques ou différents comme chaînons, qui sont choisis parmi O, S, N, et N-R^{17c} ;
ou, si m=0,
R³ et R⁵ peuvent également former, ensemble et conjointement avec l'atome de carbone auquel ils sont liés, un carbocycle ou hétérocycle de 3, 4, 5 ou 6 chaînons saturé ou partiellement insaturé,
où chacun parmi les atomes de carbone du carbocycle ou de l'hétérocycle peut être non substitué ou peut porter 1 ou 2 radicaux R^{7b} identiques ou différents, et où l'hétérocycle possède 1 ou 2 hétéroatomes ou motifs d'hétéroatomes non adjacents identiques ou différents comme chaînons, qui sont choisis parmi O, S, N, et N-R^{17c}, ou
R³ et R⁵ peuvent former ensemble =O ou =S ;
où, indépendamment de leur occurrence,
n vaut 0, 1 ou 2 ;
R⁶ est choisi dans le groupe constitué par
halogène, cyano, azido, nitro, SCN, SF₅,
C₁-C₁₀-alkyle, C₃-C₈-cycloalkyle, C₂-C₁₀-alcényle, C₂-C₁₀-alcynyle,
où chacun parmi les quatre radicaux susmentionnés peut être partiellement ou complètement halogéné et/ou peut éventuellement être substitué indépendamment les uns des autres par 1, 2 ou 3 radicaux R⁷ identiques ou différents,
OR⁸, NR^{17a}R^{17b}, S(O)ₙR⁸, S(O)ₙNR^{17a}R^{17b}, C(=O)R^{7a}, C(=O)NR^{17a}R^{17b}, C(=O)OR⁸, C(=S)R^{7a}, C(=S)NR^{17a}R^{17b}, C(=S)OR⁸, C(=S)SR⁸, C(=NR¹⁷)R^{7a}, C(=NR¹⁷)NR^{17a}R^{17b}, Si(R¹¹)₂R¹²,
phényle, éventuellement substitué par 1, 2, 3, 4 ou 5 substituants R¹⁰ identiques ou différents,
un cycle hétérocyclique de 3, 4, 5, 6 ou 7 chaînons saturé, partiellement saturé ou insaturé aromatique comprenant 1, 2 ou 3 hétéroatomes comme chaînons, qui sont identiques ou différents et choisis parmi l'oxygène, l'azote et le soufre,
où le cycle hétérocyclique est éventuellement substitué par 1, 2, 3 ou 4 substituants R¹⁰ identiques ou différents, et où le ou les atomes d'azote et/ou de soufre du cycle hétérocyclique peuvent éventuellement être oxydés ;
ou deux parmi R⁶ présents sur un carbone de cycle peuvent former ensemble =O, =CR¹³R¹⁴, =S, =NR¹⁷, =NOR¹⁶, =NNR^{9a}R^{9b} ;
ou deux R⁶ forment ensemble une chaîne C₂-C₇-alkylène linéaire, formant ainsi, conjointement avec le ou les atomes de cycle auxquels ils sont liés, un cycle de 3, 4, 5, 6, 7 ou 8 chaînons,
où 1 ou 2 motifs CH₂ de la chaîne alkylène peuvent être remplacés par 1 ou 2 motifs d'hétéroatomes choisis parmi O, S et NR^{17c} et/ou
1 ou 2 parmi les groupements CH₂ de la chaîne alkylène peuvent être remplacés par un groupement C=O, C=S et/ou C=NR¹⁷ ; et
où la chaîne alkylène est non substituée ou peut être substituée par 1, 2, 3, 4, 5 ou 6 radicaux identiques ou différents choisis dans le groupe constitué par
halogène, C₁-C₆-halogénoalkyle, C₁-C₆-alcoxy, C₁-C₆-halogénoalcoxy, C₁-C₆-alkylthio, C₁-C₆-halogénoalkylthio, C₃-C₈-cycloalkyle, C₃-C₈-halogénocycloalkyle, C₂-C₆-alcényle, C₂-C₆-halogénoalcényle, C₂-C₆-alcynyle, C₂-C₆-halogénoalcynyle,
phényle pouvant être substitué par 1, 2, 3, 4 ou 5 radicaux R¹⁰ identiques ou différents, et
un cycle hétérocyclique de 3, 4, 5, 6 ou 7 chaînons saturé, partiellement insaturé ou aromatique contenant 1, 2 ou 3 hétéroatomes ou groupements d'hétéroatomes choisis parmi N, O, S, NO, SO et SO₂, comme chaînons, où le cycle hétérocyclique peut être substitué par 1, 2, 3, 4 ou 5 radicaux R¹⁰ identiques ou différents ;
R⁷ indépendamment de son occurrence, est choisi dans le groupe constitué par
cyano, azido, nitro, -OH, -SH, -SCN, SF₅, C₁-C₆-alkyle, C₁-C₆-halogénoalkyle, C₃-C₈-cycloalkyle, C₃-C₈-halogénocycloalkyle, C₂-C₆-alcényle, C₂-C₆-halogénoalcényle, C₂-C₆-alcynyle, C₂-C₆-halogénoalcynyle, C₁-C₆-alcoxy, C₁-C₆-halogénoalcoxy, C₁-C₆-alkylthio, C₁-C₆-alkylsulfinyle, C₁-C₆-alkylsulfonyle, C₁-C₆-halogénoalkylthio, Si(R¹¹)₂R¹², OR⁸, OSO₂R⁸, S(O)ₙR⁸, S(O)ₙNR^{17a}R^{17b}, NR^{17a}R^{17b}, C(=O)NR^{17a}R^{17b}, C(=S)NR^{17a}R^{17b}, C(=O)OR⁸, C(=O)R¹⁵, C(=S)R¹⁵, C(=NR¹⁷)R¹⁵, phényle, phényl-C₁-C₄-alkyle, phénoxy,
où le cycle phényle dans les trois derniers radicaux est non substitué ou peut être substitué par 1, 2, 3, 4 ou 5 radicaux identiques ou différents choisis parmi halogène, C₁-C₆-alkyle, cyano, azido, nitro, C₁-C₁₀-alkyle, C₁-C₆-halogénoalkyle, C₁-C₆-alcoxy, C₁-C₆-halogénoalcoxy, (C₁-C₆-alcoxy) carbonyle, (C₁-C₆-alkyl)amino et di-(C₁-C₆-alkyl)amino, et
un cycle hétérocyclique de 3, 4, 5, 6 ou 7 chaînons saturé, partiellement saturé ou insaturé aromatique comprenant 1, 2 ou 3 hétéroatomes comme chaînons, qui sont identiques ou différents et choisis parmi l'oxygène, l'azote et le soufre,
où le cycle hétérocyclique est non substitué ou peut être substitué par 1, 2, 3 ou 4 radicaux identiques ou différents choisis parmi halogène, C₁-C₆-alkyle, cyano, azido, nitro, C₁-C₁₀-alkyle, C₁-C₆-halogénoalkyle, C₁-C₆-alcoxy, C₁-C₆-halogénoalcoxy, (C₁-C₆-alcoxy) carbonyle, (C₁-C₆-alkyl) amino et di-(C₁-C₆-alkyl)amino, et
où le ou les atomes d'azote et/ou de soufre du cycle hétérocyclique peuvent éventuellement être oxydés,
ou deux R⁷ présents sur un atome de carbone peuvent former ensemble =O, =CR¹³R¹⁴, =S, =NR¹⁷, =NOR¹⁶, =NNR^{9a}R^{9b},
ou deux R⁷ peuvent former un cycle carbocyclique ou hétérocyclique de 3, 4, 5, 6, 7 ou 8 chaînons saturé ou partiellement insaturé conjointement avec les atomes de carbone auxquels les deux R⁷ sont liés, où le cycle hétérocyclique comprend 1, 2 ou 3 hétéroatomes comme chaînons, qui sont identiques ou différents et choisis parmi l'oxygène, l'azote et le soufre,
où le cycle hétérocyclique est non substitué ou peut être substitué par 1, 2, 3 ou 4 radicaux identiques ou différents choisis parmi halogène, C₁-C₆-alkyle, cyano, azido, nitro, C₁-C₁₀-alkyle, C₁-C₆-halogénoalkyle, C₁-C₆-alcoxy, C₁-C₆-halogénoalcoxy, (C₁-C₆-alcoxy) carbonyle, (C₁-C₆-alkyl) amino et di-(C₁-C₆-alkyl) amino ;
R^{7a} indépendamment de son occurrence, est choisi dans le groupe constitué par
hydrogène, C₁-C₆-alkyle, C₁-C₆-halogénoalkyle, C₁-C₆-alcoxy, C₁-C₆-halogénoalcoxy, C₁-C₆-alkylthio, C₁-C₆-alkylsulfinyle, C₁-C₆-alkylsulfonyle, C₁-C₆-halogéno-alkylthio, C₃-C₈-cycloalkyle, C₃-C₈-halogénocycloalkyle, C₃-C₆-cycloalkyl-C₁-C₄-alkyle, C₂-C₆-alcényle, C₂-C₆-halogénoalcényle, C₂-C₆-alcynyle, C₂-C₆-halogénoalcynyle, phényle, éventuellement substitué par 1, 2, 3, 4 ou 5 substituants R¹⁰ identiques ou différents,
benzyle, où le motif phényle de benzyle est non substitué ou substitué par 1, 2 ou 3 radicaux identiques ou différents choisis dans le groupe constitué par halogène, CN, C₁-C₄-alkyle, C₁-C₄-halogénoalkyle, C₁-C₄-alcoxy et C₁-C₄-halogénoalcoxy, et
un cycle hétérocyclique de 3, 4, 5, 6 ou 7 chaînons saturé, partiellement saturé ou insaturé aromatique comprenant 1, 2 ou 3 hétéroatomes comme chaînons, qui sont identiques ou différents et choisis parmi l'oxygène, l'azote et le soufre,
où le cycle hétérocyclique est éventuellement substitué par 1, 2, 3 ou 4 substituants R¹⁰ identiques ou différents, et
où le ou les atomes d'azote et/ou de soufre du cycle hétérocyclique peuvent éventuellement être oxydés ;
R^{7b} est choisi dans le groupe constitué par halogène, cyano, C₁-C₁₀-alkyle, C₃-C₈-cycloalkyle, C₂-C₁₀-alcényle, C₂-C₁₀-alcynyle,
où chacun parmi les quatre radicaux susmentionnés est non substitué, partiellement ou complètement halogéné, et/ou peut être substitué par 1, 2 ou 3 radicaux R⁷ identiques ou différents,
OR⁸, NR^{17a}R^{17b}, S(O)ₙR⁸, S(O)ₙNR^{17a}R^{17b}, C(=O)R^{7a}, C(=O)NR^{17a}R^{17b}, C(=O)OR⁸, C(=S)R^{7a}, C(=S)NR^{17a}R^{17b}, C(=S)OR⁸, C(=NR¹⁷)R^{7a}, C(=NR¹⁷)NR^{17a}R^{17b}, Si(R¹¹)₂R¹² ;
phényle, éventuellement substitué par 1, 2, 3, 4 ou 5 substituants R¹⁰ identiques ou différents,
un cycle hétérocyclique de 3, 4, 5, 6 ou 7 chaînons saturé, partiellement saturé ou insaturé aromatique comprenant 1, 2 ou 3 hétéroatomes comme chaînons, qui sont identiques ou différents et choisis parmi l'oxygène, l'azote et le soufre,
où le cycle hétérocyclique est éventuellement substitué par 1, 2, 3 ou 4 substituants R¹⁰ identiques ou différents, et où le ou les atomes d'azote et/ou de soufre du cycle hétérocyclique peuvent éventuellement être oxydés,
ou deux parmi R^{7b} présents sur un carbone de cycle peuvent former ensemble =O, =S ou =CR¹³R¹⁴,
ou deux R^{7b} forment ensemble une chaîne C₂-C₇-alkylène linéaire, formant ainsi, conjointement avec le ou les atomes de cycle auxquels ils sont liés, un cycle de 3, 4, 5, 6, 7 ou 8 chaînons,
où 1 ou 2 motifs CH₂ de la chaîne alkylène peuvent être remplacés par 1 ou 2 motifs d'hétéroatomes choisis parmi O, S et NR^{17c} et/ou
1 ou 2 parmi les groupements CH₂ de la chaîne alkylène peuvent être remplacés par un groupement C=O, C=S et/ou C=NR¹⁷ ; et
où la chaîne alkylène est non substituée ou peut être substituée par 1, 2, 3, 4, 5 ou 6 radicaux identiques ou différents choisis dans le groupe constitué par
halogène, C₁-C₄-alkyle, C₁-C₆-halogénoalkyle, C₁-C₆-alcoxy, C₁-C₆-halogénoalcoxy, C₁-C₆-alkylthio, C₁-C₆-halogénoalkylthio, C₃-C₈-cycloalkyle, C₃-C₈-halogénocycloalkyle, C₂-C₆-alcényle, C₂-C₆-halogénoalcényle, C₂-C₆-alcynyle, C₂-C₆-halogénoalcynyle,
phényle pouvant être substitué par 1, 2, 3, 4 ou 5 radicaux R¹⁰ identiques ou différents, et
un cycle hétérocyclique de 3, 4, 5, 6 ou 7 chaînons saturé, partiellement insaturé ou aromatique contenant 1, 2 ou 3 hétéroatomes ou groupements d'hétéroatomes choisis parmi N, O, S, NO, SO et SO₂, comme chaînons, où le cycle hétérocyclique peut être substitué par 1, 2, 3, 4 ou 5 radicaux R¹⁰ identiques ou différents ;
R⁸ indépendamment de son occurrence, est choisi dans le groupe constitué par
hydrogène, C₁-C₆-alkyle, C₁-C₆-halogénoalkyle, C₃-C₈-cycloalkyle, C₃-C₈-cycloalkyl-C₁-C₄-alkyle, C₃-C₈-halogénocycloalkyle, C₂-C₆-alcényle, C₂-C₆-halogénoalcényle, C₂-C₆-alcynyle, C₂-C₆-halogénoalcynyle, C(=O)R¹⁵, C(=O)NR^{17a}R^{17b}, C(=S)NR^{17a}R^{17b}, C(=O)OR¹⁶,
phényle, phényl-C₁-C₄-alkyle, où le cycle phényle dans les deux radicaux susmentionnés est non substitué ou substitué par 1, 2, 3, 4 ou 5 substituants R¹⁰ identiques ou différents, et
un cycle hétérocyclique de 3, 4, 5, 6 ou 7 chaînons saturé, partiellement saturé ou insaturé aromatique comprenant 1, 2 ou 3 hétéroatomes comme chaînons, qui sont identiques ou différents et choisis parmi l'oxygène, l'azote et le soufre,
où le cycle hétérocyclique est éventuellement substitué par 1, 2, 3 ou 4 substituants R¹⁰ identiques ou différents, et
où le ou les atomes d'azote et/ou de soufre du cycle hétérocyclique peuvent éventuellement être oxydés,
R^{8a} indépendamment de son occurrence, est choisi dans le groupe constitué par
C₁-C₆-alkyle, C₃-C₈-cycloalkyle, C₃-C₈-cycloalkyl-C₁-C₄-alkyle, C₂-C₆-alcényle, C₂-C₆-alcynyle,
où chacun parmi les cinq radicaux susmentionnés est non substitué ou partiellement ou complètement halogéné, phényle, benzyle,
où le cycle phényle dans les deux radicaux susmentionnés est non substitué ou peut être substitué par 1, 2, 3, 4 ou 5 substituants R¹⁰ identiques ou différents,
et un cycle hétérocyclique de 3, 4, 5, 6 ou 7 chaînons saturé, partiellement saturé ou insaturé aromatique comprenant 1, 2 ou 3 hétéroatomes comme chaînons, qui sont identiques ou différents et choisis parmi l'oxygène, l'azote et le soufre,
où le cycle hétérocyclique est éventuellement substitué par 1, 2, 3 ou 4 substituants R¹⁰ identiques ou différents, et
où le ou les atomes d'azote et/ou de soufre du cycle hétérocyclique peuvent éventuellement être oxydés,
R⁹ indépendamment de son occurrence, est choisi dans le groupe constitué par
hydrogène, C₁-C₆-alkyle, C₁-C₆-halogénoalkyle, C₁-C₆-alcoxy, C₁-C₆-halogénoalcoxy, C₃-C₈-cycloalkyle, C₃-C₈-cycloalkyl-C₁-C₄-alkyle, C₃-C₈-halogénocycloalkyle, C₂-C₆-alcényle, C₂-C₆-halogénoalcényle, C₂-C₆-alcynyle, C₂-C₆-halogénoalcynyle,
phényle, éventuellement substitué par 1, 2, 3, 4 ou 5 substituants R¹⁰ identiques ou différents ;
un cycle hétérocyclique lié en C de 3, 4, 5, 6 ou 7 chaînons saturé, partiellement saturé ou insaturé aromatique comprenant 1, 2 ou 3 hétéroatomes comme chaînons, qui sont identiques ou différents et choisis parmi l'oxygène, l'azote et le soufre,
où le cycle hétérocyclique est éventuellement substitué par 1, 2, 3 ou 4 substituants R¹⁰ identiques ou différents, et
où le ou les atomes d'azote et/ou de soufre du cycle hétérocyclique peuvent éventuellement être oxydés,
R^{9a} et R^{9b} sont choisis chacun indépendamment l'un de l'autre dans le groupe constitué par
hydrogène, C₁-C₆-alkyle, C₁-C₆-halogénoalkyle, C₁-C₆-alcoxy, C₁-C₆-halogénoalcoxy, C₁-C₆-alkylthio, C₁-C₆-halogénoalkylthio, C₃-C₈-cycloalkyle, C₃-C₈-halogénocycloalkyle, C₃-C₈-cycloalkyl-C₁-C₄-alkyle, C₂-C₆-alcényle, C₂-C₆-halogénoalcényle, C₂-C₆-alcynyle, C₂-C₆-halogénoalcynyle,
S(O)ₙR¹⁶, -S(O)ₙNR^{17a}R^{17b}, C(=O)R¹⁵, C(=O)OR¹⁶, C(=O)NR^{17a}R^{17b}, C(=S)R¹⁵, C(=S)SR¹⁶, C(=S)NR^{17a}R^{17b}, C(=NR¹⁷)R¹⁵ ;
phényle, benzyle, 1-phénéthyle ou 2-phénéthyle,
où le cycle phényle dans les quatre radicaux susmentionnés est non substitué ou peut être substitué par 1, 2, 3, 4 ou 5 substituants R¹⁰ identiques ou différents ; et
un cycle hétérocyclique lié en C de 3, 4, 5, 6 ou 7 chaînons saturé, partiellement saturé ou insaturé aromatique comprenant 1, 2 ou 3 hétéroatomes comme chaînons, qui sont identiques ou différents et choisis parmi l'oxygène, l'azote et le soufre,
où le cycle hétérocyclique est éventuellement substitué par 1, 2, 3 ou 4 substituants R¹⁰ identiques ou différents, et
où le ou les atomes d'azote et/ou de soufre du cycle hétérocyclique peuvent éventuellement être oxydés,
ou R^{9a} et R^{9b} sont ensemble une chaîne C₂-C₇-alkylène et forment un cycle de 3, 4, 5, 6, 7 ou 8 chaînons saturé, partiellement saturé ou insaturé aromatique conjointement avec l'atome d'azote auquel ils sont liés,
où la chaîne alkylène peut contenir un ou deux hétéroatomes, qui sont, indépendamment les uns des autres, choisis parmi l'oxygène, le soufre ou l'azote, et où la chaîne alkylène peut éventuellement être substituée par 1, 2, 3 ou 4 radicaux identiques ou différents choisis parmi
halogène, C₁-C₆-alkyle, C₁-C₆-halogénoalkyle, C₁-C₆-alcoxy, C₁-C₆-halogénoalcoxy, C₁-C₆-alkylthio, C₁-C₆-halogénoalkylthio, C₃-C₈-cycloalkyle, C₃-C₈-halogénocycloalkyle, C₂-C₆-alcényle, C₂-C₆-halogénoalcényle, C₂-C₆-alcynyle, C₂-C₆-halogénoalcynyle,
phényle, éventuellement substitué par 1, 2, 3, 4 ou 5 substituants R¹⁰ identiques ou différents, et
un cycle hétérocyclique lié en C de 3, 4, 5, 6 ou 7 chaînons saturé, partiellement saturé ou insaturé aromatique comprenant 1, 2 ou 3 hétéroatomes comme chaînons, qui sont identiques ou différents et choisis parmi l'oxygène, l'azote et le soufre,
où le cycle hétérocyclique est éventuellement substitué par 1, 2, 3 ou 4 substituants R¹⁰ identiques ou différents, et
où le ou les atomes d'azote et/ou de soufre du cycle hétérocyclique peuvent éventuellement être oxydés,
ou R^{9a} et R^{9b} peuvent former ensemble un motif =CR¹³R¹⁴, =NR¹⁷, =NOR¹⁶, =NNR^{17a}R^{17b} ;
R¹⁰ indépendamment de son occurrence, est choisi dans le groupe constitué par
halogène, cyano, azido, nitro, SCN, SF₅, C₁-C₁₀-alkyle, C₁-C₄-halogénoalkyle, C₃-C₈-cycloalkyle, C₂-C₁₀-alcényle, C₂-C₁₀-alcynyle,
où chacun parmi les quatre radicaux susmentionnés peut éventuellement être substitué par 1, 2, 3, 4 ou 5 radicaux R⁷ identiques ou différents,
Si(R)₂R¹², OR¹⁶, OS(O)ₙR¹⁶, -S(O)ₙR¹⁶, S(O)ₙNR^{17a}R^{17b}, NR^{17a}R^{7b}, C(=O)R¹⁵, C(=S)R¹⁵, C(=O)OR¹⁶, -C(=NR¹⁷)R¹⁵, C(=O)NR^{17a}R^{17b}, C(=S)NR^{17a}R^{17b},
phényle,
éventuellement substitué par 1, 2, 3, 4 ou 5 radicaux identiques ou différents choisis parmi OH, halogène, cyano, nitro, C₁-C₆-alkyle, C₁-C₆-halogénoalkyle, C₁-C₆-alcoxy et C₁-C₆-halogénoalcoxy, et un cycle hétérocyclique de 3, 4, 5, 6 ou 7 chaînons saturé, partiellement saturé ou insaturé aromatique comprenant 1, 2 ou 3 hétéroatomes comme chaînons, qui sont identiques ou différents et choisis parmi l'oxygène, l'azote et le soufre,
où le cycle hétérocyclique est non substitué ou peut être substitué par 1, 2, 3, 4 ou 5 substituants identiques ou différents choisis parmi halogène, cyano, NO₂, C₁-C₆-alkyle, C₁-C₆-halogénoalkyle, C₁-C₆-alcoxy et C₁-C₆-halogénoalcoxy, et
où le ou les atomes d'azote et/ou de soufre du cycle hétérocyclique peuvent éventuellement être oxydés ;
ou deux R¹⁰ présents ensemble sur un atome de carbone de cycle d'un radical hétérocyclique saturé ou partiellement insaturé peuvent former =O, =CR¹³R¹⁴, =S, =NR¹⁷, =NOR¹⁶, =NNR^{17a}R^{17b} ;
ou deux R¹⁰ sur des atomes de carbone de cycle adjacents peuvent être ensemble un radical bivalent choisi parmi CH₂CH₂CH₂CH₂, CH=CH-CH=CH, N=CH-CH=CH, CH=N-CH=CH, N=CH-N=CH, OCH₂CH₂CH₂, OCH=CHCH₂, CH₂OCH₂CH₂, OCH₂CH₂O, OCH₂OCH₂, CH₂CH₂CH₂, CH=CHCH₂, CH₂CH₂O, CH=CHO, CH₂OCH₂, CH₂C(=O)O, C(=O) OCH₂, O(CH₂)O, SCH₂CH₂CH₂, SCH=CHCH₂, CH₂SCH₂CH₂, SCH₂CH₂S, SCH₂SCH₂, CH₂CH₂S, CH=CHS, CH₂SCH₂, CH₂C(=S)S, C(=S)SCH₂, S(CH₂)S, CH₂CH₂NR¹⁷, CH₂CH=N, CH=CH-NR¹⁷, OCH=N, SCH=N, et forment conjointement avec lesdits atomes de carbone de cycle adjacents un cycle carbocyclique ou hétérocyclique de 5 chaînons ou de 6 chaînons partiellement saturé ou insaturé, aromatique, où le cycle peut éventuellement être substitué par un ou deux substituants choisis indépendamment parmi =O, OH, CH₃, OCH₃, halogène, cyano, halogénométhyle et halogénométhoxy ;
R¹¹ et R¹² indépendamment de leur occurrence, sont choisis dans le groupe constitué par
C₁-C₆-alkyle, C₁-C₆-halogénoalkyle, C₁-C₆-alcoxy, C₁-C₆-alcoxy-C₁-C₄-alkyle, C₂-C₆-alcényle, C₂-C₆-halogénoalcényle, C₂-C₆-alcynyle, C₂-C₆-halogénoalcynyle, C₃-C₈-cycloalkyle, C₃-C₈-halogénocycloalkyle, C₃-C₈-cycloalkyl-C₁-C₄-alkyle, C₃-C₈-halogénocycloalkyl-C₁-C₄-alkyle, C₁-C₆-halogénoalcoxy-C₁-C₄-alkyle,
phényle et benzyle,
où le cycle phényle dans les deux radicaux susmentionnés est non substitué ou substitué par 1, 2, 3, 4 ou 5 radicaux identiques ou différents choisis parmi halogène, OH, cyano, NO₂, C₁-C₆-alkyle, C₁-C₆-halogénoalkyle, C₁-C₆-alcoxy et C₁-C₆-halogénoalcoxy ;
R¹³ et R¹⁴ indépendamment de leur occurrence, sont choisis dans le groupe constitué par hydrogène, halogène, CN, C₁-C₆-alkyle, C₃-C₆-cycloalkyle, C₁-C₄-alcoxy-C₁-C₄-alkyle, phényle et benzyle ;
R¹⁵ indépendamment de son occurrence, est choisi dans le groupe constitué par
hydrogène, C₁-C₆-alkyle, C₂-C₆-alcényle, C₂-C₆-alcynyle, C₃-C₈-cycloalkyle,
où les quatre radicaux aliphatiques et cycloaliphatiques susmentionnés peuvent être non substitués, partiellement ou complètement halogénés et/ou oxygénés et/ou peuvent porter 1 ou 2 radicaux identiques ou différents choisis parmi C₁-C₄-alcoxy ; C₃-C₆-cycloalkyl-C₁-C₄-alkyle, phényle, benzyle et pyridyle,
où les trois derniers radicaux peuvent être non substitués, partiellement ou complètement halogénés et/ou peuvent porter 1, 2 ou 3 substituants identiques ou différents choisis parmi C₁-C₆-alkyle, C₁-C₆-halogénoalkyle, C₁-C₆-alcoxy, C₁-C₆-halogénoalcoxy, (C₁-C₆-alcoxy)carbonyle, (C₁-C₆-alkyl) amino et di-(C₁-C₆-alkyl)amino ;
R¹⁶ indépendamment de son occurrence, est choisi dans le groupe constitué par hydrogène, C₁-C₆-alkyle, C₂-C₆-alcényle, C₂-C₆-alcynyle, C₃-C₈-cycloalkyle, C₃-C₈-cycloalkyl-C₁-C₄-alkyle,
où les cinq radicaux aliphatiques et cycloaliphatiques susmentionnés peuvent être non substitués, partiellement ou complètement halogénés et/ou oxygénés et/ou peuvent porter 1 ou 2 radicaux choisi parmi C₁-C₄-alcoxy,
phényle, benzyle et pyridyle,
où les trois derniers radicaux peuvent être non substitués, partiellement ou complètement halogénés et/ou peuvent porter 1, 2 ou 3 substituants identiques ou différents choisis parmi C₁-C₆-alkyle, C₁-C₆-halogénoalkyle, C₁-C₆-alcoxy, C₁-C₆-halogénoalcoxy, (C₁-C₆-alcoxy)carbonyle, (C₁-C₆-alkyl) amino et di-(C₁-C₆-alkyl)amino ;
R¹⁷ indépendamment de son occurrence, est choisi dans le groupe constitué par
hydrogène, C₁-C₆-alcoxy, C₁-C₆-halogénoalcoxy, C₁-C₆-alkylthio, triméthylsilyle, triéthylsilyle, tertio-butyldiméthylsilyle, C₁-C₆-alkyle, C₂-C₆-alcényle, C₂-C₆-alcynyle, C₃-C₈-cycloalkyle,
où les quatre radicaux susmentionnés peuvent être non substitués, partiellement ou complètement halogénés et/ou oxygénés et/ou peuvent porter 1 ou 2 radicaux choisis parmi C₁-C₄-alcoxy,
C₃-C₆-cycloalkyl-C₁-C₄-alkyle, phényle, benzyle, pyridyle, phénoxy,
où les quatre radicaux susmentionnés peuvent être non substitués, partiellement ou complètement halogénés et/ou peuvent porter 1, 2 ou 3 substituants identiques ou différents choisis parmi C₁-C₆-alkyle, C₁-C₆-halogénoalkyle, C₁-C₆-alcoxy, C₁-C₆-halogénoalcoxy et (C₁-C₆-alcoxy) carbonyle,
R^{17a} et R^{17b} sont choisis chacun indépendamment l'un de l'autre dans le groupe constitué par hydrogène, C₁-C₆-alcoxy, C₁-C₆-halogénoalcoxy, C₁-C₆-alkylthio, C₁-C₆-alkylsulfinyle, C₁-C₆-alkylsulfonyle, C₁-C₆-halogéno-alkylthio, triméthylsilyle, triéthylsilyle, tertio-butyldiméthylsilyle, C₁-C₆-alkyle, C₂-C₆-alcényle, C₂-C₆-alcynyle, C₃-C₈-cycloalkyle,
où les quatre radicaux susmentionnés peuvent être non substitués, partiellement ou complètement halogénés et/ou oxygénés et/ou peuvent porter 1 ou 2 radicaux identiques ou différents choisis parmi C₁-C₄-alcoxy,
phényle, benzyle, pyridyle, phénoxy,
où les quatre radicaux susmentionnés peuvent être non substitués, partiellement ou complètement halogénés et/ou peuvent porter 1, 2 ou 3 substituants identiques ou différents choisis parmi C₁-C₆-alkyle, C₁-C₆-halogénoalkyle, C₁-C₆-alcoxy, C₁-C₆-halogénoalcoxy et (C₁-C₆-alcoxy) carbonyle,
ou R^{17a} et R^{17b} peuvent être ensemble une chaîne C₂-C₆-alkylène formant un cycle de 3 à 7 chaînons saturé, partiellement saturé ou insaturé conjointement avec l'atome d'azote auquel R^{17a} et R^{17b} sont liés,
où la chaîne alkylène peut contenir 1 ou 2 hétéroatomes choisis, indépendamment les uns des autres, parmi l'oxygène, le soufre et l'azote, et peut éventuellement être substituée par halogène, C₁-C₄-halogénoalkyle, C₁-C₄-alcoxy ou C₁-C₄-halogénoalcoxy, et
où le ou les atomes d'azote et/ou de soufre du cycle hétérocyclique peuvent éventuellement être oxydés ;
ou R^{17a} et R^{17b} peuvent former ensemble un motif =CR¹³R¹⁴, =NR¹⁷ ou =NOR¹⁶ ;
R^{17c} indépendamment de son occurrence, est choisi dans le groupe constitué par
hydrogène, CN, C₁-C₆-alkyle, C₂-C₆-alcényle, C₂-C₆-alcynyle, C₃-C₈-cycloalkyle, C₃-C₈-cycloalkyl-C₁-C₄-alkyle,
où les cinq radicaux susmentionnés peuvent être non substitués, partiellement ou complètement halogénés et/ou oxygénés et/ou peuvent porter 1 ou 2 radicaux choisis indépendamment parmi C₁-C₄-alcoxy,
phényle, benzyle et pyridyle,
où les trois derniers radicaux peuvent être non substitués, partiellement ou complètement halogénés et/ou peuvent porter 1, 2 ou 3 substituants identiques ou différents choisis parmi C₁-C₆-alkyle, C₁-C₆-halogénoalkyle, C₁-C₆-alcoxy, C₁-C₆-halogénoalcoxy, (C₁-C₆-alcoxy)carbonyle, (C₁-C₆-alkyl)amino et di-(C₁-C₆-alkyl)amino ;
R¹⁸ est choisi chacun indépendamment les uns des autres dans le groupe constitué par
hydrogène, halogène, cyano, nitro, OH, SH, SCN, SF₅, C₁-C₆-alcoxy, C₁-C₆-halogénoalcoxy, C₁-C₆-alkylthio, C₁-C₆-alkylsulfinyle, C₁-C₆-alkylsulfonyle, C₁-C₆-halogéno-alkylthio, triméthylsilyle, triéthylsilyle, tertio-butyldiméthylsilyle, C₁-C₆-alkyle, C₂-C₆-alcényle, C₂-C₆-alcynyle, C₃-C₈-cycloalkyle,
où les quatre radicaux aliphatiques et cycloaliphatiques susmentionnés peuvent être non substitués, partiellement ou complètement halogénés et/ou oxygénés et/ou peuvent porter 1 ou 2 radicaux choisis parmi C₁-C₄-alcoxy ;
phényle, benzyle, pyridyle, phénoxy,
où les quatre derniers radicaux peuvent être non substitués, partiellement ou complètement halogénés et/ou peuvent porter 1, 2 ou 3 substituants choisis parmi C₁-C₆-alkyle, C₁-C₆-halogénoalkyle, C₁-C₆-alcoxy, C₁-C₆-halogénoalcoxy, (C₁-C₆-alcoxy)carbonyle, (C₁-C₆-alkyl)amino ou di-(C₁-C₆-alkyl)amino,
ou deux R¹⁸ présents sur le même atome de carbone peuvent être ensemble =O, =CH(C₁-C₄), =C(C₁-C₄-alkyl)C₁-C₄-alkyle, =N(C₁-C₆-alkyl) ou =NO(C₁-C₆-alkyl) ;
R¹⁹ est choisi chacun indépendamment les uns des autres dans le groupe constitué par
hydrogène, cyano, C₁-C₆-alcoxy, C₁-C₆-halogénoalcoxy, C₁-C₆-alkylthio, C₁-C₆-alkylsulfinyle, C₁-C₆-alkylsulfonyle, C₁-C₆-halogénoalkylthio, triméthylsilyle, triéthylsilyle, tertio-butyldiméthylsilyle, C₁-C₆-alkyle, C₂-C₆-alcényle, C₂-C₆-alcynyle, C₃-C₈-cycloalkyle,
où les quatre radicaux susmentionnés peuvent être non substitués, partiellement ou complètement halogénés et/ou oxygénés et/ou peuvent porter 1 ou 2 radicaux choisis parmi C₁-C₄-alcoxy,
phényle, benzyle, pyridyle, et phénoxy,
où les quatre derniers radicaux peuvent être non substitués, partiellement ou complètement halogénés et/ou porter 1, 2 ou 3 substituants choisis parmi C₁-C₆-alkyle, C₁-C₆-halogénoalkyle, C₁-C₆-alcoxy, C₁-C₆-halogénoalcoxy ou (C₁-C₆-alcoxy)carbonyle ;
R^{20a}, R^{20b} sont choisis chacun indépendamment l'un de l'autre dans le groupe constitué par hydrogène, cyano, C₁-C₆-alcoxy, C₁-C₆-halogénoalcoxy, C₁-C₆-alkylthio, C₁-C₆-alkylsulfinyle, C₁-C₆-alkylsulfonyle, C₁-C₆-halogénoalkylthio, triméthylsilyle, triéthylsilyle, tertio-butyldiméthylsilyle, C₁-C₆-alkyle, C₂-C₆-alcényle, C₂-C₆-alcynyle, C₃-C₈-cycloalkyle,
où les quatre radicaux aliphatiques et cycloaliphatiques susmentionnés peuvent être non substitués, partiellement ou complètement halogénés et/ou oxygénés et/ou peuvent porter 1 ou 2 radicaux identiques ou différents choisis parmi C₁-C₄-alcoxy,
phényle, benzyle, pyridyle, phénoxy,
où les quatre radicaux susmentionnés peuvent être non substitués, partiellement ou complètement halogénés et/ou porter 1, 2 ou 3 substituants identiques ou différents choisis parmi C₁-C₆-alkyle, C₁-C₆-halogénoalkyle, C₁-C₆-alcoxy, C₁-C₆-halogénoalcoxy et (C₁-C₆-alcoxy)carbonyle,
ou R^{20a} et R^{20b} peuvent être ensemble une chaîne C₂-C₆-alkylène formant un cycle de 3 à 7 chaînons saturé, partiellement saturé ou insaturé conjointement avec l'atome d'azote auquel R^{20a} et R^{20b} sont liés, où la chaîne alkylène peut contenir 1 ou 2 hétéroatomes choisis from l'oxygène, le soufre et l'azote, et peut éventuellement être substituée par halogène, C₁-C₄-halogénoalkyle, C₁-C₄-alcoxy ou C₁-C₄-halogénoalcoxy, et où le ou les atomes d'azote et/ou de soufre du cycle hétérocyclique peuvent éventuellement être oxydés ;
R^{21a}, R^{21b} sont choisis chacun indépendamment l'un de l'autre dans le groupe constitué par hydrogène, C₁-C₄-alkyle, C₁-C₆-cycloalkyle, C₁-C₄ alcoxyalkyle, phényle et benzyle ;
Hét^{#} représente un cycle hétérocyclique de 3, 4, 5, 6 ou 7 chaînons saturé, partiellement saturé ou insaturé aromatique comprenant 1, 2 ou 3 hétéroatomes comme chaînons, qui sont choisis parmi l'oxygène, l'azote et/ou le soufre,
où le cycle hétérocyclique est éventuellement substitué par 1, 2, 3, 4 ou 5 substituants R¹⁰ identiques ou différents, et où le ou les atomes d'azote et/ou de soufre du cycle hétérocyclique peuvent éventuellement être oxydés,
les stéréoisomères, tautomères et les sels de celui-ci.

2. Composé selon la revendication 1, dans lequel Hét est choisi dans le groupe constitué par les radicaux de formule suivante Hét-1 à Hét-24 : 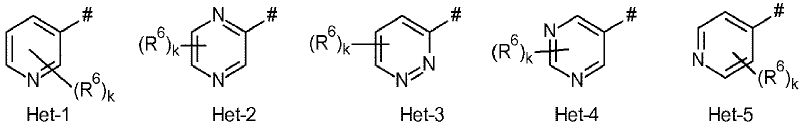 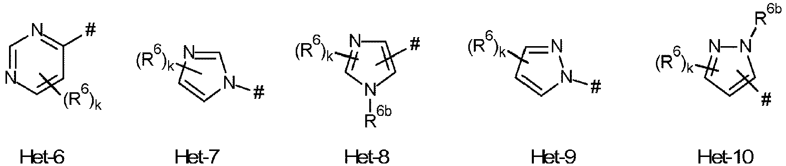 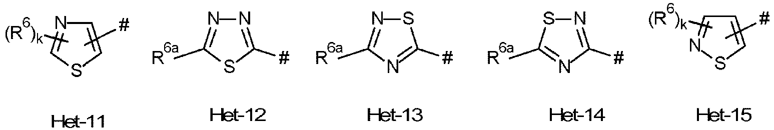 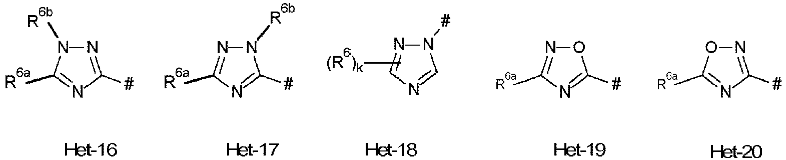 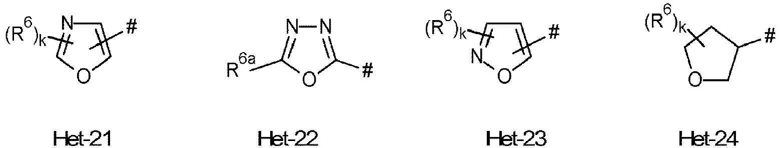
où # désigne la liaison dans la formule (I), et où k vaut 0, 1 ou 2 ; et
R^{6a} est hydrogène ou revêt l'une des significations données pour R⁶ selon la revendication 1 et
R^{6b} est hydrogène, C₁-C₄-alkyle ou C₁-C₄-halogénoalkyle.

3. Composé selon la revendication 2, dans lequel Hét est Hét-1a 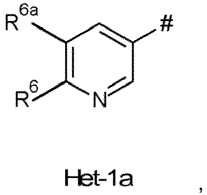
où # désigne la liaison dans la formule (I),
R⁶ est choisi parmi halogène, C₁-C₄-alkyle et C₁-C₄-halogénoalkyle, et
R^{6a} est hydrogène ou halogène, en particulier hydrogène.

4. Composé selon l'une quelconque des revendications précédentes, dans lequel
R¹ et R² sont indépendamment l'un de l'autre choisis dans le groupe constitué par hydrogène, halogène, CN, C₁-C₆-alkyle, C₃-C₆-cycloalkyle, C₁-C₆-halogénoalkyle, C₃-C₆-halogénocycloalkyle ; ou
R¹ et R² forment, conjointement avec l'atome de carbone auquel ils sont liés, un cycle carbocyclique de 3 à 5 chaînons saturé ; et où en particulier R¹ et R² sont tous deux hydrogène, ou R¹ est méthyle et R² est hydrogène.

5. Composé selon l'une quelconque des revendications précédentes, dans lequel
R³ est choisi dans le groupe constitué par hydrogène, halogène, C₁-C₆-alkyle, C₁-C₆-halogénoalkyle, C₂-C₆-alcényle, C₂-C₆-halogénoalcényle, C₃-C₆-cycloalkyle, C₃-C₆-halogénocycloalkyle, C₃-C₆-cycloalcényle, NR^{9a}R^{9b} et NR^{9a}-C(=O)R^{7a}.

6. Composé selon l'une quelconque des revendications précédentes, dans lequel m vaut 0 ou 1.

7. Composé selon l'une quelconque des revendications 1 à 4, dans lequel
m vaut 1, et
R³ et R^{4a} forment ensemble ainsi que conjointement avec la liaison existante, une double liaison entre les atomes de carbone adjacents.

8. Composé selon l'une quelconque des revendications précédentes, dans lequel
R⁵ est hydrogène, halogène, CN, NR^{9a}R^{9b}, C₁-C₆-alkyle, C₁-C₆-halogénoalkyle, C₂-C₆-alcényle, C₂-C₆-halogéno-alcényle, C₁-C₆-alcoxy, C₁-C₆-halogénoalcoxy, C₃-C₆-cycloalkyle, C₃-C₆-halogénocycloalkyle, C₃-C₆-cyclo-alcoxy, C₃-C₆-halogénocycloalcoxy, C(=O)OR⁸, C(=O)NR^{9a}R^{9b}, C(=S)NR^{9a}R^{9b}, C(=O)R^{7a}, C(=S)R^{7a},
et Q-phényle,
où phényle est non substitué ou substitué par 1, 2, 3, 4 ou 5 substituants R¹⁰ identiques ou différents.

9. Composé selon l'une quelconque des revendications 1 à 6, dans lequel
m vaut 0, et
R³ et R⁵ conjointement avec l'atome de carbone auquel ils sont liés, forment un carbocycle de 3, 4, 5 ou 6 chaînons saturé, un carbocycle de 4, 5 ou 6 chaînons insaturé, un cycle époxyde ou un cycle oxétane,
où chacun parmi les atomes de carbone du carbocycle saturé ou insaturé peut être non substitué ou peut porter 1 ou 2 radicaux R^{7b} identiques ou différents, où R^{7b} est choisi dans le groupe constitué par halogène, C₁-C₆-alkyle, C₁-C₆-alcoxy, et C₂-C₆-alcynyle,
où les atomes de carbone des radicaux aliphatiques susmentionnés peuvent éventuellement être partiellement ou complètement halogénés, et
un ou deux radicaux R^{7b} peuvent également être C₃-C₆-cycloalkyle, qui est non substitué ou porte 1, 2, 3, 4 ou 5 radicaux identiques ou différents choisis parmi
fluor, chlore ou méthyle,
phényle, éventuellement substitué par 1, 2, 3, 4 ou 5 substituants R^{10a} identiques ou différents, ou
un cycle hétérocyclique de 3, 4, 5, 6 ou 7 chaînons saturé, partiellement saturé ou insaturé aromatique comprenant 1, 2 ou 3 hétéroatomes comme chaînons, qui sont identiques ou différents et choisis parmi l'oxygène, l'azote et le soufre,
où le cycle hétérocyclique est éventuellement substitué par 1, 2, 3 ou 4 substituants R^{10a} identiques ou différents, et
où le ou les atomes d'azote et/ou de soufre du cycle hétérocyclique peuvent éventuellement être oxydés,
ou deux parmi R^{7b} présents sur un carbone de cycle forment ensemble =O ou =S,
ou deux R^{7b} forment ensemble une chaîne C₂-C₇-alkylène linéaire, formant ainsi, conjointement avec l'atome de carbone auquel ils sont liés, un cycle de 3, 4, 5 ou 6 chaînons,
où 1 ou 2 motifs CH₂ de la chaîne alkylène peuvent être remplacés par 1 ou 2 motifs d'hétéroatomes choisis indépendamment parmi O et S, et
où la chaîne alkylène est non substituée ou peut être substituée par 1, 2, 3, 4, 5 ou 6 radicaux identiques ou différents choisis dans le groupe constitué par halogène, C₁-C₄-alkyle, C₁-C₄-halogénoalkyle, C₁-C₄-alcoxy et C₁-C₄-halogénoalcoxy, et
R^{10a} est choisi dans le groupe constitué par halogène, C₁-C₄-alkyle, C₁-C₄-halogénoalkyle, C₁-C₄-alcoxy et C₁-C₄-halogénoalcoxy.

10. Composé selon l'une quelconque des revendications précédentes, dans lequel le motif de formule 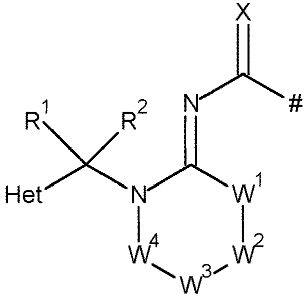 représente un radical A choisi dans le groupe constitué par W.Hét-1, W.Hét-2, W.Hét-3, W.Hét-4, W.Hét-5, W.Hét-6, W.Hét-7, W.Hét-8, W.Hét-9, W.Hét-10, W.Hét-11 et W.Hét-12 : 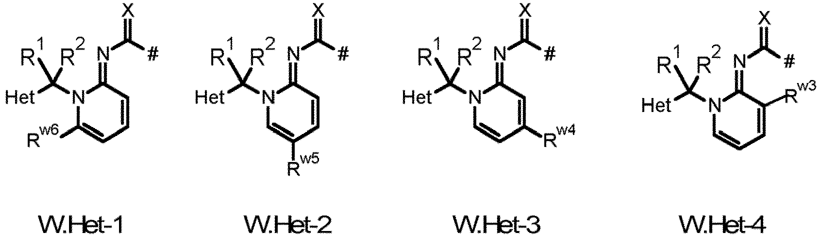 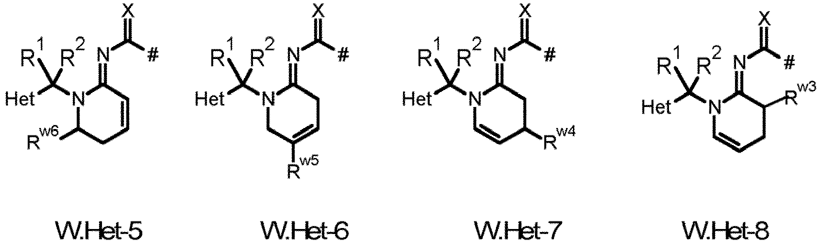 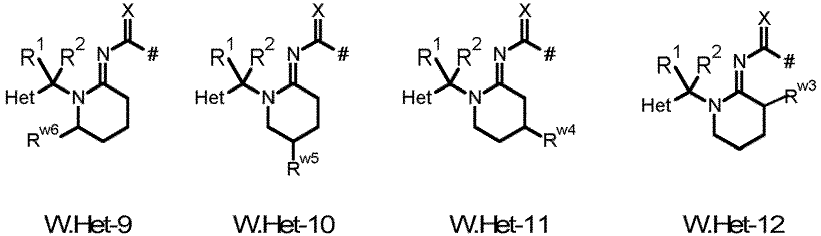
où # désigne la liaison au reste de la molécule ; R¹, R² et Hét sont tels que définis selon l'une quelconque des revendications 1 à 6, et où
R^{w3}, R^{w4}, R^{w5} et R^{w6} sont, indépendamment les uns des autres, choisis parmi hydrogène, halogène, C₁-C₄-alcoxy, C₁-C₄-halogénoalcoxy, C₁-C₄-alkyle et C₁-C₄-halogéno-alkyle, notamment hydrogène.

11. Composé selon l'une quelconque des revendications précédentes, dans lequel
X est O.

12. Composition agricole ou vétérinaire destinée à lutter contre des animaux nuisibles, comprenant au moins un composé tel que défini selon l'une quelconque des revendications 1 à 11, et au moins un véhicule acceptable liquide et/ou solide inerte, et éventuellement, si on le souhaite, au moins un agent tensioactif.

13. Utilisation d'un composé tel que défini selon l'une quelconque des revendications 1 à 11, destinée à la lutte ou au contrôle d'invertébrés nuisibles, la protection de plantes en croissance contre une attaque ou une infestation par des invertébrés nuisibles, la protection d'un matériel de propagation végétal, notamment de semences, contre des insectes du sol, ou pour protéger les racines et les pousses de plantules végétales contre des insectes du sol et foliaires, à l'exclusion d'une utilisation sur le corps humain ou animal par thérapie.

14. Méthode non thérapeutique de lutte ou de contrôle d'invertébrés nuisibles, laquelle méthode comprend la mise en contact dudit nuisible ou de sa source d'alimentation, son habitat ou ses lieux de reproduction, avec une quantité efficace sur le plan pesticide d'au moins un composé tel que défini selon l'une quelconque des revendications 1 à 11.

15. Méthode de protection de plantes en croissance contre une attaque ou une infestation par des invertébrés nuisibles, laquelle méthode comprend la mise en contact d'une plante, ou du sol ou de l'eau dans lesquels la plante se développe, avec une quantité efficace sur le plan pesticide d'au moins un composé tel que défini selon l'une quelconque des revendications 1 à 11.

16. Méthode de protection d'un matériel de propagation végétal, notamment de semences, contre des insectes du sol, et des racines et pousses de plantules contre des insectes du sol et foliaires, comprenant la mise en contact du matériel de propagation végétal avant semis et/ou après prégermination, avec au moins un composé tel que défini selon l'une quelconque des revendications 1 à 11.

17. Composé tel que défini selon l'une quelconque des revendications 1 à 11, pour une utilisation dans le traitement d'animaux infestés ou infectés par des parasites, pour la prévention d'une infection ou d'une infestation d'animaux par des parasites ou pour la protection d'animaux contre une infestation ou une infection par des parasites.
